(19) Europäisches Patentamt
European Patent Office
Office européen des brevets

(11) EP 4 613 750 A1

(12) **EUROPEAN PATENT APPLICATION**
published in accordance with Art. 153(4) EPC

(43) Date of publication:
**10.09.2025 Bulletin 2025/37**

(21) Application number: **23885847.6**

(22) Date of filing: **02.11.2023**

(51) International Patent Classification (IPC):
***C07D 487/04*** (2006.01) ***A01N 37/24*** (2006.01)
***A01N 37/38*** (2006.01) ***A01N 37/46*** (2006.01)
***A01N 37/50*** (2006.01) ***A01N 43/32*** (2006.01)
***A01N 43/36*** (2006.01) ***A01N 43/40*** (2006.01)
***A01N 43/50*** (2006.01) ***A01N 43/54*** (2006.01)
***A01N 43/56*** (2006.01) ***A01N 43/653*** (2006.01)
***A01N 43/713*** (2006.01) ***A01N 43/78*** (2006.01)
***A01N 43/80*** (2006.01) ***A01N 43/90*** (2006.01)
***A01N 47/02*** (2006.01) ***A01N 47/04*** (2006.01)
***A01N 47/14*** (2006.01) ***A01N 47/18*** (2006.01)

(52) Cooperative Patent Classification (CPC):
**A01N 37/24; A01N 37/32; A01N 37/38; A01N 37/46; A01N 37/50; A01N 43/32; A01N 43/36; A01N 43/40; A01N 43/50; A01N 43/54; A01N 43/56; A01N 43/653; A01N 43/713; A01N 43/78; A01N 43/80;** (Cont.)

(86) International application number:
**PCT/JP2023/039557**

(87) International publication number:
**WO 2024/096093 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.11.2022 JP 2022177697**
**29.05.2023 JP 2023088026**
**25.09.2023 JP 2023160980**

(71) Applicant: **Sumitomo Chemical Company, Limited**
**Tokyo 103-6020 (JP)**

(72) Inventors:
- **SATO, Natsumi**
**Takarazuka-shi, Hyogo 665-8555 (JP)**
- **MINEGISHI, Hidemitsu**
**Takarazuka-shi, Hyogo 665-8555 (JP)**
- **NISHIKAWA, Bunta**
**Takarazuka-shi, Hyogo 665-8555 (JP)**
- **SATO YANAGIHARA, Saki**
**Osaka-shi, Osaka 541-8550 (JP)**

(74) Representative: **Cabinet Beau de Loménie**
**103, rue de Grenelle / CS 90800**
**75340 Paris Cedex 07 (FR)**

(54) **HETEROCYCLIC COMPOUND AND HARMFUL ARTHROPOD CONTROL COMPOSITION CONTAINING SAME**

(57) The present invention provides a compound having excellent control efficacy against a harmful arthropod. A compound represented by formula (I)

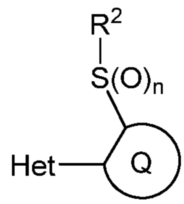

( I )

[wherein the symbols are the same as defined in the description] or an N-oxide thereof has excellent control efficacy against a harmful arthropod.

EP 4 613 750 A1

(52) Cooperative Patent Classification (CPC): (Cont.)
**A01N 43/82; A01N 43/88; A01N 43/90;**
**A01N 47/02; A01N 47/04; A01N 47/14;**
**A01N 47/18; A01N 47/22; A01N 47/24;**
**A01N 47/26; A01N 47/40; A01N 51/00;**
**A01N 53/00; A01N 55/00; A01N 57/14;**
**A01N 59/16; A01N 63/20; A01P 7/00; A01P 7/02;**
**A01P 7/04; C07D 471/04; C07D 487/04;**
**C07D 519/00**

## Description

### TECHNICAL FIELD

This application claims the priorities to and the benefits of Japanese Patent Application No. 2022-177697 filed on November 4, 2022, Japanese Patent Application No. 2023-088026 filed on May 29, 2023, and Japanese Patent Application No. 2023-160980 filed on September 25, 2023, the entire contents of which are incorporated herein by reference.

The present invention relates to heterocyclic compounds and compositions for controlling harmful arthropods comprising the same.

### BACKGROUND ART

To date, various compounds have been studied in order to control harmful arthropods. For example, Patent Document 1 discloses that certain kinds of compounds have control effects on pests.

### CITATION LIST

### PATENT DOCUMENT

Patent Document 1: WO 2016/129684 pamphlet

### SUMMARY OF THE INVENTION

### PROBLEMS TO BE SOLVED BY INVENTION

An object of the present invention is to provide compounds having excellent control efficacy against harmful arthropods.

### MEANS TO SOLVE PROBLEMS

The present invention provides the followings.

[1] A compound represented by formula (I)

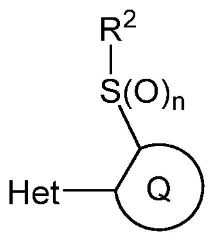

(I)

[wherein:

Q represented by the following formula

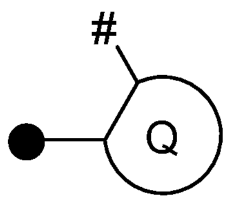

(wherein # represents the binding site to the sulfur atom, and • represents the binding site to Het) represents a group represented by formula Q1 or a group represented by formula Q2;

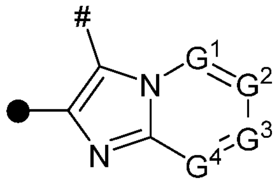
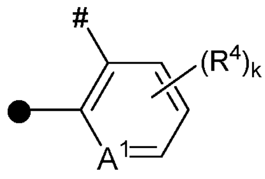

Q1 Q2

$A^1$ represents a nitrogen atom or $CR^8$ ;
$R^8$ represents a halogen atom or a hydrogen atom;
$R^2$ represents a C1-C6 alkyl group optionally substituted with one or more halogen atom(s), a cyclopropyl group, or a cyclopropylmethyl group;
n represents 0, 1, or 2;
$G^1$ represents a nitrogen atom or $CR^{3a}$;
$G^2$ represents a nitrogen atom or $CR^{3b}$;
$G^3$ represents a nitrogen atom or $CR^{3c}$;
$G^4$ represents a nitrogen atom or $CR^{3d}$;
$R^{3a}$, $R^{3b}$, $R^{3c}$, and $R^{3d}$ are identical to or different from each other, and each represent a C1-C6 chain hydrocarbon group optionally substituted with one or more substituent(s) selected from Group L, a C3-C7 cycloalkyl group optionally substituted with one or more substituent(s) selected from Group E, a phenyl group optionally substituted with one or more substituent(s) selected from Group H, a 5 or 6 membered aromatic heterocyclic group optionally substituted with one or more substituent(s) selected from Group H, $OR^{12}$, $NR^{11}R^{12}$, $NR^{11a}R^{12a}$, $NR^{24}NR^{11}R^{12}$, $NR^{24}OR^{11}$, $NR^{11}C(O)R^{13}$, $NR^{24}NR^{11}C(O)R^{13}$, $NR^{11}C(O)OR^{14}$, $NR^{24}NR^{11}C(O)OR^{14}$, $NR^{11}C(O)NR^{31}R^{32}$, $NR^{24}NR^{11}C(O)NR^{31}R^{32}$, $N=CHNR^{31}R^{32}$, $N=S(O)_pR^{15}R^{16}$, $C(O)R^{13}$, $C(O)OR^{17}$, $C(O)NR^{31}R^{32}$, $C(O)NR^{11}S(O)_2R^{23}$, $CR^{30}=NOR^{17}$, $NR^{11}CR^{24}=NOR^{17}$, $S(O)_mR^{23}$, a cyano group, a nitro group, a halogen atom, or a hydrogen atom;
p represents 0 or 1;
m represents 0, 1, or 2;
$R^{30}$ represents a C1-C6 chain hydrocarbon group optionally substituted with one or more halogen atom(s), a halogen atom, $OR^{35}$, $NR^{36}R^{37}$, or a hydrogen atom;
$R^{35}$ represents a C1-C6 chain hydrocarbon group optionally substituted with one or more halogen atom(s);
$R^{17}$ represents a C1-C6 chain hydrocarbon group optionally substituted with one or more halogen atom(s), a phenyl group optionally substituted with one or more substituent(s) selected from Group D, or a hydrogen atom;
$R^{11}$, $R^{24}$, $R^{36}$, and $R^{37}$ are identical to or different from each other, and each represent a C1-C6 chain hydrocarbon group optionally substituted with one or more halogen atom(s), or a hydrogen atom;
$R^{12}$ represents a C1-C6 chain hydrocarbon group optionally substituted with one or more substituent(s) selected from Group F, a C3-C7 cycloalkyl group optionally substituted with one or more substituent(s) selected from Group J, a C3-C7 cycloalkenyl group optionally substituted with one or more substituent(s) selected from Group J, a phenyl group optionally substituted with one or more substituent(s) selected from Group D, a 6 membered aromatic heterocyclic group optionally substituted with one or more substituent(s) selected from Group D, $S(O)_2R^{23}$, or a hydrogen atom;
$R^{23}$ represents a C1-C6 chain hydrocarbon group optionally substituted with one or more halogen atom(s), or a phenyl group optionally substituted with one or more substituent(s) selected from Group D;
$R^{11a}$ and $R^{12a}$ are combined with the nitrogen atom to which they are attached to form a 3-7 membered nonaromatic heterocyclic group optionally substituted with one or more substituent(s) selected from Group E;
$R^{13}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), a phenyl group optionally substituted with one or more substituent(s) selected from Group D, a 5 or 6 membered aromatic heterocyclic group optionally substituted with one or more substituent(s) selected from Group D, or a hydrogen atom;
$R^{14}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), or a phenyl C1-C3 alkyl group {wherein the phenyl moiety in said phenyl C1-C3 alkyl group is optionally substituted with one or more substituent(s) selected from Group D};
$R^{15}$ and $R^{16}$ are identical to or different from each other, and each represent a C1-C6 alkyl group optionally substituted with one or more halogen atom(s);

$R^{31}$ represents a C1-C6 alkyl group optionally substituted with one or more halogen atom(s), or a hydrogen atom;
$R^{32}$ represents a C1-C6 chain hydrocarbon group optionally substituted with one or more substituent(s) selected from Group F, a C3-C7 cycloalkyl group optionally substituted with one or more substituent(s) selected from Group J, $S(O)_2R^{23}$, or a hydrogen atom;
$R^4$ represents a C1-C6 chain hydrocarbon group optionally substituted with one or more substituent(s) selected from Group $L^2$, a C3-C7 cycloalkyl group optionally substituted with one or more substituent(s) selected from Group $E^2$, a phenyl group optionally substituted with one or more substituent(s) selected from Group $H^2$, a 5 or 6 membered aromatic heterocyclic group optionally substituted with one or more substituent(s) selected from Group $H^2$, $OR^{42}$, $NR^{41}R^{42}$, $NR^{41a}R^{42a}$, $NR^{54}NR^{41}R^{42}$, $NR^{54}OR^{41}$, $NR^{41}C(O)R^{43}$, $NR^{54}NR^{41}C(O)R^{43}$, $NR^{41}C(O)OR^{44}$, $NR^{54}NR^{41}C(O)OR^{44}$, $NR^{41}C(O)NR^{61}R^{62}$, $NR^{54}NR^{41}C(O)NR^{61}R^{62}$, $N=CHNR^{61}R^{62}$, $N=S(O)_rR^{45}R^{46}$, $C(O)R^{43}$, $C(O)OR^{47}$, $C(O)NR^{61}R^{62}$, $C(O)NR^{41}S(O)_2R^{53}$, $CR^{60}=NOR^{47}$, $NR^{41}CR^{54}=NOR^{47}$, $S(O)_tR^{53}$, a cyano group, a nitro group, or a halogen atom;
r represents 0 or 1;
t represents 0, 1, or 2;
$R^{60}$ represents a C1-C6 chain hydrocarbon group optionally substituted with one or more halogen atom(s), a halogen atom, $OR^{65}$, $NR^{66}R^{67}$, or a hydrogen atom;
$R^{65}$ represents a C1-C6 chain hydrocarbon group optionally substituted with one or more halogen atom(s);
$R^{47}$ represents a C1-C6 chain hydrocarbon group optionally substituted with one or more halogen atom(s), a phenyl group optionally substituted with one or more substituent(s) selected from Group $D^2$, or a hydrogen atom;
$R^{41}$, $R^{54}$, $R^{66}$, and $R^{67}$ are identical to or different from each other, and each represent a C1-C6 chain hydrocarbon group optionally substituted with one or more halogen atom(s), or a hydrogen atom;
$R^{42}$ represents a C1-C6 chain hydrocarbon group optionally substituted with one or more substituent(s) selected from Group $F^2$, a C3-C7 cycloalkyl group optionally substituted with one or more substituent(s) selected from Group $J^2$, a C3-C7 cycloalkenyl group optionally substituted with one or more substituent(s) selected from Group $J^2$, a phenyl group optionally substituted with one or more substituent(s) selected from Group $D^2$, a 6 membered aromatic heterocyclic group optionally substituted with one or more substituent(s) selected from Group $D^2$, a hydrogen atom, or $S(O)_2R^{53}$;
$R^{53}$ represents a C1-C6 chain hydrocarbon group optionally substituted with one or more halogen atom(s), or a phenyl group optionally substituted with one or more substituent(s) selected from Group $D^2$;
$R^{41a}$ and $R^{42a}$ are combined with the nitrogen atom to which they are attached to form a 3-7 membered nonaromatic heterocyclic group optionally substituted with one or more substituent(s) selected from Group $E^2$;
$R^{43}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), a phenyl group optionally substituted with one or more substituent(s) selected from Group $D^2$, a 5 or 6 membered aromatic heterocyclic group optionally substituted with one or more substituent(s) selected from Group $D^2$, or a hydrogen atom;
$R^{44}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), or a phenyl C1-C3 alkyl group {wherein the phenyl moiety in said phenyl C1-C3 alkyl group is optionally substituted with one or more substituent(s) selected from Group $D^2$};
$R^{45}$ and $R^{46}$ are identical to or different from each other, and each represent a C1-C6 alkyl group optionally substituted with one or more halogen atom(s);
$R^{61}$ represents a C1-C6 alkyl group optionally substituted with one or more halogen atom(s), or a hydrogen atom;
$R^{62}$ represents a C1-C6 chain hydrocarbon group optionally substituted with one or more substituent(s) selected from Group $F^2$, a C3-C7 cycloalkyl group optionally substituted with one or more substituent(s) selected from Group $J^2$, $S(O)_2R^{53}$, or a hydrogen atom;
k represents 0, 1, 2, or 3, wherein when k represents 2 or 3, then two or three $R^4$ are identical to or different from each other;
when two $R^4$ are bound to adjacent carbon atoms, then said two $R^4$ are optionally combined with two carbon atoms to which they are attached to form a benzene ring, a pyrrole ring, a furan ring, a thiophene ring, a pyrazole ring, an imidazole ring, an oxazole ring, an isoxazole ring, a thiazole ring, an oxadiazole ring, a thiadiazole ring, a pyridine ring, a pyridazine ring, a pyrimidine ring, or a pyrazine ring {wherein said benzene ring, said pyrrole ring, said furan ring, said thiophene ring, said pyrazole ring, said imidazole ring, said oxazole ring, said isoxazole ring, said thiazole ring, said pyridine ring, said pyridazine ring, said pyrimidine ring, and said pyrazine ring are optionally substituted with one or more substituent(s) selected from Group $H^2$}, or a triazole ring optionally substituted with one or more substituent(s) selected from Group P;
Het represents a group represented by formula Het1 or a group represented by formula Het2;

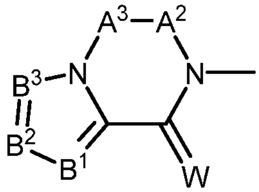 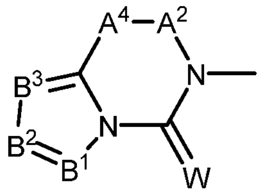

Het1 Het2

$A^2$ represents $-NR^{5a}-$ or $-CR^{7a}R^{7b}-$;
$A^3$ represents $-CR^{7c}R^{7d}-$, $-CR^{7e}R^{7f}-CR^{7g}R^{7h}-*^1$, or $-CR^{7i}R^{7j}-CR^{7k}R^{7l}-CR^{7m}R^{7n}-*^1$;
$*^1$ represents the binding position to $A^2$;
$A^4$ represents $-NR^{5b}-$, $-CR^{7o}R^{7p}-$, $-CR^{7q}R^{7r}-CR^{7s}R^{7t}-*^2$, $-NR^{5c}-CR^{7u}R^{7v}-*^2$, $-CR^{7w}R^{7x}-CR^{7y}R^{7z}-CR^{7aa}R^{7bb}-*^2$, or $-NR^{5d}-CR^{7cc}R^{7dd}-CR^{7ee}R^{7ff}-*^2$;
$*^2$ represents the binding position to $A^2$;
$R^{5a}$, $R^{5b}$, $R^{5c}$, and $R^{5d}$ are identical to or different from each other, and each represent a C1-C6 chain hydrocarbon group optionally substituted with one or more substituent(s) selected from Group K, a C1-C6 alkoxy group optionally substituted with one or more halogen atom(s), a C3-C7 cycloalkyl group optionally substituted with one or more halogen atom(s), a (C3-C7 cycloalkyl) C1-C6 alkyl group optionally substituted with one or more halogen atom(s), or a hydrogen atom;
$R^{7a}$, $R^{7b}$, $R^{7c}$, $R^{7d}$, $R^{7e}$, $R^{7f}$, $R^{7g}$, $R^{7h}$, $R^{7i}$, $R^{7j}$, $R^{7k}$, $R^{7l}$, $R^{7m}$, $R^{7n}$, $R^{7o}$, $R^{7p}$, $R^{7q}$, $R^{7r}$, $R^{7s}$, $R^{7t}$, $R^{7u}$, $R^{7v}$, $R^{7w}$, $R^{7x}$, $R^{7y}$, $R^{7z}$, $R^{7aa}$, $R^{7bb}$, $R^{7cc}$, $R^{7dd}$, $R^{7ee}$, and $R^{7ff}$ are identical to or different from each other, and each represent a C1-C6 chain hydrocarbon group optionally substituted with one or more substituent(s) selected from the group consisting of a cyano group and a halogen atom, a C3-C4 cycloalkyl group optionally substituted with one or more substituent(s) selected from the group consisting of a cyano group and a halogen atom, a nitro group, $OR^{69}$, $NR^{69}R^{70}$, a cyano group, a halogen atom, or a hydrogen atom;
$R^{7a}$ and $R^{7b}$, $R^{7c}$ and $R^{7d}$, $R^{7e}$ and $R^{7f}$, $R^{7g}$ and $R^{7h}$, $R^{7i}$ and $R^{7j}$, $R^{7k}$ and $R^{7l}$, $R^{7m}$ and $R^{7n}$, $R^{7o}$ and $R^{7p}$, $R^{7q}$ and $R^{7r}$, $R^{7s}$ and $R^{7t}$, $R^{7u}$ and $R^{7v}$, $R^{7w}$ and $R^{7x}$, $R^{7y}$ and $R^{7z}$, $R^{7aa}$ and $R^{7bb}$, $R^{7cc}$ and $R^{7dd}$, and $R^{7ee}$ and $R^{7ff}$ are each optionally combined with the carbon atom to which they are attached to form a C3-C7 cycloalkyl group optionally substituted with one or more substituent(s) selected from the group consisting of a cyano group and a halogen atom;
$R^{69}$ represents a C1-C6 chain hydrocarbon group optionally substituted with one or more substituent(s) selected from Group T, a C3-C7 cycloalkyl group optionally substituted with one or more substituent(s) selected from Group U, a C3-C7 cycloalkenyl group optionally substituted with one or more substituent(s) selected from Group U, a phenyl group optionally substituted with one or more substituent(s) selected from Group V, a 6 membered aromatic heterocyclic group optionally substituted with one or more substituent(s) selected from Group V, or a hydrogen atom;
$R^{70}$ represents a C1-C6 chain hydrocarbon group optionally substituted with one or more halogen atom(s), or a hydrogen atom;
W represents an oxygen atom or a sulfur atom;
$B^1$ represents $CR^{6a}$ or a nitrogen atom;
$B^2$ represents $CR^{6b}$ or a nitrogen atom;
$B^3$ represents $CR^{6c}$ or a nitrogen atom;
$R^{6a}$, $R^{6b}$, and $R^{6c}$ are identical to or different from each other, and each represent a C1-C6 chain hydrocarbon group optionally substituted with one or more substituent(s) selected from Group $L^3$, a C3-C7 cycloalkyl group optionally substituted with one or more substituent(s) selected from Group $E^3$, a phenyl group optionally substituted with one or more substituent(s) selected from Group $H^3$, a 5 or 6 membered aromatic heterocyclic group optionally substituted with one or more substituent(s) selected from Group $H^3$, $OR^{72}$, $NR^{71}R^{72}$, $NR^{71a}R^{72a}$, $NR^{84}NR^{71}R^{72}$, $NR^{84}OR^{71}$, $NR^{71}C(O)R^{73}$, $NR^{84}NR^{71}C(O)R^{73}$, $NR^{71}C(O)OR^{74}$, $NR^{84}NR^{71}C(O)OR^{74}$, $NR^{71}C(O)NR^{91}R^{92}$, $NR^{84}NR^{71}C(O)NR^{91}R^{92}$, $N=CHNR^{91}R^{92}$, $N=S(O)_qR^{75}R^{76}$, $C(O)R^{73}$, $C(O)OR^{77}$, $C(O)NR^{91}R^{92}$, $C(O)NR^{71}S(O)_2R^{83}$, $CR^{90}=NOR^{77}$, $NR^{71}CR^{84}=NOR^{77}$, $S(O)_vR^{83}$, a cyano group, a nitro group, a halogen atom, or a hydrogen atom;
q represents 0 or 1;
v represents 0, 1, or 2;
$R^{90}$ represents a C1-C6 chain hydrocarbon group optionally substituted with one or more halogen atom(s), a halogen atom, $OR^{95}$, $NR^{96}R^{97}$, or a hydrogen atom;
$R^{95}$ represents a C1-C6 chain hydrocarbon group optionally substituted with one or more halogen atom(s);
$R^{77}$ represents a C1-C6 chain hydrocarbon group optionally substituted with one or more halogen atom(s), a

phenyl group optionally substituted with one or more substituent(s) selected from Group $D^3$, or a hydrogen atom; $R^{71}$, $R^{84}$, $R^{96}$, and $R^{97}$ are identical to or different from each other, and each represent a C1-C6 chain hydrocarbon group optionally substituted with one or more halogen atom(s), or a hydrogen atom;

$R^{72}$ represents a C1-C6 chain hydrocarbon group optionally substituted with one or more substituent(s) selected from Group $F^3$, a C3-C7 cycloalkyl group optionally substituted with one or more substituent(s) selected from Group $J^3$, a C3-C7 cycloalkenyl group optionally substituted with one or more substituent(s) selected from Group $J^3$, a phenyl group optionally substituted with one or more substituent(s) selected from Group $D^3$, a 6 membered aromatic heterocyclic group optionally substituted with one or more substituent(s) selected from Group $D^3$, $S(O)_2R^{83}$, or a hydrogen atom;

$R^{83}$ represents a C1-C6 chain hydrocarbon group optionally substituted with one or more halogen atom(s), or a phenyl group optionally substituted with one or more substituent(s) selected from Group $D^3$;

$R^{71a}$ and $R^{72a}$ are combined with the nitrogen atom to which they are attached to form a 3-7 membered nonaromatic heterocyclic group optionally substituted with one or more substituent(s) selected from Group $E^3$;

$R^{73}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), a phenyl group optionally substituted with one or more substituent(s) selected from Group $D^3$, a 5 or 6 membered aromatic heterocyclic group optionally substituted with one or more substituent(s) selected from Group $D^3$, or a hydrogen atom;

$R^{74}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), or a phenyl C1-C3 alkyl group {wherein the phenyl moiety in said phenyl C1-C3 alkyl group is optionally substituted with one or more substituent(s) selected from Group $D^3$};

$R^{75}$ and $R^{76}$ are identical to or different from each other, and each represent a C1-C6 alkyl group optionally substituted with one or more halogen atom(s);

$R^{91}$ represents a C1-C6 alkyl group optionally substituted with one or more halogen atom(s), or a hydrogen atom; and

$R^{92}$ represents a C1-C6 chain hydrocarbon group optionally substituted with one or more substituent(s) selected from Group $F^3$, a C3-C7 cycloalkyl group optionally substituted with one or more substituent(s) selected from Group $J^3$, $S(O)_2R^{83}$, or a hydrogen atom;

Group L: a group consisting of a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C3-C6 cycloalkyl group optionally substituted with one or more halogen atom(s), a cyano group, a hydroxy group, and a halogen atom;

Group M: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), and a halogen atom;

Group D: a group consisting of a C1-C6 chain hydrocarbon group optionally substituted with one or more halogen atom(s), a hydroxy group, a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a sulfanyl group, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), an amino group, $NHR^{21}$, $NR^{21}R^{22}$, $C(O)R^{21}$, $OC(O)R^{21}$, $C(O)OR^{21}$, a cyano group, a nitro group, and a halogen atom;

wherein $R^{21}$ and $R^{22}$ are identical to or different from each other, and each represent a C1-C6 alkyl group optionally substituted with one or more halogen atom(s);

Group E: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a halogen atom, an oxo group, a hydroxy group, a cyano group, and a nitro group;

Group F: a group consisting of a C1-C6 alkoxy group optionally substituted with one or more halogen atom(s), a phenyl group optionally substituted with one or more substituent(s) selected from Group D, a 5 or 6 membered aromatic heterocyclic group optionally substituted with one or more substituent(s) selected from Group D, a C3-C7 cycloalkyl group optionally substituted with one or more halogen atom(s), a 3-7 membered nonaromatic heterocyclic group optionally substituted with one or more substituent(s) selected from Group M, an amino group, $NHR^{21}$, $NR^{21}R^{22}$, a halogen atom, and a cyano group;

Group H: a group consisting of a C1-C6 alkyl group optionally substituted with one or more halogen atom(s), $OR^{10}$, $NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, an amino group, and a 5 or 6 membered aromatic heterocyclic group optionally substituted with one or more substituent(s) selected from Group W;

$R^9$ represents a C1-C6 alkyl group optionally substituted with one or more halogen atom(s), or a C3-C6 cycloalkyl group optionally substituted with one or more halogen atom(s); and

$R^{10}$ represents a C1-C6 alkyl group optionally substituted with one or more halogen atom(s), a C3-C6 cycloalkyl group optionally substituted with one or more halogen atom(s), or a hydrogen atom;

Group P: a group consisting of a C2-C6 chain hydrocarbon group optionally substituted with one or more halogen atom(s), a C3-C6 cycloalkyl group optionally substituted with one or more halogen atom(s), a phenyl group optionally substituted with one or more substituent(s) selected from Group $D^2$, a 5 or 6 membered aromatic heterocyclic group optionally substituted with one or more substituent(s) selected from Group $D^2$, a C2-C6 alkylcarbonyl group optionally substituted with one or more halogen atom(s), a C2-C6 alkoxycarbonyl group optionally substituted with one or more halogen atom(s), an aminocarbonyl group, a (C1-C6 alkyl)aminocarbonyl group optionally substituted with one or more halogen atom(s), a methyl group, and a di(C1-C4 alkyl)aminocarbonyl group optionally substituted with one or more halogen atom(s);

Group J: a group consisting of a C1-C6 alkyl group optionally substituted with one or more halogen atom(s), a halogen atom, and a cyano group;

Group K: a group consisting of a C1-C6 alkoxy group optionally substituted with one or more halogen atom(s), a phenyl group optionally substituted with one or more substituent(s) selected from Group D, a 5 or 6 membered aromatic heterocyclic group optionally substituted with one or more substituent(s) selected from Group D, a C3-C7 cycloalkyl group optionally substituted with one or more halogen atom(s), and a 3-7 membered nonaromatic heterocyclic group optionally substituted with one or more substituent(s) selected from Group M;

Group T: a group consisting of a C1-C6 alkoxy group optionally substituted with one or more halogen atom(s), a phenyl group optionally substituted with one or more substituent(s) selected from Group D, a 5 or 6 membered aromatic heterocyclic group optionally substituted with one or more substituent(s) selected from Group D, a C3-C7 cycloalkyl group optionally substituted with one or more halogen atom(s), a 3-7 membered nonaromatic heterocyclic group optionally substituted with one or more substituent(s) selected from Group M, an amino group, $NHR^{21}$, $NR^{21}R^{22}$, a halogen atom, and a cyano group;

Group U: a group consisting of a C1-C6 alkyl group optionally substituted with one or more halogen atom(s), a halogen atom, and a cyano group;

Group V: a group consisting of a C1-C6 chain hydrocarbon group optionally substituted with one or more halogen atom(s), a hydroxy group, a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a sulfanyl group, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), an amino group, $NHR^{21}$, $NR^{21}R^{22}$, $C(O)R^{21}$, $OC(O)R^{21}$, $C(O)OR^{21}$, a cyano group, a nitro group, and a halogen atom;

Group W: a group consisting of a C1-C6 chain hydrocarbon group optionally substituted with one or more halogen atom(s), a hydroxy group, a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a sulfanyl group, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), an amino group, $NHR^{21}$, $NR^{21}R^{22}$, $C(O)R^{21}$, $OC(O)R^{21}$, $C(O)OR^{21}$, a cyano group, a nitro group, and a halogen atom;

Group $L^2$: a group consisting of a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C3-C6 cycloalkyl group optionally substituted with one or more halogen atom(s), a cyano group, a hydroxy group, and a halogen atom;

Group $D^2$: a group consisting of a C1-C6 chain hydrocarbon group optionally substituted with one or more halogen atom(s), a hydroxy group, a C1-C6 alkoxy group optionally substituted with one or more halogen atom(s), a C3-C6 alkenyloxy group optionally substituted with one or more halogen atom(s), a C3-C6 alkynyloxy group optionally

substituted with one or more halogen atom(s), a sulfanyl group, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), an amino group, $NHR^{21}$, $NR^{21}R^{22}$, $C(O)R^{21}$, $OC(O)R^{21}$, $C(O)OR^{21}$, a cyano group, a nitro group, and a halogen atom;

Group $E^2$: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a halogen atom, an oxo group, a hydroxy group, a cyano group, and a nitro group;

Group $F^2$: a group consisting of a C1-C6 alkoxy group optionally substituted with one or more halogen atom(s), a phenyl group optionally substituted with one or more substituent(s) selected from Group D, a 5 or 6 membered aromatic heterocyclic group optionally substituted with one or more substituent(s) selected from Group D, a C3-C7 cycloalkyl group optionally substituted with one or more halogen atom(s), a 3-7 membered nonaromatic heterocyclic group optionally substituted with one or more substituent(s) selected from Group M, an amino group, $NHR^{21}$, $NR^{21}R^{22}$, a halogen atom, and a cyano group;

Group $H^2$: a group consisting of a C1-C6 alkyl group optionally substituted with one or more halogen atom(s), $OR^{10}$, $NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, an amino group, and a 5 or 6 membered aromatic heterocyclic group optionally substituted with one or more substituent(s) selected from Group W;

Group $J^2$: a group consisting of a C1-C6 alkyl group optionally substituted with one or more halogen atom(s), a halogen atom, and a cyano group;

Group $L^3$: a group consisting of a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C3-C6 cycloalkyl group optionally substituted with one or more halogen atom(s), a cyano group, a hydroxy group, and a halogen atom;

Group $D^3$: a group consisting of a C1-C6 chain hydrocarbon group optionally substituted with one or more halogen atom(s), a hydroxy group, a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a sulfanyl group, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), an amino group, $NHR^{21}$, $NR^{21}R^{22}$, $C(O)R^{21}$, $OC(O)R^{21}$, $C(O)OR^{21}$, a cyano group, a nitro group, and a halogen atom;

Group $E^3$: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a halogen atom, an oxo group, a hydroxy group, a cyano group, and a nitro group;

Group $F^3$: a group consisting of a C1-C6 alkoxy group optionally substituted with one or more halogen atom(s), a phenyl group optionally substituted with one or more substituent(s) selected from Group D, a 5 or 6 membered aromatic heterocyclic group optionally substituted with one or more substituent(s) selected from Group D, a C3-C7 cycloalkyl group optionally substituted with one or more halogen atom(s), a 3-7 membered nonaromatic heterocyclic group optionally substituted with one or more substituent(s) selected from Group M, an amino group, $NHR^{21}$, $NR^{21}R^{22}$, a halogen atom, and a cyano group;

Group $H^3$: a group consisting of a C1-C6 alkyl group optionally substituted with one or more halogen atom(s), $OR^{10}$, $NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, an amino group, and a 5 or 6 membered aromatic heterocyclic group optionally substituted with one or more substituent(s) selected from Group W;

Group $J^3$: a group consisting of a C1-C6 alkyl group optionally substituted with one or more halogen atom(s), a halogen atom, and a cyano group]

(hereinafter referred to as "Present compound N" or "Compound N of the present invention") or an N-oxide thereof (hereinafter the compound represented by formula (I) or an N-oxide thereof is referred to as "Present compound" or "Compound of the present invention").

[2] The compound or an N-oxide thereof according to [1], wherein Q represents the group represented by formula Q1.

[3] The compound or an N-oxide thereof according to [1], wherein Q represents the group represented by formula Q2.

[4] The compound or an N-oxide thereof according to [1], wherein

Q represents the group represented by formula Q1;
$G^1$ represents $CR^{3a}$;
$G^2$ represents $CR^{3b}$;
$G^3$ represents $CR^{3c}$; and
$G^4$ represents a nitrogen atom or $CR^{3d}$.

[5] The compound or an N-oxide thereof according to [1], wherein

Q represents the group represented by formula Q1;
$G^1$ represents $CR^{3a}$;
$G^2$ represents $CR^{3b}$;
$G^3$ represents $CR^{3c}$; and
$G^4$ represents $CR^{3d}$.

[6] The compound or an N-oxide thereof according to [1], wherein

Q represents the group represented by formula Q2; and
$A^1$ represents a nitrogen atom.

[7] The compound or an N-oxide thereof according to [1], wherein

Q represents the group represented by formula Q2; and
$A^1$ represents $CR^8$.

[8] The compound or an N-oxide thereof according to any one of [1] to [7], wherein

Het represents the group represented by formula Het1;
$A^2$ represents $-CR^{7a}R^{7b}-$;
$A^3$ represents $-CR^{7c}R^{7d}-$ or $-CR^{7e}R^{7f}-CR^{7g}R^{7h}-*^1$; and
W represents an oxygen atom.

[9] The compound or an N-oxide thereof according to any one of [1] to [7], wherein

Het represents the group represented by formula Het2;
$A^2$ represents $-CR^{7a}R^{7b}-$;
$A^4$ represents $-CR^{7o}R^{7p}-$ or $-CR^{7q}R^{7r}-CR^{7s}R^{7t}-*^2$; and
W represents an oxygen atom.

[10] A composition for controlling a harmful arthropod comprising the compound or an N-oxide thereof according to any one of [1] to [9] and an inert carrier.
[11] A composition comprising one or more ingredient(s) selected from the group consisting of Group (a), Group (b), Group (c), and Group (d), and the compound or an N-oxide thereof according to any one of [1] to [9]:

Group (a): a group consisting of insecticidal active ingredients, miticidal active ingredients, and nematicidal active ingredients;
Group (b): fungicidal active ingredients;
Group (c): plant growth regulatory ingredients;
Group (d): repellent ingredients.

[12] A method for controlling a harmful arthropod which comprises applying an effective amount of the compound or an N-oxide thereof according to any one of [1] to [9] or an effective amount of the composition according to [11] to a harmful arthropod or a habitat where a harmful arthropod lives.
[13] A seed or a vegetative reproductive organ holding an effective amount of the compound or an N-oxide thereof according to any one of [1] to [9] or an effective amount of the composition according to [11].
[14] A compound represented by formula (II)

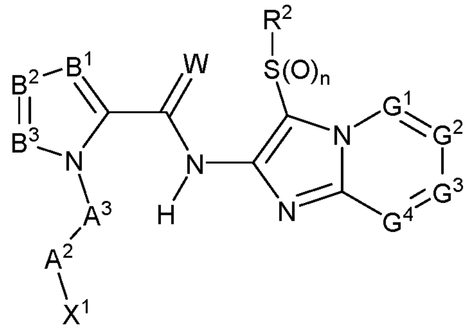

( II )

[wherein:

$X^1$ represents a chlorine atom, a bromine atom, an iodine atom, or a hydroxy group; and
the other symbols are the same as defined in [1]] or a salt thereof

(hereinafter the compound represented by formula (II) or a salt thereof is referred to as "Intermediate A").
[15] A compound represented by formula (III)

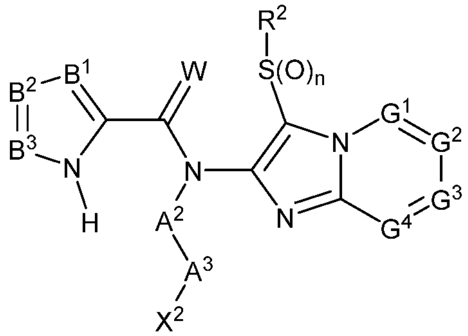

( III )

[wherein:

$X^2$ represents a chlorine atom, a bromine atom, an iodine atom, or a hydroxy group; and
the other symbols are the same as defined in [1]] or a salt thereof

(hereinafter the compound represented by formula (III) or a salt thereof is referred to as "Intermediate B").

EFFECT OF INVENTION

According to the present invention, harmful arthropods can be controlled.

MODE FOR CARRYING OUT THE INVENTION

The substituents in the present invention are explained as follows.
The term of "halogen atom" represents a fluorine atom, a chlorine atom, a bromine atom, or an iodine atom.
When a substituent is substituted with two or more halogen atoms or substituents, these halogen atoms or substituents may be identical to or different from each other.
The expression of "CX-CY" as described herein means that the number of carbon atom is X to Y. For example, the expression of "C1-C6" means that the number of carbon atom is 1 to 6.
The term of "chain hydrocarbon group" represents an alkyl group, an alkenyl group, or an alkynyl group.
Examples of the term of "alkyl group" include a methyl group, an ethyl group, a propyl group, an isopropyl group, a 1,1-dimethylpropyl group, a 1,2-dimethylpropyl group, a 1-ethylpropyl group, a butyl group, a sec-butyl group, a tert-butyl group, a pentyl group, and a hexyl group.
Examples of the term of "alkenyl group" include a vinyl group, a 1-propenyl group, a 2-propenyl group, a 1-methyl-1-propenyl group, a 1-methyl-2-propenyl group, a 1,2-dimethyl-1-propenyl group, a 1-ethyl-2-propenyl group, a 3-butenyl group, a 4-pentenyl group, and a 5-hexenyl group.
Examples of the term of "alkynyl group" include an ethynyl group, a 1-propynyl group, a 2-propynyl group, a 1-methyl-2-propynyl group, a 1,1-dimethyl-2-propynyl group, a 1-ethyl-2-propynyl group, a 2-butynyl group, a 4-pentynyl group, and a 5-hexynyl group.

Examples of the term of "alkoxy group" include a methoxy group, an ethoxy group, a propoxy group, an isopropoxy group, a butoxy group, a tert-butoxy group, a pentyloxy group, and a hexyloxy group.

Examples of the term of "alkenyloxy group" include a 2-propenyloxy group, a 2-butenyloxy group, and a 5-hexenyloxy group.

Examples of the term of "alkynyloxy group" include a 2-propynyloxy group, a 2-butynyloxy group, and a 5-hexynyloxy group.

Examples of the term of "cycloalkyl group" include a cyclopropyl group, a cyclobutyl group, a cyclopentyl group, a cyclohexyl group, and a cycloheptyl group.

Examples of the term of "cycloalkenyl group" include a cyclopropenyl group, a cyclobutenyl group, a cyclopentenyl group, a cyclohexenyl group, and a cycloheptenyl group.

Examples of the term of "3-7 membered nonaromatic heterocyclic group" include an aziridinyl group, an oxiranyl group, a thiiranyl group, an azetidinyl group, an oxetanyl group, a thietanyl group, a pyrrolidinyl group, a tetrahydrofuranyl group, a tetrahydrothienyl group, a piperidyl group, a pyranyl group, a dihydropyranyl group, a tetrahydropyranyl group, a tetrahydrothiopyranyl group, an azepanyl group, an oxepanyl group, a thiepanyl group, a pyrazolinyl group, a pyrazolidinyl group, an imidazolinyl group, an imidazolidinyl group, an oxazolinyl group, a thiazolinyl group, an oxazolidinyl group, a thiazolidinyl group, an isoxazolinyl group, an isoxazolidinyl group, an isothiazolinyl group, an isothiazolidinyl group, a dioxolyl group, a dioxolanyl group, a dioxanyl group, a morpholinyl group, a thiomorpholinyl group, and a piperazinyl group.

Examples of the term of "5 or 6 membered aromatic heterocyclic group" include a pyrrolyl group, a furyl group, a thienyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a pyridyl group, a pyridazinyl group, a pyrimidinyl group, a pyrazinyl group, a triazinyl group, and a tetrazinyl group.

Examples of the term of "6 membered aromatic heterocyclic group" include a pyridyl group, a pyridazinyl group, a pyrimidinyl group, a pyrazinyl group, a triazinyl group, and a tetrazinyl group.

Examples of the term of "(C3-C6 cycloalkyl) C1-C3 alkyl group optionally substituted with one or more halogen atom(s) " include a cyclopropylmethyl group, a (2-fluorocyclopropyl)methyl group, a cyclopropyl(fluoro)methyl group, and a (2-fluorocyclopropyl) (fluoro)methyl group.

The term of "(C3-C7 cycloalkyl) C1-C6 alkyl group optionally substituted with one or more halogen atom(s)" refers to a group in which the (C3-C7 cycloalkyl) and/or the(C1-C6 alkyl) is/are optionally substituted with one or more halogen atom(s), and examples thereof include a (2,2-difluorocyclopropyl)methyl group, a 2-cyclopropyl-1,1,2,2-tetrafluoroethyl group, a 2-(2,2-difluorocyclopropyl)-1,1,2,2-tetrafluoroethyl group, a (2,2-difluorocyclopropyl)propyl group, a (2,2-difluorocyclopropyl)butyl group, a (2,2-difluorocyclopropyl)pentyl group, and a (2,2-difluorocyclopropyl)hexyl group.

Examples of the terms of "phenyl C1-C3 alkyl group {wherein the phenyl moiety in said phenyl C1-C3 alkyl group is optionally substituted with one or more substituent(s) selected from Group D}", "phenyl C1-C3 alkyl group {wherein the phenyl moiety in said phenyl C1-C3 alkyl group is optionally substituted with one or more substituent(s) selected from Group $D^2$}", and "phenyl C1-C3 alkyl group {wherein the phenyl moiety in said phenyl C1-C3 alkyl group is optionally substituted with one or more substituent(s) selected from Group $D^3$}" include a benzyl group, a 2-fluorobenzyl group, a 4-chlorobenzyl group, a 4-(trifluoromethyl)benzyl group, and a 2-[4-(trifluoromethyl)phenyl]ethyl group.

Examples of the term of "alkylsulfanyl group" include a methylsulfanyl group, an ethylsulfanyl group, a propylsulfanyl group, and an isopropylsulfanyl group.

Examples of the term of "alkylsulfinyl group" include a methylsulfinyl group, an ethylsulfinyl group, a propylsulfinyl group, and an isopropylsulfinyl group.

Examples of the term of "alkylsulfonyl group" include a methylsulfonyl group, an ethylsulfonyl group, a propylsulfonyl group, and an isopropylsulfonyl group.

Examples of the term of "alkylcarbonyl group" include an acetyl group, a propanoyl group, a 2-methylpropanoyl group, and a hexanoyl group.

Examples of the term of "alkoxycarbonyl group" include a methoxycarbonyl group, an ethoxycarbonyl group, an isopropoxycarbonyl group, and a pentyloxycarbonyl group.

Examples of the term of "(C1-C6 alkyl)aminocarbonyl group optionally substituted with one or more halogen atom(s)" include a methylaminocarbonyl group, a trifluoromethylaminocarbonyl group, an isopropylaminocarbonyl group, and a hexylaminocarbonyl group.

Examples of the term of "di(C1-C4 alkyl)aminocarbonyl group optionally substituted with one or more halogen atom(s)" include a dimethylaminocarbonyl group, a methylethylaminocarbonyl group, a trifluoromethylethylaminocarbonyl group, and a diisopropylaminocarbonyl group.

Examples of the N-oxide of the compound represented by formula (I) include the compound represented by the following formula.

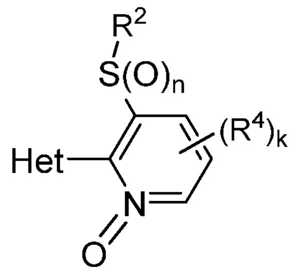

[wherein the symbols are the same as defined above.]

The Present compound may optionally have one or more stereoisomer(s). Examples of the stereoisomer(s) include enantiomers, diastereomers, and geometric isomers. The Present compound encompasses each stereoisomer and mixtures of stereoisomers at any ratio.

The Present compound, the compound represented by formula (II), and the compound represented by formula (III) may optionally form an acid addition salt. Examples of the acid to form the acid addition salt include inorganic acids such as hydrogen chloride, phosphoric acid, and sulfuric acid; and organic acids such as acetic acid, trifluoroacetic acid, benzoic acid, and p-toluenesulfonic acid. Such acid addition salt may be prepared by mixing the Present compound, the compound represented by formula (II), or the compound represented by formula (III) with an acid.

Aspects of the Present compound N include the following compounds.

[Aspect 1] The Present compound N, wherein W represents an oxygen atom.
[Aspect 2] The Present compound N, wherein $R^2$ represents a C1-C6 alkyl group.
[Aspect 3] The Present compound N, wherein $R^2$ represents an ethyl group.
[Aspect 4] The Present compound N, wherein Q represents the group represented by Q1.
[Aspect 5] The Present compound N, wherein Q represents the group represented by Q2.
[Aspect 6] The Present compound N, wherein Het represents the group represented by Het1.
[Aspect 7] The Present compound N, wherein Het represents the group represented by Het2.
[Aspect 8] The Present compound N, wherein

Q represents the group represented by Q1;
the combination of $G^1$, $G^2$, $G^3$, and $G^4$ represents
a combination wherein $G^1$ represents $CR^{3a}$, $G^2$ represents $CR^{3b}$, $G^3$ represents $CR^{3c}$, and $G^4$ represents a nitrogen atom;
a combination wherein $G^1$ represents $CR^{3a}$, $G^2$ represents $CR^{3b}$, $G^3$ represents a nitrogen atom, and $G^4$ represents $CR^{3d}$;
a combination wherein $G^1$ represents $CR^{3a}$, $G^2$ represents a nitrogen atom, $G^3$ represents $CR^{3c}$, and $G^4$ represents $CR^{3d}$;
a combination wherein $G^1$ represents a nitrogen atom, $G^2$ represents $CR^{3b}$, $G^3$ represents $CR^{3c}$, and $G^4$ represents $CR^{3d}$; or
a combination wherein $G^1$ represents $CR^{3a}$, $G^2$ represents $CR^{3b}$, $G^3$ represents $CR^{3c}$, and $G^4$ represents $CR^{3d}$;
$R^{3a}$, $R^{3c}$, and $R^{3d}$ each represent a hydrogen atom; and
$R^{3b}$ represents a C1-C6 alkyl group optionally substituted with one or more halogen atom(s), a C3-C7 cycloalkyl group optionally substituted with one or more substituent(s) selected from the group consisting of a halogen atom and a cyano group, a phenyl group optionally substituted with one or more halogen atom(s), a 5 or 6 membered aromatic heterocyclic group optionally substituted with one or more halogen atom(s), or a halogen atom.

[Aspect 9] The Present compound N, wherein

Q represents the group represented by Q1;
$G^1$ represents $CR^{3a}$;
$G^2$ represents $CR^{3b}$;
$G^3$ represents $CR^{3c}$;
$G^4$ represents a nitrogen atom or $CR^{3d}$;
$R^{3a}$, $R^{3c}$, and $R^{3d}$ each represent a hydrogen atom; and
$R^{3b}$ represents a C1-C6 alkyl group optionally substituted with one or more halogen atom(s), or a halogen atom.

[Aspect 10] The Present compound N, wherein

Q represents the group represented by Q1;
$G^1$ represents $CR^{3a}$;
$G^2$ represents $CR^{3b}$;
$G^3$ represents $CR^{3c}$;
$G^4$ represents $CR^{3d}$;
$R^{3a}$, $R^{3c}$, and $R^{3d}$ each represent a hydrogen atom; and
$R^{3b}$ represents a C1-C6 alkyl group optionally substituted with one or more halogen atom(s), or a halogen atom.

[Aspect 11] The Present compound N, wherein

Q represents the group represented by Q1;
the combination of $G^1$, $G^2$, $G^3$, and $G^4$ represents
a combination wherein $G^1$ represents $CR^{3a}$, $G^2$ represents $CR^{3b}$, $G^3$ represents $CR^{3c}$, and $G^4$ represents a nitrogen atom;
a combination wherein $G^1$ represents $CR^{32}$, $G^2$ represents $CR^{3b}$, $G^3$ represents a nitrogen atom, and $G^4$ represents $CR^{3d}$ ;
a combination wherein $G^1$ represents $CR^{3a}$, $G^2$ represents a nitrogen atom, $G^3$ represents $CR^{3c}$, and $G^4$ represents $CR^{3d}$ ;
a combination wherein $G^1$ represents a nitrogen atom, $G^2$ represents $CR^{3b}$, $G^3$ represents $CR^{3c}$, and $G^4$ represents $CR^{3d}$; or
a combination wherein $G^1$ represents $CR^{3a}$, $G^2$ represents $CR^{3b}$, $G^3$ represents $CR^{3c}$, and $G^4$ represents $CR^{3d}$;
$R^{3a}$, $R^{3b}$, and $R^{3d}$ each represent a hydrogen atom; and
$R^{3c}$ represents a halogen atom.

[Aspect 12] The Present compound N, wherein

Q represents the group represented by Q1;
$G^1$ represents $CR^{3a}$;
$G^2$ represents $CR^{3b}$;
$G^3$ represents $CR^{3c}$;
$G^4$ represents a nitrogen atom or $CR^{3d}$;
$R^{3a}$, $R^{3b}$, and $R^{3d}$ each represent a hydrogen atom; and
$R^{3c}$ represents a halogen atom.

[Aspect 13] The Present compound N, wherein

Q represents the group represented by Q1;
$G^1$ represents $CR^{33}$;
$G^2$ represents $CR^{3b}$;
$G^3$ represents $CR^{3c}$;
$G^4$ represents $CR^{3d}$;
$R^{3a}$, $R^{3b}$, and $R^{3d}$ represent a hydrogen atom; and
$R^{3c}$ represents a halogen atom.

[Aspect 14] The Present compound N, wherein

Q represents the group represented by Q2; and
$A^1$ represents a nitrogen atom.

[Aspect 15] The Present compound N, wherein

Q represents the group represented by Q2; and
$A^1$ represents $CR^8$.

[Aspect 16] The Present compound N, wherein

Q represents the group represented by Q2; and
$R^4$ represents a C3-C7 cycloalkyl group optionally substituted with one or more substituent(s) selected from

Group $E^2$, a phenyl group optionally substituted with one or more substituent(s) selected from Group $H^2$, a 5 or 6 membered aromatic heterocyclic group optionally substituted with one or more substituent(s) selected from Group $H^2$, a halogen atom, or a hydrogen atom.

[Aspect 17] The Present compound N, wherein

Q represents the group represented by Q2;
$A^1$ represents a nitrogen atom or $CR^8$;
k represents 2;
two $R^4$ are adjacent with each other; and
two $R^4$ are combined with two carbon atoms to which they are attached to form a benzene ring, a pyridine ring, a pyridazine ring, a pyrimidine ring, or a pyrazine ring {wherein said benzene ring, said pyridine ring, said pyridazine ring, said pyrimidine ring, and said pyrazine ring are optionally substituted with one or more substituent(s) selected from Group $H^2$}.

[Aspect 18] The Present compound N, wherein

Q represents the group represented by Q2; and
$R^4$ represents a C3-C7 cycloalkyl group optionally substituted with one or more substituent(s) selected from Group $E^2$, a phenyl group optionally substituted with one or more substituent(s) selected from Group $H^2$, a 5 or 6 membered aromatic heterocyclic group optionally substituted with one or more substituent(s) selected from Group $H^2$, or a halogen atom.

[Aspect 19] The Present compound N, wherein

$G^1$ represents $CR^{33}$;
$G^2$ represents $CR^{3b}$;
$G^3$ represents $CR^{3c}$;
$G^4$ represents $CR^{3d}$;
$R^{3a}$, $R^{3c}$, and $R^{3d}$ each represent a hydrogen atom;
$R^{3b}$ represents a halogen atom;
$A^1$ represents a nitrogen atom;
k represents 0 or 1; and
$R^4$ represents a phenyl group optionally substituted with one or more substituent(s) selected from the group consisting of a cyano group and a halogen atom, a 5 or 6 membered aromatic heterocyclic group optionally substituted with one or more substituent(s) selected from the group consisting of a cyano group and a halogen atom, a C3-C7 cycloalkyl group, or a halogen atom.

[Aspect 20] The Present compound N, wherein

Q represents the group represented by Q1;
$G^1$ represents $CR^{33}$;
$G^2$ represents $CR^{3b}$;
$G^3$ represents $CR^{3c}$;
$G^4$ represents $CR^{3d}$;
$R^{3a}$, $R^{3c}$, and $R^{3d}$ each represent a hydrogen atom; and
$R^{3b}$ represents a halogen atom.

[Aspect 21] The Present compound N, wherein

Q represents the group represented by Q2;
$A^1$ represents a nitrogen atom;
k represents 0 or 1; and
$R^4$ represents a phenyl group optionally substituted with one or more substituent(s) selected from the group consisting of a cyano group and a halogen atom, a 5 or 6 membered aromatic heterocyclic group optionally substituted with one or more substituent(s) selected from the group consisting of a cyano group and a halogen atom, a C3-C7 cycloalkyl group, or a halogen atom.

[Aspect 22] The Present compound N, wherein

Q represents the group represented by Q2;
$A^1$ represents a nitrogen atom;
k represents 0 or 1; and
$R^4$ represents a phenyl group optionally substituted with one or more substituent(s) selected from the group consisting of a cyano group and a halogen atom, a pyridyl group optionally substituted with one or more substituent(s) selected from the group consisting of a cyano group and a halogen atom, or a pyrimidinyl group optionally substituted with one or more substituent(s) selected from the group consisting of a cyano group and a halogen atom.

[Aspect 23] The Present compound N, wherein

Q represents the group represented by Q2;
$A^1$ represents a nitrogen atom;
k represents 0 or 1; and
$R^4$ represents a phenyl group optionally substituted with one or more substituent(s) selected from the group consisting of a cyano group and a halogen atom, or a pyridyl group optionally substituted with one or more substituent(s) selected from the group consisting of a cyano group and a halogen atom.

[Aspect 24] The Present compound N, wherein

$G^1$ represents $CR^{33}$;
$G^2$ represents $CR^{3b}$;
$G^3$ represents $CR^{3c}$;
$G^4$ represents $CR^{3d}$;
$R^{3a}$, $R^{3c}$, and $R^{3d}$ each represent a hydrogen atom;
$R^{3b}$ represents a halogen atom;
$A^1$ represents a nitrogen atom;
k represents 0 or 1; and
$R^4$ represents a phenyl group optionally substituted with one or more substituent(s) selected from the group consisting of a cyano group and a halogen atom, a 5 or 6 membered aromatic heterocyclic group optionally substituted with one or more substituent(s) selected from the group consisting of a cyano group and a halogen atom, a C3-C7 cycloalkyl group, $OR^{42}$, $NR^{41}R^{42}$, $NR^{41}C(O)R^{43}$, $NR^{41}C(O)OR^{44}$, or a halogen atom.

[Aspect 25] The Present compound N, wherein

$G^1$ represents $CR^{33}$;
$G^2$ represents $CR^{3b}$;
$G^3$ represents $CR^{3c}$;
$G^4$ represents $CR^{3d}$;
$R^{3a}$, $R^{3c}$, and $R^{3d}$ each represent a hydrogen atom;
$R^{3b}$ represents a halogen atom;
$A^1$ represents a nitrogen atom;
k represents 0 or 1;
$R^4$ represents a phenyl group optionally substituted with one or more substituent(s) selected from the group consisting of a cyano group and a halogen atom, a pyridyl group optionally substituted with one or more substituent(s) selected from the group consisting of a cyano group and a halogen atom, $OR^{42}$, $NR^{41}R^{42}$, $NR^{41}C(O)R^{43}$, or $NR^{41}C(O)OR^{44}$;
$R^{41}$ represents a C1-C6 alkyl group or a hydrogen atom;
$R^{42}$ represents a hydrogen atom;
$R^{43}$ represents a C1-C6 alkyl group or a C3-C7 cycloalkyl group; and
$R^{44}$ represents a C1-C6 alkyl group.

[Aspect 26] The Present compound N, wherein

Q represents the group represented by Q2;
$A^1$ represents a nitrogen atom;

k represents 0 or 1; and
$R^4$ represents a phenyl group optionally substituted with one or more substituent(s) selected from the group consisting of a cyano group and a halogen atom, a 5 or 6 membered aromatic heterocyclic group optionally substituted with one or more substituent(s) selected from the group consisting of a cyano group and a halogen atom, a C3-C7 cycloalkyl group, $OR^{42}$, $NR^{41}R^{42}$, $NR^{41}C(O)R^{43}$, $NR^{41}C(O)OR^{44}$, or a halogen atom.

[Aspect 27] The Present compound N, wherein

Q represents the group represented by Q2;
$A^1$ represents a nitrogen atom;
k represents 0 or 1;
$R^4$ represents a phenyl group optionally substituted with one or more substituent(s) selected from the group consisting of a cyano group and a halogen atom, a pyridyl group optionally substituted with one or more substituent(s) selected from the group consisting of a cyano group and a halogen atom, $OR^{42}$, $NR^{41}R^{42}$, $NR^{41}C(O)R^{43}$, or $NR^{41}C(O)OR^{44}$;
$R^{41}$ represents a C1-C6 alkyl group or a hydrogen atom;
$R^{42}$ represents a hydrogen atom;
$R^{43}$ represents a C1-C6 alkyl group or a C3-C7 cycloalkyl group; and
$R^{44}$ represents a C1-C6 alkyl group.

[Aspect 28] The Present compound N, wherein

Q represents the group represented by formula Q1 or a group represented by Q2a

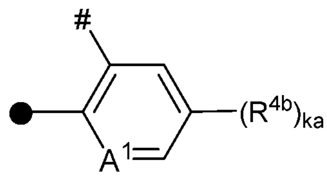

Q2a

$G^1$ represents $CR^{3a}$;
$G^2$ represents $CR^{3b}$;
$G^3$ represents $CR^{3c}$;
$G^4$ represents $CR^{3d}$;
$R^{3a}$, $R^{3c}$, and $R^{3d}$ each represent a hydrogen atom;
$R^{3b}$ represents a halogen atom;
$A^1$ represents a nitrogen atom;
ka represents 0 or 1; and
$R^{4b}$ represents a phenyl group optionally substituted with one or more substituent(s) selected from the group consisting of a cyano group and a halogen atom, a 5 or 6 membered aromatic heterocyclic group optionally substituted with one or more substituent(s) selected from the group consisting of a cyano group and a halogen atom, a C3-C7 cycloalkyl group, $OR^{42}$, $NR^{41}R^{42}$, $NR^{41}C(O)R^{43}$, $NR^{41}C(O)OR^{44}$, or a halogen atom.

[Aspect 29] The Present compound N, wherein

Q represents the group represented by Q1 or the group represented by the above formula Q2a;
$G^1$ represents $CR^{33}$;
$G^2$ represents $CR^{3b}$;
$G^3$ represents $CR^{3c}$;
$G^4$ represents $CR^{3d}$;
$R^{3a}$, $R^{3c}$, and $R^{3d}$ each represent a hydrogen atom;
$R^{3b}$ represents a halogen atom;
$A^1$ represents a nitrogen atom;
ka represents 0 or 1;
$R^{4b}$ represents a phenyl group optionally substituted with one or more substituent(s) selected from the group

consisting of a cyano group and a halogen atom, a pyridyl group optionally substituted with one or more substituent(s) selected from the group consisting of a cyano group and a halogen atom, $OR^{42}$, $NR^{41}R^{42}$, $NR^{41}C(O)R^{43}$, or $NR^{41}C(O)OR^{44}$;
$R^{41}$ represents a C1-C6 alkyl group or a hydrogen atom;
$R^{42}$ represents a hydrogen atom;
$R^{43}$ represents a C1-C6 alkyl group or a C3-C7 cycloalkyl group; and
$R^{44}$ represents a C1-C6 alkyl group.

[Aspect 30] The Present compound N, wherein

Q represents the group represented by the above formula Q2a;
$A^1$ represents a nitrogen atom;
ka represents 0 or 1; and
$R^{4b}$ represents a phenyl group optionally substituted with one or more substituent(s) selected from the group consisting of a cyano group and a halogen atom, a 5 or 6 membered aromatic heterocyclic group optionally substituted with one or more substituent(s) selected from the group consisting of a cyano group and a halogen atom, a C3-C7 cycloalkyl group, $OR^{42}$, $NR^{41}R^{42}$, $NR^{41}C(O)R^{43}$, $NR^{41}C(O)OR^{44}$, or a halogen atom.

[Aspect 31] The Present compound N, wherein

Q represents the group represented by the above formula Q2a;
$A^1$ represents a nitrogen atom;
ka represents 0 or 1;
$R^{4b}$ represents a phenyl group optionally substituted with one or more substituent(s) selected from the group consisting of a cyano group and a halogen atom, a pyridyl group optionally substituted with one or more substituent(s) selected from the group consisting of a cyano group and a halogen atom, $OR^{42}$, $NR^{41}R^{42}$, $NR^{41}C(O)R^{43}$, or $NR^{41}C(O)OR^{44}$;
$R^{41}$ represents a C1-C6 alkyl group or a hydrogen atom;
$R^{42}$ represents a C1-C6 alkyl group optionally substituted with one or more halogen atom(s), or a hydrogen atom;
$R^{43}$ represents a C1-C6 alkyl group or a C3-C7 cycloalkyl group; and
$R^{44}$ represents a C1-C6 alkyl group.

[Aspect 32] The Present compound N, wherein

Q represents the group represented by the above formula Q2a;
$A^1$ represents a nitrogen atom;
ka represents 0 or 1;
$R^{4b}$ represents a phenyl group optionally substituted with one or more substituent(s) selected from the group consisting of a cyano group and a halogen atom, a pyridyl group optionally substituted with one or more substituent(s) selected from the group consisting of a cyano group and a halogen atom, $OR^{42}$, $NR^{41}R^{42}$, $NR^{41}C(O)R^{43}$, or $NR^{41}C(O)OR^{44}$;
$R^{41}$ represents a C1-C6 alkyl group or a hydrogen atom;
$R^{42}$ represents a hydrogen atom;
$R^{43}$ represents a C1-C6 alkyl group or a C3-C7 cycloalkyl group; and
$R^{44}$ represents a C1-C6 alkyl group.

[Aspect 33] The compound according to the Aspect 2, wherein W represents an oxygen atom.
[Aspect 34] The compound according to the Aspect 3, wherein W represents an oxygen atom.
[Aspect 35] The compound according to the Aspect 4, wherein W represents an oxygen atom.
[Aspect 36] The compound according to the Aspect 5, wherein W represents an oxygen atom.
[Aspect 37] The compound according to the Aspect 6, wherein W represents an oxygen atom.
[Aspect 38] The compound according to the Aspect 7, wherein W represents an oxygen atom.
[Aspect 39] The compound according to the Aspect 8, wherein W represents an oxygen atom.
[Aspect 40] The compound according to the Aspect 9, wherein W represents an oxygen atom.
[Aspect 41] The compound according to the Aspect 10, wherein W represents an oxygen atom.
[Aspect 42] The compound according to the Aspect 11, wherein W represents an oxygen atom.
[Aspect 43] The compound according to the Aspect 12, wherein W represents an oxygen atom.
[Aspect 44] The compound according to the Aspect 13, wherein W represents an oxygen atom.

[Aspect 45] The compound according to the Aspect 14, wherein W represents an oxygen atom.
[Aspect 46] The compound according to the Aspect 15, wherein W represents an oxygen atom.
[Aspect 47] The compound according to the Aspect 16, wherein W represents an oxygen atom.
[Aspect 48] The compound according to the Aspect 17, wherein W represents an oxygen atom.
[Aspect 49] The compound according to the Aspect 18, wherein W represents an oxygen atom.
[Aspect 50] The compound according to the Aspect 19, wherein W represents an oxygen atom.
[Aspect 51] The compound according to the Aspect 20, wherein W represents an oxygen atom.
[Aspect 52] The compound according to the Aspect 21, wherein W represents an oxygen atom.
[Aspect 53] The compound according to the Aspect 22, wherein W represents an oxygen atom.
[Aspect 54] The compound according to the Aspect 23, wherein W represents an oxygen atom.
[Aspect 55] The compound according to the Aspect 24, wherein W represents an oxygen atom.
[Aspect 56] The compound according to the Aspect 25, wherein W represents an oxygen atom.
[Aspect 57] The compound according to the Aspect 26, wherein W represents an oxygen atom.
[Aspect 58] The compound according to the Aspect 27, wherein W represents an oxygen atom.
[Aspect 59] The compound according to the Aspect 28, wherein W represents an oxygen atom.
[Aspect 60] The compound according to the Aspect 29, wherein W represents an oxygen atom.
[Aspect 61] The compound according to the Aspect 30, wherein W represents an oxygen atom.
[Aspect 62] The compound according to the Aspect 31, wherein W represents an oxygen atom.
[Aspect 63] The compound according to the Aspect 32, wherein W represents an oxygen atom.
[Aspect 64] The compound according to the Aspect 4, wherein $R^2$ represents a C1-C6 alkyl group.
[Aspect 65] The compound according to the Aspect 5, wherein $R^2$ represents a C1-C6 alkyl group.
[Aspect 66] The compound according to the Aspect 6, wherein $R^2$ represents a C1-C6 alkyl group.
[Aspect 67] The compound according to the Aspect 7, wherein $R^2$ represents a C1-C6 alkyl group.
[Aspect 68] The compound according to the Aspect 8, wherein $R^2$ represents a C1-C6 alkyl group.
[Aspect 69] The compound according to the Aspect 9, wherein $R^2$ represents a C1-C6 alkyl group.
[Aspect 70] The compound according to the Aspect 10, wherein $R^2$ represents a C1-C6 alkyl group.
[Aspect 71] The compound according to the Aspect 11, wherein $R^2$ represents a C1-C6 alkyl group.
[Aspect 72] The compound according to the Aspect 12, wherein $R^2$ represents a C1-C6 alkyl group.
[Aspect 73] The compound according to the Aspect 13, wherein $R^2$ represents a C1-C6 alkyl group.
[Aspect 74] The compound according to the Aspect 14, wherein $R^2$ represents a C1-C6 alkyl group.
[Aspect 75] The compound according to the Aspect 15, wherein $R^2$ represents a C1-C6 alkyl group.
[Aspect 76] The compound according to the Aspect 16, wherein $R^2$ represents a C1-C6 alkyl group.
[Aspect 77] The compound according to the Aspect 17, wherein $R^2$ represents a C1-C6 alkyl group.
[Aspect 78] The compound according to the Aspect 18, wherein $R^2$ represents a C1-C6 alkyl group.
[Aspect 79] The compound according to the Aspect 19, wherein $R^2$ represents a C1-C6 alkyl group.
[Aspect 80] The compound according to the Aspect 20, wherein $R^2$ represents a C1-C6 alkyl group.
[Aspect 81] The compound according to the Aspect 21, wherein $R^2$ represents a C1-C6 alkyl group.
[Aspect 82] The compound according to the Aspect 22, wherein $R^2$ represents a C1-C6 alkyl group.
[Aspect 83] The compound according to the Aspect 23, wherein $R^2$ represents a C1-C6 alkyl group.
[Aspect 84] The compound according to the Aspect 24, wherein $R^2$ represents a C1-C6 alkyl group.
[Aspect 85] The compound according to the Aspect 25, wherein $R^2$ represents a C1-C6 alkyl group.
[Aspect 86] The compound according to the Aspect 26, wherein $R^2$ represents a C1-C6 alkyl group.
[Aspect 87] The compound according to the Aspect 27, wherein $R^2$ represents a C1-C6 alkyl group.
[Aspect 88] The compound according to the Aspect 28, wherein $R^2$ represents a C1-C6 alkyl group.
[Aspect 89] The compound according to the Aspect 29, wherein $R^2$ represents a C1-C6 alkyl group.
[Aspect 90] The compound according to the Aspect 30, wherein $R^2$ represents a C1-C6 alkyl group.
[Aspect 91] The compound according to the Aspect 31, wherein $R^2$ represents a C1-C6 alkyl group.
[Aspect 92] The compound according to the Aspect 32, wherein $R^2$ represents a C1-C6 alkyl group.
[Aspect 93] The compound according to the Aspect 4, wherein $R^2$ represents an ethyl group.
[Aspect 94] The compound according to the Aspect 5, wherein $R^2$ represents an ethyl group.
[Aspect 95] The compound according to the Aspect 6, wherein $R^2$ represents an ethyl group.
[Aspect 96] The compound according to the Aspect 7, wherein $R^2$ represents an ethyl group.
[Aspect 97] The compound according to the Aspect 8, wherein $R^2$ represents an ethyl group.
[Aspect 98] The compound according to the Aspect 9, wherein $R^2$ represents an ethyl group.
[Aspect 99] The compound according to the Aspect 10, wherein $R^2$ represents an ethyl group.
[Aspect 100] The compound according to the Aspect 11, wherein $R^2$ represents an ethyl group.
[Aspect 101] The compound according to the Aspect 12, wherein $R^2$ represents an ethyl group.
[Aspect 102] The compound according to the Aspect 13, wherein $R^2$ represents an ethyl group.

[Aspect 103] The compound according to the Aspect 14, wherein $R^2$ represents an ethyl group.
[Aspect 104] The compound according to the Aspect 15, wherein $R^2$ represents an ethyl group.
[Aspect 105] The compound according to the Aspect 16, wherein $R^2$ represents an ethyl group.
[Aspect 106] The compound according to the Aspect 17, wherein $R^2$ represents an ethyl group.
[Aspect 107] The compound according to the Aspect 18, wherein $R^2$ represents an ethyl group.
[Aspect 108] The compound according to the Aspect 19, wherein $R^2$ represents an ethyl group.
[Aspect 109] The compound according to the Aspect 20, wherein $R^2$ represents an ethyl group.
[Aspect 110] The compound according to the Aspect 21, wherein $R^2$ represents an ethyl group.
[Aspect 111] The compound according to the Aspect 22, wherein $R^2$ represents an ethyl group.
[Aspect 112] The compound according to the Aspect 23, wherein $R^2$ represents an ethyl group.
[Aspect 113] The compound according to the Aspect 24, wherein $R^2$ represents an ethyl group.
[Aspect 114] The compound according to the Aspect 25, wherein $R^2$ represents an ethyl group.
[Aspect 115] The compound according to the Aspect 26, wherein $R^2$ represents an ethyl group.
[Aspect 116] The compound according to the Aspect 27, wherein $R^2$ represents an ethyl group.
[Aspect 117] The compound according to the Aspect 28, wherein $R^2$ represents an ethyl group.
[Aspect 118] The compound according to the Aspect 29, wherein $R^2$ represents an ethyl group.
[Aspect 119] The compound according to the Aspect 30, wherein $R^2$ represents an ethyl group.
[Aspect 120] The compound according to the Aspect 31, wherein $R^2$ represents an ethyl group.
[Aspect 121] The compound according to the Aspect 32, wherein $R^2$ represents an ethyl group.
[Aspect 122] The compound according to the Aspect 4, wherein

W represents an oxygen atom; and
$R^2$ represents an ethyl group.

[Aspect 123] The compound according to the Aspect 5, wherein

W represents an oxygen atom; and
$R^2$ represents an ethyl group.

[Aspect 124] The compound according to the Aspect 6, wherein

W represents an oxygen atom; and
$R^2$ represents an ethyl group.

[Aspect 125] The compound according to the Aspect 7, wherein

W represents an oxygen atom; and
$R^2$ represents an ethyl group.

[Aspect 126] The compound according to the Aspect 8, wherein

W represents an oxygen atom; and
$R^2$ represents an ethyl group.

[Aspect 127] The compound according to the Aspect 9, wherein

W represents an oxygen atom; and
$R^2$ represents an ethyl group.

[Aspect 128] The compound according to the Aspect 10, wherein

W represents an oxygen atom; and
$R^2$ represents an ethyl group.

[Aspect 129] The compound according to the Aspect 11, wherein

W represents an oxygen atom; and
$R^2$ represents an ethyl group.

[Aspect 130] The compound according to the Aspect 12, wherein

W represents an oxygen atom; and
$R^2$ represents an ethyl group.

[Aspect 131] The compound according to the Aspect 13, wherein

W represents an oxygen atom; and
$R^2$ represents an ethyl group.

[Aspect 132] The compound according to the Aspect 14, wherein

W represents an oxygen atom; and
$R^2$ represents an ethyl group.

[Aspect 133] The compound according to the Aspect 15, wherein

W represents an oxygen atom; and
$R^2$ represents an ethyl group.

[Aspect 134] The compound according to the Aspect 16, wherein

W represents an oxygen atom; and
$R^2$ represents an ethyl group.

[Aspect 135] The compound according to the Aspect 17, wherein

W represents an oxygen atom; and
$R^2$ represents an ethyl group.

[Aspect 136] The compound according to the Aspect 18, wherein

W represents an oxygen atom; and
$R^2$ represents an ethyl group.

[Aspect 137] The compound according to the Aspect 19, wherein

W represents an oxygen atom; and
$R^2$ represents an ethyl group.

[Aspect 138] The compound according to the Aspect 20, wherein

W represents an oxygen atom; and
$R^2$ represents an ethyl group.

[Aspect 139] The compound according to the Aspect 21, wherein

W represents an oxygen atom; and
$R^2$ represents an ethyl group.

[Aspect 140] The compound according to the Aspect 22, wherein

W represents an oxygen atom; and
$R^2$ represents an ethyl group.

[Aspect 141] The compound according to the Aspect 23, wherein

W represents an oxygen atom; and

$R^2$ represents an ethyl group.

[Aspect 142] The compound according to the Aspect 24, wherein

W represents an oxygen atom; and
$R^2$ represents an ethyl group.

[Aspect 143] The compound according to the Aspect 25, wherein

W represents an oxygen atom; and
$R^2$ represents an ethyl group.

[Aspect 144] The compound according to the Aspect 26, wherein

W represents an oxygen atom; and
$R^2$ represents an ethyl group.

[Aspect 145] The compound according to the Aspect 27, wherein

W represents an oxygen atom; and
$R^2$ represents an ethyl group.

[Aspect 146] The compound according to the Aspect 28, wherein

W represents an oxygen atom; and
$R^2$ represents an ethyl group.

[Aspect 147] The compound according to the Aspect 29, wherein

W represents an oxygen atom; and
$R^2$ represents an ethyl group.

[Aspect 148] The compound according to the Aspect 30, wherein

W represents an oxygen atom; and
$R^2$ represents an ethyl group.

[Aspect 149] The compound according to the Aspect 31, wherein

W represents an oxygen atom; and
$R^2$ represents an ethyl group.

[Aspect 150] The compound according to the Aspect 32, wherein

W represents an oxygen atom; and
$R^2$ represents an ethyl group.

[Aspect 151] The compound according to the Aspect 6, wherein Q represents the group represented by Q1.
[Aspect 152] The compound according to the Aspect 7, wherein Q represents the group represented by Q1.
[Aspect 153] The compound according to the Aspect 6, wherein Q represents the group represented by Q2.
[Aspect 154] The compound according to the Aspect 7, wherein Q represents the group represented by Q2.
[Aspect 155] The compound according to the Aspect 8, wherein Het represents the group represented by Het1.
[Aspect 156] The compound according to the Aspect 9, wherein Het represents the group represented by Het1.
[Aspect 157] The compound according to the Aspect 10, wherein Het represents the group represented by Het1.
[Aspect 158] The compound according to the Aspect 11, wherein Het represents the group represented by Het1.
[Aspect 159] The compound according to the Aspect 12, wherein Het represents the group represented by Het1.
[Aspect 160] The compound according to the Aspect 13, wherein Het represents the group represented by Het1.
[Aspect 161] The compound according to the Aspect 14, wherein Het represents the group represented by Het1.

[Aspect 162] The compound according to the Aspect 15, wherein Het represents the group represented by Het1.
[Aspect 163] The compound according to the Aspect 16, wherein Het represents the group represented by Het1.
[Aspect 164] The compound according to the Aspect 17, wherein Het represents the group represented by Het1.
[Aspect 165] The compound according to the Aspect 18, wherein Het represents the group represented by Het1.
[Aspect 166] The compound according to the Aspect 19, wherein Het represents the group represented by Het1.
[Aspect 167] The compound according to the Aspect 20, wherein Het represents the group represented by Het1.
[Aspect 168] The compound according to the Aspect 21, wherein Het represents the group represented by Het1.
[Aspect 169] The compound according to the Aspect 22, wherein Het represents the group represented by Het1.
[Aspect 170] The compound according to the Aspect 23, wherein Het represents the group represented by Het1.
[Aspect 171] The compound according to the Aspect 24, wherein Het represents the group represented by Het1.
[Aspect 172] The compound according to the Aspect 25, wherein Het represents the group represented by Het1.
[Aspect 173] The compound according to the Aspect 26, wherein Het represents the group represented by Het1.
[Aspect 174] The compound according to the Aspect 27, wherein Het represents the group represented by Het1.
[Aspect 175] The compound according to the Aspect 28, wherein Het represents the group represented by Het1.
[Aspect 176] The compound according to the Aspect 29, wherein Het represents the group represented by Het1.
[Aspect 177] The compound according to the Aspect 30, wherein Het represents the group represented by Het1.
[Aspect 178] The compound according to the Aspect 31, wherein Het represents the group represented by Het1.
[Aspect 179] The compound according to the Aspect 32, wherein Het represents the group represented by Het1.
[Aspect 180] The compound according to the Aspect 8, wherein Het represents the group represented by Het2.
[Aspect 181] The compound according to the Aspect 9, wherein Het represents the group represented by Het2.
[Aspect 182] The compound according to the Aspect 10, wherein Het represents the group represented by Het2.
[Aspect 183] The compound according to the Aspect 11, wherein Het represents the group represented by Het2.
[Aspect 184] The compound according to the Aspect 12, wherein Het represents the group represented by Het2.
[Aspect 185] The compound according to the Aspect 13, wherein Het represents the group represented by Het2.
[Aspect 186] The compound according to the Aspect 14, wherein Het represents the group represented by Het2.
[Aspect 187] The compound according to the Aspect 15, wherein Het represents the group represented by Het2.
[Aspect 188] The compound according to the Aspect 16, wherein Het represents the group represented by Het2.
[Aspect 189] The compound according to the Aspect 17, wherein Het represents the group represented by Het2.
[Aspect 190] The compound according to the Aspect 18, wherein Het represents the group represented by Het2.
[Aspect 191] The compound according to the Aspect 19, wherein Het represents the group represented by Het2.
[Aspect 192] The compound according to the Aspect 20, wherein Het represents the group represented by Het2.
[Aspect 193] The compound according to the Aspect 21, wherein Het represents the group represented by Het2.
[Aspect 194] The compound according to the Aspect 22, wherein Het represents the group represented by Het2.
[Aspect 195] The compound according to the Aspect 23, wherein Het represents the group represented by Het2.
[Aspect 196] The compound according to the Aspect 24, wherein Het represents the group represented by Het2.
[Aspect 197] The compound according to the Aspect 25, wherein Het represents the group represented by Het2.
[Aspect 198] The compound according to the Aspect 26, wherein Het represents the group represented by Het2.
[Aspect 199] The compound according to the Aspect 27, wherein Het represents the group represented by Het2.
[Aspect 200] The compound according to the Aspect 28, wherein Het represents the group represented by Het2.
[Aspect 201] The compound according to the Aspect 29, wherein Het represents the group represented by Het2.
[Aspect 202] The compound according to the Aspect 30, wherein Het represents the group represented by Het2.
[Aspect 203] The compound according to the Aspect 31, wherein Het represents the group represented by Het2.
[Aspect 204] The compound according to the Aspect 32, wherein Het represents the group represented by Het2.
[Aspect 205] The compound according to any one of the Aspects 1 to 5, Aspects 8 to 154, or the Present compound N, wherein

Het represents the group represented by Het1; and
the combination of $B^1$, $B^2$, and $B^3$ represents
a combination wherein $B^1$ represents $CR^{6a}$, $B^2$ represents $CR^{6b}$, and $B^3$ represents $CR^{6c}$;
a combination wherein $B^1$ represents a nitrogen atom, $B^2$ represents $CR^{6b}$, and $B^3$ represents $CR^{6c}$;
a combination wherein $B^1$ represents $CR^{6a}$, $B^2$ represents a nitrogen atom, and $B^3$ represents $CR^{6c}$; or
a combination wherein $B^1$ represents $CR^{6a}$, $B^2$ represents $CR^{6b}$, and $B^3$ represents a nitrogen atom.

[Aspect 206] The compound according to any one of the Aspects 1 to 5, Aspects 8 to 154, or the Present compound N, wherein

Het represents the group represented by Het1;
the combination of $B^1$, $B^2$, and $B^3$ represents

a combination wherein $B^1$ represents $CR^{6a}$, $B^2$ represents $CR^{6b}$, and $B^3$ represents $CR^{6c}$;
a combination wherein $B^1$ represents a nitrogen atom, $B^2$ represents $CR^{6b}$, and $B^3$ represents $CR^{6c}$;
a combination wherein $B^1$ represents $CR^{6a}$, $B^2$ represents a nitrogen atom, and $B^3$ represents $CR^{6c}$; or
a combination wherein $B^1$ represents $CR^{6a}$, $B^2$ represents $CR^{6b}$, and $B^3$ represents a nitrogen atom; and
$R^{6a}$, $R^{6b}$, and $R^{6c}$ are identical to or different from each other, and each represent a C1-C6 chain hydrocarbon group optionally substituted with one or more halogen atom(s), a C3-C7 cycloalkyl group optionally substituted with one or more substituent(s) selected from the group consisting of a halogen atom and a cyano group, $OR^{72}$, or a hydrogen atom.

[Aspect 207] The compound according to any one of the Aspects 1 to 5, Aspects 8 to 154, or the Present compound N, wherein

Het represents the group represented by Het1;
$B^1$ represents $CR^{6a}$;
$B^2$ represents $CR^{6b}$;
$B^3$ represents $CR^{6c}$;
$R^{6a}$ represents a hydrogen atom; and
$R^{6b}$ and $R^{6c}$ are identical to or different from each other, and each represent a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect 208] The compound according to any one of the Aspects 1 to 5, Aspects 8 to 154, or the Present compound N, wherein

Het represents the group represented by Het1;
$B^1$ represents a nitrogen atom;
$B^2$ represents $CR^{6b}$;
$B^3$ represents $CR^{6c}$;
$R^{6b}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$; and
$R^{6c}$ represents a hydrogen atom.

[Aspect 209] The compound according to any one of the Aspects 1 to 5, Aspects 8 to 154, or the Present compound N, wherein

Het represents the group represented by Het1;
$B^1$ represents a nitrogen atom;
$B^2$ represents $CR^{6b}$;
$B^3$ represents $CR^{6c}$;
$R^{6b}$ represents a hydrogen atom; and
$R^{6c}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect 210] The compound according to any one of the Aspects 1 to 5, Aspects 8 to 154, or the Present compound N, wherein

Het represents the group represented by Het1;
$B^1$ represents $CR^{6a}$;
$B^2$ represents a nitrogen atom;
$B^3$ represents $CR^{6c}$; and
$R^{6a}$ and $R^{6c}$ are identical to or different from each other, and each represent a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect 211] The compound according to any one of the Aspects 1 to 5, Aspects 8 to 154, or the Present compound N, wherein

Het represents the group represented by Het1;
$B^1$ represents $CR^{6a}$;
$B^2$ represents $CR^{6b}$;
$B^3$ represents a nitrogen atom;
$R^{6a}$ represents a hydrogen atom; and
$R^{6b}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect 212] The compound according to any one of the Aspects 1 to 5, Aspects 8 to 154, or the Present compound N, wherein

Het represents the group represented by Het1;
$B^1$ represents $CR^{6a}$;
$B^2$ represents $CR^{6b}$;
$B^3$ represents a nitrogen atom;
$R^{6a}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$; and
$R^{6b}$ represents a hydrogen atom.

[Aspect 213] The compound according to any one of the Aspects 1 to 5, Aspects 8 to 154, or the Present compound N, wherein

Het represents the group represented by Het2;
the combination of $B^1$, $B^2$, and $B^3$ represents
a combination wherein $B^1$ represents $CR^{6a}$, $B^2$ represents $CR^{6b}$, and $B^3$ represents $CR^{6c}$;
a combination wherein $B^1$ represents a nitrogen atom, $B^2$ represents $CR^{6b}$, and $B^3$ represents $CR^{6c}$;
a combination wherein $B^1$ represents $CR^{6a}$, $B^2$ represents a nitrogen atom, and $B^3$ represents $CR^{6c}$; or
a combination wherein $B^1$ represents $CR^{6a}$, $B^2$ represents $CR^{6b}$, and $B^3$ represents a nitrogen atom; and
$R^{6a}$, $R^{6b}$, and $R^{6c}$ are identical to or different from each other, and each represent a C1-C6 chain hydrocarbon group optionally substituted with one or more halogen atom(s), a C3-C7 cycloalkyl group optionally substituted with one or more substituent(s) selected from the group consisting of a halogen atom and a cyano group, $OR^{72}$, or a hydrogen atom.

[Aspect 214] The compound according to any one of the Aspects 1 to 5, Aspects 8 to 154, or the Present compound N, wherein

Het represents the group represented by Het2;
$B^1$ represents $CR^{6a}$;
$B^2$ represents $CR^{6b}$;
$B^3$ represents a nitrogen atom;
$R^{6a}$ represents a hydrogen atom; and
$R^{6b}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect 215] The compound according to any one of the Aspects 1 to 5, Aspects 8 to 154, or the Present compound N, wherein

Het represents the group represented by Het1; and
the combination of $A^2$ and $A^3$ represents
a combination wherein $A^2$ represents $-CR^{7a}R^{7b}-$ and $A^3$ represents $-CR^{7c}R^{7d}-$;
a combination wherein $A^2$ represents $-CR^{7a}R^{7b}-$ and $A^3$ represents $-CR^{7e}R^{7f}-CR^{7g}R^{7h}-^{*1}$ (wherein $^{*1}$ represents the binding position to $A^2$);
a combination wherein $A^2$ represents $-NR^{5a}-$ and $A^3$ represents $-CR^{7c}R^{7d}-$; or
a combination wherein $A^2$ represents $-NR^{5a}-$ and $A^3$ represents $-CR^{7e}R^{7f}-CR^{7g}R^{7h}-^{*1}$ (wherein $^{*1}$ represents the binding position to $A^2$).

[Aspect 216] The compound according to any one of the Aspects 1 to 5, Aspects 8 to 154, or the Present compound N, wherein

Het represents the group represented by Het1; and
the combination of $A^2$ and $A^3$ represents
a combination wherein $A^2$ represents $-CR^{7a}R^{7b}-$ and $A^3$ represents $-CR^{7c}R^{7d}-$; or
a combination wherein $A^2$ represents $-CR^{7a}R^{7b}-$ and $A^3$ represents $-CR^{7e}R^{7f}-CR^{7g}R^{7h}-*^1$ (wherein $*^1$ represents the binding position to $A^2$).

[Aspect 217] The compound according to any one of the Aspects 1 to 5, Aspects 8 to 154, or the Present compound N, wherein

Het represents the group represented by Het2; and
the combination of $A^2$ and $A^4$ represents
a combination wherein $A^2$ represents $-CR^{7a}R^{7b}-$ and $A^4$ represents $-CR^{7o}R^{7p}-$;
a combination wherein $A^2$ represents $-CR^{7a}R^{7b}-$ and $A^4$ represents $-CR^{7q}R^{7r}-CR^{7s}R^{7t}-*^2$ (wherein $*^2$ represents the binding position to $A^2$);
a combination wherein $A^2$ represents $-CR^{7a}R^{7b}-$ and $A^4$ represents $-NR^{5c}-CR^{7u}R^{7v}-*^2$ (wherein $*^2$ represents the binding position to $A^2$);
a combination wherein $A^2$ represents $-NR^{5a}-$ and $A^4$ represents $-CR^{7o}R^{7p}-$; or
a combination wherein $A^2$ represents $-NR^{5a}-$ and $A^4$ represents $-CR^{7q}R^{7r}-CR^{7s}R^{7t}-*^2$ (wherein $*^2$ represents the binding position to $A^2$).

[Aspect 218] The compound according to any one of the Aspects 1 to 5, Aspects 8 to 154, or the Present compound N, wherein

Het represents the group represented by Het2; and
the combination of $A^2$ and $A^4$ represents
a combination wherein $A^2$ represents $-CR^{7a}R^{7b}-$ and $A^4$ represents $-CR^{7o}R^{7p}-$; or
a combination wherein $A^2$ represents $-CR^{7a}R^{7b}-$ and $A^4$ represents $-CR^{7q}R^{7r}-CR^{7s}R^{7t}-*^2$ (wherein $*^2$ represents the binding position to $A^2$).

[Aspect 219] The compound according to any one of the Aspects 1 to 5, Aspects 8 to 154, or the Present compound N, wherein

Het represents the group represented by Het1;
the combination of $A^2$ and $A^3$ represents
a combination wherein $A^2$ represents $-CR^{7a}R^{7b}-$ and $A^3$ represents $-CR^{7c}R^{7d}-$; or
a combination wherein $A^2$ represents $-CR^{7a}R^{7b}-$ and $A^3$ represents $-CR^{7e}R^{7f}-CR^{7g}R^{7h}-*^1$ (wherein $*^1$ represents the binding position to $A^2$); and
$R^{7a}$, $R^{7b}$, $R^{7c}$, $R^{7d}$, $R^{7e}$, $R^{7f}$, $R^{7g}$, and $R^{7h}$ are identical to or different from each other, and each represent a C1-C6 alkyl group or a hydrogen atom.

[Aspect 220] The compound according to any one of the **Aspects 1** to 5, Aspects 8 to 154, or the Present compound N, wherein

Het represents the group represented by Het1;
$A^2$ represents $-CR^{7a}R^{7b}-$;
$A^3$ represents $-CR^{7c}R^{7d}-$;
$R^{7a}$ and $R^{7b}$, and $R^{7c}$ and $R^{7d}$ are each optionally combined with the carbon atom to which they are attached to form a C3-C4 cycloalkyl group; or
Het represents the group represented by Het1;
$A^2$ represents $-CR^{7a}R^{7b}-$;
$A^3$ represents $-CR^{7e}R^{7f}-CR^{7g}R^{7h}-*^1$ (wherein $*^1$ represents the binding position to $A^2$); and
$R^{7a}$ and $R^{7b}$, $R^{7e}$ and $R^{7f}$, and $R^{7g}$ and $R^{7h}$ are each optionally combined with the carbon atom to which they are attached to form a C3-C4 cycloalkyl group.

[Aspect 221] The compound according to any one of the Aspects 1 to 5, Aspects 8 to 154, or the Present compound N,

wherein

Het represents the group represented by Het2;
the combination of $A^2$ and $A^4$ represents
a combination wherein $A^2$ represents $-CR^{7a}R^{7b}-$ and $A^4$ represents $-CR^{7o}R^{7p}-$; or
a combination wherein $A^2$ represents $-CR^{7a}R^{7b}-$ and $A^4$ represents $-CR^{7q}R^{7r}-CR^{7s}R^{7t}-*^2$ (wherein $*^2$ represents the binding position to $A^2$); and
$R^{7a}$, $R^{7b}$, $R^{7o}$, $R^{7p}$, $R^{7q}$, R7r, $R^{7s}$, and $R^{7t}$ are identical to or different from each other, and each represent a C1-C6 alkyl group or a hydrogen atom.

[Aspect 222] The compound according to any one of the Aspects 1 to 5, Aspects 8 to 154, or the Present compound N, wherein

Het represents the group represented by Het2;
$A^2$ represents $-CR^{7a}R^{7b}-$;
$A^4$ represents $-CR^{7o}R^{7p}-$;
$R^{7a}$ and $R^{7b}$, and $R^{7o}$ and $R^{7p}$ are each optionally combined with the carbon atom to which they are attached to form a C3-C4 cycloalkyl group; or
Het represents the group represented by Het2;
$A^2$ represents $-CR^{7a}R^{7b}-$;
$A^4$ represents $-CR^{7q}R^{7r}-CR^{7s}R^{7t}-*^2$ (wherein $*^2$ represents the binding position to $A^2$); and
$R^{7a}$ and $R^{7b}$, $R^{7q}$ and $R^{7r}$, and $R^{7s}$ and $R^{7t}$ are each optionally combined with the carbon atom to which they are attached to form a C3-C4 cycloalkyl group.

[Aspect 223] The compound according to any one of the Aspects 1 to 5, Aspects 8 to 154, or the Present compound N, wherein

Het represents the group represented by Het1;
$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 chain hydrocarbon group optionally substituted with one or more halogen atom(s), or $OR^{72}$; and
$R^{72}$ represents a C1-C6 chain hydrocarbon group optionally substituted with a halogen atom.

[Aspect 224] The compound according to any one of the Aspects 1 to 5, Aspects 8 to 154, or the Present compound N, wherein

Het represents the group represented by Het2;
$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 chain hydrocarbon group optionally substituted with one or more halogen atom(s), or $OR^{72}$; and
$R^{72}$ represents a C1-C6 chain hydrocarbon group optionally substituted with a halogen atom.

[Aspect 225] The compound according to any one of the Aspects 1 to 5, Aspects 8 to 154, or the Present compound N, wherein

Het represents the group represented by Het1;
$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 chain hydrocarbon group optionally substituted with one or more halogen atom(s), a C3-C7 cycloalkyl group optionally substituted with one or more substituent(s) selected from the group consisting of a

halogen atom and a cyano group, a halogen atom, or $OR^{72}$; and
$R^{72}$ represents a C1-C6 chain hydrocarbon group optionally substituted with a halogen atom.

[Aspect 226] The compound according to any one of the Aspects 1 to 5, Aspects 8 to 154, or the Present compound N, wherein

Het represents the group represented by Het2;
$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, a halogen atom, or $OR^{72}$; and
$R^{72}$ represents a C1-C6 chain hydrocarbon group optionally substituted with a halogen atom.

[Aspect 227] The compound according to any one of the Aspects 1 to 5, Aspects 8 to 154, or the Present compound N, wherein

Het represents the group represented by Het1;
$A^2$ represents $-CR^{7a}R^{7b}-$;
$A^3$ represents $-CR^{7c}R^{7d}-$; and
$R^{7a}$, $R^{7b}$, $R^{7c}$, and $R^{7d}$ each represent a hydrogen atom.

[Aspect 228] The compound according to any one of the Aspects 1 to 5, Aspects 8 to 154, or the Present compound N, wherein

Het represents the group represented by Het1;
$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 chain hydrocarbon group optionally substituted with one or more halogen atom(s), or $OR^{72}$;
$R^{72}$ represents a C1-C6 chain hydrocarbon group optionally substituted with a halogen atom;
$A^2$ represents $-CR^{7a}R^{7b}-$;
$A^3$ represents $-CR^{7c}R^{7d}-$; and
$R^{7a}$, $R^{7b}$, $R^{7c}$, and $R^{7d}$ each represent a hydrogen atom.

[Aspect 229] The compound according to any one of the Aspects 1 to 5, Aspects 8 to 154, or the Present compound N, wherein

Het represents the group represented by Het1;
$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 alkyl group optionally substituted with one or more halogen atom(s), $OR^{72}$, or a halogen atom;
$R^{72}$ represents a C1-C6 alkyl group optionally substituted with a halogen atom;
$A^2$ represents $-CR^{7a}R^{7b}-$;
$A^3$ represents $-CR^{7c}R^{7d}-$; and
$R^{7a}$, $R^{7b}$, $R^{7c}$, and $R^{7d}$ each represent a hydrogen atom.

Aspects of the Intermediate A include the following compounds.

[Aspect A1] The compound according to the Intermediate A, wherein W represents an oxygen atom.
[Aspect A2] The compound according to the Intermediate A, wherein $R^2$ represents a C1-C6 alkyl group.

[Aspect A3] The compound according to the Intermediate A, wherein $R^2$ represents an ethyl group.

[Aspect A4] The compound according to the Intermediate A, wherein $X^1$ represents a chlorine atom, a bromine atom, or an iodine atom.

[Aspect A5] The compound according to the Intermediate A, wherein $X^1$ represents a hydroxy group.

[Aspect A6] The compound according to the Intermediate A, wherein

the combination of $G^1$, $G^2$, $G^3$, and $G^4$ represents
a combination wherein $G^1$ represents $CR^{3a}$, $G^2$ represents $CR^{3b}$, $G^3$ represents $CR^{3c}$, and $G^4$ represents a nitrogen atom;
a combination wherein $G^1$ represents $CR^{3a}$, $G^2$ represents $CR^{3b}$, $G^3$ represents a nitrogen atom, and $G^4$ represents $CR^{3d}$;
a combination wherein $G^1$ represents $CR^{3a}$, $G^2$ represents a nitrogen atom, $G^3$ represents $CR^{3c}$, and $G^4$ represents $CR^{3d}$;
a combination wherein $G^1$ represents a nitrogen atom, $G^2$ represents $CR^{3b}$, $G^3$ represents $CR^{3c}$, and $G^4$ represents $CR^{3d}$; or
a combination wherein $G^1$ represents $CR^{3a}$, $G^2$ represents $CR^{3b}$, $G^3$ represents $CR^{3c}$, and $G^4$ represents $CR^{3d}$;
$R^{3a}$, $R^{3c}$, and $R^{3d}$ each represent a hydrogen atom; and
$R^{3b}$ represents a C1-C6 alkyl group optionally substituted with one or more halogen atom(s), a C3-C7 cycloalkyl group optionally substituted with one or more substituent(s) selected from the group consisting of a halogen atom and a cyano group, a phenyl group optionally substituted with one or more halogen atom(s), a 5 or 6 membered aromatic heterocyclic group optionally substituted with one or more halogen atom(s), or a halogen atom.

[Aspect A7] The compound according to the Intermediate A, wherein

$G^1$ represents $CR^{3a}$;
$G^2$ represents $CR^{3b}$;
$G^3$ represents $CR^{3c}$;
$G^4$ represents a nitrogen atom or $CR^{3d}$;
$R^{3a}$, $R^{3c}$, and $R^{3d}$ each represent a hydrogen atom; and
$R^{3b}$ represents a C1-C6 alkyl group optionally substituted with one or more halogen atom(s), or a halogen atom.

[Aspect A8] The compound according to the Intermediate A, wherein

$G^1$ represents $CR^{3a}$;
$G^2$ represents $CR^{3b}$;
$G^3$ represents $CR^{3c}$;
$G^4$ represents $CR^{3d}$;
$R^{3a}$, $R^{3c}$, and $R^{3d}$ each represent a hydrogen atom; and
$R^{3b}$ represents a C1-C6 alkyl group optionally substituted with one or more halogen atom(s), or a halogen atom.

[Aspect A9] The compound according to the Intermediate A, wherein

the combination of $G^1$, $G^2$, $G^3$, and $G^4$ represents
a combination wherein $G^1$ represents $CR^{3a}$, $G^2$ represents $CR^{3b}$, $G^3$ represents $CR^{3c}$, and $G^4$ represents a nitrogen atom;
a combination wherein $G^1$ represents $CR^{3a}$, $G^2$ represents $CR^{3b}$, $G^3$ represents a nitrogen atom, and $G^4$ represents $CR^{3d}$;
a combination wherein $G^1$ represents $CR^{3a}$, $G^2$ represents a nitrogen atom, $G^3$ represents $CR^{3c}$, and $G^4$ represents $CR^{3d}$;
a combination wherein $G^1$ represents a nitrogen atom, $G^2$ represents $CR^{3b}$, $G^3$ represents $CR^{3c}$, and $G^4$ represents $CR^{3d}$; or
a combination wherein $G^1$ represents $CR^{3a}$, $G^2$ represents $CR^{3b}$, $G^3$ represents $CR^{3c}$, and $G^4$ represents $CR^{3d}$;
$R^{3a}$, $R^{3b}$, and $R^{3d}$ each represent a hydrogen atom; and
$R^{3c}$ represents a halogen atom.

[Aspect A10] The compound according to the Intermediate A, wherein

$G^1$ represents $CR^{3a}$;

$G^2$ represents $CR^{3b}$;
$G^3$ represents $CR^{3c}$;
$G^4$ represents a nitrogen atom or $CR^{3d}$;
$R^{3a}$, $R^{3b}$, and $R^{3d}$ each represent a hydrogen atom; and
$R^{3c}$ represents a halogen atom.

[Aspect A11] The compound according to the Intermediate A, wherein

$G^1$ represents $CR^{3a}$;
$G^2$ represents $CR^{3b}$;
$G^3$ represents $CR^{3c}$;
$G^4$ represents $CR^{3d}$;
$R^{3a}$, $R^{3b}$, and $R^{3d}$ each represent a hydrogen atom; and
$R^{3c}$ represents a halogen atom.

[Aspect A12] The compound according to the Intermediate A, wherein

$G^1$ represents $CR^{3a}$;
$G^2$ represents $CR^{3b}$;
$G^3$ represents $CR^{3c}$;
$G^4$ represents $CR^{3d}$;
$R^{3a}$, $R^{3c}$, and $R^{3d}$ each represent a hydrogen atom; and
$R^{3b}$ represents a halogen atom.

[Aspect A13] The compound according to the Aspect A2, wherein W represents an oxygen atom.
[Aspect A14] The compound according to the Aspect A3, wherein W represents an oxygen atom.
[Aspect A15] The compound according to the Aspect A4, wherein W represents an oxygen atom.
[Aspect A16] The compound according to the Aspect A5, wherein W represents an oxygen atom.
[Aspect A17] The compound according to the Aspect A6, wherein W represents an oxygen atom.
[Aspect A18] The compound according to the Aspect A7, wherein W represents an oxygen atom.
[Aspect A19] The compound according to the Aspect A8, wherein W represents an oxygen atom.
[Aspect A20] The compound according to the Aspect A9, wherein W represents an oxygen atom.
[Aspect A21] The compound according to the Aspect A10, wherein W represents an oxygen atom.
[Aspect A22] The compound according to the Aspect A11, wherein W represents an oxygen atom.
[Aspect A23] The compound according to the Aspect A12, wherein W represents an oxygen atom.
[Aspect A24] The compound according to the Aspect A4, wherein $R^2$ represents a C1-C6 alkyl group.
[Aspect A25] The compound according to the Aspect A5, wherein $R^2$ represents a C1-C6 alkyl group.
[Aspect A26] The compound according to the Aspect A6, wherein $R^2$ represents a C1-C6 alkyl group.
[Aspect A27] The compound according to the Aspect A7, wherein $R^2$ represents a C1-C6 alkyl group.
[Aspect A28] The compound according to the Aspect A8, wherein $R^2$ represents a C1-C6 alkyl group.
[Aspect A29] The compound according to the Aspect A9, wherein $R^2$ represents a C1-C6 alkyl group.
[Aspect A30] The compound according to the Aspect A10, wherein $R^2$ represents a C1-C6 alkyl group.
[Aspect A31] The compound according to the Aspect A11, wherein $R^2$ represents a C1-C6 alkyl group.
[Aspect A32] The compound according to the Aspect A12, wherein $R^2$ represents a C1-C6 alkyl group.
[Aspect A33] The compound according to the Aspect A4, wherein $R^2$ represents an ethyl group.
[Aspect A34] The compound according to the Aspect A5, wherein $R^2$ represents an ethyl group.
[Aspect A35] The compound according to the Aspect A6, wherein $R^2$ represents an ethyl group.
[Aspect A36] The compound according to the Aspect A7, wherein $R^2$ represents an ethyl group.
[Aspect A37] The compound according to the Aspect A8, wherein $R^2$ represents an ethyl group.
[Aspect A38] The compound according to the Aspect A9, wherein $R^2$ represents an ethyl group.
[Aspect A39] The compound according to the Aspect A10, wherein $R^2$ represents an ethyl group.
[Aspect A40] The compound according to the Aspect A11, wherein $R^2$ represents an ethyl group.
[Aspect A41] The compound according to the Aspect A12, wherein $R^2$ represents an ethyl group.
[Aspect A42] The compound according to the Aspect A4, wherein

W represents an oxygen atom; and
$R^2$ represents an ethyl group.

[Aspect A43] The compound according to the Aspect A5, wherein

W represents an oxygen atom; and
$R^2$ represents an ethyl group.

[Aspect A44] The compound according to the Aspect A6, wherein

W represents an oxygen atom; and
$R^2$ represents an ethyl group.

[Aspect A45] The compound according to the Aspect A7, wherein

W represents an oxygen atom; and
$R^2$ represents an ethyl group.

[Aspect A46] The compound according to the Aspect A8, wherein

W represents an oxygen atom; and
$R^2$ represents an ethyl group.

[Aspect A47] The compound according to the Aspect A9, wherein

W represents an oxygen atom; and
$R^2$ represents an ethyl group.

[Aspect A48] The compound according to the Aspect A10, wherein

W represents an oxygen atom; and
$R^2$ represents an ethyl group.

[Aspect A49] The compound according to the Aspect A11, wherein

W represents an oxygen atom; and
$R^2$ represents an ethyl group.

[Aspect A50] The compound according to the Aspect A12, wherein

W represents an oxygen atom; and
$R^2$ represents an ethyl group.

[Aspect A51] The compound according to the Aspect A6, wherein $X^1$ represents a chlorine atom, a bromine atom, or an iodine atom.
[Aspect A52] The compound according to the Aspect A7, wherein $X^1$ represents a chlorine atom, a bromine atom, or an iodine atom.
[Aspect A53] The compound according to the Aspect A8, wherein $X^1$ represents a chlorine atom, a bromine atom, or an iodine atom.
[Aspect A54] The compound according to the Aspect A9, wherein $X^1$ represents a chlorine atom, a bromine atom, or an iodine atom.
[Aspect A55] The compound according to the Aspect A10, wherein $X^1$ represents a chlorine atom, a bromine atom, or an iodine atom.
[Aspect A56] The compound according to the Aspect A11, wherein $X^1$ represents a chlorine atom, a bromine atom, or an iodine atom.
[Aspect A57] The compound according to the Aspect A12, wherein $X^1$ represents a chlorine atom, a bromine atom, or an iodine atom.
[Aspect A58] The compound according to the Aspect A6, wherein $X^1$ represents a hydroxy group.
[Aspect A59] The compound according to the Aspect A7, wherein $X^1$ represents a hydroxy group.
[Aspect A60] The compound according to the Aspect A8, wherein $X^1$ represents a hydroxy group.
[Aspect A61] The compound according to the Aspect A9, wherein $X^1$ represents a hydroxy group.

[Aspect A62] The compound according to the Aspect A10, wherein $X^1$ represents a hydroxy group.
[Aspect A63] The compound according to the Aspect A11, wherein $X^1$ represents a hydroxy group.
[Aspect A64] The compound according to the Aspect A12, wherein $X^1$ represents a hydroxy group.
[Aspect A65] The compound according to any one of the Aspects A1 to A64 or the Intermediate A, wherein

the combination of $B^1$, $B^2$, and $B^3$ represents
a combination wherein $B^1$ represents $CR^{6a}$, $B^2$ represents $CR^{6b}$, and $B^3$ represents $CR^{6c}$;
a combination wherein $B^1$ represents a nitrogen atom, $B^2$ represents $CR^{6b}$, and $B^3$ represents $CR^{6c}$;
a combination wherein $B^1$ represents $CR^{6a}$, $B^2$ represents a nitrogen atom, and $B^3$ represents $CR^{6c}$; or
a combination wherein $B^1$ represents $CR^{6a}$, $B^2$ represents $CR^{6b}$, and $B^3$ represents a nitrogen atom.

[Aspect A66] The compound according to any one of the Aspects A1 to A64 or the Intermediate A, wherein

the combination of $B^1$, $B^2$, and $B^3$ represents
a combination wherein $B^1$ represents $CR^{6a}$, $B^2$ represents $CR^{6b}$, and $B^3$ represents $CR^{6c}$;
a combination wherein $B^1$ represents a nitrogen atom, $B^2$ represents $CR^{6b}$, and $B^3$ represents $CR^{6c}$;
a combination wherein $B^1$ represents $CR^{6a}$, $B^2$ represents a nitrogen atom, and $B^3$ represents $CR^{6c}$; or
a combination wherein $B^1$ represents $CR^{6a}$, $B^2$ represents $CR^{6b}$, and $B^3$ represents a nitrogen atom; and
$R^{6a}$, $R^{6b}$, and $R^{6c}$ are identical to or different from each other, and each represent a C1-C6 chain hydrocarbon group optionally substituted with one or more halogen atom(s), a C3-C7 cycloalkyl group optionally substituted with one or more substituent(s) selected from the group consisting of a halogen atom and a cyano group, $OR^{72}$, or a hydrogen atom.

[Aspect A67] The compound according to any one of the Aspects A1 to A64 or the Intermediate A, wherein

$B^1$ represents $CR^{6a}$;
$B^2$ represents $CR^{6b}$;
$B^3$ represents $CR^{6c}$;
$R^{6a}$ represents a hydrogen atom; and
$R^{6b}$ and $R^{6c}$ are identical to or different from each other, and each represent a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect A68] The compound according to any one of the Aspects A1 to A64 or the Intermediate A, wherein

$B^1$ represents a nitrogen atom;
$B^2$ represents $CR^{6b}$;
$B^3$ represents $CR^{6c}$;
$R^{6b}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$; and
$R^{6c}$ represents a hydrogen atom.

[Aspect A69] The compound according to any one of the Aspects A1 to A64 or the Intermediate A, wherein

$B^1$ represents a nitrogen atom;
$B^2$ represents $CR^{6b}$;
$B^3$ represents $CR^{6c}$;
$R^{6b}$ represents a hydrogen atom; and
$R^{6c}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect A70] The compound according to any one of the Aspects A1 to A64 or the Intermediate A, wherein

$B^1$ represents $CR^{6a}$;
$B^2$ represents a nitrogen atom;
$B^3$ represents $CR^{6c}$; and

$R^{6a}$ and $R^{6c}$ are identical to or different from each other, and each represent a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect A71] The compound according to any one of the Aspects A1 to A64 or the Intermediate A, wherein

$B^1$ represents $CR^{6a}$;
$B^2$ represents $CR^{6b}$;
$B^3$ represents a nitrogen atom;
$R^{6a}$ represents a hydrogen atom; and
$R^{6b}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect A72] The compound according to any one of the Aspects A1 to A64 or the Intermediate A, wherein

$B^1$ represents $CR^{6a}$;
$B^2$ represents $CR^{6b}$;
$B^3$ represents a nitrogen atom;
$R^{6a}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$; and
$R^{6b}$ represents a hydrogen atom.

[Aspect A73] The compound according to any one of the Aspects A1 to A64 or the Intermediate A, wherein

the combination of $A^2$ and $A^3$ represents
a combination wherein $A^2$ represents $-CR^{7a}R^{7b}-$ and $A^3$ represents $-CR^{7c}R^{7d}-$;
a combination wherein $A^2$ represents $-CR^{7a}R^{7b}-$ and $A^3$ represents $-CR^{7e}R^{7f}-CR^{7g}R^{7h}-*^1$ (wherein $*^1$ represents the binding position to $A^2$);
a combination wherein $A^2$ represents $-NR^{5a}-$ and $A^3$ represents $-CR^{7c}R^{7d}-$; or
a combination wherein $A^2$ represents $-NR^{5a}-$ and $A^3$ represents $-CR^{7e}R^{7f}-CR^{7g}R^{7h}-*^1$ (wherein $*^1$ represents the binding position to $A^2$).

[Aspect A74] The compound according to any one of the Aspects A1 to A64 or the Intermediate A, wherein

the combination of $A^2$ and $A^3$ represents
a combination wherein $A^2$ represents $-CR^{7a}R^{7b}-$ and $A^3$ represents $-CR^{7c}R^{7d}-$; or
a combination wherein $A^2$ represents $-CR^{7a}R^{7b}-$ and $A^3$ represents $-CR^{7e}R^{7f}-CR^{7g}R^{7h}-*^1$ (wherein $*^1$ represents the binding position to $A^2$).

[Aspect A75] The compound according to any one of the Aspects A1 to A64 or the Intermediate A, wherein

the combination of $A^2$ and $A^3$ represents
a combination wherein $A^2$ represents $-CR^{7a}R^{7b}-$ and $A^3$ represents $-CR^{7c}R^{7d}-$; or
a combination wherein $A^2$ represents $-CR^{7a}R^{7b}-$ and $A^3$ represents $-CR^{7e}R^{7f}-CR^{7g}R^{7h}-*^1$ (wherein $*^1$ represents the binding position to $A^2$); and
$R^{7a}$, $R^{7b}$, $R^{7c}$, $R^{7d}$, $R^{7e}$, $R^{7f}$, $R^7g$, and $R^{7h}$ are identical to or different from each other, and each represent a C1-C6 alkyl group or a hydrogen atom.

[Aspect A76] The compound according to any one of the Aspects A1 to A64 or the Intermediate A, wherein

$A^2$ represents $-CR^{7a}R^{7b}-$;
$A^3$ represents $-CR^{7c}R^{7d}-$; and
$R^{7a}$ and $R^{7b}$, and $R^{7c}$ and $R^{7d}$ are each optionally combined with the carbon atom to which they are attached to form a C3-C4 cycloalkyl group; or
$A^2$ represents $-CR^{7a}R^{7b}-$;
$A^3$ represents $-CR^{7e}R^{7f}-CR^{7g}R^{7h}-*^1$ (wherein $*^1$ represents the binding position to $A^2$) ; and

$R^{7a}$ and $R^{7b}$, $R^{7e}$ and $R^{7f}$, and $R^{7g}$ and $R^{7h}$ are each optionally combined with the carbon atom to which they are attached to form a C3-C4 cycloalkyl group.

[Aspect A77] The compound according to any one of the Aspects A1 to A64 or the Intermediate A, wherein

$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 chain hydrocarbon group optionally substituted with one or more halogen atom(s), or $OR^{72}$; and
$R^{72}$ represents a C1-C6 chain hydrocarbon group optionally substituted with a halogen atom.

[Aspect A78] The compound according to any one of the Aspects A1 to A64 or the Intermediate A, wherein

$A^2$ represents $-CR^{7a}R^{7b}-$;
$A^3$ represents $-CR^{7c}R^{7d}-$; and
$R^{7a}$, $R^{7b}$, $R^{7c}$, and $R^{7d}$ each represent a hydrogen atom.

[Aspect A79] The compound according to any one of the Aspects A1 to A64 or the Intermediate A, wherein

$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 chain hydrocarbon group optionally substituted with one or more halogen atom(s), or $OR^{72}$;
$R^{72}$ represents a C1-C6 chain hydrocarbon group optionally substituted with a halogen atom;
$A^2$ represents $-CR^{7a}R^{7b}-$;
$A^3$ represents $-CR^{7c}R^{7d}-$; and
$R^{7a}$, $R^{7b}$, $R^{7c}$, and $R^{7d}$ each represent a hydrogen atom.

[Aspect A80] The compound according to any one of the Aspects A1 to A64 or the Intermediate A, wherein

$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 alkyl group optionally substituted with one or more halogen atom(s), or $OR^{72}$;
$R^{72}$ represents a C1-C6 alkyl group optionally substituted with a halogen atom;
$A^2$ represents $-CR^{7a}R^{7b}-$;
$A^3$ represents $-CR^{7c}R^{7d}-$; and
$R^{7a}$, $R^{7b}$, $R^{7c}$, and $R^{7d}$ each represent a hydrogen atom.

[Aspect A81] The compound according to any one of the Aspects A1 to A64 or the Intermediate A, wherein

$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 chain hydrocarbon group optionally substituted with one or more halogen atom(s), a halogen atom, or $OR^{72}$; and
$R^{72}$ represents a C1-C6 chain hydrocarbon group optionally substituted with a halogen atom.

Aspects of the Intermediate B include the following compounds.

[Aspect B1] The compound according to the Intermediate B, wherein W represents an oxygen atom.
[Aspect B2] The compound according to the Intermediate B, wherein $R^2$ represents a C1-C6 alkyl group.

[Aspect B3] The compound according to the Intermediate B, wherein $R^2$ represents an ethyl group.
[Aspect B4] The compound according to the Intermediate B, wherein $X^2$ represents a chlorine atom, a bromine atom, or an iodine atom.
[Aspect B5] The compound according to the Intermediate B, wherein $X^2$ represents a hydroxy group.
[Aspect B6] The compound according to the Intermediate B, wherein

the combination of $G^1$, $G^2$, $G^3$, and $G^4$ represents
a combination wherein $G^1$ represents $CR^{3a}$, $G^2$ represents $CR^{3b}$, $G^3$ represents $CR^{3c}$, and $G^4$ represents a nitrogen atom;
a combination wherein $G^1$ represents $CR^{3a}$, $G^2$ represents $CR^{3b}$, $G^3$ represents a nitrogen atom, and $G^4$ represents $CR^{3d}$;
a combination wherein $G^1$ represents $CR^{3a}$, $G^2$ represents a nitrogen atom, $G^3$ represents $CR^{3c}$, and $G^4$ represents $CR^{3d}$;
a combination wherein $G^1$ represents a nitrogen atom, $G^2$ represents $CR^{3b}$, $G^3$ represents $CR^{3c}$, and $G^4$ represents $CR^{3d}$; or
a combination wherein $G^1$ represents $CR^{3a}$, $G^2$ represents $CR^{3b}$, $G^3$ represents $CR^{3c}$, and $G^4$ represents $CR^{3d}$;
$R^{3a}$, $R^{3c}$, and $R^{3d}$ each represent a hydrogen atom; and
$R^{3b}$ represents a C1-C6 alkyl group optionally substituted with one or more halogen atom(s), a C3-C7 cycloalkyl group optionally substituted with one or more substituent(s) selected from the group consisting of a halogen atom and a cyano group, a phenyl group optionally substituted with one or more halogen atom(s), a 5 or 6 membered aromatic heterocyclic group optionally substituted with one or more halogen atom(s), or a halogen atom.

[Aspect B7] The compound according to the Intermediate B, wherein

$G^1$ represents $CR^{3a}$;
$G^2$ represents $CR^{3b}$;
$G^3$ represents $CR^{3c}$;
$G^4$ represents a nitrogen atom or $CR^{3d}$;
$R^{3a}$, $R^{3c}$, and $R^{3d}$ each represent a hydrogen atom; and
$R^{3b}$ represents a C1-C6 alkyl group optionally substituted with one or more halogen atom(s), or a halogen atom.

[Aspect B8] The compound according to the Intermediate B, wherein

$G^1$ represents $CR^{3a}$;
$G^2$ represents $CR^{3b}$;
$G^3$ represents $CR^{3c}$;
$G^4$ represents $CR^{3d}$;
$R^{3a}$, $R^{3c}$, and $R^{3d}$ each represent a hydrogen atom; and
$R^{3b}$ represents a C1-C6 alkyl group optionally substituted with one or more halogen atom(s), or a halogen atom.

[Aspect B9] The compound according to the Intermediate B, wherein

the combination of $G^1$, $G^2$, $G^3$, and $G^4$ represents
a combination wherein $G^1$ represents $CR^{3a}$, $G^2$ represents $CR^{3b}$, $G^3$ represents $CR^{3c}$, and $G^4$ represents a nitrogen atom;
a combination wherein $G^1$ represents $CR^{3a}$, $G^2$ represents $CR^{3b}$, $G^3$ represents a nitrogen atom, and $G^4$ represents $CR^{3d}$;
a combination wherein $G^1$ represents $CR^{3a}$, $G^2$ represents a nitrogen atom, $G^3$ represents $CR^{3c}$, and $G^4$ represents $CR^{3d}$;
a combination wherein $G^1$ represents a nitrogen atom, $G^2$ represents $CR^{3b}$, $G^3$ represents $CR^{3c}$, and $G^4$ represents $CR^{3d}$; or
a combination wherein $G^1$ represents $CR^{3a}$, $G^2$ represents $CR^{3b}$, $G^3$ represents $CR^{3c}$, and $G^4$ represents $CR^{3d}$;
$R^{3a}$, $R^{3b}$, and $R^{3d}$ each represent a hydrogen atom; and
$R^{3c}$ represents a halogen atom.

[Aspect B10] The compound according to the Intermediate B, wherein

$G^1$ represents $CR^{3a}$;

$G^2$ represents $CR^{3b}$;
$G^3$ represents $CR^{3c}$;
$G^4$ represents a nitrogen atom or $CR^{3d}$;
$R^{3a}$, $R^{3b}$, and $R^{3d}$ each represent a hydrogen atom; and
$R^{3c}$ represents a halogen atom.

[Aspect B11] The compound according to the Intermediate B, wherein

$G^1$ represents $CR^{3a}$;
$G^2$ represents $CR^{3b}$;
$G^3$ represents $CR^{3c}$;
$G^4$ represents $CR^{3d}$;
$R^{3a}$, $R^{3b}$, and $R^{3d}$ each represent a hydrogen atom; and
$R^{3c}$ represents a halogen atom.

[Aspect B12] The compound according to the Intermediate B, wherein

$G^1$ represents $CR^{3a}$;
$G^2$ represents $CR^{3b}$;
$G^3$ represents $CR^{3c}$;
$G^4$ represents $CR^{3d}$;
$R^{3a}$, $R^{3c}$, and $R^{3d}$ each represent a hydrogen atom; and
$R^{3b}$ represents a halogen atom.

[Aspect B13] The compound according to the Aspect B2, wherein W represents an oxygen atom.
[Aspect B14] The compound according to the Aspect B3, wherein W represents an oxygen atom.
[Aspect B15] The compound according to the Aspect B4, wherein W represents an oxygen atom.
[Aspect B16] The compound according to the Aspect B5, wherein W represents an oxygen atom.
[Aspect B17] The compound according to the Aspect B6, wherein W represents an oxygen atom.
[Aspect B18] The compound according to the Aspect B7, wherein W represents an oxygen atom.
[Aspect B19] The compound according to the Aspect B8, wherein W represents an oxygen atom.
[Aspect B20] The compound according to the Aspect B9, wherein W represents an oxygen atom.
[Aspect B21] The compound according to the Aspect B10, wherein W represents an oxygen atom.
[Aspect B22] The compound according to the Aspect B11, wherein W represents an oxygen atom.
[Aspect B23] The compound according to the Aspect B12, wherein W represents an oxygen atom.
[Aspect B24] The compound according to the Aspect B4, wherein $R^2$ represents a C1-C6 alkyl group.
[Aspect B25] The compound according to the Aspect B5, wherein $R^2$ represents a C1-C6 alkyl group.
[Aspect B26] The compound according to the Aspect B6, wherein $R^2$ represents a C1-C6 alkyl group.
[Aspect B27] The compound according to the Aspect B7, wherein $R^2$ represents a C1-C6 alkyl group.
[Aspect B28] The compound according to the Aspect B8, wherein $R^2$ represents a C1-C6 alkyl group.
[Aspect B29] The compound according to the Aspect B9, wherein $R^2$ represents a C1-C6 alkyl group.
[Aspect B30] The compound according to the Aspect B10, wherein $R^2$ represents a C1-C6 alkyl group.
[Aspect B31] The compound according to the Aspect B11, wherein $R^2$ represents a C1-C6 alkyl group.
[Aspect B32] The compound according to the Aspect B12, wherein $R^2$ represents a C1-C6 alkyl group.
[Aspect B33] The compound according to the Aspect B4, wherein $R^2$ represents an ethyl group.
[Aspect B34] The compound according to the Aspect B5, wherein $R^2$ represents an ethyl group.
[Aspect B35] The compound according to the Aspect B6, wherein $R^2$ represents an ethyl group.
[Aspect B36] The compound according to the Aspect B7, wherein $R^2$ represents an ethyl group.
[Aspect B37] The compound according to the Aspect B8, wherein $R^2$ represents an ethyl group.
[Aspect B38] The compound according to the Aspect B9, wherein $R^2$ represents an ethyl group.
[Aspect B39] The compound according to the Aspect B10, wherein $R^2$ represents an ethyl group.
[Aspect B40] The compound according to the Aspect B11, wherein $R^2$ represents an ethyl group.
[Aspect B41] The compound according to the Aspect B12, wherein $R^2$ represents an ethyl group.
[Aspect B42] The compound according to the Aspect B4, wherein

W represents an oxygen atom; and
$R^2$ represents an ethyl group.

[Aspect B43] The compound according to the Aspect B5, wherein

W represents an oxygen atom; and
$R^2$ represents an ethyl group.

[Aspect B44] The compound according to the Aspect B6, wherein

W represents an oxygen atom; and
$R^2$ represents an ethyl group.

[Aspect B45] The compound according to the Aspect B7, wherein

W represents an oxygen atom; and
$R^2$ represents an ethyl group.

[Aspect B46] The compound according to the Aspect B8, wherein

W represents an oxygen atom; and
$R^2$ represents an ethyl group.

[Aspect B47] The compound according to the Aspect B9, wherein

W represents an oxygen atom; and
$R^2$ represents an ethyl group.

[Aspect B48] The compound according to the Aspect B10, wherein

W represents an oxygen atom; and
$R^2$ represents an ethyl group.

[Aspect B49] The compound according to the Aspect B11, wherein

W represents an oxygen atom; and
$R^2$ represents an ethyl group.

[Aspect B50] The compound according to the Aspect B12, wherein

W represents an oxygen atom; and
$R^2$ represents an ethyl group.

[Aspect B51] The compound according to the Aspect B6, wherein $X^2$ represents a chlorine atom, a bromine atom, or an iodine atom.
[Aspect B52] The compound according to the Aspect B7, wherein $X^2$ represents a chlorine atom, a bromine atom, or an iodine atom.
[Aspect B53] The compound according to the Aspect B8, wherein $X^2$ represents a chlorine atom, a bromine atom, or an iodine atom.
[Aspect B54] The compound according to the Aspect B9, wherein $X^2$ represents a chlorine atom, a bromine atom, or an iodine atom.
[Aspect B55] The compound according to the Aspect B10, wherein $X^2$ represents a chlorine atom, a bromine atom, or an iodine atom.
[Aspect B56] The compound according to the Aspect B11, wherein $X^2$ represents a chlorine atom, a bromine atom, or an iodine atom.
[Aspect B57] The compound according to the Aspect B12, wherein $X^2$ represents a chlorine atom, a bromine atom, or an iodine atom.
[Aspect B58] The compound according to the Aspect B6, wherein $X^2$ represents a hydroxy group.
[Aspect B59] The compound according to the Aspect B7, wherein $X^2$ represents a hydroxy group.
[Aspect B60] The compound according to the Aspect B8, wherein $X^2$ represents a hydroxy group.
[Aspect B61] The compound according to the Aspect B9, wherein $X^2$ represents a hydroxy group.

[Aspect B62] The compound according to the Aspect B10, wherein $X^2$ represents a hydroxy group.
[Aspect B63] The compound according to the Aspect B11, wherein $X^2$ represents a hydroxy group.
[Aspect B64] The compound according to the Aspect B12, wherein $X^2$ represents a hydroxy group.
[Aspect B65] The compound according to any one of the Aspects B1 to B64 or the Intermediate B, wherein

the combination of $B^1$, $B^2$, and $B^3$ represents
a combination wherein $B^1$ represents $CR^{6a}$, $B^2$ represents $CR^{6b}$, and $B^3$ represents $CR^{6c}$;
a combination wherein $B^1$ represents a nitrogen atom, $B^2$ represents $CR^{6b}$, and $B^3$ represents $CR^{6c}$;
a combination wherein $B^1$ represents $CR^{6a}$, $B^2$ represents a nitrogen atom, and $B^3$ represents $CR^{6c}$; or
a combination wherein $B^1$ represents $CR^{6a}$, $B^2$ represents $CR^{6b}$, and $B^3$ represents a nitrogen atom.

[Aspect B66] The compound according to any one of the Aspects B1 to B64 or the Intermediate B, wherein

the combination of $B^1$, $B^2$, and $B^3$ represents
a combination wherein $B^1$ represents $CR^{6a}$, $B^2$ represents $CR^{6b}$, and $B^3$ represents $CR^{6c}$;
a combination wherein $B^1$ represents a nitrogen atom, $B^2$ represents $CR^{6b}$, and $B^3$ represents $CR^{6c}$;
a combination wherein $B^1$ represents $CR^{6a}$, $B^2$ represents a nitrogen atom, and $B^3$ represents $CR^{6c}$; or
a combination wherein $B^1$ represents $CR^{6a}$, $B^2$ represents $CR^{6b}$, and $B^3$ represents a nitrogen atom; and
$R^{6a}$, $R^{6b}$, and $R^{6c}$ are identical to or different from each other, and each represent a C1-C6 chain hydrocarbon group optionally substituted with one or more halogen atom(s), a C3-C7 cycloalkyl group optionally substituted with one or more substituent(s) selected from the group consisting of a halogen atom and a cyano group, $OR^{72}$, or a hydrogen atom.

[Aspect B67] The compound according to any one of the Aspects B1 to B64 or the Intermediate B, wherein

$B^1$ represents $CR^{6a}$;
$B^2$ represents $CR^{6b}$;
$B^3$ represents $CR^{6c}$;
$R^{6a}$ represents a hydrogen atom; and
$R^{6b}$ and $R^{6c}$ are identical to or different from each other, and each represent a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect B68] The compound according to any one of the Aspects B1 to B64 or the Intermediate B, wherein

$B^1$ represents a nitrogen atom;
$B^2$ represents $CR^{6b}$;
$B^3$ represents $CR^{6c}$;
$R^{6b}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$; and
$R^{6c}$ represents a hydrogen atom.

[Aspect B69] The compound according to any one of the Aspects B1 to B64 or the Intermediate B, wherein

$B^1$ represents a nitrogen atom;
$B^2$ represents $CR^{6b}$;
$B^3$ represents $CR^{6c}$;
$R^{6b}$ represents a hydrogen atom; and
$R^{6c}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect B70] The compound according to any one of the Aspects B1 to B64 or the Intermediate B, wherein

$B^1$ represents $CR^{6a}$;
$B^2$ represents a nitrogen atom;
$B^3$ represents $CR^{6c}$; and

$R^{6a}$ and $R^{6c}$ are identical to or different from each other, and each represent a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect B71] The compound according to any one of the Aspects B1 to B64 or the Intermediate B, wherein

$B^1$ represents $CR^{6a}$;
$B^2$ represents $CR^{6b}$;
$B^3$ represents a nitrogen atom;
$R^{6a}$ represents a hydrogen atom; and
$R^{6b}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$.

[Aspect B72] The compound according to any one of the Aspects B1 to B64 or the Intermediate B, wherein

$B^1$ represents $CR^{6a}$;
$B^2$ represents $CR^{6b}$;
$B^3$ represents a nitrogen atom; and
$R^{6a}$ represents a C1-C6 chain hydrocarbon group optionally substituted with one or more halogen atom(s), a C3-C7 cycloalkyl group optionally substituted with one or more substituent(s) selected from the group consisting of a halogen atom and a cyano group, or $OR^{72}$; and
$R^{6b}$ represents a hydrogen atom.

[Aspect B73] The compound according to any one of the Aspects B1 to B64 or the Intermediate B, wherein

the combination of $A^2$ and $A^3$ represents
a combination wherein $A^2$ represents $-CR^{7a}R^{7b}-$ and $A^3$ represents $-CR^{7c}R^{7d}-$;
a combination wherein $A^2$ represents $-CR^{7a}R^{7b}-$ and $A^3$ represents $-CR^{7e}R^{7f}-CR^{7g}R^{7h}-*^1$ (wherein $*^1$ represents the binding position to $A^2$);
a combination wherein $A^2$ represents $-NR^{5a}-$ and $A^3$ represents $-CR^{7c}R^{7d}-$; or
a combination wherein $A^2$ represents $-NR^{5a}-$ and $A^3$ represents $-CR^{7e}R^{7f}-CR^{7g}R^{7h}-*^1$ (wherein $*^1$ represents the binding position to $A^2$).

[Aspect B74] The compound according to any one of the Aspects B1 to B64 or the Intermediate B, wherein

the combination of $A^2$ and $A^3$ represents
a combination wherein $A^2$ represents $-CR^{7a}R^{7b}-$ and $A^3$ represents $-CR^{7c}R^{7d}-$; or
a combination wherein $A^2$ represents $-CR^{7a}R^{7b}-$ and $A^3$ represents $-CR^{7e}R^{7f}-CR^{7g}R^{7h}-*^1$ (wherein $*^1$ represents the binding position to $A^2$).

[Aspect B75] The compound according to any one of the Aspects B1 to B64 or the Intermediate B, wherein

the combination of $A^2$ and $A^3$ represents
a combination wherein $A^2$ represents $-CR^{7a}R^{7b}-$ and $A^3$ represents $-CR^{7c}R^{7d}-$; or
a combination wherein $A^2$ represents $-CR^{7a}R^{7b}-$ and $A^3$ represents $-CR^{7e}R^{7f}-CR^{7g}R^{7h}-*^1$ (wherein $*^1$ represents the binding position to $A^2$); and
R7a, $R^{7b}$, $R^{7c}$, $R^{7d}$, R7e, $R^{7f}$, $R^{7g}$, and $R^{7h}$ are identical to or different from each other, and each represent a C1-C6 alkyl group or a hydrogen atom.

[Aspect B76] The compound according to any one of the Aspects B1 to B64 or the Intermediate B, wherein

$A^2$ represents $-CR^{7a}R^{7b}-$;
$A^3$ represents $-CR^{7c}R^{7d}-$; and
$R^{7a}$ and $R^{7b}$, and $R^{7c}$ and $R^{7d}$ are each optionally combined with the carbon atom to which they are attached to form a C3-C4 cycloalkyl group; or
$A^2$ represents $-CR^{7a}R^{7b}-$
$A^3$ represents $-CR^{7e}R^{7f}-CR^{7g}R^{7h}-*^1$ (wherein $*^1$ represents the binding position to $A^2$); and

$R^{7a}$ and $R^{7b}$, $R^{7e}$ and $R^{7f}$, and $R^{7g}$ and $R^{7h}$ are each optionally combined with the carbon atom to which they are attached to form a C3-C4 cycloalkyl group.

[Aspect B77] The compound according to any one of the Aspects B1 to B64 or the Intermediate B, wherein

$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 chain hydrocarbon group optionally substituted with one or more halogen atom(s), or $OR^{72}$; and
$R^{72}$ represents a C1-C6 chain hydrocarbon group optionally substituted with a halogen atom.

[Aspect B78] The compound according to any one of the Aspects B1 to B64 or the Intermediate B, wherein

$A^2$ represents $-CR^{7a}R^{7b}-$;
$A^3$ represents $-CR^{7c}R^{7d}-$; and
$R^{7a}$, $R^{7b}$, $R^{7c}$, and $R^{7d}$ each represent a hydrogen atom.

[Aspect B79] The compound according to any one of the Aspects B1 to B64 or the Intermediate B, wherein

$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 chain hydrocarbon group optionally substituted with one or more halogen atom(s), or $OR^{72}$;
$R^{72}$ represents a C1-C6 chain hydrocarbon group optionally substituted with a halogen atom;
$A^2$ represents $-CR^{7a}R^{7b}-$;
$A^3$ represents $-CR^{7c}R^{7d}-$; and
$R^{7a}$, $R^{7b}$, $R^{7c}$, and $R^{7d}$ each represent a hydrogen atom.

[Aspect B80] The compound according to any one of the Aspects B1 to B64 or the Intermediate B, wherein

$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 alkyl group optionally substituted with one or more halogen atom(s), or $OR^{72}$;
$R^{72}$ represents a C1-C6 alkyl group optionally substituted with a halogen atom;
$A^2$ represents $-CR^{7a}R^{7b}-$;
$A^3$ represents $-CR^{7c}R^{7d}-$; and
$R^{7a}$, $R^{7b}$, $R^{7c}$, and $R^{7d}$ each represent a hydrogen atom.

[Aspect B81] The compound according to any one of the Aspects B1 to B64 or the Intermediate B, wherein

$B^1$ represents $CR^{6a}$;
$B^2$ represents $CR^{6b}$;
$B^3$ represents a nitrogen atom;
$R^{6a}$ represents a hydrogen atom;
$R^{6b}$ represents a C1-C6 chain hydrocarbon group optionally substituted with one or more halogen atom(s), a halogen atom, or $OR^{72}$; and
$R^{72}$ represents a C1-C6 chain hydrocarbon group optionally substituted with a halogen atom.

Next, production methods of the Present compounds are described.

Production method 1

A compound represented by formula (I-b) (hereinafter referred to as "Compound (I-b)") or a compound represented by formula (I-c) (hereinafter referred to as "Compound (I-c)") may be prepared by reacting a compound represented by formula (I-a) (hereinafter referred to as "Compound (I-a)") with an oxidizing agent.

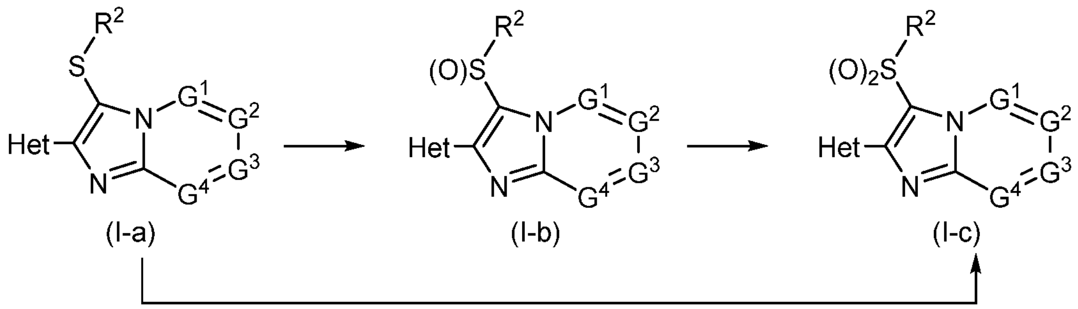

[wherein the symbols are the same as defined above.]

First, a method for producing the Compound (I-b) from the Compound (I-a) is described.

The reaction is usually carried out in a solvent. Examples of the solvent include halogenated hydrocarbons such as dichloromethane and chloroform (hereinafter collectively referred to as "halogenated hydrocarbons"); nitriles such as acetonitrile (hereinafter collectively referred to as "nitriles"); alcohol such as methanol and ethanol (hereinafter collectively referred to as "alcohols"); acetic acid; water; and mixtures of two or more of them.

Examples of the oxidizing agent include sodium periodate, m-chloroperbenzoic acid (hereinafter referred to as "mCPBA"), and hydrogen peroxide.

When hydrogen peroxide is used as the oxidizing agent, a base or a catalyst may be used as needed.

Examples of the base include sodium carbonate. When a base is used in the reaction, the base is usually used at a ratio of 0.01 to 1 mol relative to 1 mol of the Compound (I-a).

Examples of the catalyst include tungstic acid and sodium tungstate. When a catalyst is used in the reaction, the catalyst is usually used at a ratio of 0.01 to 0.5 mol relative to 1 mol of the Compound (I-a).

In the reaction, the oxidizing agent is usually used at a ratio of 1 to 1.2 mol relative to 1 mol of the Compound (I-a).

The reaction temperature is usually within the range of -20 to 80°C. The reaction time is usually within the range of 0.1 to 12 hour(s).

When the reaction is completed, to the reaction mixture is added water, the resulting mixture is subjected to extraction with organic solvent(s), and the resulting organic layer is washed with an aqueous solution of a reducing agent (for example, sodium sulfite or sodium thiosulfate) and an aqueous solution of a base (for example, sodium hydrogen carbonate) as needed. The resulting organic layer may be dried and/or concentrated to give the Compound (I-b).

Next, a method for producing the Compound (I-c) from the Compound (I-b) is described.

The reaction is usually carried out in a solvent. Examples of the solvent include halogenated hydrocarbons, nitriles, alcohols, acetic acid, water, and mixtures of two or more of them.

Examples of the oxidizing agent include mCPBA and hydrogen peroxide.

When hydrogen peroxide is used as the oxidizing agent, a base or a catalyst may be used as needed.

Examples of the base include sodium carbonate. When a base is used in the reaction, the base is usually used at a ratio of 0.01 to 1 mol relative to 1 mol of the Compound (I-b).

Examples of the catalyst include sodium tungstate. When a catalyst is used in the reaction, the catalyst is usually used at a ratio of 0.01 to 0.5 mol relative to 1 mol of the Compound (I-b).

In the reaction, the oxidizing agent is usually used at a ratio of 1 to 2 mol relative to 1 mol of the Compound (I-b).

The reaction temperature is usually within the range of -20 to 120°C. The reaction time is usually within the range of 0.1 to 12 hour(s).

When the reaction is completed, to the reaction mixture is added water, the resulting mixture is subjected to extraction with organic solvent(s), and the resulting organic layer is washed with an aqueous solution of a reducing agent (for example, sodium sulfite or sodium thiosulfate) and an aqueous solution of a base (for example, sodium hydrogen carbonate) as needed. The resulting organic layer may be dried and/or concentrated to give the Compound (I-c).

Also, the Compound (I-c) may be prepared in one step reaction (one-pot) by reacting the Compound (I-a) with an oxidizing agent.

The reaction may be carried out according to the method for producing the Compound (I-c) from the Compound (I-b) by usually using the oxidizing agent at a ratio of 2 to 5 mol relative to 1 mol of the Compound (I-a).

Production method 2

A compound represented by formula (I-S-1) (hereinafter referred to as "Compound (I-S-1)") may be prepared by reacting a compound represented by formula (I-O-1) (hereinafter referred to as "Compound (I-O-1)") with a sulfating agent.

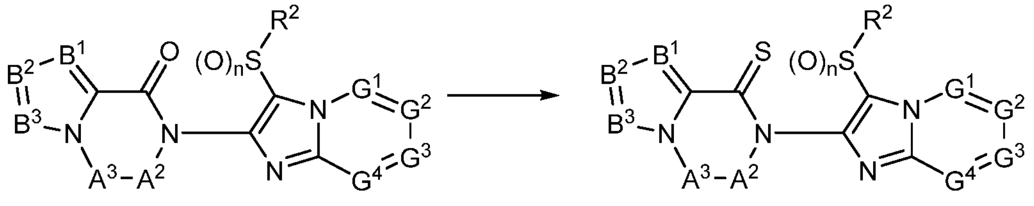

(I-O-1) (I-S-1)

[wherein the symbols are the same as defined above.]

The reaction is carried out in a solvent or in the absence of a solvent. Examples of the solvent include ethers such as tetrahydrofuran (hereinafter referred to as "THF"), methyl tert-butyl ether (hereinafter referred to as "MTBE"), ethylene glycol dimethyl ether, 1,4-dioxane, and cyclopentyl methyl ether (hereinafter collectively referred to as "ethers"); halogenated hydrocarbons; aromatic hydrocarbons such as toluene and xylene (hereinafter collectively referred to as "aromatic hydrocarbons"); nitriles; nitrogen-containing aromatic compounds such as pyridine, picoline, lutidine, and quinoline (hereinafter collectively referred to as "nitrogen-containing aromatic compounds"); and mixtures of two or more of them.

Examples of the sulfating agent include phosphorus pentasulfide and Lawesson's reagent (2,4-bis(4-methoxyphenyl)-1,3-dithia-2,4-diphosphetane-2,4-disulfide).

In the reaction, the sulfating agent is usually used at a ratio of 1 to 3 mol relative to 1 mol of the Compound (I-O-1).

The reaction temperature is usually within the range of 0 to 200°C. The reaction time is usually within the range of 1 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-S-1).

Production method 3

A compound represented by formula (I-S-2) (hereinafter referred to as "Compound (I-S-2)") may be prepared by reacting a compound represented by formula (I-O-2) (hereinafter referred to as "Compound (I-O-2)") with a sulfating agent.

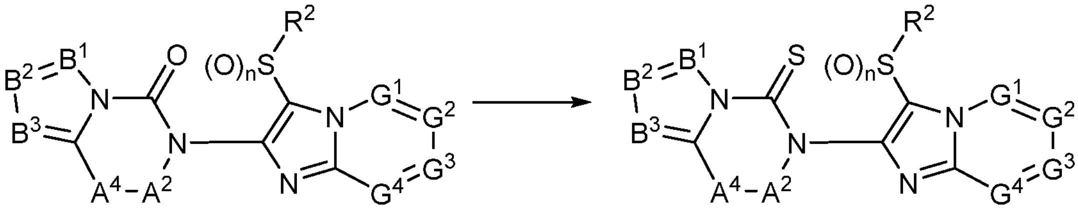

(I-O-2) (I-S-2)

[wherein the symbols are the same as defined above.]

The Compound (I-S-2) may be prepared according to the Production method 2 by using the Compound (I-O-2) instead of the Compound (I-O-1).

Production method 4

The Compound (I-O-1) may be prepared by reacting a compound represented by formula (M-1) (hereinafter referred to as "Compound (M-1)") with a compound represented by formula (M-2) (hereinafter referred to as "Compound (M-2)") in the presence of a base.

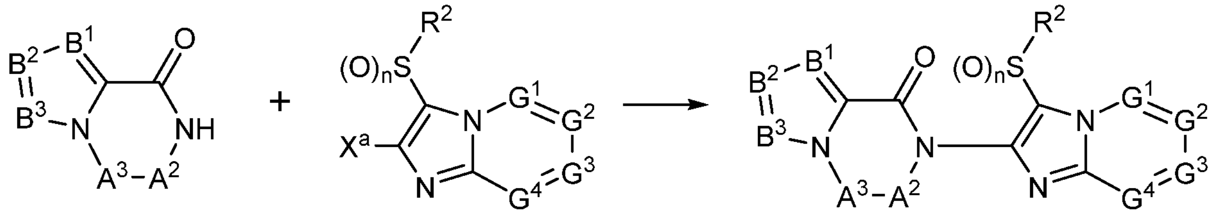

(M-1) (M-2) (I-O-1)

[wherein $X^a$ represents a leaving group such as a fluorine atom, a chlorine atom, a bromine atom, and an iodine atom; and the other symbols are the same as defined above.]

The reaction is usually carried out in a solvent. Examples of the solvent include ethers; aromatic hydrocarbons; nitriles; aprotic polar solvents such as N,N-dimethylformamide (hereinafter referred to as "DMF"), N-methylpyrrolidone (hereinafter referred to as "NMP"), and dimethyl sulfoxide (hereinafter referred to as "DMSO") (hereinafter collectively referred to as "aprotic polar solvents"); and mixtures of two or more of them.

Examples of the base include organic bases such as triethylamine, N,N-diisopropylethylamine, pyridine, and 4-(dimethylamino)pyridine (hereinafter collectively referred to as "organic bases"); alkali metal carbonates such as sodium carbonate, potassium carbonate, and cesium carbonate (hereinafter collectively referred to as "alkali metal carbonates"); and alkali metal hydrides such as sodium hydride (hereinafter collectively referred to as "alkali metal hydrides").

The reaction may also be carried out in the presence of a metal catalyst as needed. Examples of the metal catalyst include copper catalysts such as copper(I) iodide, copper(I) bromide, copper(I) chloride, copper(I) oxide, copper(I) trifluoromethanesulfonate benzene complex, tetrakis(acetonitrile)copper(I) hexafluorophosphate, and copper(I) thiophene-2-carboxylate; nickel catalysts such as bis(cyclooctadiene)nickel(0) and nickel(II) chloride; and palladium catalysts such as palladium(II) acetate, tetrakis(triphenylphosphine)palladium(0), and tris(dibenzylideneaceton)dipalladium(0). When a metal catalyst is used in the reaction, the metal catalyst is usually used at a ratio of 0.01 to 0.5 mol relative to 1 mol of the Compound (M-1).

The reaction may also be carried out in the presence of a ligand as needed. Examples of the ligand include triphenylphosphine, 4,5-bis(diphenylphosphino)-9,9-dimethylxanthene (hereinafter referred to as "Xantphos"), 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, and N,N'-dimethylethylenediamine. When a ligand is used in the reaction, the ligand is usually used at a ratio of 0.01 to 0.5 mol relative to 1 mol of the Compound (M-1).

In the reaction, the Compound (M-2) is usually used at a ratio of 0.8 to 1.2 mol, and the base is usually used at a ratio of 1 to 3 mol, relative to 1 mol of the Compound (M-1).

The reaction temperature is usually within the range of -20 to 150°C. The reaction time is usually within the range of 0.5 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-O-1).

The Compound (M-1) is a commercially available compound or may be prepared according to the method(s) described in, for example, WO 2013/192346 pamphlet.

Production method 5

The Compound (I-O-2) may be prepared by reacting a compound represented by formula (M-3) (hereinafter referred to as "Compound (M-3)") with the Compound (M-2) in the presence of a base.

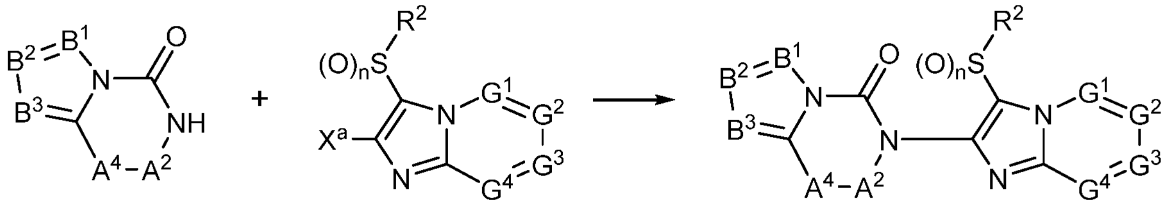

(M-3) (M-2) (I-O-2)

[wherein the symbols are the same as defined above.]

The reaction may be carried out according to the Production method 4 by using the Compound (M-3) instead of the Compound (M-1).

The Compound (M-3) is a commercially available compound or may be prepared by using known method(s).

Production method 6

A compound represented by formula (I-n0-1) (hereinafter referred to as "Compound (I-n0-1)") may be prepared according to the following scheme.

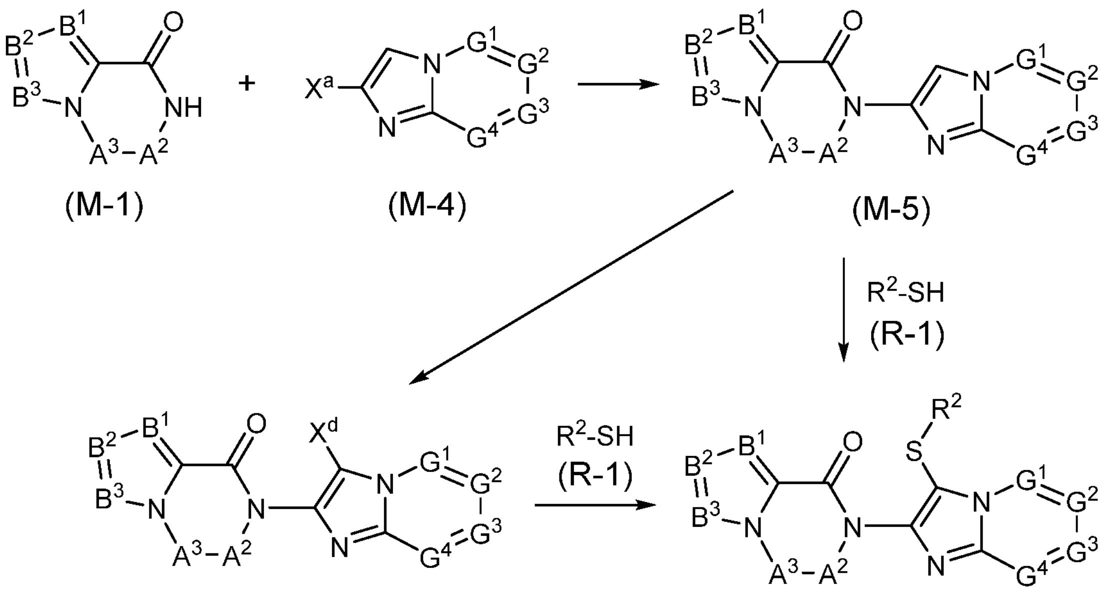


(M-1) (M-4) (M-5)

(M-6) (I-n0-1)

[wherein $X^d$ represents a chlorine atom, a bromine atom, or an iodine atom; and the other symbols are the same as defined above.]

First, a method for producing a compound represented by formula (M-5) (hereinafter referred to as "Compound (M-5)") is described.

The Compound (M-5) may be prepared according to the Production method 4 by using a compound represented by formula (M-4) (hereinafter referred to as "Compound (M-4)") instead of the Compound (M-2).

Next, a method for producing a compound represented by formula (M-6) (hereinafter referred to as "Compound (M-6)") is described.

The Compound (M-6) may be prepared by reacting the Compound (M-5) with a halogenating agent.

The reaction is usually carried out in a solvent. Examples of the solvent include alcohols, nitriles, ethers, aromatic hydrocarbons, aprotic polar solvents, halogenated hydrocarbons, water, and mixtures of two or more of them.

Examples of the halogenating agent include chlorine, bromine, iodine, N-chlorosuccinimide, N-bromosuccinimide, and N-iodosuccinimide.

In the reaction, the halogenating agent is usually used at a ratio of 1 to 20 mol relative to 1 mol of the Compound (M-5).

The reaction temperature is usually within the range of -20 to 200°C. The reaction time is usually within the range of 0.1 to 72 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (M-6).

Next, a method for producing the Compound (I-n0-1) from the Compound (M-5) is described.

The Compound (I-n0-1) may be prepared by reacting the Compound (M-5), a compound represented by formula (R-1) (hereinafter referred to as "Compound (R-1)"), and a halogenating agent.

The reaction is usually carried out in a solvent. Examples of the solvent include alcohols, nitriles, ethers, aromatic hydrocarbons, aprotic polar solvents, halogenated hydrocarbons, water, and mixtures of two or more of them.

Examples of the halogenating agent include chlorine, bromine, iodine, N-chlorosuccinimide, N-bromosuccinimide, and N-iodosuccinimide.

In the reaction, the Compound (R-1) is usually used at a ratio of 1 to 20 mol, and the halogenating agent is usually used at a ratio of 1 to 20 mol, relative to 1 mol of the Compound (M-5).

The reaction temperature is usually within the range of -20 to 200°C. The reaction time is usually within the range of 0.1 to 72 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-n0-1).

The Compound (R-1) is a commercially available compound or may be prepared by using known method(s).

Next, a method for producing the Compound (I-n0-1) from the Compound (M-6) is described.

The Compound (I-n0-1) may also be prepared by reacting the Compound (M-6) with the Compound (R-1) in the presence of a metal catalyst and a base.

The reaction is usually carried out in a solvent. Examples of the solvent include alcohols, nitriles, ethers, aromatic hydrocarbons, aprotic polar solvents, water, and mixtures of two or more of them.

Examples of the metal catalyst include palladium catalysts such as tetrakis(triphenylphosphine)palladium(0), [1,1'-bis(diphenylphosphino)ferrocene]palladium(II) dichloride, tris(dibenzylideneaceton)dipalladium(0), and palladium(II) acetate; nickel catalysts such as bis(cyclooctadiene)nickel(0) and nickel(II) chloride; and copper catalysts such as copper(I) iodide and copper(I) chloride.

Examples of the base include alkali metal hydrides, alkali metal carbonates, and organic bases.

In the reaction, a ligand may also be used. Examples of the ligand include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, and 1,10-phenanthroline. When a ligand is used in the reaction, the ligand is usually used at a ratio of 0.01 to 1 mol relative to 1 mol of the Compound (M-6).

In the reaction, the Compound (R-1) is usually used at a ratio of 1 to 20 mol, the metal catalyst is usually used at a ratio of 0.01 to 0.5 mol, and the base is usually used at a ratio of 0.1 to 5 mol, relative to 1 mol of the Compound (M-6).

The reaction temperature is usually within the range of -20 to 200°C. The reaction time is usually within the range of 0.1 to 72 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-n0-1).

Production method 7

A compound represented by formula (I-n0-2) (hereinafter referred to as "Compound (I-n0-2)") may be prepared according to the following scheme.

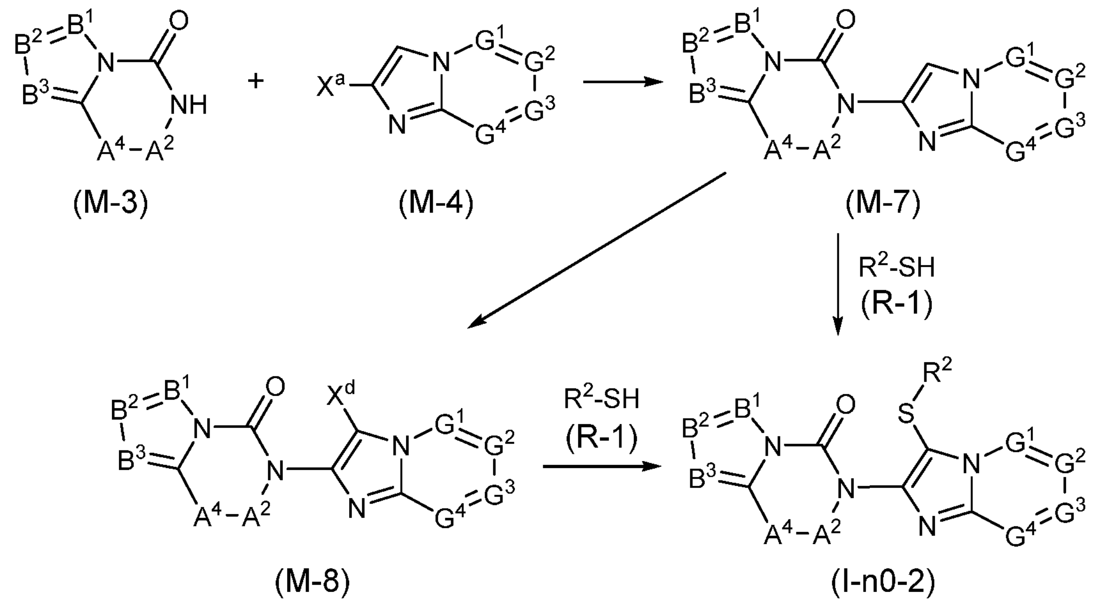


(M-3) (M-4) (M-7) (M-8) (I-n0-2)

[wherein the symbols are the same as defined above.]

A compound represented by formula (M-7) (hereinafter referred to as "Compound (M-7)") may be prepared according to the Production method 6 by using the Compound (M-3) instead of the Compound (M-1).

A compound represented by formula (M-8) (hereinafter referred to as "Compound (M-8)") may be prepared according to the method for producing the Compound (M-6) from the Compound (M-5) in the Production method 6 by using the Compound (M-7) instead of the Compound (M-5).

The Compound (I-n0-2) may be prepared according to the method for producing the Compound (I-n0-1) from the Compound (M-5) in the Production method 6 by using the Compound (M-7) instead of the Compound (M-5).

The Compound (I-n0-2) may also be prepared according to the method for producing the Compound (I-n0-1) from the Compound (M-6) in the Production method 6 by using the Compound (M-8) instead of the Compound (M-6).

Production method 8

The Compound (I-O-1) may also be prepared by reacting a compound represented by formula (M-9-X) (hereinafter referred to as "Compound (M-9-X)") with a base.

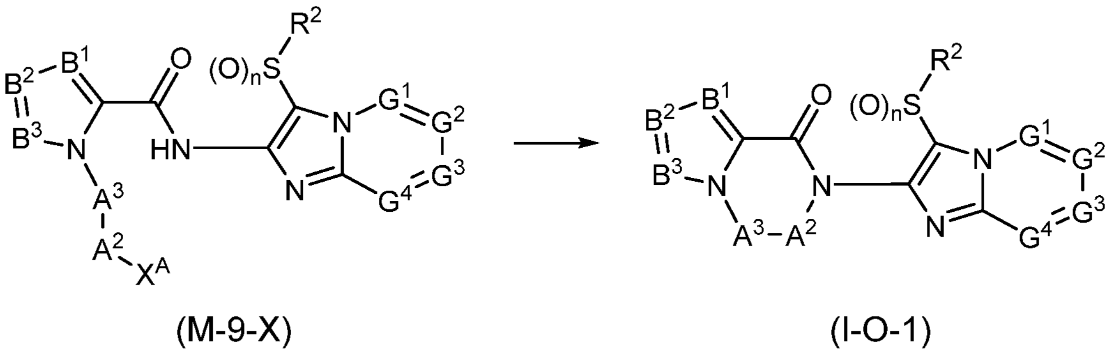

(M-9-X) (I-O-1)

[wherein $X^A$ represents a chlorine atom, a bromine atom, or an iodine atom; and the other symbols are the same as defined above.]

The reaction is usually carried out in a solvent. Examples of the solvent include ethers; aromatic hydrocarbons; nitriles; aprotic polar solvents; and mixtures of two or more of them.

Examples of the base include organic bases; alkali metal carbonates; and alkali metal hydrides.

The reaction may also be carried out in the presence of a metal catalyst as needed. Examples of the metal catalyst include copper catalysts such as copper(I) iodide, copper(I) bromide, copper(I) chloride, copper(I) oxide, copper(I) trifluoromethanesulfonate benzene complex, tetrakis(acetonitrile)copper(I) hexafluorophosphate, and copper(I) thiophene-2-carboxylate; nickel catalysts such as bis(cyclooctadiene)nickel(0) and nickel(II) chloride; and palladium catalysts

such as palladium(II) acetate, tetrakis(triphenylphosphine)palladium(0), and tris(dibenzylideneaceton)dipalladium(0). When a metal catalyst is used in the reaction, the metal catalyst is usually used at a ratio of 0.01 to 0.5 mol relative to 1 mol of the Compound (M-9-X).

The reaction may also be carried out in the presence of a ligand as needed. Examples of the ligand include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, and N,N'-dimethylethylenediamine. When a ligand is used in the reaction, the ligand is usually used at a ratio of 0.01 to 0.5 mol relative to 1 mol of the Compound (M-9-X).

In the reaction, the base is usually used at a ratio of 1 to 5 mol relative to 1 mol of the Compound (M-9-X).

The reaction temperature is usually within the range of -20 to 150°C. The reaction time is usually within the range of 0.5 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-O-1).

Production method 9

The Compound (I-O-1) may also be prepared by subjecting a compound represented by formula (M-9-OH) (hereinafter referred to as "Compound (M-9-OH)") to intramolecular condensation in the presence of a condensing agent.

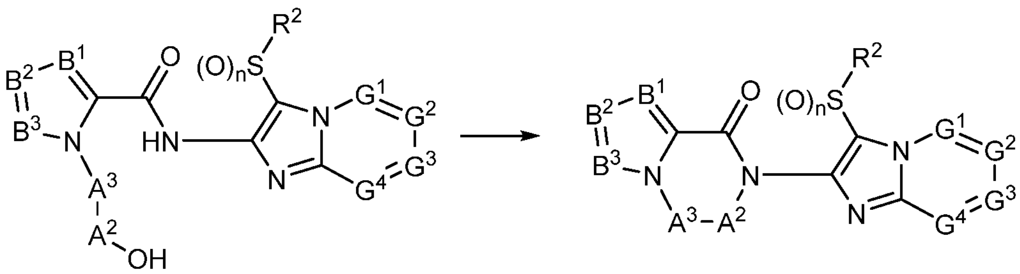

(M-9-OH) (I-O-1)

[wherein the symbols are the same as defined above.]

The reaction is usually carried out in a solvent. Examples of the solvent include ethers; halogenated hydrocarbons; aromatic hydrocarbons; esters such as ethyl acetate and butyl acetate (hereinafter collectively referred to as "esters"); nitriles; aprotic polar solvents; nitrogen-containing aromatic compounds; and mixtures of two or more of them.

Examples of the condensing agent to be used in the reaction include a mixture of triphenylphosphine and an azodiester compound. Examples of the azodiester compound include diethyl azodicarboxylate.

In the reaction, the condensing agent is usually used at a ratio of 1 to 5 mol relative to 1 mol of the Compound (M-9-OH).

The reaction temperature is usually within the range of -20 to 200°C. The reaction time is usually within the range of 0.1 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-O-1).

Production method 10

The Compound (I-O-1)") may also be prepared by reacting a compound represented by formula (M-10-X) (hereinafter referred to as "Compound (M-10-X)") with a base.

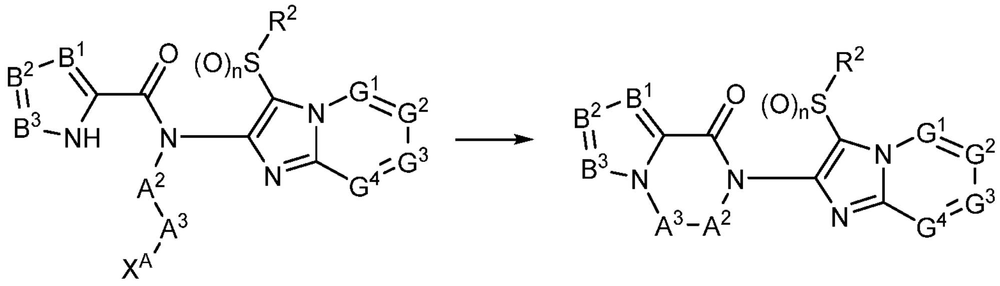

(M-10-X) (I-O-1)

[wherein the symbols are the same as defined above.]

The Compound (I-O-1) may also be prepared according to the Production method 8 by using the Compound (M-10-X) instead of the Compound (M-9-X).

Production method 11

The Compound (I-O-1) may also be prepared by subjecting a compound represented by formula (M-10-OH) (hereinafter referred to as "Compound (M-10-OH)") to intramolecular condensation in the presence of a condensing agent.

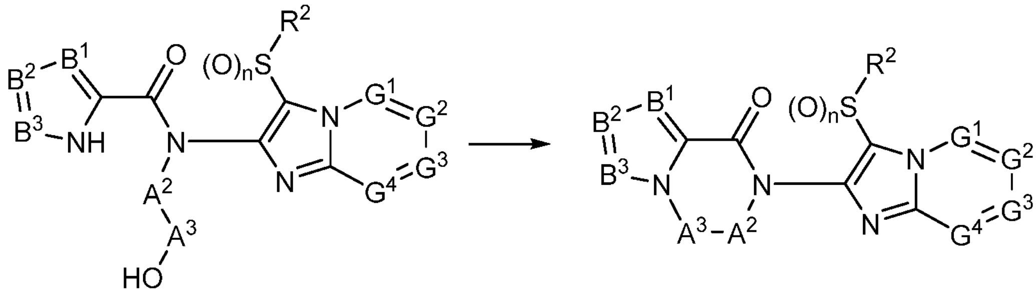

(M-10-OH) (I-O-1)

[wherein the symbols are the same as defined above.]

The Compound (I-O-1) may also be prepared according to the Production method 9 by using the Compound (M-10-OH) instead of the Compound (M-9-OH).

Production method 12

A compound represented by formula (I-b-p) (hereinafter referred to as "Compound (I-b-p)") or a compound represented by formula (I-c-p) (hereinafter referred to as "Compound (I-c-p)") may be prepared by reacting a compound represented by formula (I-a-p) (hereinafter referred to as "Compound (I-a-p)") with an oxidizing agent.

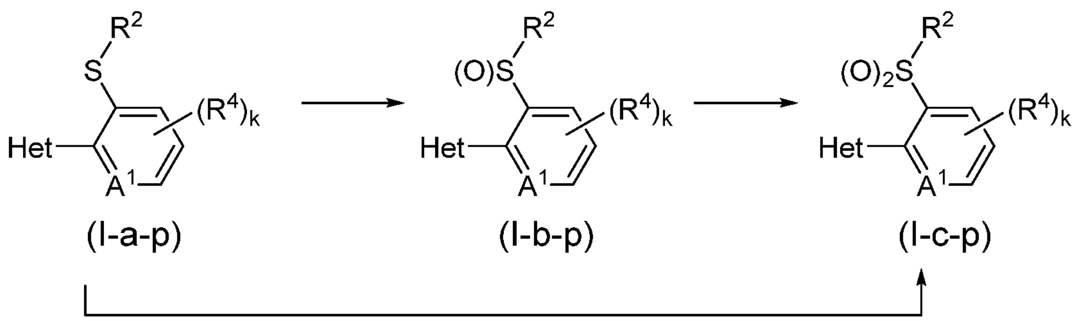

[wherein the symbols are the same as defined above.]

The Compound (I-b-p) may be prepared according to the Production method 1 by using the Compound (I-a-p) instead of the Compound (I-a).

The Compound (I-c-p) may be prepared according to the Production method 1 by using the Compound (I-b-p)

instead of the Compound (I-b).

The Compound (I-c-p) may also be prepared according to the Production method 1 by using the Compound (I-a-p) instead of the Compound (I-a).

Production method 13

A compound represented by formula (I-S-1p) (hereinafter referred to as "Compound (I-S-1p)") may be prepared by reacting a compound represented by formula (I-O-1p) (hereinafter referred to as "Compound (I-O-1p)") with a sulfating agent.

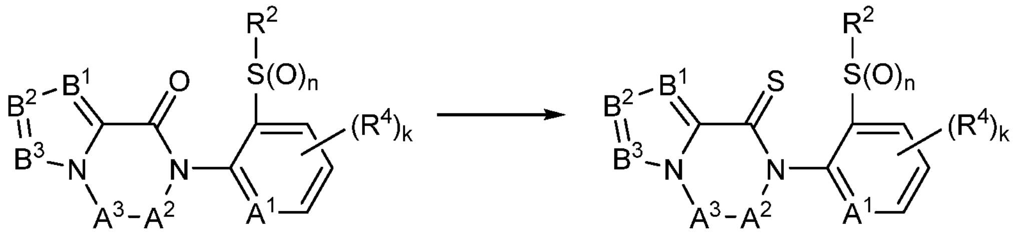

(I-O-1p) (I-S-1p)

[wherein the symbols are the same as defined above.]

The Compound (I-S-1p) may be prepared according to the Production method 2 by using the Compound (I-O-1p) instead of the Compound (I-O-1).

Production method 14

A compound represented by formula (I-S-2p) (hereinafter referred to as "Compound (I-S-2p)") may be prepared by reacting a compound represented by formula (I-O-2p) (hereinafter referred to as "Compound (I-O-2p)") with a sulfating agent.

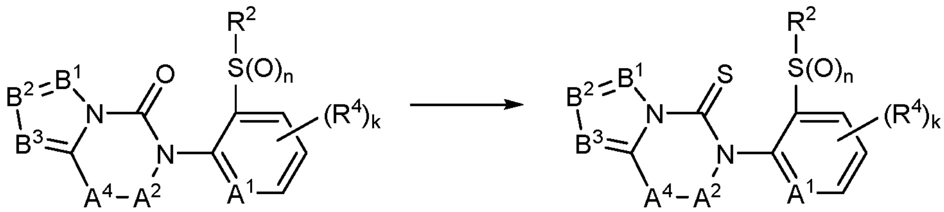

(I-O-2p) (I-S-2p)

[wherein the symbols are the same as defined above.]

The Compound (I-S-2p) may be prepared according to the Production method 2 by using the Compound (I-O-2p) instead of the Compound (I-O-1).

Production method 15

The Compound (I-O-1p) may be prepared by reacting the Compound (M-1) with a compound represented by formula (M-11) (hereinafter referred to as "Compound (M-11)") in the presence of a base.

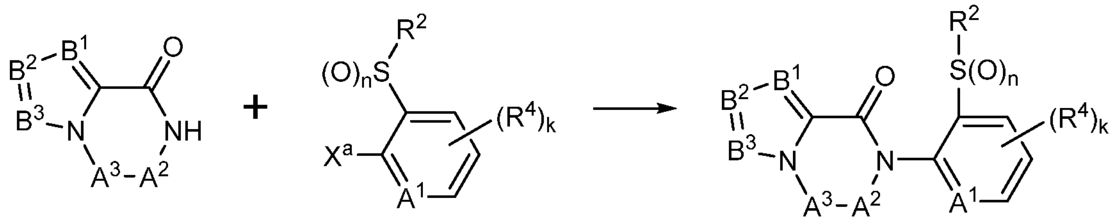

(M-1) (M-11) (I-O-1p)

[wherein the symbols are the same as defined above.]

The Compound (I-O-1p) may be prepared according to the Production method 4 by using the Compound (M-11) instead of the Compound (M-2).

Production method 16

The Compound (I-O-2p) may be prepared by reacting the Compound (M-3) with the Compound (M-11) in the presence of a base.

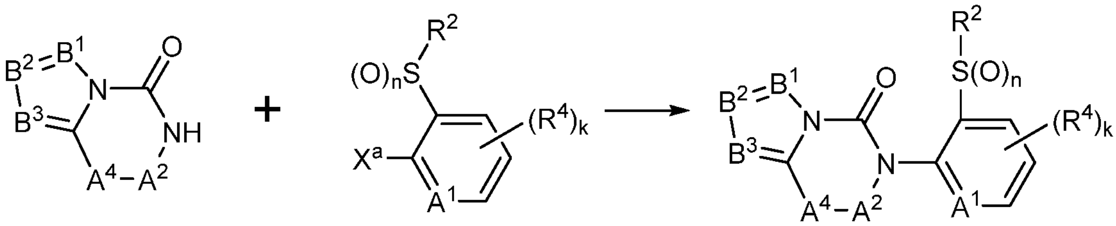

(M-3) (M-11) (I-O-2p)

[wherein the symbols are the same as defined above.]

The Compound (I-O-2p) may be prepared according to the Production method 5 by using the Compound (M-11) instead of the Compound (M-2).

Production method 17

A compound represented by formula (I-n0-1p) (hereinafter referred to as "Compound (I-n0-1p)") may be prepared according to the following scheme.

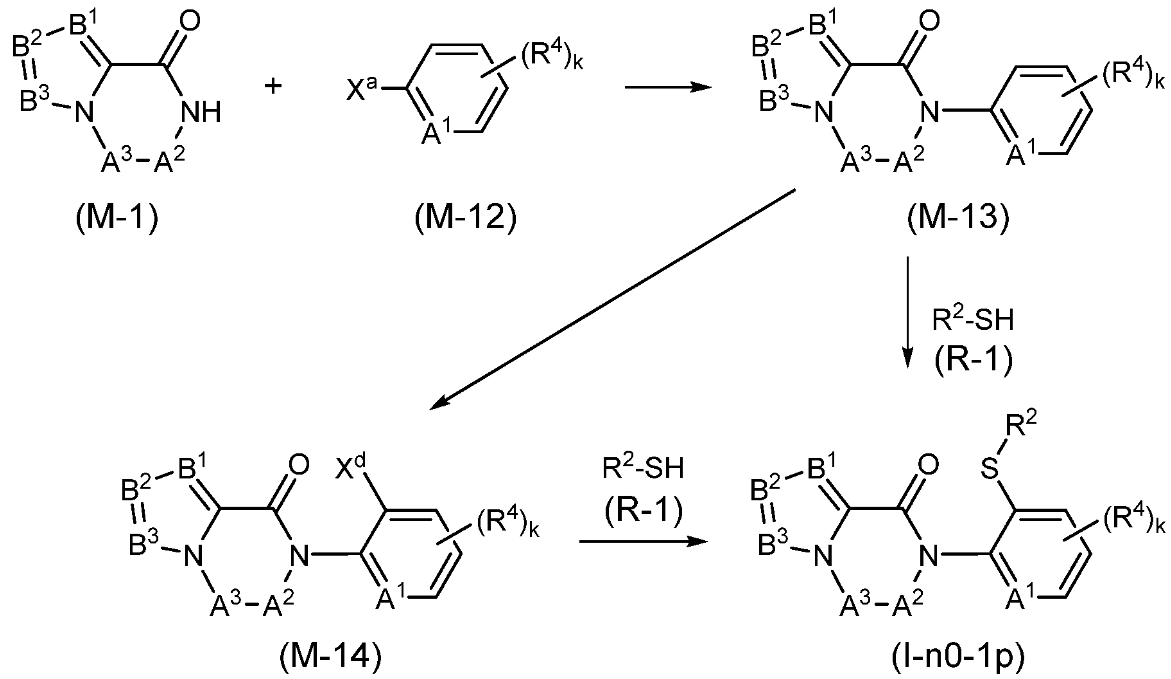


[wherein the symbols are the same as defined above.]

A compound represented by formula (M-13) (hereinafter referred to as "Compound (M-13)") may be prepared according to the Production method 6 by using the Compound (M-12) instead of the Compound (M-4).

The Compound (M-12) is a commercially available compound or may be prepared by using known method(s).

A compound represented by formula (M-14) (hereinafter referred to as "Compound (M-14)") may be prepared according to the method for producing the Compound (M-6) from the Compound (M-5) in the Production method 6 by using the Compound (M-13) instead of the Compound (M-5).

The Compound (I-n0-1p) may also be prepared according to the method for producing the Compound (I-n0-1) from the Compound (M-5) in the Production method 6 by using the Compound (M-13) instead of the Compound (M-5).

The Compound (I-n0-1p) may also be prepared according to the method for producing the Compound (I-n0-1) from the Compound (M-6) in the Production method 6 by using the Compound (M-14) instead of the Compound (M-6).

Production method 18

A compound represented by formula (I-n0-2p) (hereinafter referred to as "Compound (I-n0-2p)") may be prepared according to the following scheme.

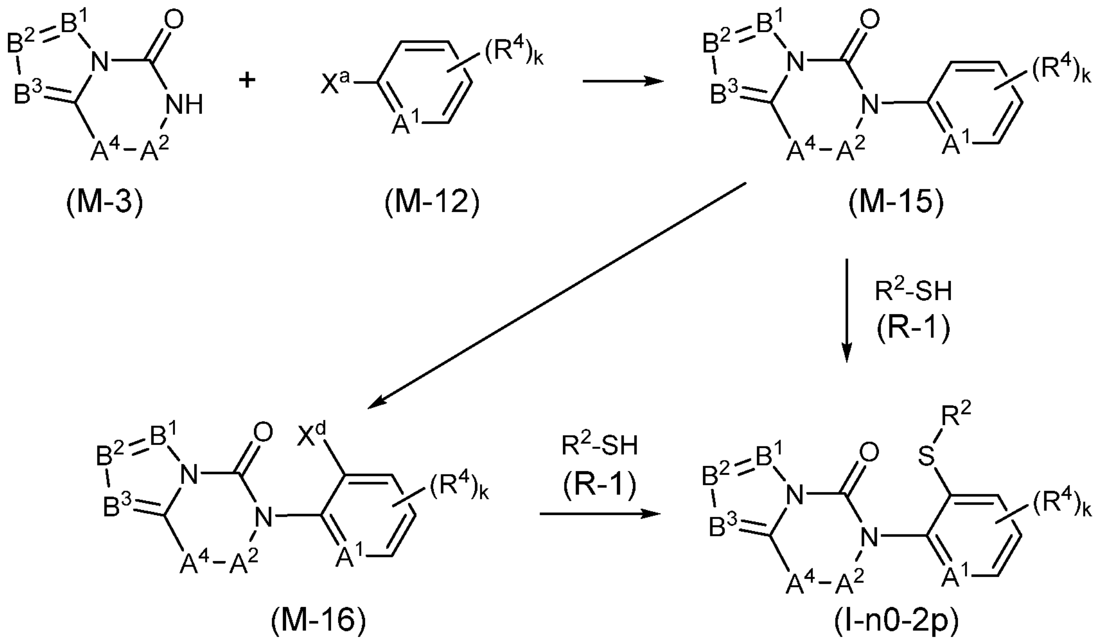


(M-3) (M-12) (M-15) (M-16) (I-n0-2p)

[wherein the symbols are the same as defined above.]

A compound represented by formula (M-15) (hereinafter referred to as "Compound (M-15)") may be prepared according to the Production method 7 by using the Compound (M-12) instead of the Compound (M-4).

A compound represented by formula (M-16) (hereinafter referred to as "Compound (M-16)") may be prepared according to the method for producing the Compound (M-8) from the Compound (M-7) in the Production method 7 by using the Compound (M-15) instead of the Compound (M-7).

The Compound (I-n0-2p) may also be prepared according to the method for producing the Compound (I-n0-2) from the Compound (M-7) in the Production method 7 by using the Compound (M-15) instead of the Compound (M-7).

The Compound (I-n0-2p) may also be prepared according to the method for producing the Compound (I-n0-2) from the Compound (M-8) in the Production method 7 by using the Compound (M-16) instead of the Compound (M-8).

Production method 19

The Compound (I-O-1p) may also be prepared by reacting a compound represented by formula (M-17-X) (hereinafter referred to as "Compound (M-17-X)") with a base.

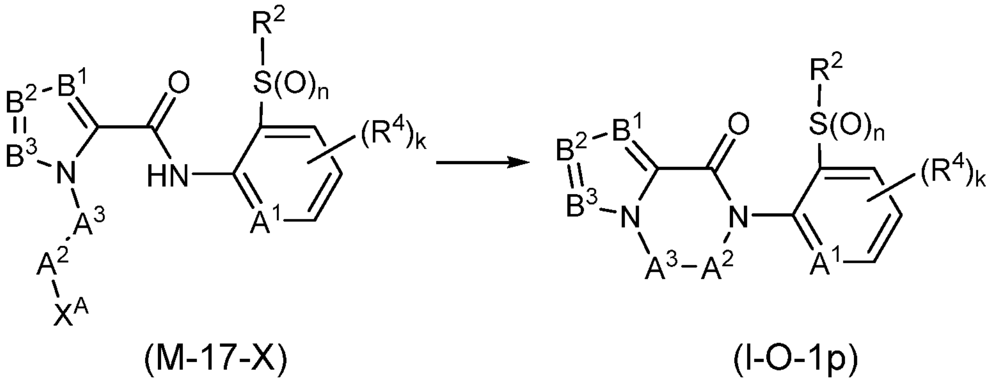

(M-17-X) (I-O-1p)

[wherein the symbols are the same as defined above.]

The Compound (I-O-1p) may be prepared according to the Production method 8 by using the Compound (M-17-X) instead of the Compound (M-9-X).

Production method 20

The Compound (I-O-1p) may also be prepared by subjecting a compound represented by formula (M-17-OH)

(hereinafter referred to as "Compound (M-17-OH)") to intramolecular condensation in the presence of a condensing agent.

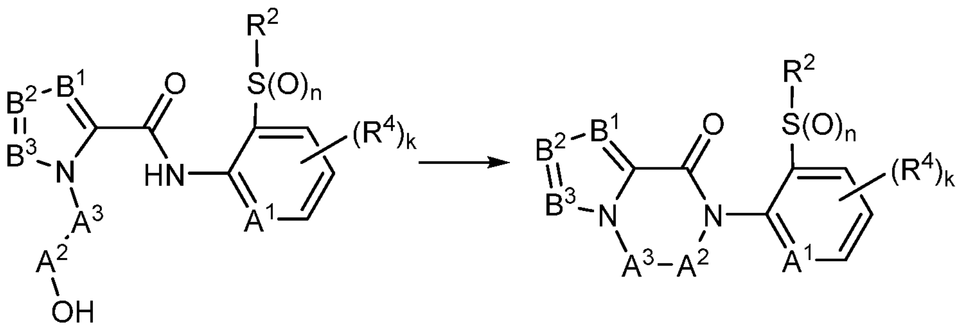

(M-17-OH) (I-O-1p)

[wherein the symbols are the same as defined above.]

The Compound (I-O-1p) may be prepared according to the Production method 9 by using the Compound (M-17-OH) instead of the Compound (M-9-OH).

Production method 21

The Compound (I-O-1p) may also be prepared by reacting a compound represented by formula (M-18-X) (hereinafter referred to as "Compound (M-18-X)") with a base.

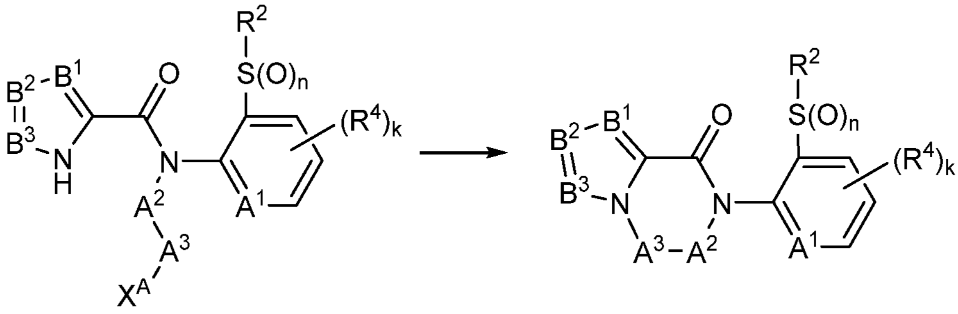

(M-18-X) (I-O-1p)

[wherein the symbols are the same as defined above.]

The Compound (I-O-1p) may be prepared according to the Production method 10 by using the Compound (M-18-X) instead of the Compound (M-10-X).

Production method 22

The Compound (I-O-1p) may also be prepared by subjecting a compound represented by formula (M-18-OH) (hereinafter referred to as "Compound (M-18-OH)") to intramolecular condensation in the presence of a condensing agent.

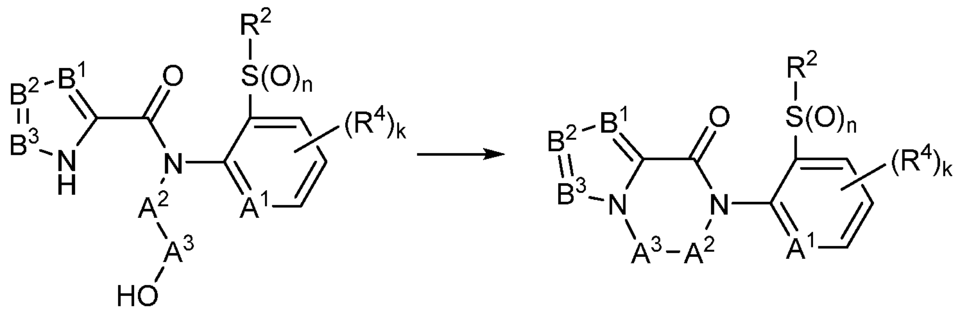

(M-18-OH) (I-O-1p)

[wherein the symbols are the same as defined above.]

The Compound (I-O-1p) may also be prepared according to the Production method 11 by using the Compound (M-18-OH) instead of the Compound (M-10-OH).

Production method 23

An N-oxide of the compound represented by formula (I) may be prepared by reacting the compound represented by formula (I) with an oxidizing agent. The reaction may be carried out according to the method(s) described in, for example, the Production method 1, US Patent Application Publication No. 2018/0009778, or WO 2016/121970 pamphlet.

Production method 24

A compound represented by formula (I-O-1ph) (hereinafter referred to as "Compound (I-O-1ph)") may be prepared by reacting a compound represented by formula (I-O-1pp) (hereinafter referred to as "Compound (I-O-1pp)") with a compound represented by formula (Ph-BOH) (hereinafter referred to as "Compound (Ph-BOH)") or a compound represented by formula (Ph-Bpin) (hereinafter referred to as "Compound (Ph-Bpin)") in the presence of a metal catalyst and a base.

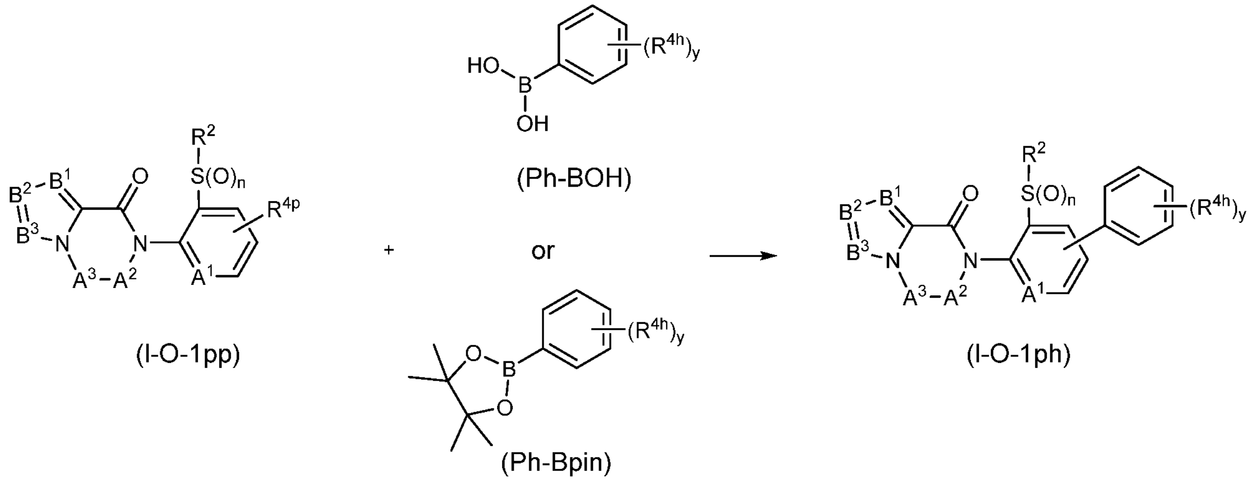

(I-O-1pp) + (Ph-BOH) or (Ph-Bpin) (I-O-1ph)

[wherein $R^{4p}$ represents a chlorine atom, a bromine atom, or an iodine atom; $R^{4h}$ represents a substituent selected from Group $H^2$; y represents 0, 1, 2, 3, 4, or 5; and the other symbols are the same as defined above.]

The reaction is usually carried out in a solvent. Examples of the solvent to be used in the reaction include ethers, aromatic hydrocarbons, nitriles, aprotic polar solvents, water, and mixtures of two or more of them.

Examples of the base to be used in the reaction include organic bases; alkali metal carbonates; alkali metal hydrides; and alkali metal phosphates such as potassium phosphate (hereinafter collectively referred to as "alkali metal phosphates").

Examples of the metal catalyst to be used in the reaction include nickel catalysts such as bis(cyclooctadiene) nickel(0) and nickel(II) chloride; and palladium catalysts such as palladium(II) acetate, tetrakis(triphenylphosphine) palladium(0), tris(dibenzylideneaceton)dipalladium(0), and [1,1'-bis(diphenylphosphino)ferrocene]dichloropalladium(II).

The reaction may also be carried out in the presence of a ligand as needed. Examples of the ligand include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, and N,N'-dimethylethylenediamine. When a ligand is used in the reaction, the ligand is usually used at a ratio of 0.01 to 0.5 mol relative to 1 mol of the Compound (I-O-1pp).

In the reaction, the Compound (Ph-BOH) or the Compound (Ph-Bpin) is usually used at a ratio of 1 to 5 mol, the metal catalyst is usually used at a ratio of 0.01 to 0.5 mol, and the base is usually used at a ratio of 1 to 5 mol, relative to 1 mol of the Compound (I-O-1pp).

The reaction temperature is usually within the range of -20 to 150°C. The reaction time is usually within the range of 0.5 to 24 hour(s).

When the reaction is completed, the reaction mixture may be subjected to a work-up such as adding water to the reaction mixture, subjecting the resulting mixture to extraction with organic solvent(s), and drying and/or concentrating the

resulting organic layer to give the Compound (I-O-1ph).

Production method 25

A compound represented by formula (I-O-2ph) (hereinafter referred to as "Compound (I-O-2ph)") may be prepared by reacting a compound represented by formula (I-O-2pp) (hereinafter referred to as "Compound (I-O-2pp)") with the Compound (Ph-BOH) or the Compound (Ph-Bpin) in the presence of a metal catalyst and a base.

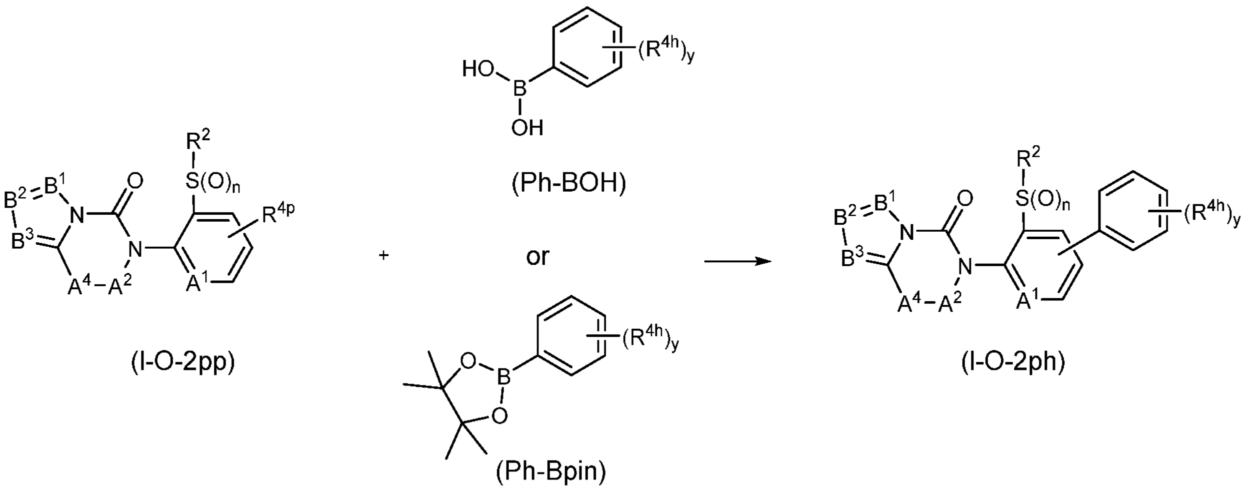

(I-O-2pp) + (Ph-BOH) or (Ph-Bpin) → (I-O-2ph)

[wherein the symbols are the same as defined above.]

The Compound (I-O-2ph) may be prepared according to the Production method 24 by using the Compound (I-O-2pp) instead of the Compound (I-O-1pp).

Production method 26

A compound represented by formula (I-O-1Ar) (hereinafter referred to as "Compound (I-O-1Ar)") may be prepared according to the following scheme.

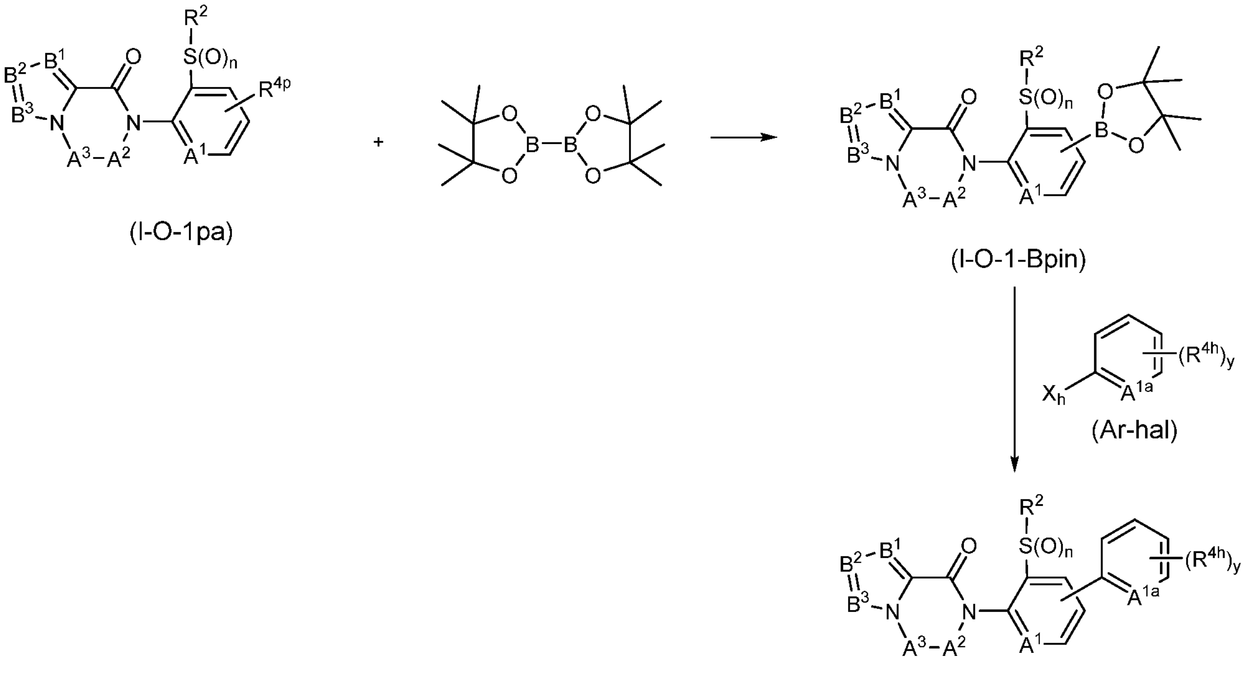

(I-O-1pa)

(I-O-1-Bpin)

(Ar-hal)

(I-O-1Ar)

[wherein $A^{1a}$ represents $CR^{1a}$ or a nitrogen atom; $R^{1a}$ represents a hydrogen atom or a substituent selected from Group $H^2$ ; $X_h$ represents a halogen atom; and the other symbols are the same as defined above.]

First, a method for producing a compound represented by formula (I-O-1-Bpin) (hereinafter referred to as "Compound (I-O-1-Bpin)") from a compound represented by formula (I-O-1pa) (hereinafter referred to as "Compound (I-O-1pa)") is described.

The Compound (I-O-1-Bpin) may be prepared by reacting the Compound (I-O-1pa) with Bis(pinacolato)diboron in the presence of a metal catalyst and a base.

The reaction is usually carried out in a solvent. Examples of the solvent to be used in the reaction include ethers, aromatic hydrocarbons, nitriles, aprotic polar solvents, and mixtures of two or more of them.

Examples of the base to be used in the reaction include organic bases, alkali metal carbonates, alkali metal hydrides, and alkali metal phosphates.

Examples of the metal catalyst to be used in the reaction include palladium catalysts such as [1,1'-bis(diphenylphosphino)ferrocene]dichloropalladium(II) .

In the reaction, Bis(pinacolato)diboron is usually used at a ratio of 1 to 3 mol, the metal catalyst is usually used at a ratio of 0.01 to 0.5 mol, and the base is usually used at a ratio of 1 to 5 mol, relative to 1 mol of the Compound (I-O-1pa).

The reaction temperature is usually within the range of -20 to 150°C. The reaction time is usually within the range of 0.5 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-O-1-Bpin).

Next, a method for producing the Compound (I-O-1Ar) from the Compound (I-O-1-Bpin) is described.

The Compound (I-O-1Ar) may be prepared by reacting the Compound (I-O-1-Bpin) with a compound represented by formula (Ar-hal) (hereinafter referred to as "Compound (Ar-hal)") in the presence of a metal catalyst and a base.

The reaction is usually carried out in a solvent. Examples of the solvent to be used in the reaction include ethers, aromatic hydrocarbons, nitriles, aprotic polar solvents, water, and mixtures of two or more of them.

Examples of the base to be used in the reaction include organic bases, alkali metal carbonates, alkali metal hydrides, and alkali metal phosphates.

Examples of the metal catalyst to be used in the reaction include palladium catalysts such as palladium(II) acetate, tetrakis(triphenylphosphine)palladium(0), tris(dibenzylideneaceton)dipalladium(0), and [1,1'-bis(diphenylphosphino)ferrocene]dichloropalladium(II) .

The reaction may also be carried out in the presence of a ligand as needed. Examples of the ligand include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, and N,N'-dimethylethylenediamine. When a ligand is used in the reaction, the ligand is usually used at a ratio of 0.01 to 0.5 mol relative to 1 mol of the Compound (I-O-1-Bpin).

In the reaction, the Compound (Ar-hal) is usually used at a ratio of 1 to 5 mol, the metal catalyst is usually used at a ratio of 0.01 to 0.5 mol, and the base is usually used at a ratio of 1 to 5 mol, relative to 1 mol of the Compound (I-O-1-Bpin).

The reaction temperature is usually within the range of -20 to 150°C. The reaction time is usually within the range of 0.5 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-O-1Ar).

Production method 27

A compound represented by formula (I-O-2Ar) (hereinafter referred to as "Compound (I-O-2Ar)") may be prepared according to the following scheme.

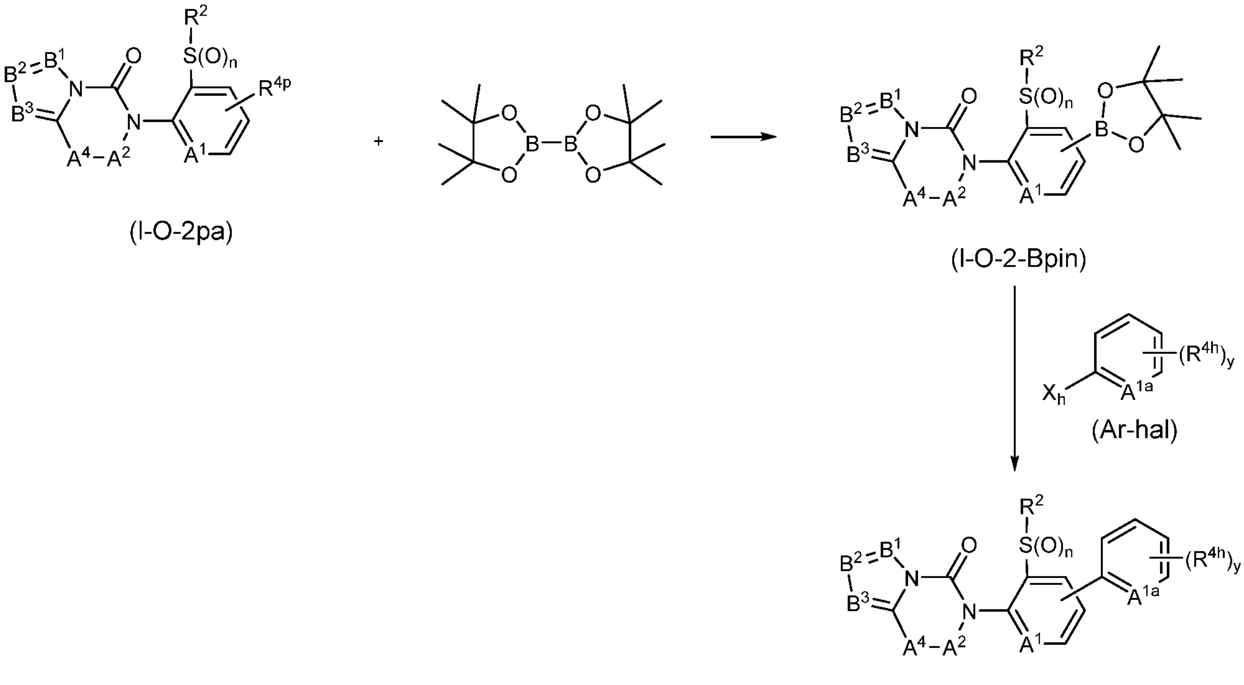

(I-O-2pa)

(I-O-2-Bpin)

(Ar-hal)

(I-O-2Ar)

[wherein the symbols are the same as defined above.]

The Compound (I-O-2Ar) may be prepared according to the Production method 26 from a compound represented by formula (I-O-2-Bpin) prepared according to the Production method 26, by using a compound represented by formula (I-O-2pa) instead of the Compound (I-O-1pa).

Production method 28

A compound represented by formula (I-O-1NBoc) (hereinafter referred to as "Compound (I-O-1NBoc)") may be prepared by reacting the Compound (I-O-1pp) with tert-butyl carbamate in the presence of a metal catalyst and a base.

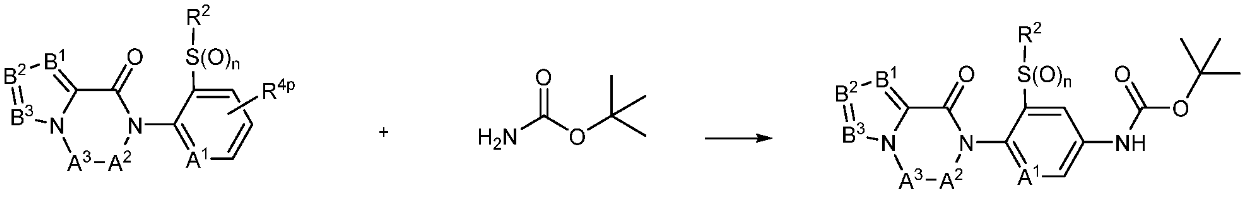

(I-O-1pp)

(I-O-1NBoc)

[wherein the symbols are the same as defined above.]

The reaction is usually carried out in a solvent. Examples of the solvent to be used in the reaction include ethers, aromatic hydrocarbons, nitriles, aprotic polar solvents, and mixtures of two or more of them.

Examples of the base to be used in the reaction include organic bases, alkali metal carbonates, alkali metal hydrides, and alkali metal phosphates.

Examples of the metal catalyst to be used in the reaction include nickel catalysts such as bis(cyclooctadiene) nickel(0) and nickel(II) chloride; palladium catalysts such as palladium(II) acetate, tetrakis(triphenylphosphine)palladium(0), tris(dibenzylideneaceton)dipalladium(0), and [1,1'-bis(diphenylphosphino)ferrocene]dichloropalladium(II) .

The reaction may also be carried out in the presence of a ligand as needed. Examples of the ligand include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, and N,N'-dimethylethylenediamine. When a ligand is used in the reaction, the ligand is usually used at a ratio of 0.01 to 0.5 mol relative to 1 mol of the Compound (I-O-1pp).

In the reaction, tert-butyl carbamate is usually used at a ratio of 1 to 5 mol, the metal catalyst is usually used at a

ratio of 0.01 to 0.5 mol, and the base is usually used at a ratio of 1 to 5 mol, relative to 1 mol of the Compound (I-O-1pp).

The reaction temperature is usually within the range of -20 to 150°C. The reaction time is usually within the range of 0.5 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-O-1NBoc).

Production method 29

A compound represented by formula (I-O-2NBoc) (hereinafter referred to as "Compound (I-O-2NBoc)") may be prepared by reacting the Compound (I-O-2pp) with tert-butyl carbamate in the presence of a metal catalyst and a base.

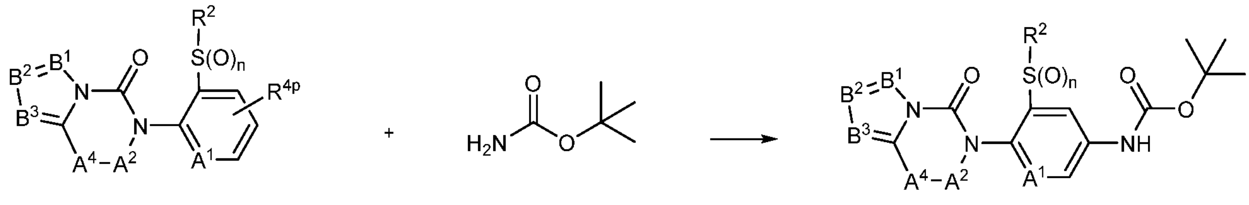

(I-O-2pp) (I-O-2NBoc)

[wherein the symbols are the same as defined above.]

The Compound (I-O-2NBoc) may be prepared according to the Production method 28 by using the Compound (I-O-2pp) instead of the Compound (I-O-1pp).

Production method 30

A compound represented by formula (I-O-1$NH_2$) (hereinafter referred to as "Compound (I-O-1$NH_2$)") may be prepared by reacting the Compound (I-O-1NBoc) with an acid.

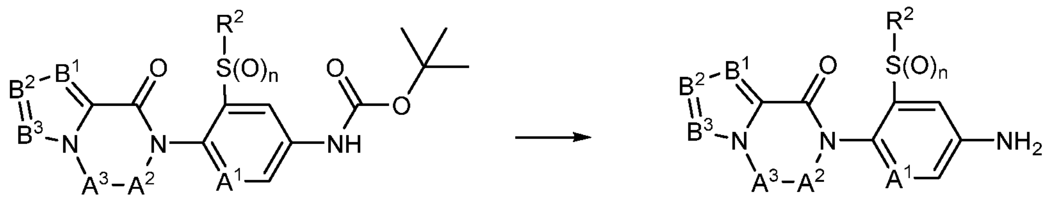

(I-O-1NBoc) (I-O-1$NH_2$)

[wherein the symbols are the same as defined above.]

The reaction is usually carried out in a solvent. Examples of the solvent to be used in the reaction include halogenated hydrocarbons, ethers, alcohols, water, and mixtures of two or more of them.

Examples of the acid to be used in the reaction include mineral acids such as hydrochloric acid; and carboxylic acids such as trifluoroacetic acid.

In the reaction, the acid is usually used at a ratio of 1 to 50 mol relative to 1 mol of the Compound (I-O-1NBoc).

The reaction temperature is usually within the range of 0 to 120°C. The reaction time is usually within the range of 0.1 to 24 hour(s).

When the reaction is completed, the reaction mixture may be subjected to a work-up such as subjecting the reaction mixture to extraction with organic solvent(s), and drying and/or concentrating the resulting organic layer to give the Compound (I-O-1$NH_2$).

Production method 31

A compound represented by formula (I-O-2$NH_2$) (hereinafter referred to as "Compound (I-O-2$NH_2$)") may be prepared by reacting the Compound (I-O-2NBoc) with an acid.

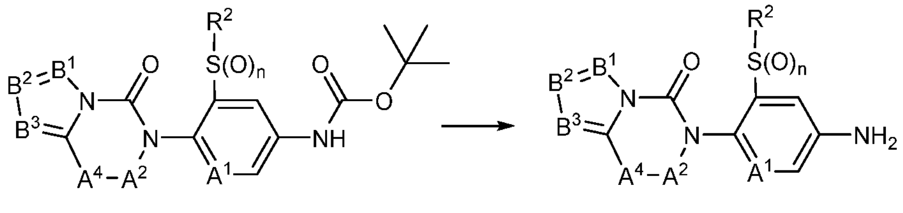

(I-O-2NBoc) (I-O-2$NH_2$)

[wherein the symbols are the same as defined above.]

The Compound (I-O-2$NH_2$) may be prepared according to the Production method 30 by using the Compound (I-O-2NBoc) instead of the Compound (I-O-1NBoc).

Production method 32

A compound represented by formula (I-O-1-NHamide) (hereinafter referred to as "Compound (I-O-1-NHamide)") may be prepared by reacting the Compound (I-Q-1$NH_2$) with a compound represented by formula (R-13) (hereinafter referred to as "Compound (R-13)").

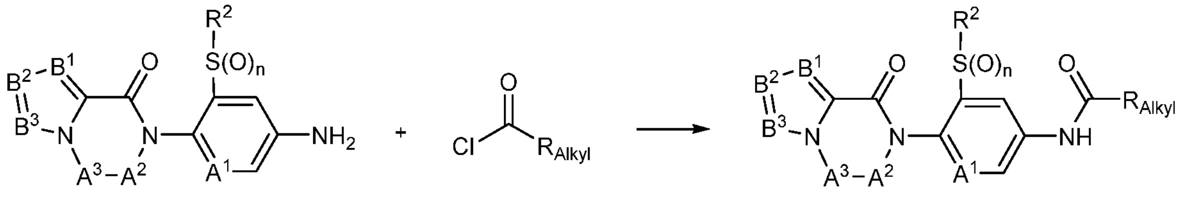

(I-O-1$NH_2$) (R-13) (I-O-1-NHamide)

[wherein $R_{Alkyl}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), a phenyl group optionally substituted with one or more substituent(s) selected from Group $D^2$, or a 5 or 6 membered aromatic heterocyclic group optionally substituted with one or more substituent(s) selected from Group $D^2$; and the other symbols are the same as defined above.]

The reaction is usually carried out in a solvent. Examples of the solvent to be used in the reaction include ethers; aliphatic hydrocarbons such as hexane, heptane, and octane (hereinafter collectively referred to as "aliphatic hydrocarbons"); aromatic hydrocarbons; halogenated hydrocarbons; esters; nitriles; aprotic polar solvents; nitrogen-containing aromatic compounds; and mixtures of two or more of them.

In the reaction, a base may be used as needed. Examples of the base to be used in the reaction include alkali metal carbonates; alkali metal hydroxides such as sodium hydroxide and potassium hydroxide (hereinafter collectively referred to as "alkali metal hydroxides"); and organic bases. When a base is used in the reaction, the base is usually used at a ratio of 1 to 2 mol relative to 1 mol of the Compound (I-Q-1$NH_2$).

In the reaction, the Compound (R-13) is usually used at a ratio of 0.8 to 2 mol relative to 1 mol of the Compound (I-O-1$NH_2$).

The reaction temperature is usually within the range of -20 to 200°C. The reaction time is usually within the range of 0.1 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-O-1-NHamide).

The Compound (R-13) is a commercially available compound or may be prepared by using known method(s).

Production method 33

A compound represented by formula (I-O-2-NHamide) (hereinafter referred to as "Compound (I-O-2-NHamide)") may be prepared by reacting the Compound (I-O-2$NH_2$) with the Compound (R-13).

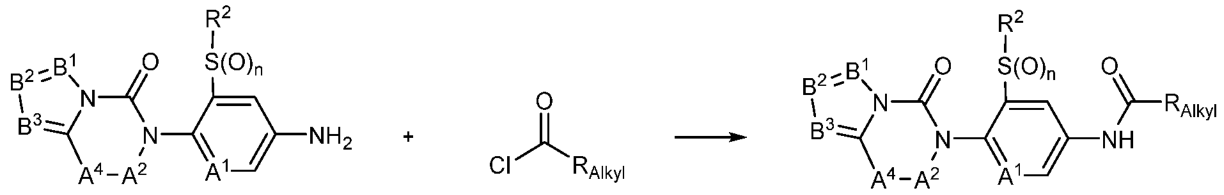

(I-O-2NH$_2$) (R-13) (I-O-2-NHamide)

[wherein the symbols are the same as defined above.]

The Compound (I-O-2-NHamide) may be prepared according to the Production method 32 by using the Compound (I-O-2NH$_2$) instead of the Compound (I-O-1NH$_2$).

Production method 34

A compound represented by formula (I-O-1-amide) (hereinafter referred to as "Compound (I-O-1-amide)") may be prepared by reacting the Compound (I-O-1-NHamide) with the Compound (R-14) in the presence of a base.

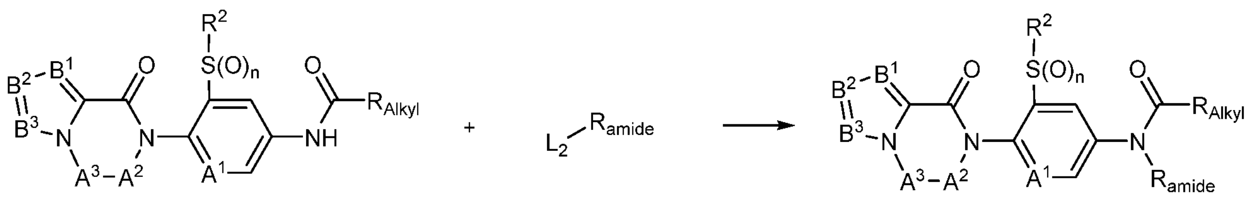

(I-O-1-NHamide) (R-14) (I-O-1-amide)

[wherein $L_2$ represents a leaving group such as a chlorine atom, a bromine atom, and an iodine atom; $R_{amide}$ represents a C1-C6 chain hydrocarbon group optionally substituted with one or more halogen atom(s); and the other symbols are the same as defined above.]

The reaction is usually carried out in a solvent. Examples of the solvent to be used in the reaction include ethers, aromatic hydrocarbons, nitriles, aprotic polar solvents, and mixtures of two or more of them.

Examples of the base to be used in the reaction include alkali metal hydrides, alkali metal carbonates, and organic bases.

In the reaction, the Compound (R-14) is usually used at a ratio of 1 to 5 mol, and the base is usually used at a ratio of 1 to 5 mol, relative to 1 mol of the Compound (I-O-1-NHamide).

The reaction temperature is usually within the range of 0 to 100°C. The reaction time is usually within the range of 0.1 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-O-1-amide).

The Compound (R-14) is a commercially available compound or may be prepared by using known method(s).

Production method 35

A compound represented by formula (I-O-2-amide) (hereinafter referred to as "Compound (I-O-2-amide)") may be prepared by reacting the Compound (I-O-2-NHamide) with the Compound (R-14) in the presence of a base.

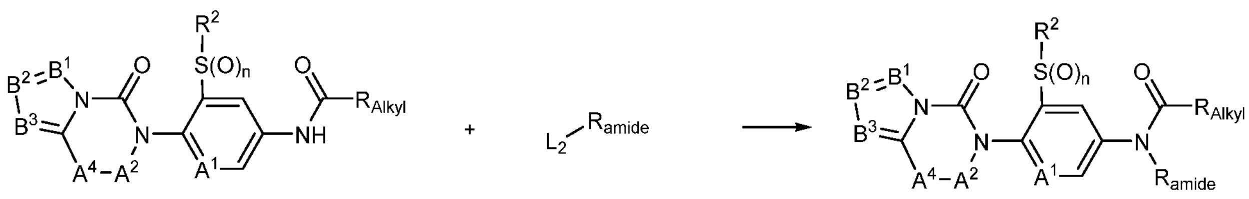

(I-O-2-NHamide) (R-14) (I-O-2-amide)

[wherein the symbols are the same as defined above.]

The Compound (I-O-2-amide) may be prepared according to the Production method 34 by using the Compound (I-O-2-NHamide) instead of the Compound (I-O-1-NHamide).

Production method 36

A compound represented by formula (I-O-1-OH) (hereinafter referred to as "Compound (I-O-1-OH)") may be prepared by reacting the Compound (I-O-1-OBn) with hydrogen in the presence of a catalyst.

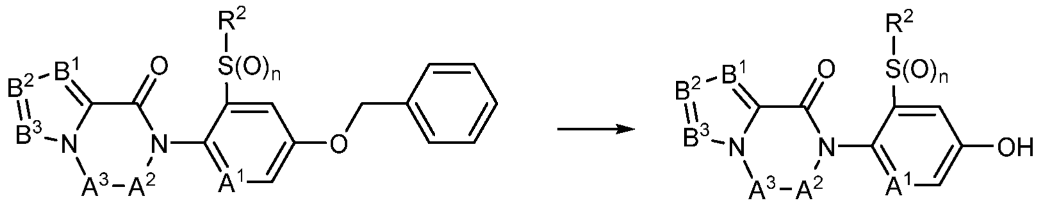

(I-O-1-OBn) (I-O-1-OH)

[wherein the symbols are the same as defined above.]

In the reaction, hydrogen gas is used. The pressure of hydrogen gas is usually within the range of 1 to 100 atm.

The reaction is usually carried out in a solvent. Examples of the solvent to be used in the reaction include ethers, esters, alcohols, water, and mixtures of two or more of them.

Examples of the catalyst to be used in the reaction include transition metal compounds such as palladium carbon, palladium(II) hydroxide, Raney nickel, and platinum oxide.

In the reaction, the catalyst is usually used at a ratio of 0.001 to 0.5 mol relative to 1 mol of the Compound (I-O-1-OBn).

In the reaction, an acid, a base, or the like may also be used as needed.

Examples of the acid include acetic acid and hydrochloric acid, and examples of the base include tertiary amines such as triethylamine. When an acid is used in the reaction, the acid is usually used at a ratio of 0.001 to 0.5 mol relative to 1 mol of the Compound (I-O-1-OBn). When a base is used in the reaction, the base is usually used at a ratio of 0.1 to 5 mol relative to 1 mol of the Compound (I-O-1-OBn).

The reaction temperature is usually within the range of -20 to 100°C. The reaction time is usually within the range of 0.1 to 24 hour(s).

When the reaction is completed, the reaction mixture may be subjected to a work-up such as filtering the reaction mixture, subjecting the resulting mixture to extraction with organic solvent(s) as needed, and drying and/or concentrating the resulting organic layer to give the Compound (I-O-1-OH).

Production method 37

A compound represented by formula (I-O-2-OH) (hereinafter referred to as "Compound (I-O-2-OH)") may be prepared by reacting the Compound (I-O-2-OBn) with hydrogen in the presence of a catalyst.

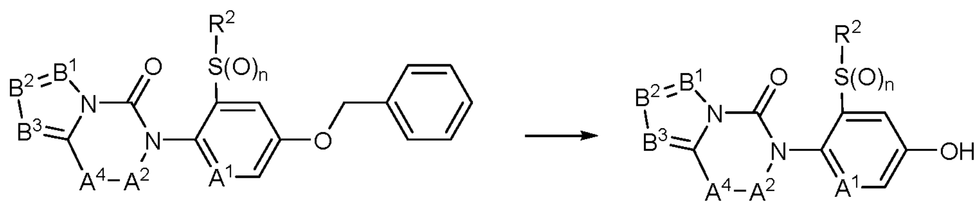

(I-O-2-OBn) (I-O-2-OH)

[wherein the symbols are the same as defined above.]

The Compound (I-O-2-OH) may be prepared according to the Production method 36 by using the Compound (I-O-2-OBn) instead of the Compound (I-O-1-OBn).

Production method 38

A compound represented by formula (I-O-1-OAlkyl) (hereinafter referred to as "Compound (I-O-1-OAlkyl)") may be prepared by reacting the Compound (I-O-1-OH) with a compound represented by formula (R-15) (hereinafter referred to as "Compound (R-15)") in the presence of a base.

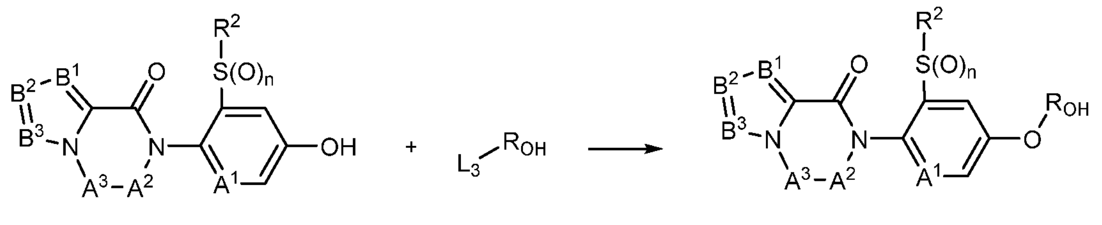

(I-O-1-OH) (R-15) (I-O-1-OAlkyl)

[wherein $L_3$ represents a leaving group such as a chlorine atom, a bromine atom, and an iodine atom; $R_{OH}$ represents a C1-C6 chain hydrocarbon group optionally substituted with one or more substituent(s) selected from Group $F^2$, a C3-C7 cycloalkyl group optionally substituted with one or more substituent(s) selected from Group $J^2$, a C3-C7 cycloalkenyl group optionally substituted with one or more substituent(s) selected from Group $J^2$, a phenyl group optionally substituted with one or more substituent(s) selected from Group $D^2$, or a 6 membered aromatic heterocyclic group optionally substituted with one or more substituent(s) selected from Group $D^2$; and the other symbols are the same as defined above.]

The reaction is usually carried out in a solvent. Examples of the solvent to be used in the reaction include ethers, aromatic hydrocarbons, nitriles, aprotic polar solvents, and mixtures of two or more of them.

Examples of the base to be used in the reaction include alkali metal hydrides, alkali metal carbonates, and organic bases.

In the reaction, the Compound (R-15) is usually used at a ratio of 1 to 5 mol, and the base is usually used at a ratio of 1 to 5 mol, relative to 1 mol of the Compound (I-O-1-OH).

The reaction temperature is usually within the range of 0 to 100°C. The reaction time is usually within the range of 0.1 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-O-1-OAlkyl).

The Compound (R-15) is a commercially available compound or may be prepared by using known method(s).

Production method 39

A compound represented by formula (I-O-2-OAlkyl) (hereinafter referred to as "Compound (I-O-2-OAlkyl)") may be prepared by reacting the Compound (I-O-2-OH) with the Compound (R-15) in the presence of a base.

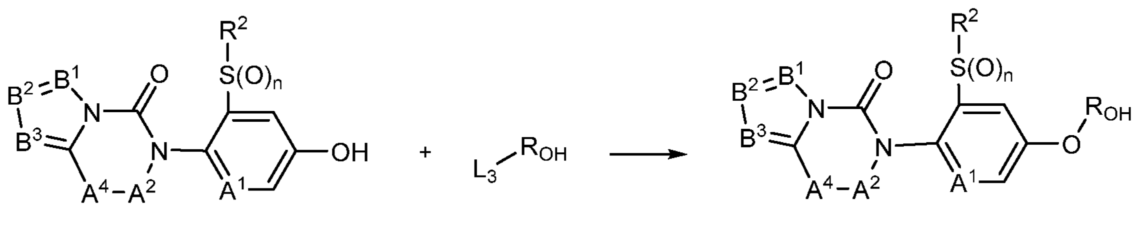

(I-O-2-OH) (R-15) (I-O-2-OAlkyl)

[wherein the symbols are the same as defined above.]

The Compound (I-O-2-OAlkyl) may be prepared according to the Production method 38 by using the Compound (I-O-2-OH) instead of the Compound (I-O-1-OH).

Hereinafter, methods for producing production intermediates are described.

Reference Production method 1

A compound represented by formula (M-2-n1) and a compound represented by formula (M-2-n2) may be

prepared by reacting a compound represented by formula (M-2-n0) with an oxidizing agent.

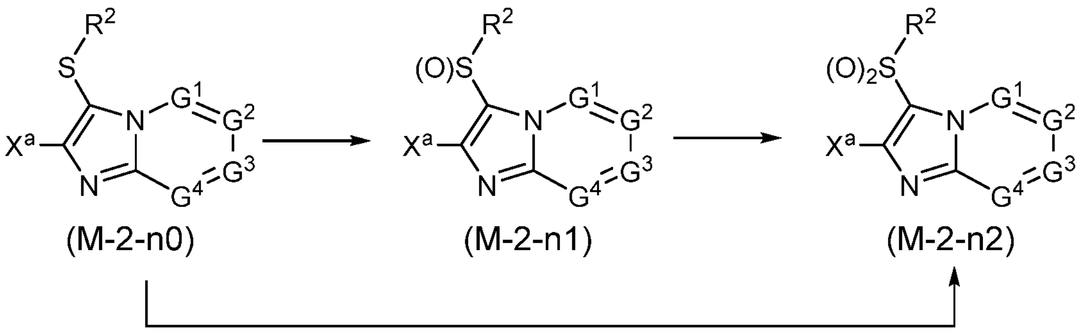

(M-2-n0) (M-2-n1) (M-2-n2)

[wherein the symbols are the same as defined above.]

These reactions may be carried out according to the Production method 1.

Reference Production method 2

A compound represented by formula (M-2-d) (hereinafter referred to as "Compound (M-2-d)") may be prepared according to the following scheme.

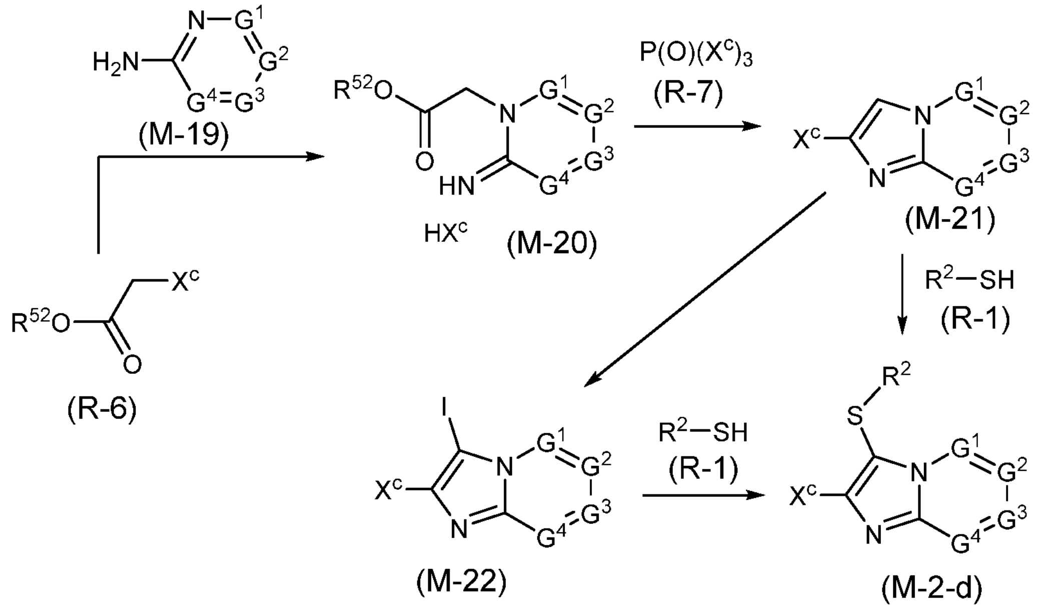


(M-19) (R-6) (M-20) (M-21) (M-22) (M-2-d)

[wherein $X^c$ represents a chlorine atom or a bromine atom; $R^{52}$ represents a hydrogen atom, a methyl group, or an ethyl group; and the other symbols are the same as defined above.]

A compound represented by formula (M-20) (hereinafter referred to as "Compound (M-20)") may be prepared by reacting a compound represented by formula (M-19) (hereinafter referred to as "Compound (M-19)") with a compound represented by formula (R-6) (hereinafter referred to as "Compound (R-6)").

The reaction is usually carried out in a solvent. Examples of the solvent include aromatic hydrocarbons, alcohols, nitriles, and mixtures of two or more of them.

In the reaction, the Compound (M-19) is usually used at a ratio of 1 to 10 mol relative to 1 mol of the Compound (R-6).

The reaction temperature is usually within the range of 0 to 200°C. The reaction time is usually within the range of 0.1 to 48 hour(s).

When the reaction is completed, the reaction mixture may be subjected to a usual work-up to give the Compound (M-20).

The Compound (R-6) and the Compound (M-19) are commercially available compounds or may be prepared by using known method(s).

A compound represented by formula (M-21) (hereinafter referred to as "Compound (M-21)") may be prepared by

reacting the Compound (M-20) with a compound represented by formula (R-7) (hereinafter referred to as "Compound (R-7)").

The reaction is usually carried out in a solvent. Examples of the solvent include aromatic hydrocarbons, nitriles, and mixtures of two or more of them.

In the reaction, the Compound (R-7) is usually used at a ratio of 1 to 10 mol relative to 1 mol of the Compound (M-20).

The reaction temperature is usually within the range of 60 to 120°C. The reaction time is usually within the range of 0.1 to 48 hour(s).

When the reaction is completed, the reaction mixture may be subjected to a usual work-up to give the Compound (M-21).

The Compound (R-7) is a commercially available compound or may be prepared by using known method(s).

A compound represented by formula (M-22) (hereinafter referred to as "Compound (M-22)") may be prepared by reacting the Compound (M-21) with N-iodosuccinimide. The reaction may be carried out according to the method for producing the Compound (M-6) from the Compound (M-5) in the Production method 6.

The Compound (M-2-d) may be prepared by reacting the Compound (M-21) or the Compound (M-22) with the Compound (R-1). These reactions may be carried out according to the method for producing the Compound (I-n0-1) from the Compound (M-5) or the Compound (M-6) in the Production method 6.

Reference Production method 3

A compound represented by formula (M-2-f) may be prepared by reacting a compound represented by formula (M-2-e) (hereinafter referred to as "Compound (M-2-e)") with silver fluoride in the presence of a metal catalyst.

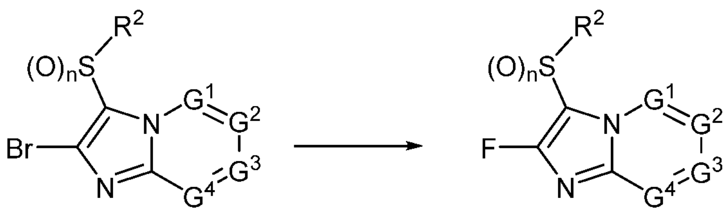

(M-2-e) (M-2-f)

[wherein the symbols are the same as defined above.]

The reaction may be carried out according to the method(s) described in, for example, Journal of the American Chemical Society, 2014, 136, 3792.

Reference Production method 4

A compound represented by formula (M-2-g) may be prepared by reacting the Compound (M-2-e) with sodium iodide in the presence of a metal catalyst.

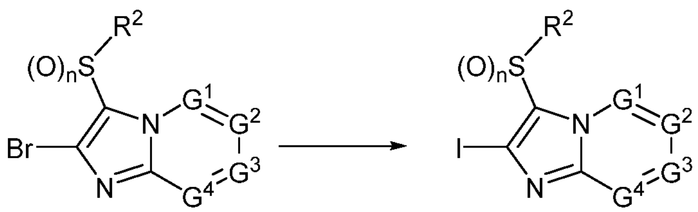

(M-2-e) (M-2-g)

[wherein the symbols are the same as defined above.]

The reaction may be carried out according to the method(s) described in, for example, Journal of the American Chemical Society, 2002, 124, 14844.

Reference Production method 5

The Compound (M-9-X) may be prepared by reacting a compound represented by formula (M-23) (hereinafter

referred to as "Compound (M-23)") with a compound represented by formula (M-24) (hereinafter referred to as "Compound (M-24)") in the presence of a condensing agent.

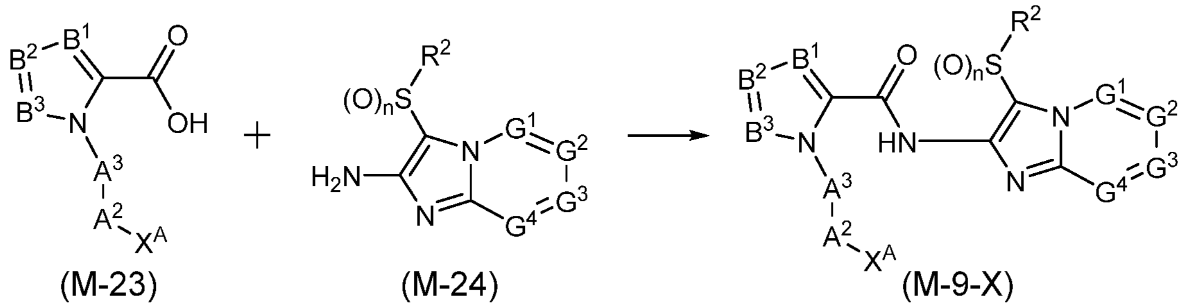

(M-23) (M-24) (M-9-X)

[wherein the symbols are the same as defined above.]

The reaction is usually carried out in a solvent. Examples of the solvent to be used in the reaction include ethers, halogenated hydrocarbons, aromatic hydrocarbons, esters, nitriles, aprotic polar solvents, nitrogen-containing aromatic compounds, and mixtures of two or more of them.

Examples of the condensing agent to be used in the reaction include carbodiimide compounds such as 1-ethyl-3-[3-(dimethylamino)propyl]carbodiimide hydrochloride and 1,3-dicyclohexylcarbodiimide.

In the reaction, a catalyst may be used as needed. Examples of the catalyst to be used in the reaction include 1-hydroxybenzotriazole. When a catalyst is used in the reaction, the catalyst is usually used at a ratio of 0.01 to 0.5 mol relative to 1 mol of the Compound (M-24).

In the reaction, a base may be used as needed. Examples of the base include organic bases. When a base is used in the reaction, the base is usually used at a ratio of 1 to 2 mol relative to 1 mol of the Compound (M-24).

In the reaction, the Compound (M-23) is usually used at a ratio of 1 to 2 mol, and the condensing agent is usually used at a ratio of 1 to 2 mol, relative to 1 mol of the Compound (M-24).

The reaction temperature is usually within the range of 0 to 200°C. The reaction time is usually within the range of 0.1 to 12 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (M-9-X).

Reference Production method 6

The Compound (M-23) may be prepared by hydrolyzing a compound represented by formula (M-25) (hereinafter referred to as "Compound (M-25)").

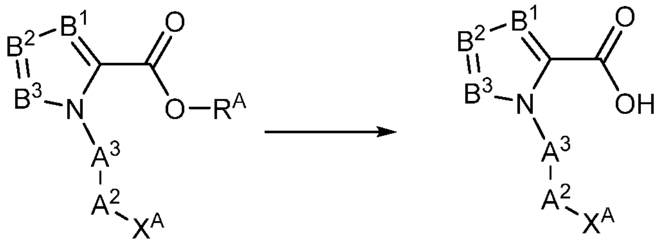

(M-25) (M-23)

[wherein $R^A$ represents a C1-C3 alkyl group such as a methyl group and an ethyl group; and the other symbols are the same as defined above.]

In case of hydrolysis with an acid, the reaction is usually carried out by using an aqueous solution of an acid as a solvent.

Examples of the acid to be used in the reaction include mineral acids such as hydrochloric acid, nitric acid, phosphoric acid, and sulfuric acid; and carboxylic acids such as acetic acid and trifluoroacetic acid.

The reaction temperature is usually within the range of 0 to 100°C. The reaction time is usually within the range of 0.1 to 24 hour(s).

When the reaction is completed, the reaction mixture may be subjected to a work-up such as subjecting the reaction mixture to extraction with organic solvent(s), and drying and/or concentrating the resulting organic layer to give the

Compound (M-23).

In case of hydrolysis with a base, the reaction is usually carried out in the presence of a solvent.

Examples of the solvent to be used in the reaction include ethers, alcohols, water, and mixtures of two or more of them.

Examples of the base to be used in the reaction include alkali metal hydroxides.

In the reaction, the base is usually used at a ratio of 1 to 10 mol relative to 1 mol of the Compound (M-25).

The reaction temperature is usually within the range of 0 to 120°C. The reaction time is usually within the range of 0.1 to 24 hour(s).

When the reaction is completed, the reaction solution may be subjected to a work-up such as acidifying the reaction solution, then subjecting the resulting mixture to extraction with organic solvent(s), and drying and/or concentrating the resulting organic layer to give the Compound (M-23).

Reference Production method 7

The Compound (M-25) may be prepared by reacting a compound represented by formula (M-26) (hereinafter referred to as "Compound (M-26)") with a compound represented by formula (R-8) (hereinafter referred to as "Compound (R-8)") in the presence of a condensing agent.

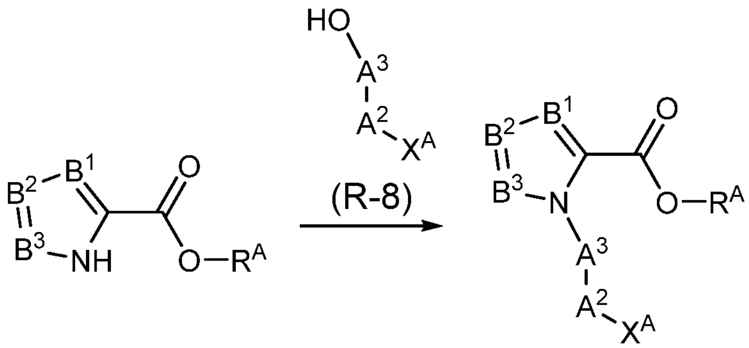

(M-26) (M-25)

[wherein the symbols are the same as defined above.]

The reaction is usually carried out in a solvent. Examples of the solvent include ethers, halogenated hydrocarbons, aromatic hydrocarbons, esters, nitriles, aprotic polar solvents, nitrogen-containing aromatic compounds, and mixtures of two or more of them.

Examples of the condensing agent to be used in the reaction include a mixture of triphenylphosphine and an azodiester compound. Examples of the azodiester compound include diethyl azodicarboxylate.

In the reaction, the Compound (R-8) is usually used at a ratio of 1 to 5 mol, and the condensing agent is usually used at a ratio of 1 to 5 mol, relative to 1 mol of the Compound (M-26).

The reaction temperature is usually within the range of -20 to 200°C. The reaction time is usually within the range of 0.1 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (M-25).

The Compound (M-26) is a commercially available compound or may be prepared by using known method(s).

The Compound (R-8) is a commercially available compound or may be prepared by using known method(s).

Reference Production method 8

The Compound (M-24) may be prepared by reacting the Compound (M-2-h) with ammonia.

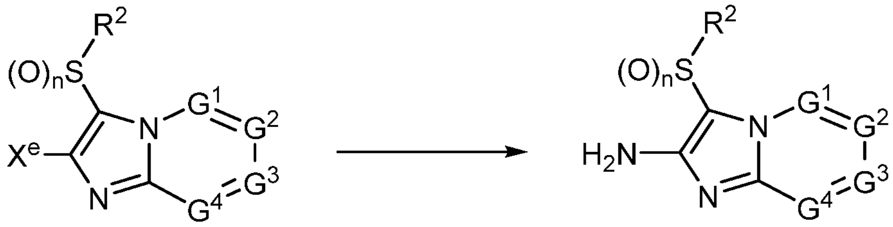

(M-2-h) (M-24)

[wherein $X^e$ represents a fluorine atom or a chlorine atom; and the other symbols are the same as defined above.]

The reaction is usually carried out in a solvent. Examples of the solvent to be used in the reaction include ethers, nitriles, aprotic polar solvents, alcohols, water, and mixtures of two or more of them.

In the reaction, a base may be used as needed. Examples of the base to be used in the reaction include organic bases and alkali metal carbonates. When a base is used in the reaction, the base is usually used at a ratio of 0.1 to 5 mol relative to 1 mol of the Compound (M-2-h).

Ammonia may also be used as a solution such as aqueous ammonia and a solution of ammonia in methanol.

In the reaction, ammonia is usually used at a ratio of 1 to 100 mol relative to 1 mol of the Compound (M-2-h).

The reaction temperature is usually within the range of -20 to 100°C. The reaction time is usually within the range of 0.1 to 48 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (M-24).

Reference Production method 9

The Compound (M-9-X) may also be prepared by reacting a compound represented by formula (M-27) (hereinafter referred to as "Compound (M-27)") with the Compound (M-24).

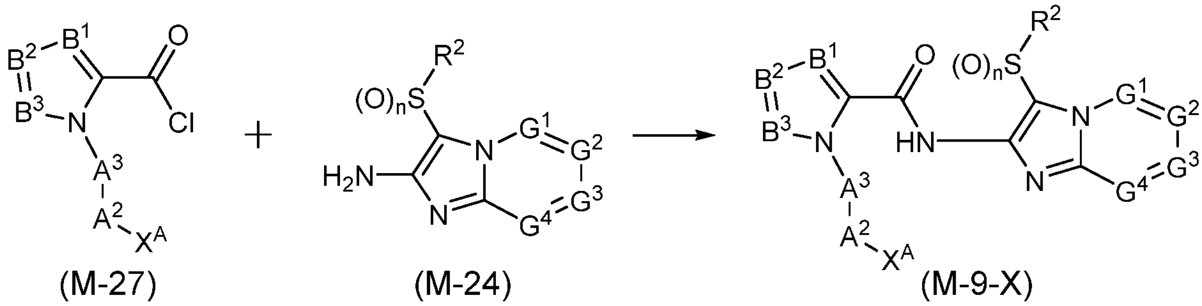

(M-27) (M-24) (M-9-X)

[wherein the symbols are the same as defined above.]

The reaction is usually carried out in a solvent. Examples of the solvent to be used in the reaction include ethers; aliphatic hydrocarbons; aromatic hydrocarbons; halogenated hydrocarbons; esters; nitriles; aprotic polar solvents; and mixtures of two or more of them.

In the reaction, a base may be used as needed. Examples of the base to be used in the reaction include alkali metal carbonates; alkali metal hydroxides; and organic bases. When a base is used in the reaction, the base is usually used at a ratio of 1 to 2 mol relative to 1 mol of the Compound (M-24).

In the reaction, the Compound (M-27) is usually used at a ratio of 0.8 to 1.2 mol relative to 1 mol of the Compound (M-24).

The reaction temperature is usually within the range of -20 to 200°C. The reaction time is usually within the range of 0.1 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (M-9-X).

Reference Production method 10

The Compound (M-27) may be prepared by reacting the Compound (M-23) in the presence of a chlorinating agent.

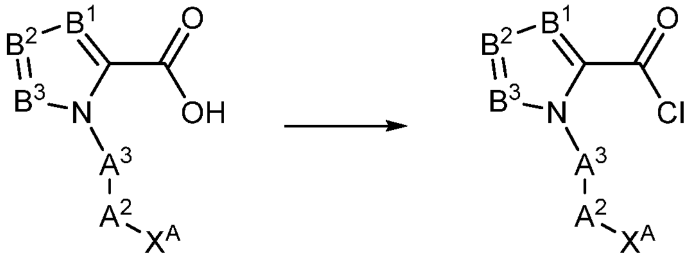

(M-23) (M-27)

[wherein the symbols are the same as defined above.]

The reaction is usually carried out in a solvent. Examples of the solvent to be used in the reaction include ethers, aromatic hydrocarbons, halogenated hydrocarbons, and mixtures of two or more of them.

Examples of the chlorinating agent to be used in the reaction include thionyl chloride, oxalyl dichloride, and phosphorus oxychloride.

In the reaction, the chlorinating agent is usually used at a ratio of 1 to 5 mol relative to 1 mol of the Compound (M-23).

The reaction temperature is usually within the range of 0 to 100°C. The reaction time is usually within the range of 0.1 to 24 hour(s).

When the reaction is completed, the solvent may be distilled away to give the Compound (M-27).

Reference Production method 11

The Compound (M-9-OH) may be prepared by reacting a compound represented by formula (M-9-P) (hereinafter referred to as "Compound (M-9-P)") with an acid.

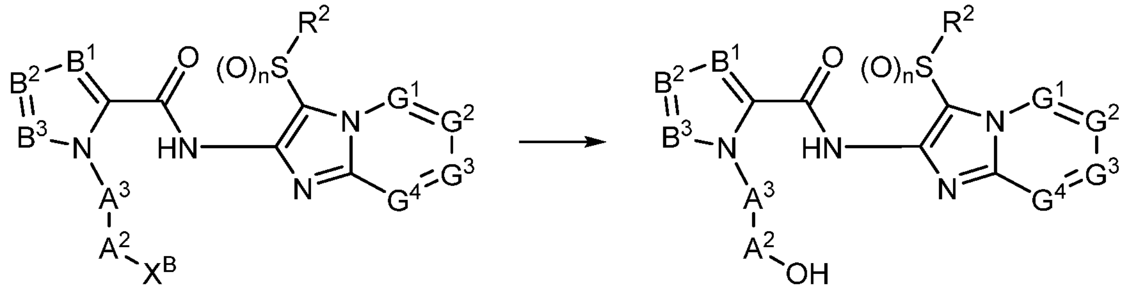

(M-9-P) (M-9-OH)

[wherein $X^B$ represents $OR^B$; $R^B$ represents a protecting group such as a tetrahydropyranyl group, a methoxymethyl group, a 2-methoxyethoxymethyl group, a tertbutyldimethylsilyl group, or a tribenzylsilyl group; and the other symbols are the same as defined above.]

The reaction is usually carried out in a solvent. Examples of the solvent to be used in the reaction include ethers, alcohols, water, and mixtures of two or more of them.

Examples of the acid to be used in the reaction include mineral acids such as hydrochloric acid, nitric acid, phosphoric acid, and sulfuric acid; and carboxylic acids such as acetic acid and trifluoroacetic acid.

The reaction temperature is usually within the range of 0 to 120°C. The reaction time is usually within the range of 0.1 to 24 hour(s).

In the reaction, the acid is usually used at a ratio of 1 to 10 mol relative to 1 mol of the Compound (M-9-P).

When the reaction is completed, the reaction mixture may be subjected to a work-up such as subjecting the reaction mixture to extraction with organic solvent(s), and drying and/or concentrating the resulting organic layer to give the Compound (M-9-OH).

Reference Production method 12

The Compound (M-9-P) may be prepared by reacting a compound represented by formula (M-28) (hereinafter referred to as "Compound (M-28)") with the Compound (M-24) in the presence of a condensing agent.

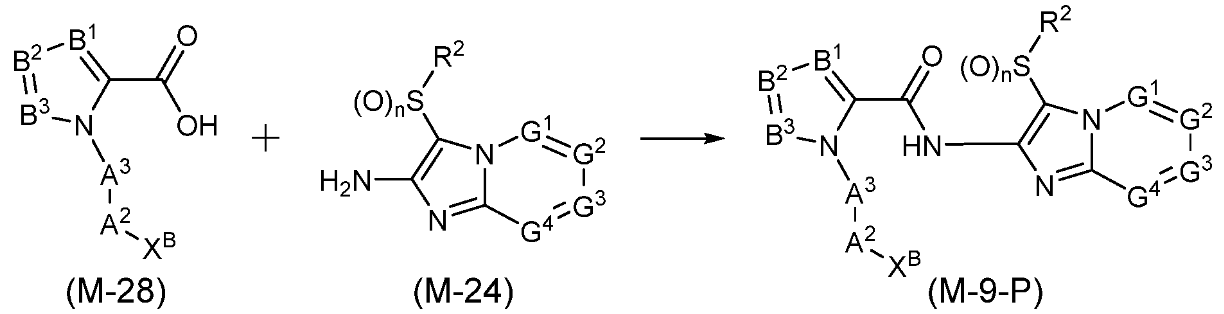

(M-28) (M-24) (M-9-P)

[wherein the symbols are the same as defined above.]

The Compound (M-9-P) may be prepared according to the Reference Production method 5 by using the Compound (M-28) instead of the Compound (M-23).

Reference Production method 13

The Compound (M-28) may be prepared by hydrolyzing a compound represented by formula (M-29) (hereinafter referred to as "Compound (M-29)") in the presence of a base.

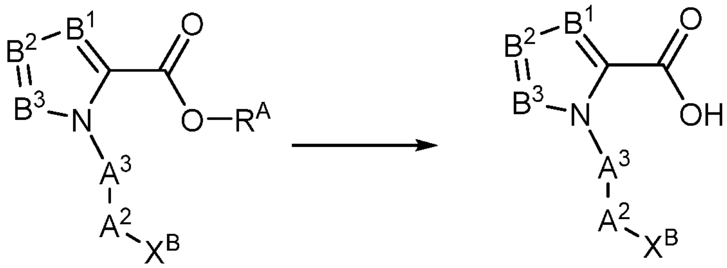

(M-29) (M-28)

[wherein the symbols are the same as defined above.]

The reaction is usually carried out in a solvent. Examples of the solvent to be used in the reaction include ethers, alcohols, water, and mixtures of two or more of them.

Examples of the base to be used in the reaction include alkali metal hydroxides.

In the reaction, the base is usually used at a ratio of 1 to 10 mol relative to 1 mol of the Compound (M-29).

The reaction temperature is usually within the range of 0 to 120°C. The reaction time is usually within the range of 0.1 to 24 hour(s).

When the reaction is completed, the reaction solution may be subjected to a work-up such as acidifying the reaction solution, then subjecting the resulting mixture to extraction with organic solvent(s), and drying and/or concentrating the resulting organic layer to give the Compound (M-28).

Reference Production method 14

The Compound (M-29) may be prepared by reacting the Compound (M-26) with a compound represented by formula (R-9) (hereinafter referred to as "Compound (R-9)") in the presence of a condensing agent.

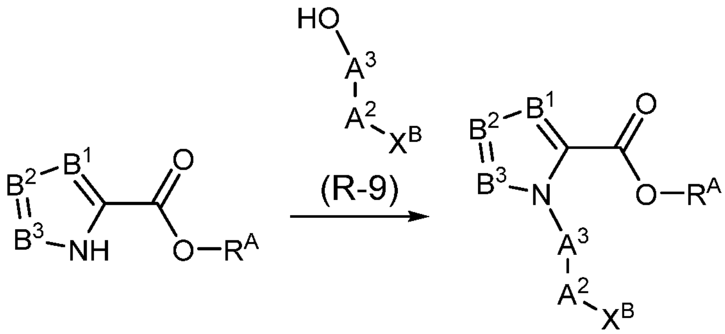


(M-26) (M-29)

[wherein the symbols are the same as defined above.]

The Compound (M-29) may be prepared according to the Reference Production method 7 by using the Compound (R-9) instead of the Compound (R-8).

The Compound (R-9) is a commercially available compound or may be prepared by using known method(s).

Reference Production method 15

The Compound (M-10-X) may be prepared by reacting the Compound (M-10-OH) in the presence of a halogenating agent.

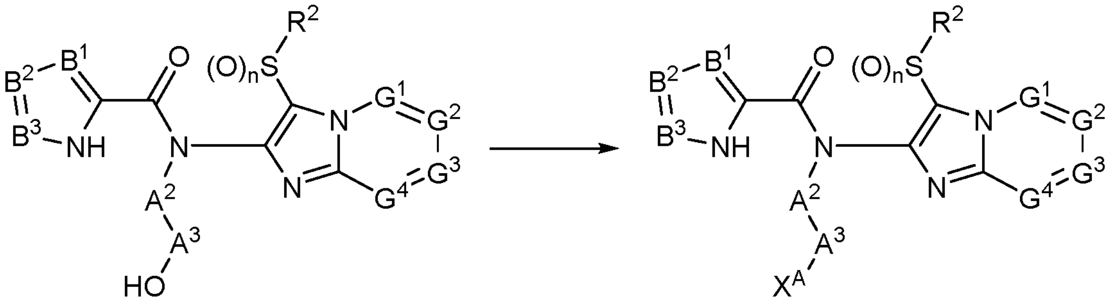

(M-10-OH) (M-10-X)

[wherein the symbols are the same as defined above.]

The reaction is usually carried out in a solvent. Examples of the solvent to be used in the reaction include ethers, alcohols, aromatic hydrocarbons, halogenated hydrocarbons, aprotic polar solvents, water, and mixtures of two or more of them.

Examples of the halogenating agent to be used in the reaction include hydrogen halides such as hydrogen chloride; phosphorus halide such as phosphorus tribromide; thionyl halide such as thionyl chloride; a mixture of triphenylphosphine and carbon tetrachloride; and a mixture of triphenylphosphine and carbon tetrabromide.

In the reaction, the halogenating agent is usually used at a ratio of 1 to 10 mol relative to 1 mol of the Compound (M-10-OH).

In the reaction, a base may be used as needed. Examples of the base to be used in the reaction include organic bases. When a base is used in the reaction, the base is usually used at a ratio of 1 to 2 mol relative to 1 mol of the Compound (M-10-OH).

The reaction temperature is usually within the range of -20 to 150°C. The reaction time is usually within the range of 0.1 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (M-10-X).

Reference Production method 16

The Compound (M-10-OH) may be prepared by reacting a compound represented by formula (M-10-P) (hereinafter referred to as "Compound (M-10-P) with an acid.

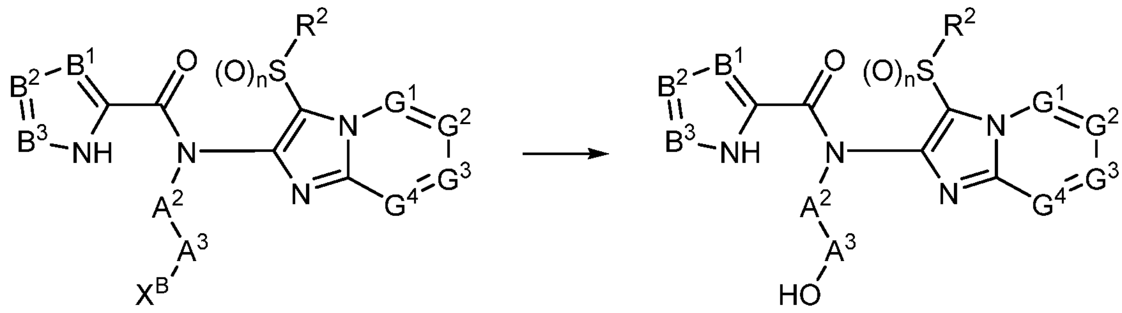

(M-10-P) (M-10-OH)

[wherein the symbols are the same as defined above.]

The Compound (M-10-OH) may be prepared according to the Reference Production method 11 by using the Compound (M-10-P) instead of the Compound (M-9-P).

Reference Production method 17

The Compound (M-10-P) may be prepared by reacting a compound represented by formula (M-30) (hereinafter referred to as "Compound (M-30)") with a compound represented by formula (M-33) (hereinafter referred to as "Compound (M-33)") in the presence of a condensing agent.

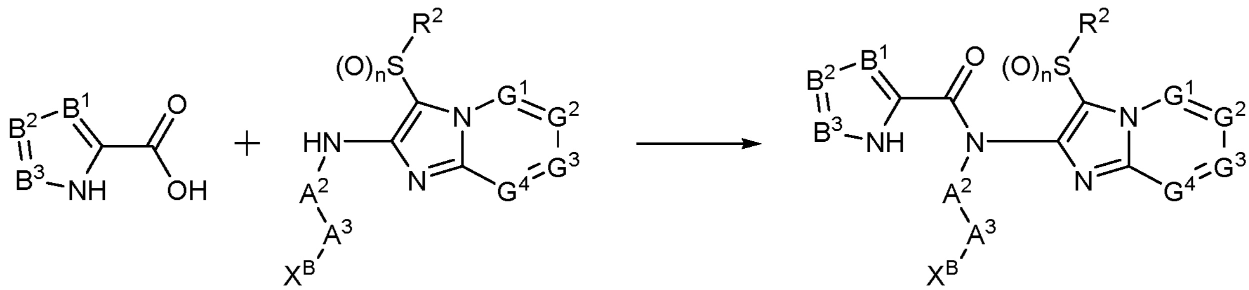

(M-30) (M-33) (M-10-P)

[wherein the symbols are the same as defined above.]

The Compound (M-10-P) may be prepared according to the Reference Production method 5 by using the Compound (M-30) instead of the Compound (M-23) and by using the Compound (M-33) instead of the Compound (M-24).

The Compound (M-30) is a commercially available compound or may be prepared by using known method(s).

Reference Production method 18

The Compound (M-10-P) may also be prepared by reacting a compound represented by formula (M-32) (hereinafter referred to as "Compound (M-32)") with the Compound (M-33).

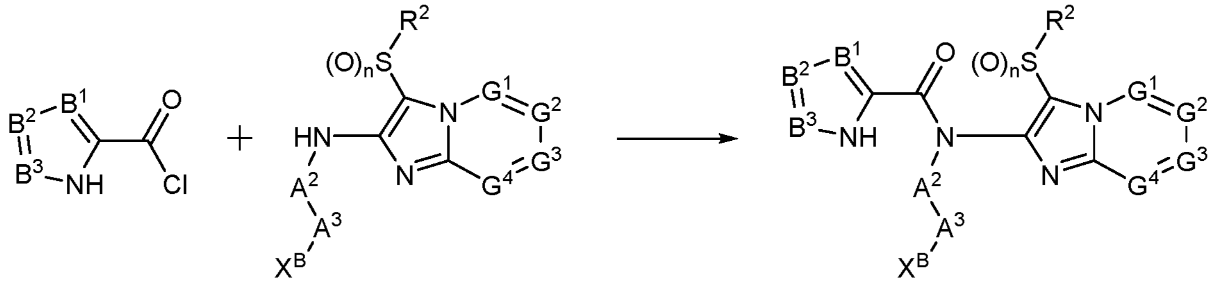

(M-32) (M-33) (M-10-P)

[wherein the symbols are the same as defined above.]

The Compound (M-10-P) may be prepared according to the Reference Production method 9 by using the

Compound (M-32) instead of the Compound (M-27) and by using the Compound (M-33) instead of the Compound (M-24).

The Compound (M-32) is a commercially available compound or may be prepared by using known method(s).

Reference Production method 19

The Compound (M-33) may be prepared by reacting the Compound (M-24) with a compound represented by formula (R-12) (hereinafter referred to as "Compound (R-12)") in the presence of a base.

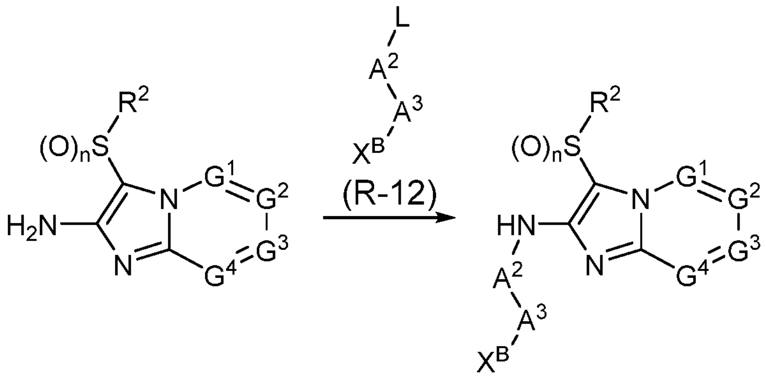


(M-24) (M-33)

[wherein L represents a leaving group such as a chlorine atom and a bromine atom; and the other symbols are the same as defined above.]

The reaction is usually carried out in a solvent. Examples of the solvent to be used in the reaction include ethers, aromatic hydrocarbons, nitriles, aprotic polar solvents, and mixtures of two or more of them.

Examples of the base to be used in the reaction include alkali metal hydrides, alkali metal carbonates, and organic bases.

In the reaction, the Compound (R-12) is usually used at a ratio of 1 to 5 mol, and the base is usually used at a ratio of 1 to 2 mol, relative to 1 mol of the Compound (M-24).

The reaction temperature is usually within the range of 0 to 100°C. The reaction time is usually within the range of 0.1 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (M-33).

The Compound (R-12) is a commercially available compound or may be prepared by using known method(s).

Reference Production method 20

The Compound (M-2-n0) may be prepared according to the following scheme.

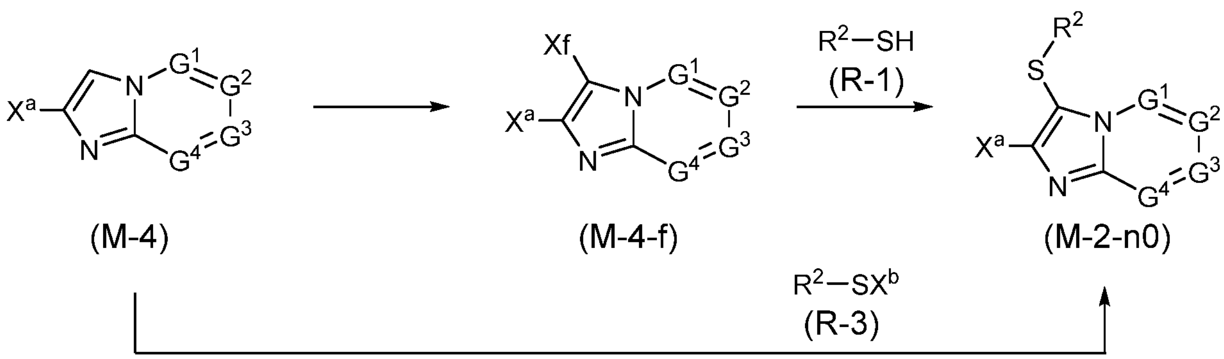


(M-4) (M-4-f) (M-2-n0)

[wherein $X^b$ represents $SR^2$ or a hydrogen atom; $X^f$ represents a chlorine atom, a bromine atom, or an iodine atom; and the other symbols are the same as defined above.]

First, a step for producing a compound represented by formula (M-4-f) (hereinafter referred to as "Compound (M-4-f)") from the Compound (M-4) is described.

The Compound (M-4-f) may be prepared by reacting the Compound (M-4) with a halogenating agent.

The reaction is usually carried out in a solvent. Examples of the solvent to be used in the reaction include alcohols, nitriles, ethers, aromatic hydrocarbons, aprotic polar solvents, halogenated hydrocarbons, water, and mixtures of two or more of them.

Examples of the halogenating agent include chlorine, bromine, iodine, N-chlorosuccinimide, N-bromosuccinimide, and N-iodosuccinimide.

In the reaction, the halogenating agent is usually used at a ratio of 1 to 20 mol relative to 1 mol of the Compound (M-4).

The reaction temperature is usually within the range of -20 to 200°C. The reaction time is usually within the range of 0.1 to 72 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (M-4-f).

Next, a step for producing the Compound (M-2-n0) from the Compound (M-4-f) is described.

The Compound (M-2-n0) may be prepared by reacting the Compound (M-4-f) with the Compound (R-1) in the presence of a metal catalyst and a base.

The reaction is usually carried out in a solvent. Examples of the solvent to be used in the reaction include alcohols, nitriles, ethers, aromatic hydrocarbons, aprotic polar solvents, water, and mixtures of two or more of them.

Examples of the metal catalyst to be used in the reaction include palladium catalysts such as tetrakis(triphenylphosphine)palladium(0), [1,1'-bis(diphenylphosphino)ferrocene]palladium(II) dichloride, tris(dibenzylideneaceton)dipalladium(0), and palladium(II) acetate; nickel catalysts such as bis(cyclooctadiene)nickel(0) and nickel(II) chloride; and copper catalysts such as copper(I) iodide and copper(I) chloride.

Examples of the base to be used in the reaction include alkali metal hydrides, alkali metal carbonates, and organic bases.

In the reaction, a ligand may also be used. Examples of the ligand include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, and 1,10-phenanthroline. When a ligand is used in the reaction, the ligand is usually used at a ratio of 0.01 to 1 mol relative to 1 mol of the Compound (M-4-f).

In the reaction, the Compound (R-1) is usually used at a ratio of 1 to 20 mol, the metal catalyst is usually used at a ratio of 0.01 to 0.5 mol, and the base is usually used at a ratio of 0.1 to 5 mol, relative to 1 mol of the Compound (M-4-f).

The reaction temperature is usually within the range of -20 to 200°C. The reaction time is usually within the range of 0.1 to 72 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (M-2-n0).

Next, a step for producing the Compound (M-2-n0) from the Compound (M-4) is described.

The Compound (M-2-n0) may also be prepared by reacting the Compound (M-4) with a compound represented by formula (R-3) (hereinafter referred to as "Compound (R-3)") in the presence of a halogenating agent.

The reaction is usually carried out in a solvent. Examples of the solvent to be used in the reaction include alcohols, nitriles, ethers, aromatic hydrocarbons, aprotic polar solvents, water, and mixtures of two or more of them.

Examples of the halogenating agent include bromine, iodine, sodium bromide, potassium bromide, sodium iodide, and potassium iodide.

In the reaction, an oxidizing agent may be used as needed. Examples of the oxidizing agent to be used in the reaction include hydrogen peroxide, tert-butyl hydroperoxide, and DMSO. When an oxidizing agent is used in the reaction, the oxidizing agent is usually used at a ratio of 1 to 20 mol relative to 1 mol of the Compound (M-4).

In the reaction, the Compound (R-3) is usually used at a ratio of 0.5 to 10 mol, and the halogenating agent is usually used at a ratio of 0.05 to 10 mol, relative to 1 mol of the Compound (M-4).

The reaction temperature is usually within the range of 0 to 200°C. The reaction time is usually within the range of 0.1 to 72 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (M-2-n0).

The Compound (R-3) is known or may be prepared according to known method(s).

The Compound (M-4) is known or may be prepared according to the method described in WO 2015/157093 pamphlet, WO 2016/109706 pamphlet, Organic & Biomolecular Chemistry, 2017, 15, 4199., European Journal of Medicinal Chemistry, 2016, 123, 916., or the like.

Reference Production method 21

A compound represented by formula (M-11-n1) (hereinafter referred to as "Compound (M-11-n1)") or a compound represented by formula (M-11-n2) (hereinafter referred to as "Compound (M-11-n2)") may be prepared by reacting the Compound (M-11-n0) with an oxidizing agent. The Compound (M-11-n2) may also be prepared by reacting the Compound (M-11-n1) with an oxidizing agent.

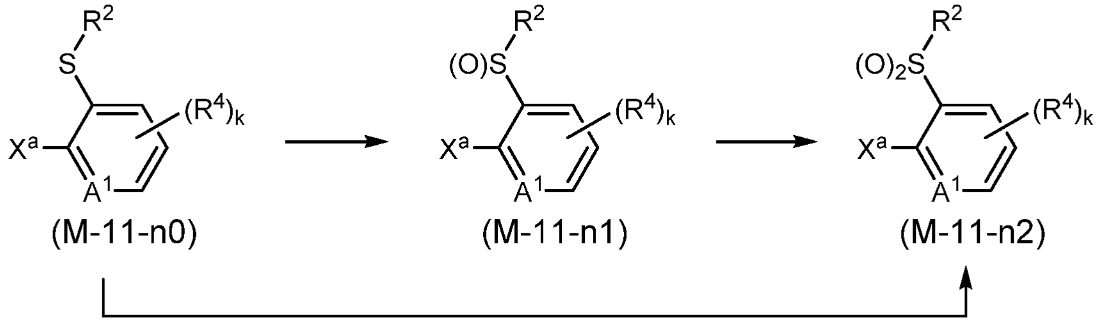

(M-11-n0) (M-11-n1) (M-11-n2)

[wherein the symbols are the same as defined above.]

These reactions may be carried out according to the Production method 1.

Reference Production method 22

The Compound (M-17-X) may be prepared by reacting the Compound (M-23) with a compound represented by formula (M-34) (hereinafter referred to as "Compound (M-34)") in the presence of a condensing agent.

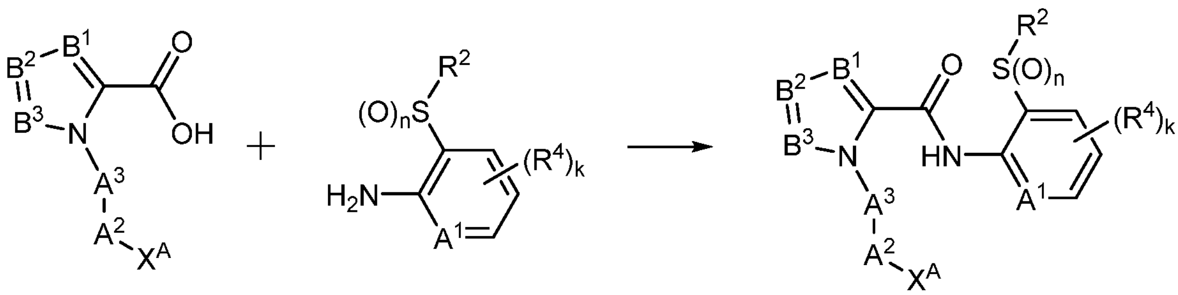

(M-23) (M-34) (M-17-X)

[wherein the symbols are the same as defined above.]

The Compound (M-17-X) may be prepared according to the Reference Production method 5 by using the Compound (M-34) instead of the Compound (M-24).

Reference Production method 23

The Compound (M-17-X) may also be prepared by reacting the Compound (M-27) with the Compound (M-34).

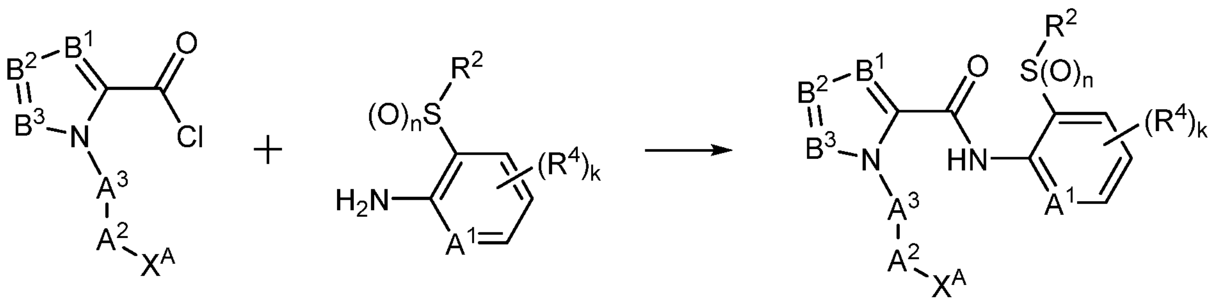

(M-27) (M-34) (M-17-X)

[wherein the symbols are the same as defined above.]

The Compound (M-17-X) may be prepared according to the Reference Production method 9 by using the Compound (M-34) instead of the Compound (M-24).

Reference Production method 24

The Compound (M-34) may be prepared by reacting a compound represented by formula (M-11-h) (hereinafter referred to as "Compound (M-11-h)") with ammonia.

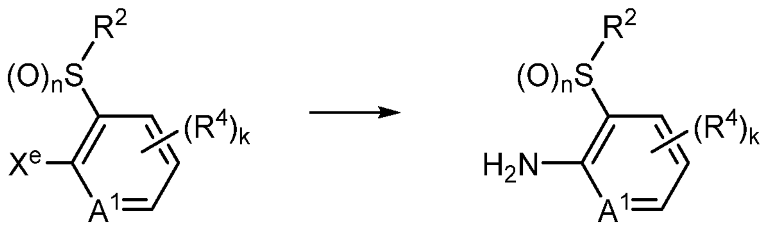

(M-11-h) (M-34)

[wherein the symbols are the same as defined above.]

The Compound (M-34) may be prepared according to the Reference Production method 8 by using the Compound (M-11-h) instead of the Compound (M-2-h).

Reference Production method 25

The Compound (M-11-n0) may be prepared according to the following scheme.

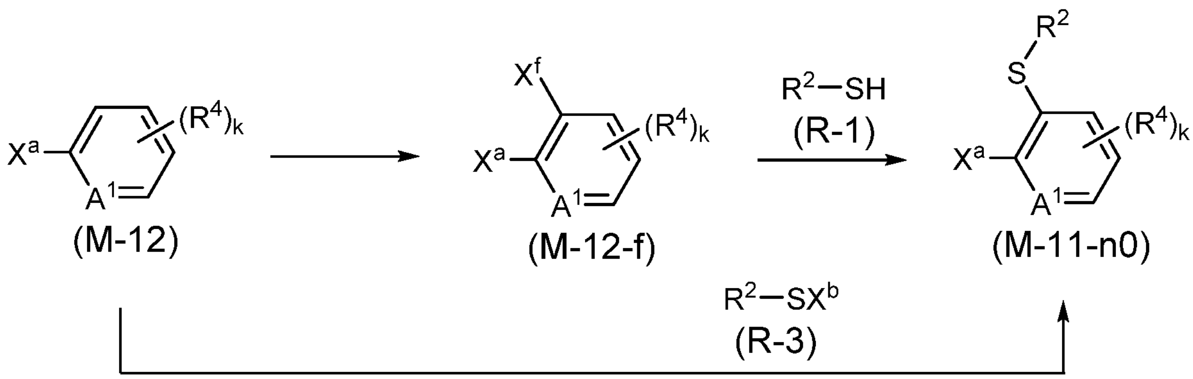


[wherein the symbols are the same as defined above.]

These reactions may be carried out according to the Reference Production method 20.

Reference Production method 26

The Compound (M-17-OH) may be prepared by reacting a compound represented by formula (M-17-P) (hereinafter referred to as "Compound (M-17-P)") with an acid.

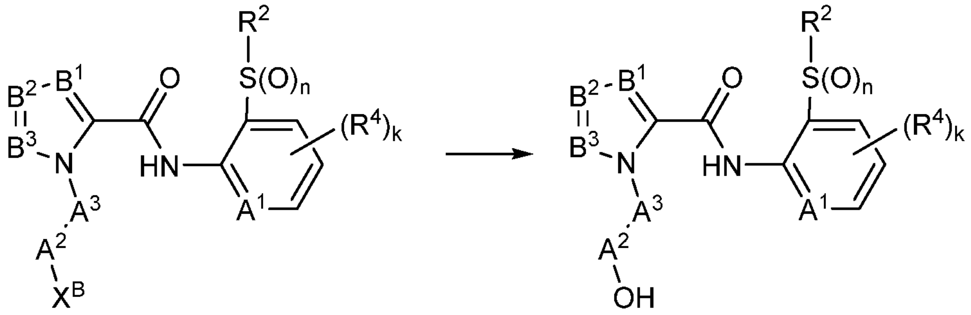

(M-17-P) (M-17-OH)

[wherein the symbols are the same as defined above.]

The Compound (M-17-OH) may be prepared according to the Reference Production method 11 by using the Compound (M-17-P) instead of the Compound (M-9-P).

Reference Production method 27

The Compound (M-17-P) may be prepared by reacting a compound represented by formula (M-28) (hereinafter referred to as "Compound (M-28)") with the Compound (M-34) in the presence of a condensing agent.

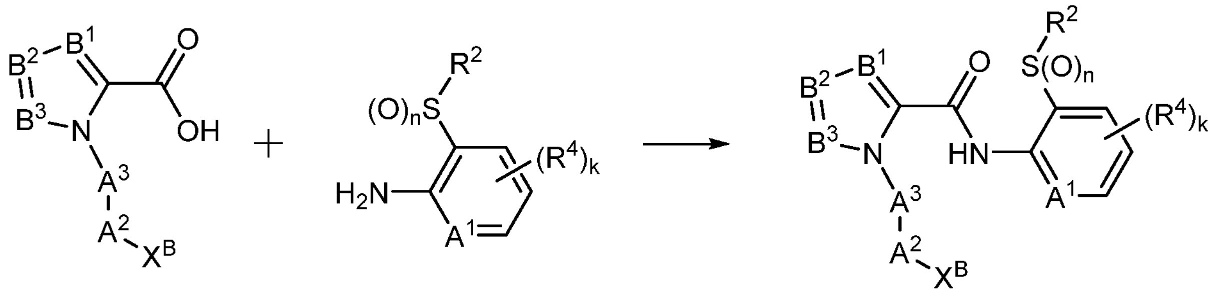

(M-28) (M-34) (M-17-P)

[wherein the symbols are the same as defined above.]

The Compound (M-17-P) may be prepared according to the Reference Production method 12 by using the Compound (M-34) instead of the Compound (M-24).

Reference Production method 28

The Compound (M-18-OH) may be prepared by reacting a compound represented by formula (M-18-P) (hereinafter referred to as "Compound (M-18-P)") with an acid.

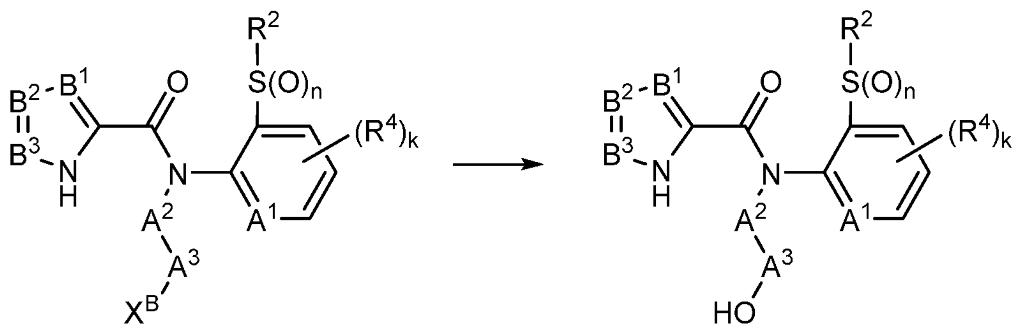

(M-18-P) (M-18-OH)

[wherein the symbols are the same as defined above.]

The Compound (M-18-OH) may be prepared according to the Reference Production method 11 by using the

Compound (M-18-P) instead of the Compound (M-9-P).

Reference Production method 29

The Compound (M-18-P) may be prepared by reacting the Compound (M-30) with a compound represented by formula (M-35) (hereinafter referred to as "Compound (M-35)") in the presence of a condensing agent.

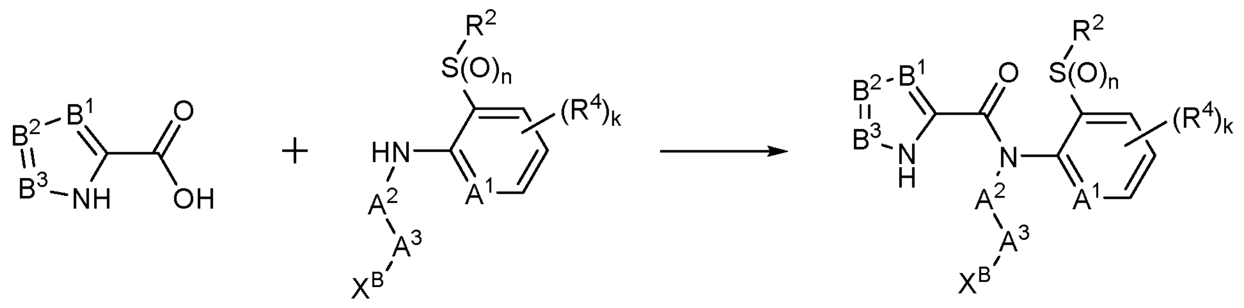

(M-30) (M-35) (M-18-P)

[wherein the symbols are the same as defined above.]

The Compound (M-18-P) may be prepared according to the Reference Production method 5 by using the Compound (M-30) instead of the Compound (M-23) and by using the Compound (M-35) instead of the Compound (M-24).

Reference Production method 30

The Compound (M-18-P) may also be prepared by reacting the Compound (M-32) with the Compound (M-35).

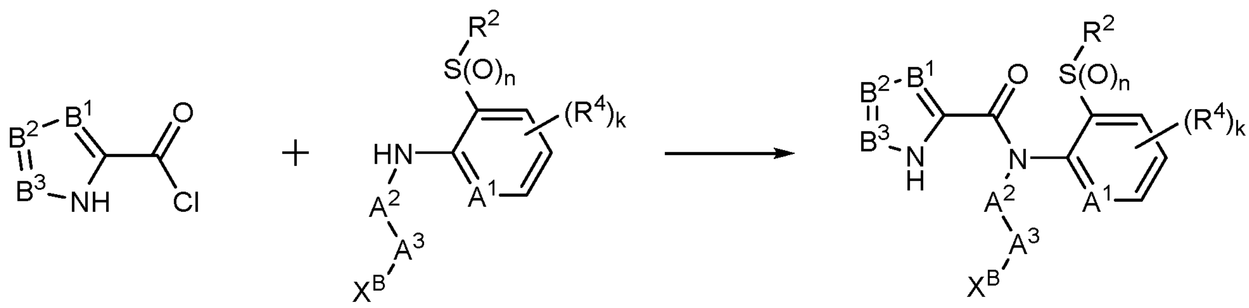

(M-32) (M-35) (M-18-P)

[wherein the symbols are the same as defined above.]

The Compound (M-18-P) may be prepared according to the Reference Production method 9 by using the Compound (M-32) instead of the Compound (M-27) and by using the Compound (M-35) instead of the Compound (M-24).

Reference Production method 31

The Compound (M-35) may be prepared by reacting the Compound (M-34) with the Compound (R-12) in the presence of a base.

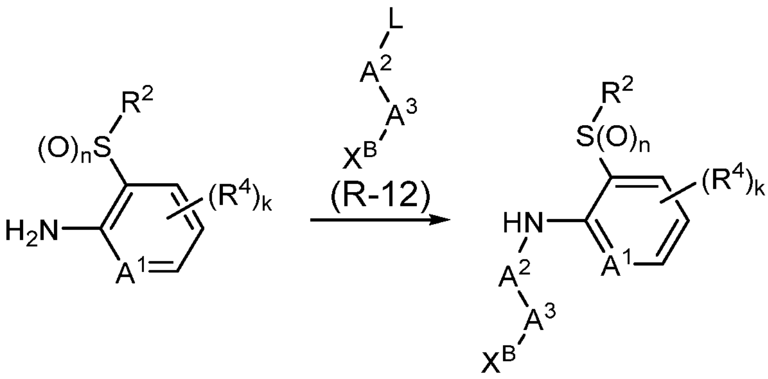

(M-34) (M-35)

[wherein the symbols are the same as defined above.]

The Compound (M-35) may be prepared according to the Reference Production method 19 by using the Compound (M-34) instead of the Compound (M-24).

Reference Production method 32

The Compound (M-18-X) may be prepared by reacting the Compound (M-18-OH) in the presence of a halogenating agent.

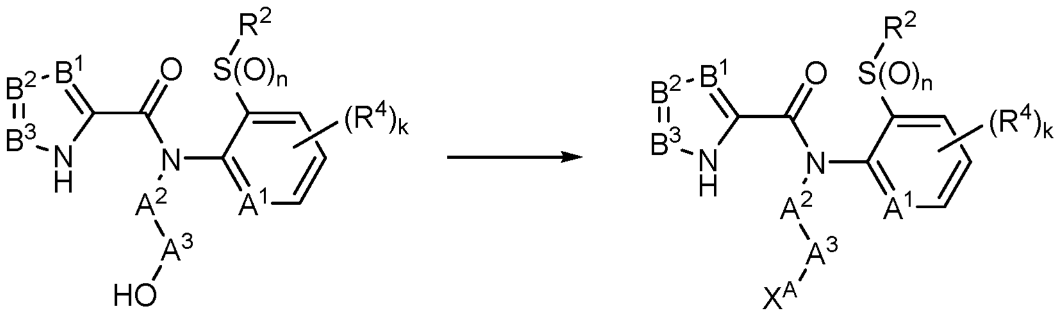

(M-18-OH) (M-18-X)

[wherein the symbols are the same as defined above.]

The Compound (M-18-X) may be prepared according to the Reference Production method 15 by using the Compound (M-18-OH) instead of the Compound (M-10-OH).

The Compound (M-2) may also be prepared according to the method(s) described in, for example, WO 2020/158889 pamphlet.

Reference Production method 33

The Compound (M-1) may also be prepared according to the following scheme.

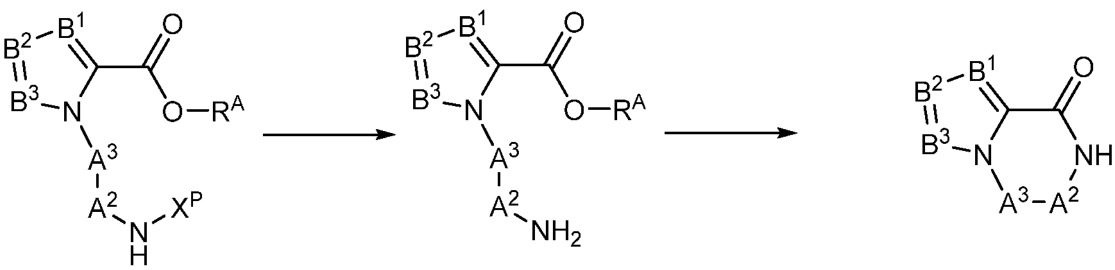

(M-29-N-PG) (M-29-N) (M-1)

[wherein $X^P$ represents a protecting group such as a tert-butoxycarbonyl group; and the other symbols are the same as defined above.]

First, a method for producing a compound represented by formula (M-29-N) (hereinafter referred to as "Compound (M-29-N)") from a compound represented by formula (M-29-N-PG) (hereinafter referred to as "Compound (M-29-N-PG)") is described.

The Compound (M-29-N) may be prepared by reacting the Compound (M-29-N-PG) with an acid.

The reaction is usually carried out in a solvent. Examples of the solvent to be used in the reaction include ethers, aromatic hydrocarbons, nitriles, aprotic polar solvents, and mixtures of two or more of them.

Examples of the acid to be used in the reaction include mineral acids such as hydrochloric acid, nitric acid, phosphoric acid, and sulfuric acid; and carboxylic acids such as acetic acid and trifluoroacetic acid.

In the reaction, the acid is usually used at a ratio of 1 to 30 mol relative to 1 mol of the Compound (M-29-N-PG).

The reaction temperature is usually within the range of 0 to 100°C. The reaction time is usually within the range of 0.1 to 24 hour(s).

When the reaction is completed, the reaction mixture may be subjected to a work-up such as concentrating the reaction mixture to give the Compound (M-29-N).

Next, a method for producing the Compound (M-1) from the Compound (M-29-N) is described.

The Compound (M-1) may be prepared by reacting the Compound (M-29-N) with a base.

The reaction is usually carried out in a solvent. Examples of the solvent to be used in the reaction include ethers, aromatic hydrocarbons, nitriles, aprotic polar solvents, water, and mixtures of two or more of them.

Examples of the base to be used in the reaction include alkali metal carbonates.

In the reaction, the base is usually used at a ratio of 1 to 30 mol relative to 1 mol of the Compound (M-29-N).

The reaction temperature is usually within the range of 0 to 100°C. The reaction time is usually within the range of 0.1 to 24 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (M-1).

The Present compound may be mixed with or used in combination with one or more ingredient(s) selected from the group consisting of the following Group (a), Group (b), Group (c), and Group (d) (hereinafter referred to as "Present ingredient").

When the Present compound is mixed with or used in combination with the Present ingredient, they are used simultaneously, separately, or at time intervals with each other.

When the Present compound is used simultaneously with the Present ingredient, the Present compound and the Present ingredient may be contained in separate formulations or contained in one formulation.

One aspect of the present invention provides a composition comprising one or more ingredient(s) selected from the group consisting of Group (a), Group (b), Group (c), and Group (d) (i.e., Present ingredient), and the Present compound (hereinafter referred to as "Composition A").

Group (a) is a group consisting of acetylcholinesterase inhibitors (for example, carbamate insecticides and organophosphate insecticides), GABA-gated chloride channel blockers (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mite growth inhibitors, microbial disruptors of insect midgut membranes, inhibitors of mitochondrial ATP synthase, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, mitochondrial complexes I, II, III, and IV electron transport inhibitors, voltage-dependent sodium channel blockers, inhibitors of acetyl CoA carboxylase, ryanodine receptor modulators (for example, diamide insecticides), chordotonal organ modulators, and microbial insecticides, and other insecticidal active ingredients, miticidal active ingredients, and nematicidal active ingredients. These ingredients are described in the classification on the basis of action mechanism by IRAC.

Group (b) is a group consisting of nucleic acids synthesis inhibitors (for example, phenylamide fungicides and acylamino acid fungicides), cell division and cytoskeleton inhibitors (for example, MBC fungicides), respiration inhibitors (for example, QoI fungicides and QiI fungicides), amino acids synthesis and protein synthesis inhibitors (for example, anilino-pyrimidine fungicides), signal transduction inhibitors, lipid synthesis and membrane synthesis inhibitors, sterol biosynthesis inhibitors (for example, DMI fungicides such as triazole fungicides), cell wall biosynthesis inhibitors, melanin synthesis inhibitors, plant defense inducers, fungicides with multi-site contact activity, microbial fungicides, and other fungicidal active ingredients. These ingredients are described in the classification on the basis of action mechanism by FRAC.

Group (c) is a group of plant growth regulatory ingredients (including mycorrhizal fungi and root nodule bacteria).

Group (d) is a group of repellent ingredients.

Hereinafter, examples of the combination of the Present ingredient and the Present compound are described. For example, "alanycarb + SX" indicates a combination of alanycarb and SX.

The abbreviation of "SX" indicates any one of the Present compound selected from the Compound groups SX1 to SX1840. Also, all of the following Present ingredient are known ingredients, and may be obtained from commercially available formulations, or may be prepared by known methods. When the Present ingredient is a microorganism, it may also be available from a bacterial authority depository. Further, the number in parentheses represents the CAS RN (registered trademark).

Combinations of the Present ingredient in the above Group (a) and the Present compound:

abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC(2-methyl-4,6-dinitrophenol) + SX, doramectin + SX, dried leaves of Dryopteris filix-mas + SX, emamectin-benzoate + SX, empenthrin + SX, endosulfan + SX, EPN(O-ethyl O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, flupyroxystrobin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hvla peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, ledprona + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX,

sulfiflumin + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, N-[4-chloro-2-(pyridin-3-yl)-1,3-thiazol-5-yl]-N-ethyl-3-(methanesulfonyl)propanamide + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, BT crop protein Cry1Ab + SX, BT crop protein CrylAc + SX, BT crop protein CrylFa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, Adoxophyes orana GV(granulovirus) strain BV-0001 + SX, Anticarsia gemmatalis MNPV(multiple nucleocapsid nucleopolyhedrovirus) + SX, Autographa californica MNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV(nucleopolyhedrovirus) strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua MNPV + SX, Spodoptera littoralis MNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306 + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX,

Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

Combination of the Present ingredient in the above Group (b) and the Present compound:
acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Allium sativum + SX, extract of the cotyledons of lupine plantlets ("BLAD") + SX, extract of Equisetum arvense + SX, extract of Melaleuca alternifolia + SX, extract of Reynoutria sachalinensis + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl] ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl) butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl) carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl} methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl) pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)amino]-N-(2,2-dimethylcyclobutyl)-5-methylthiazole-4-carboxamide + SX, 2-[(2,6-difluoropyridin-4-yl)amino]-N-(2,2-dimethylcyclobutyl)-5-methylthiazole-4-carboxamide + SX, 2-{[(oxetan-3-yl)carbonyl](2,6-difluoropyridin-4-yl)amino}-N-(2,2-dimethylcyclobutyl)-5-methylthiazole-4-carboxamide + SX, 2-{[(oxolan-3-yl)carbonyl](2,6-difluoropyridin-4-yl)amino}-N-(2,2-dimethylcyclobutyl)-5-methylthiazole-4-carboxamide + SX, 2-{[(oxan-4-yl)carbonyl](2,6-difluoropyridin-4-yl)amino}-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2-methylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2,2-dimethylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(5-fluoro-2-methoxyphenyl)-2-(2-cyanoethoxy)ethyl]-5-[1-(isopropoxyimino)ethyl]-2,6-dioxo-3,6-dihydropyrimidin-1(2H)-yl}propan-2-yl)-2-methylpropanamide + SX, methyl ({5-[1-(2,6-difluoro-4-isopropylphenyl)-1H-pyrazol-3-yl]-2-methylphenyl}methyl)carbamate + SX, methyl ({5-[1-(2,6-difluoro-4-cyclopropylphenyl)-1H-pyrazol-3-yl]-2-methylphenyl}methyl)carbamate + SX, methyl ({5-[1-(2,6-difluoro-4-methoxyphenyl)-1H-pyrazol-3-yl]-2-methylphenyl}methyl)carbamate + SX, methyl (Z)-2-(5-cyclopentyl-2-methylphenoxy)-3-methoxyprop-2-enoate + SX, methyl (Z)-2-(5-cyclohexyl-2-methylphenoxy)-3-methoxyprop-2-enoate + SX, methyl (Z)-2-[(3-isopropyl-1H-pyrazol-1-yl)-2-methylphenoxy]-3-methoxyprop-2-enoate + SX, 1-(4,5-dimethyl-1H-benzimidazol-1-yl)-4,4-difluoro-3,3-dimethyl-3,4-dihydroisoquinoline + SX, 1-(4,5-dimethyl-1H-benzimidazol-1-yl)-4,4,5-trifluoro-3,3-dimethyl-3,4-dihydroisoquinoline + SX, 1-(pyrazolo[1,5-a]pyridin-3-yl)-4,4-difluoro-3,3-dimethyl-3,4-dihydroisoquinoline + SX, 1-(6,7-dimethylpyrazolo[1,5-a]pyridin-3-yl)-6-fluoro-3,3-dimethylisoquinolin-4(3H)-one + SX, methyl (2Z)-3-methoxy-2-[(4-methyl[1,1'-biphenyl]-3-yl)oxy]prop-2-enoate + SX, Agrobacterium radiobactor strain K1026 + SX, Agrobacterium radiobactor strain K84 + SX, Bacillus amyloliquefaciens strain

PTA-4838 (Aveo (registered trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 +

SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

Combination of the Present ingredient in the above Group (c) and the Present compound:
1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butylate + SX, methyl 5-(trifluoromethyl)benzo[b]thiophene-2-carboxylate + SX, 3-[(6-chloro-4-phenylquinazolin-2-yl)amino]propan-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus

irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combination of the Present ingredient in the above Group (d) and the Present compound:
anthraquinone + SX, deet + SX, icaridin + SX.

The ratio of the Present compound to the Present ingredient includes, but not limited thereto, as a ratio by weight (the Present compound : the Present ingredient) 1,000 : 1 to 1 : 1,000, 500 : 1 to 1 : 500, 100 : 1 to 1 : 100, 50 : 1, 20 : 1, 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9, 1 : 10, 1 : 20, 1 : 50, and the others.

The Present compound can control harmful arthropods such as harmful insects and harmful mites, harmful nematodes, and harmful mollusks. Examples of the harmful arthropods, harmful nematodes, and harmful mollusks include the followings.

Hemiptera:

from the family Delphacidae, for example, small brown planthopper (Laodelphax striatellus), brown planthopper (Nilaparvata lugens), white-backed planthopper (Sogatella furcifera), corn planthopper (Peregrinus maidis), cereal leafhopper (Javesella pellucida), sugarcane leafhopper (Perkinsiella saccharicida), Tagosodes orizicolus, and Stenocranus pacificus;

from the family Cicadellidae, for example, green rice leafhopper (Nephotettix cincticeps), green paddy leafhopper (Nephotettix virescens), rice leafhopper (Nephotettix nigropictus), zigzag-striped leafhopper (Recilia dorsalis), tea green leafhopper (Empoasca onukii), potato leafhopper (Empoasca fabae), corn leafhopper (Dalbulus maidis), rice leafhopper (Cofana spectra), and Amrasca biguttula biguttula;

from the family Aphrophoridae, for example, European spittlebug (Philaenus spumarius);

from the family Cercopidae, for example, Mahanarva posticata and Mahanarva fimbriolata;

from the family Aphididae, for example, bean aphid (Aphis fabae), soybean aphid (Aphis glycines), cotton aphid (Aphis gossypii), green apple aphid (Aphis pomi), apple aphid (Aphis spiraecola), green peach aphid (Myzus persicae), leaf-curling plum aphid (Brachycaudus helichrysi), cabbage aphid (Brevicoryne brassicae), rosy apple aphid (Dysaphis plantaginea), false cabbage aphid (Lipaphis erysimi), potato aphid (Macrosiphum euphorbiae), foxglove aphid (Aulacorthum solani), lettuce aphid (Nasonovia ribisnigri), grain aphid (Rhopalosiphum padi), corn aphid (Rhopalosiphum maidis), brown citrus aphid (Toxoptera citricida), mealy plum aphid (Hyalopterus pruni), cane aphid (Melanaphis sacchari), black rice root aphid (Tetraneura nigriabdominalis), sugarcane cottony aphid (Ceratovacuna lanigera), apple woolly aphid (Eriosoma lanigerum), and English grain aphid (Sitobion avenae) ;

from the family Phylloxeridae, for example, grapevine phylloxera (Daktulosphaira vitifoliae), Pecan phylloxera (Phylloxera devastatrix), Pecan leaf phylloxera (Phylloxera notabilis), and Southern pecan leaf phylloxera (Phylloxera russelae);

from the family Adelgidae, for example, hemlock woolly aphid (Adelges tsugae), balsam woolly aphid (Adelges piceae), and Aphrastasia pectinatae;

from the family Pentatomidae, for example, black rice bug (Scotinophara lurida), black paddy bug (Scotinophara coarctata), common green stink bug (Nezara antennata), white-spotted spined bug (Eysarcoris aeneus), lewis spined bug (Eysarcoris lewisi), white-spotted bug (Eysarcoris ventralis), Eysarcoris annamita, brown marmorated stink bug (Halyomorpha halys), green plant bug (Nezara viridula), brown stink bug (Euschistus heros), red-banded stink bug (Piezodorus guildinii), Oebalus pugnax, Dichelops melacanthus, and red-banded shield bug (Piezodorus hybneri);

from the family Cydnidae, for example, Scaptocoris castanea;

from the family Alydidae, for example, bean bug (Riptortus clavatus), corbett rice bug (Leptocorisa chinensis), and rice bug (Leptocorisa acuta);

from the family Coreidae, for example, Cletus punctiger and Australian leaf-footed bug (Leptoglossus australis);

from the family Lygaeidae, for example, oriental chinch bug (Cavelerius saccharivorus), seed bug (Togo hemipterus), and chinch bug (Blissus leucopterus);

from the family Miridae, for example, rice leaf bug (Trigonotylus caelestialium), sorghum plant bug (Stenotus rubrovittatus), wheat leaf bug (Stenodema calcarata), and American tarnished plant bug (Lygus lineolaris);

from the family Aleyrodidae, for example, greenhouse whitefly (Trialeurodes vaporariorum), tobacco whitefly (Bemisia tabaci), citrus whitefly (Dialeurodes citri), citrus spiny whitefly (Aleurocanthus spiniferus), tea spiny whitefly (Aleurocanthus camelliae), and Pealius euryae;

from the family Diaspididae, for example, Abgrallaspis cyanophylli, red scale (Aonidiella aurantii), San José scale (Diaspidiotus perniciosus), white peach scale (Pseudaulacaspis pentagona), arrowhead scale (Unaspis yanonensis), and citrus snow scale (Unaspis citri);
from the family Coccidae, for example, pink wax scale (Ceroplastes rubens);
from the family Margarodidae, for example, fluted scale (Icerya purchasi) and seychelles fluted scale (Icerya seychellarum);
from the family Pseudococcidae, for example, solanum mealybug (Phenacoccus solani), cotton mealybug (Phenacoccus solenopsis), Japanese mealybug (Planococcus kraunhiae), white peach scale (Pseudococcus comstocki), citrus mealybug (Planococcus citri), currant mealybug (Pseudococcus calceolariae), long-tailed mealybug (Pseudococcus longispinus), and tuttle mealybug (Brevennia rehi);
from the family Psyllidae, for example, citrus psylla (Diaphorina citri), two-spotted citrus psyllid (Trioza erytreae), pear sucker (Cacopsylla pyrisuga), Cacopsylla chinensis, potato psyllid (Bactericera cockerelli), and Pear psylla (Cacopsylla pyricola);
from the family Tingidae, for example, sycamore lace bug (Corythucha ciliata), aster tingid (Corythucha marmorata), Japanese pear lace bug (Stephanitis nashi), and azalea lace bug (Stephanitis pyrioides);
from the family Cimicidae, for example, common bed bug (Cimex lectularius) and tropical bed bug (Cimex hemipterus);
from the family Cicadidae, for example, Quesada gigas;
from the family Reduviidae, for example, Triatoma infestans, large kissing bug (Triatoma rubrofasciata), Triatoma dimidiata, and Rhodonius prolixus;

and the others.

Lepidoptera:

from the family Crambidae, for example, rice stem borer (Chilo suppressalis), Dark-headed stem borer (Chilo polychrysus), white stem borer (Scirpophaga innotata), yellow paddy borer (Scirpophaga incertulas), Rupela albina, rice leaf roller (Cnaphalocrocis medinalis), Marasmia patnalis, rice leaf roller (Marasmia exigua), cotton leaf roller (Notarcha derogata), corn borer (Ostrinia furnacalis), European corn borer (Ostrinia nubilalis), cabbage webworm (Hellula undalis), grape leafroller (Herpetogramma luctuosale), bluegrass webworm (Parapediasia teterrellus), rice case-worm (Nymphula depunctalis), Sugarcane borer (Diatraea saccharalis), and eggplant fruit borer (Leucinodes orbonalis);
from the family Pyralidae, for example, lesser cornstalk borer (Elasmopalpus lignosellus), mealworm moth (Plodia interpunctella), persimmon bark borer (Euzophera batangensis), and fig moth (Cadra cautella);
from the family Noctuidae, for example, cotton worm (Spodoptera litura), beet armyworm (Spodoptera exigua), rice armyworm (Mythimna separata), cabbage moth (Mamestra brassicae), pink borer (Sesamia inferens), grass armyworm (Spodoptera mauritia), green rice caterpillar (Naranga aenescens), fall armyworm (Spodoptera frugiperda), true armyworm (Spodoptera exempta), semitropical armyworm (Spodoptera eridania), black cutworm (Agrotis ipsilon), turnip moth (Agrotis segetum), beet worm (Autographa nigrisigna), rice looper (Plusia festucae), Soybean looper (Chrysodeixis includens), Trichoplusia spp., Heliothis spp. (such as tobacco budworm (Heliothis virescens)), Helicoverpa spp. (such as tobacco budworm (Helicoverpa armigera), corn earworm (Helicoverpa zea)), velvet-bean caterpillar (Anticarsia gemmatalis), cotton leafworm (Alabama argillacea), and hop vine borer (Hydraecia immanis);
from the family Pieridae, for example, common cabbage worm (Pieris rapae);
from the family Tortricidae, for example, oriental fruit moth (Grapholita molesta), Grapholita dimorpha, soybean moth (Leguminivora glycinivorella), Matsumuraeses azukivora, summer fruit tortrix (Adoxophyes orana fasciata), smaller tea tortrix (Adoxophyes honmai), Japanese tea tortrix (Homona magnanima), apple tortrix (Archips fuscocupreanus), codling moth (Cydia pomonella), sugarcane shoot borer (Tetramoera schistaceana), bean shoot borer (Epinotia aporema), citrus fruit borer (Citripestis sagittiferella), and European grapevine moth (Lobesia botrana);
from the family Gracillariidae, for example, tea leaf roller (Caloptilia theivora) and Asiatic apple leaf miner (Phyllonorycter ringoniella);
from the family Carposinidae, for example, peach fruit moth (Carposina sasakii);
from the family Lyonetiidae, for example, coffee leaf miner (Leucoptera coffeella), peach leaf miner (Lyonetia clerkella), and Lyonetia prunifoliella;
from the family Lymantriidae, for example, Lymantria spp. (such as gypsy moth (Lymantria dispar)) and Euproctis spp. (such as tea lymantriid (Euproctis pseudoconspersa));
from the family Plutellidae, for example, diamondback moth (Plutella xylostella);
from the family Gelechiidae, for example, peach worm (Anarsia lineatella), sweetpotato leaf folder (Helcystogramma triannulella), pink bollworm (Pectinophora gossypiella), potato moth (Phthorimaea operculella), and South American tomato moth (Tuta absoluta);

from the family Arctiidae, for example, American white moth (Hyphantria cunea);
from the family Castniidae, for example, giant sugarcane borer (Telchin licus);
from the family Cossidae, for example, Cossus insularis;
from the family Geometridae, for example, Ascotis selenaria;
from the family Limacodidae, for example, blue-striped nettle grub (Parasa lepida);
from the family Stathmopodidae, for example, persimmon fruit moth (Stathmopoda masinissa);
from the family Sphingidae, for example, tobacco hornworm (Acherontia lachesis);
from the family Sesiidae, for example, Nokona feralis, cherry borer (Synanthedon hector), and Synanthedon tenuis;
from the family Hesperiidae, for example, rice skipper (Parnara guttata);
from the family Tineidae, for example, casemaking clothes moth (Tinea translucens), common clothes moth (Tineola bisselliella);

and the others.

Thysanoptera:

from the family Thripidae, for example, western flower thrips (Frankliniella occidentalis), oriental thrips (Thrips palmi), yellow tea thrips (Scirtothrips dorsalis), onion thrips (Thrips tabaci), eastern flower thrips (Frankliniella intonsa), rice thrips (Stenchaetothrips biformis), Echinothrips americanus, and avocado thrips (Scirtothrips perseae);
from the family Phlaeothripidae, for example, aculeated rice thrips (Haplothrips aculeatus);

and the others.

Diptera:

from the family Anthomyiidae, for example, seedcorn maggot (Delia platura), onion maggot (Delia antiqua), and beet leaf miner (Pegomya cunicularia);
from the family Ulidiidae, for example, sugarbeet root maggot (Tetanops myopaeformis);
from the family Agromyzidae, for example, rice leaf miner (Agromyza oryzae), tomato leaf miner (Liriomyza sativae), chrysanthemum leaf miner (Liriomyza trifolii), and pea leafminer (Chromatomyia horticola);
from the family Chloropidae, for example, rice stem maggot (Chlorops oryzae);
from the family Tephritidae, for example, melon fly (Bactrocera cucurbitae), oriental fruit fly (Bactrocera dorsalis), Malaysian fruit fly (Bactrocera latifrons), olive fruit fly (Bactrocera oleae), Queensland fruit fly (Bactrocera tryoni), Mediterranean fruit fly (Ceratitis capitata), apple maggot (Rhagoletis pomonella), and Japanese cherry fruit fly (Rhacochlaena japonica);
from the family Ephydridae, for example, smaller rice leaf miner (Hydrellia griseola), whorl maggot (Hydrellia philippina), and paddy stem maggot (Hydrellia sasakii);
from the family Drosophilidae, for example, cherry drosophila (Drosophila suzukii), and common fruit fly (Drosophila melanogaster);
from the family Phoridae, for example, Megaselia spiracularis;
from the family Psychodidae, for example, Clogmia albipunctata;
from the family Sciaridae, for example, Bradysia difformis and sciarid fly (Bradysia odoriphaga);
from the family Cecidomyiidae, for example, hessian fly (Mayetiola destructor) and paddy gall fly (Orseolia oryzae);
from the family Diopsidae, for example, Diopsis macrophthalma;
from the family Glossinidae, for example, Glossina palpalis and Glossina morsitans;
from the family Simuliidae, for example, Simulium japonicum and Simulium damnosum;
from the subfamily Phlebotominae;
from the family Tipulidae, for example, rice crane fly (Tipula aino), common cranefly (Tipula oleracea), and European cranefly (Tipula paludosa);
from the family Culicidae, for example, southern house mosquito (Culex pipiens pallens), Culex tritaeniorhynchus, Culex pipiens f. molestus, brown house mosquito (Culex quinquefasciatus), northern house mosquito (Culex pipiens pipiens), Culex vishnui, Asian tiger mosquito (Aedes albopictus), dengue mosquito (Aedes aegypti), Chinese malaria mosquito (Anopheles sinensis), Anopheles gambiae, Anopheles stephensi, Anopheles coluzzii, Anopheles albimanus, Anopheles sundaicus, Anopheles arabiensis, Anopheles funestus, Anopheles darlingi, Anopheles farauti, and Anopheles minimus;
from the family Simulidae, for example, Prosimulium yezoensis and Simulium ornatum;
from the family Tabanidae, for example, Tabanus trigonus;
from the family Muscidae, for example, house fly (Musca domestica), false stable fly (Muscina stabulans), biting house fly (Stomoxys calcitrans), and buffalo fly (Haematobia irritans);
from the family Calliphoridae;

from the family Sarcophagidae;
from the family Chironomidae, for example, Chironomus plumosus, Chironomus yoshimatsui, and Glyptotendipes tokunagai;
from the family Fannidae;

and the others.

Coleoptera:

from the family Chrysomelidae, for example, Diabrotica spp. (such as western corn rootworm (Diabrotica virgifera virgifera), southern corn rootworm (Diabrotica undecimpunctata howardi), northern corn rootworm (Diabrotica barberi), Mexican corn rootworm (Diabrotica virgifera zeae), banded cucumber beetle (Diabrotica balteata), cucurbit beetle (Diabrotica speciosa)), bean leaf beetle (Cerotoma trifurcata), barley leaf beetle (Oulema melanopus), cucurbit leaf beetle (Aulacophora femoralis), striped flea beetle (Phyllotreta striolata), cabbage flea beetle (Phyllotreta cruciferae), western black flea beetle (Phyllotreta pusilla), cabbage stem flea beetle (Psylliodes chrysocephala), hop flea beetle (Psylliodes punctulata), Colorado potato beetle (Leptinotarsa decemlineata), rice leaf beetle (Oulema oryzae), grape colaspis (Colaspis brunnea), corn flea beetle (Chaetocnema pulicaria), sweet-potato flea beetle (Chaetocnema confinis), potato flea beetle (Epitrix cucumeris), rice leaf beetle (Dicladispa armigera), southern corn leaf beetle (Myochrous denticollis), Laccoptera quadrimaculata, tobacco flea beetle (Epitrix hirtipennis), brassica leaf beetle (Phaedon brassicae), and two-striped leaf beetle (Medythia nigrobilineata);
from the family Carabidae, for example, Seedcorn beetle (Stenolophus lecontei) and slender seed-corn ground beetle (Clivina impressifrons);
from the family Scarabaeidae, for example, cupreus chafer (Anomala cuprea), soybean beetle (Anomala rufocuprea), Anomala albopilosa, Japanese beetle (Popillia japonica), yellowish elongate chafer (Heptophylla picea), European Chafer (Rhizotrogus majalis), Tomarus gibbosus, Holotrichia spp., Phyllophaga spp. (such as June beetle (Phyllophaga crinita)), and Diloboderus spp. (such as Diloboderus abderus);
from the family Anthriibidae, for example, coffee bean weevil (Araecerus coffeae);
from the family Aponidae, for example, sweet-potato weevil (Cylas formicarius);
from the family Bruchidae, for example, Mexican bean weevil (Zabrotes subfasciatus);
from the family Scolytidae, for example, pine beetle (Tomicus piniperda) and coffee berry borer (Hypothenemus hampei) ;
from the family Curculionidae, for example, West Indian sweet-potato weevil (Euscepes postfasciatus), alfalfa weevil (Hypera postica), maize wevil (Sitophilus zeamais), rice weevil (Sitophilus oryzae), grain weevil (Sitophilus granarius), rice plant weevil (Echinocnemus squameus), rice water weevil (Lissorhoptrus oryzophilus), Rhabdoscelus lineaticollis, boll weevil (Anthonomus grandis), nunting billbug (Sphenophorus venatus), southern corn billbug (Sphenophorus callosus), soybean stalk weevil (Sternechus subsignatus), sugarcane weevil (Sphenophorus levis), rusty gourd-shaped weevil (Scepticus griseus), brown gourd-shaped weevil (Scepticus uniformis), Aracanthus spp. (such as Aracanthus mourei), and cotton root borer (Eutinobothrus brasiliensis);
from the family Tenebrionidae, for example, red meal beetle (Tribolium castaneum), mason beetle (Tribolium confusum), and lesser mealworm (Alphitobius diaperinus);
from the family Coccinellidae, for example, twenty-eight-spotted ladybird (Epilachna vigintioctopunctata);
from the family Bostrychidae, for example, common powder-post beetle (Lyctus brunneus), and lesser grain borer (Rhizopertha dominica);
from the family Ptinidae;
from the family Cerambycidae, for example, citrus long-horned beetle (Anoplophora malasiaca), Migdolus fryanus, and peach borer (Aromia bungii);
from the family Elateridae, for example, Melanotus okinawensis, barley wireworm (Agriotes fuscicollis), Melanotus legatus, Anchastus spp., Conoderus spp., Ctenicera spp., Limonius spp., and Aeolus spp.;
from the family Staphylinidae, for example, Paederus fuscipes;
from the family Dermestidae, for example, varied carpet beetle (Anthrenus verbasci), hide beetle (Dermestes maculates), and khapra beetle (Trogoderma granarium);
from the family Anobiidae, for example, tobacco beetle (Lasioderma serricorne), and biscuit beetle (Stegobium paniceum);
from the family Laemophloeidae, for example, flat grain beetle (Cryptolestes ferrugineus);
from the family Silvanidae, for example, saw-toothed grain beetle (Oryzaephilus surinamensis);
from the family Nitidulidae, for example, blossom beetle (Brassicogethes aeneus);

and the others.

Orthoptera:

from the family Acrididae, for example, oriental migratory locust (Locusta migratoria), Moroccan locust (Dociostaurus maroccanus), Australian plague locust (Chortoicetes terminifera), red locust (Nomadacris septemfasciata), brown locust (Locustana pardalina), tree locust (Anacridium melanorhodon), Italian locust (Calliptamus italicus), differential grasshopper (Melanoplus differentialis), two-striped grasshopper (Melanoplus bivittatus), migratory grasshopper (Melanoplus sanguinipes), red-legged grasshopper (Melanoplus femurrubrum), clear-winged grasshopper (Camnula pellucida), desert locust (Schistocerca gregaria), Yellow-winged locust (Gastrimargus musicus), spur-throated locust (Austracris guttulosa), Japanese grasshopper (Oxya yezoensis), rice grasshopper (Oxya japonica), and Bombay locust (Patanga succincta);
from the family Gryllotalpidae, for example, oriental mole cricket (Gryllotalpa orientalis);
from the family Gryllidae, for example, house cricket (Acheta domestica), and emma field cricket (Teleogryllus emma);
from the family Tettigoniidae, for example, mormon cricket (Anabrus simplex);

and the others.
Hymenoptera:

from the family Tenthredinidae, for example, beet sawfly (Athalia rosae) and nippon cabbage sawfly (Athalia japonica);
from the family Formicidae, for example, Solenopsis spp. (such as red imported fire ant (Solenopsis invicta), tropical fire ant (Solenopsis geminata)), Atta spp. (such as brown leaf-cutting ant (Atta capiguara), Acromyrmex spp., Paraponera clavata, black house ant (Ochetellus glaber), little red ant (Monomorium pharaonis), Argentine ant (Linepithema humile), Formica japonica, Pristomyrmex punctutus, Pheidole noda, big-headed ant (Pheidole megacephala), Camponotus spp. (such as Camponotus japonicus, Camponotus obscuripes), Pogonomyrmex spp. (such as western harvester ant (Pogonomyrmex occidentalis)), Wasmania spp. (such as Wasmania auropunctata), and long-legged ant (Anoplolepis gracilipes);
from the family Vespidae, for example, Asian giant hornet (Vespa mandarinia), Vespa simillima, Vespa analis, Asian hornet (Vespa velutina), and Polistes jokahamae;
from the family Siricidae, for example, pine wood wasp (Urocerus gigas);
from the family Bethylidae;

and the others.
Blattodea:

from the family Ectobiidae, for example, German cockroach (Blattella germanica);
from the family Blattidae, for example, smoky-brown cockroach (Periplaneta fuliginosa), American cockroach (Periplaneta americana), Australian cockroach (Periplaneta australasiae), brown cockroach (Periplaneta brunnea), and black cockroach (Blatta orientalis);
from the family Termitidae, for example, Japanese termite (Reticulitermes speratus), Formosan termite (Coptotermes formosanus), western drywood termite (Incisitermes minor), Cryptotermes domesticus, Odontotermes formosanus, Neotermes koshunensis, Glyptotermes satsumensis, Glyptotermes nakajimai, Glyptotermes fuscus, Hodotermopsis sjostedti, Coptotermes guangzhouensis, Reticulitermes amamianus, Reticulitermes miyatakei, Reticulitermes kanmonensis, Nasutitermes takasagoensis, Pericapritermes nitobei, Sinocapritermes mushae, and Cornitermes cumulans;

and the others.
Siphonaptera:

from the family Pulicidae, for example, human flea (Pulex irritans), cat flea (Ctenocephalides felis), dog flea (Ctenocephalides canis), oriental rat flea (Xenopsylla cheopis), and chicken flea (Echidnophaga gallinacea);
from the family Pulicidae, for example, chigoe flea (Tunga penetrans);
from the family Ceratophyllidae, for example, European rat flea (Nosopsyllus fasciatus);

and the others.
Psocodae:

from the family Pediculidae, for example, head louse (Pediculus humanus capitis);
from the family Pthiridae, for example, crab louse (Pthirus pubis);
from the family Haematopinidae, for example, short-nosed cattle louse (Haematopinus eurysternus) and pig louse (Haematopinus suis);

from the family Linognathidae, for example, blue cattle louse (Linognathus vituli), sheep face louse (Linognathus ovillus), and capillate louse (Solenopotes capillatus);
from the family Bovicoliidae, for example, cattle biting louse (Bovicola bovis), sheep biting louse (Bovicola ovis), Bovicola breviceps, Damalinia forficula, and Werneckiella spp.;
from the family Trichodectidae, for example, dog biting louse (Trichodectes canis) and cat louse (Felicola subrostratus);
from the family Menoponidae, for example, common chicken louse (Menopon gallinae), chicken body louse (Menacanthus stramineus), and Trinoton spp.;
from the family Trimenoponidae, for example, Cummingsia spp.;
from the family Trogiidae, for example, death watch (Trogium pulsatorium);
from the family Liposcelidae or Liposcelididae, for example, book louse (Liposcelis corrodens), Liposcelis bostrychophila, Liposcelis pearmani, and Liposcelis entomophila;

and the other.
Thysanura:
from the family Lepismatidae, for example, oriental silverfish (Ctenolepisma villosa) and moth fish (Lepisma saccharina); and the others.
Acari:

from the family Tetranychidae, for example, common red spider mite (Tetranychus urticae), kanzawa spider mite (Tetranychus kanzawai), red spider mite (Tetranychus evansi), citrus red mite (Panonychus citri), fruit-tree red spider mite (Panonychus ulmi), and Oligonychus spp.;
from the family Eriophyidae, for example, Japanese citrus rust mite (Aculops pelekassi), Phyllocoptruta citri, tomato mite (Aculops lycopersici), purple mite (Calacarus carinatus), tea rust mite (Acaphylla theavagrans), Eriophyes chibaensis, apple bud mite (Aculus schlechtendali), Aceria diospyri, Aceria tosichella, and Shevtchenkella sp.;
from the family Tarsonemidae, for example, broad mite (Polyphagotarsonemus latus);
from the family Tenuipalpidae, for example, Brevipalpus phoenicis;
from the family Tuckerellidae;
from the family Ixodidae, for example, Haemaphysalis longicornis, Haemaphysalis flava, Haemaphysalis japonica, Haemaphysalis campanulata, American dog tick (Dermacentor variabilis), Dermacentor taiwanensis, Rocky Mountain wood tick (Dermacentor andersoni), netted tick (Dermacentor reticulatus), Ixodes ovatus, Ixodes persulcatus, black-legged tick (Ixodes scapularis), Ixodes pacificus, Ixodes holocyclus, Ixodes ricinus, lone star tick (Amblyomma americanum), gulf coast tick (Amblyomma maculatum), Rhipicephalus microplus, cattle tick (Rhipicephalus annulatus), brown dog tick (Rhipicephalus sanguineus), Rhipicephalus appendiculatus, and Rhipicephalus decoloratus;
from the family Argasidae, for example, fowl tick (Argas persicus), Ornithodoros hermsi, and Ornithodoros turicata;
from the family Acaridae, for example, cereal mite (Tyrophagus putrescentiae) and grassland mite (Tyrophagus similis);
from the family Pyroglyphidae, for example, American house dust mite (Dermatophagoides farinae) and European house dust mite (Dermatophagoides pteronyssinus);
from the family Cheyletidae, for example, Cheyletus eruditus, Cheyletus malaccensis, Chelacaropsis moorei, and Cheyletiella yasguri;
from the family Psoroptidae, for example, sheep scab mite (Psoroptes ovis), horse psoroptic mange mite (Psoroptes equi), Knemidocoptes mutans, ear mange mite (Otodectes cynotis), and Chorioptes spp.;
from the family Sarcoptidae, for example, Notoedres cati, Notoedres muris, and itch mite (Sarcoptes scabiei);
from the family Listrophoridae, for example, Listrophorus gibbus;
from the family Dermanyssidae, for example, bird mite (Dermanyssus gallinae);
from the family Macronyssidae, for example, feather mite (Ornithonyssus sylviarum) and tropical rat mite (Ornithonyssus bacoti);
from the family Varroidae, for example, Varroa jacobsoni;
from the family Demodicidae, for example, dog follicle mite (Demodex canis) and cat follicle mite (Demodex cati);
from the family Trombiculidae, for example, Leptotrombidium akamushi, Leptotrombidium pallidum, and Leptotrombidium scutellare;

and the others.
Araneae:

from the family Eutichuridae, for example, Cheiracanthium japonicum;
from the family Theridiidae, for example, red-back spider (Latrodectus hasseltii);

and the others.

Polydesmida:

from the family Paradoxosomatidae, for example, flat-backed millipede (Oxidus gracilis) and Nedyopus tambanus; and the others.

Isopoda:

from the family Armadillidiidae, for example, common pill bug (Armadillidium vulgare);

and the others.

Chilopoda:

from the family Scutigeridae, for example, Thereuonema hilgendorfi;
from the family Scolopendridae, for example, giant tropical centipede (Scolopendra subspinipes);
from the family Ethopolyidae, for example, Bothropolys rugosus;

and the others.

Gastropoda:

from the family Limacidae, for example, tree slug (Limax marginatus) and garden tawny slug (Limax flavus);
from the family Philomycidae, for example, Meghimatium bilineatum;
from the family Ampullariidae, for example, golden apple snail (Pomacea canaliculata);
from the family Lymnaeidae, for example, Austropeplea ollula;

and the others.

Nematoda:

from the family Aphelenchoididae, for example, rice white-tip nematode (Aphelenchoides besseyi);
from the family Pratylenchidae, for example, root lesion nematode (Pratylenchus coffeae), Pratylenchus brachyurus, California meadow nematode (Pratylenchus neglectus), and Radopholus similis;
from the family Heteroderidae, for example, javanese root-knot nematode (Meloidogyne javanica), southern root-knot nematode (Meloidogyne incognita), guava root-knot nematodes (Meloidogyne enterolobii), northern root-knot nematode (Meloidogyne hapla), soybean cyst nematode (Heterodera glycines), potato cyst nematode (Globodera rostochiensis), white potato cyst nematode (Globodera pallida), and Columbia root-knot nematode (Meloidogyne chitwoodi);
from the family Hoplolaimidae, for example, Rotylenchulus reniformis;
from the family Anguinidae, for example, strawberry bud nematode (Nothotylenchus acris), and stem nematode (Ditylenchus dipsaci);
from the family Tylenchulidae, for example, citrus nematode (Tylenchulus semipenetrans);
from the family Longidoridae, for example, dagger nematode (Xiphinema index);
from the family Trichodoridae;
from the family Parasitaphelenchidae, for example, pine wilt disease (Bursaphelenchus xylophilus);

and the others.

The harmful arthropods such as harmful insects and harmful mites, harmful mollusks, and harmful nematodes may be those having a reduced susceptibility to or a developed resistance to an insecticide, a miticide, a molluscicide, or a nematicide.

The method for controlling harmful arthropods of the present invention is carried out by applying an effective amount of the Present compound or the Composition A to a harmful arthropod directly and/or a habitat thereof (for example, plant bodies, soil, an interior of a house, or animal bodies). Examples of the method for controlling harmful arthropods of the present invention include foliage treatment, soil treatment, root treatment, shower treatment, smoking treatment, water surface treatment, and seed treatment.

The Present compound or the Composition A is usually used by mixing it with inert carrier(s) such as solid carrier(s), liquid carrier(s), and gaseous carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, a tablet, an aerosol formulation, a resin formulation, or the like. In addition to these formulations, the Present compound or the Composition A may be used by formulating it into a dosage form described in Manual on development and use of FAO and WHO Specifications for pesticides, FAO Plant Production and Protection Papers- 271-276, prepared by the FAO/WHO Joint Meeting on Pesticide Specifications, 2016, ISSN:0259-2517.

These formulations usually comprise 0.0001 to 99% by weight ratio of the Present compound or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the gaseous carrier include fluorocarbon, butane gas, LPG (liquefied petroleum gas), dimethyl ether, nitrogen, and carbon dioxide.

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Also, adjuvant(s) may be used as ingredient(s) for enhancing or assisting the efficacy of the Present compound. Specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

In the present invention, examples of the plant include whole plant, stem and leaf, flower, ear, fruit, tree stem, branch, crown, seed, vegetative reproductive organ, and seedling.

A vegetative reproductive organ means a part of plant such as root, stem, and leaf which has a growth capability even when said part is separated from the plant body and placed into soil. Examples of the vegetative reproductive organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Examples of a method for controlling harmful arthropods by applying an effective amount of the Present compound or the Composition A to soil include a method of applying an effective amount of the Present compound or the Composition A to soil before planting plants or after planting plants. Specifically, examples of the application method include planting hole treatment (for example, spraying into planting holes and soil mixing after planting hole treatment), plant foot treatment (for example, plant foot spraying, soil mixing after plant foot treatment, irrigation at plant foot, and plant foot treatment at a later seeding raising stage), planting furrow treatment (for example, planting furrow spraying and soil mixing after planting furrow treatment), planting row treatment (for example, planting row spraying, soil mixing after planting row treatment, and planting row spraying at a growing stage), planting row treatment at the time of sowing (for example, planting row spraying at the time of sowing and soil mixing after planting row treatment at the time of sowing), broadcast treatment (for example, overall soil surface spraying and soil mixing after broadcast treatment), side-article treatment, treatment of water surface (for example, application to water surface and application to water surface after flooding), other soil spraying treatment (for example, spraying of a granular formulation on leaves at a growing stage, spraying under a canopy or around a tree stem, spraying on the soil surface, mixing with surface soil, spraying into seed holes, spraying on the ground surfaces of furrows, and spraying between plants), other irrigation treatment (for example, soil irrigation, irrigation at a seedling raising stage, chemical solution injection treatment, irrigation of a plant part just above the ground, chemical solution drip irrigation, and chemigation), seedling raising box treatment (for example, spraying into a seedling raising box, irrigation of a seedling raising box, and flooding into a seedling raising box with chemical solution),

seedling raising tray treatment (for example, spraying on a seedling raising tray, irrigation of a seedling raising tray, and flooding into a seedling raising tray with chemical solution), seedbed treatment (for example, spraying on a seedbed, irrigation of a seedbed, spraying on a lowland rice nursery, and immersion of seedlings), seedbed soil incorporation treatment (for example, mixing with seedbed soil, mixing with seedbed soil before sowing, spraying at sowing before covering with soil, spraying at sowing after covering with soil, and mixing with covering with soil), and other treatment (for example, mixing with culture soil, plowing under, mixing with surface soil, mixing with soil at the place where raindrops fall from a canopy, treatment at a planting position, spraying of a granule formulation on flower clusters, and mixing with a paste fertilizer).

Examples of the application to seeds (or seed treatments) include an application of the Present compound or the Composition A to seeds or vegetative reproductive organs, and specific examples thereof include spraying treatment in which a suspension of the Present compound or the Composition A is sprayed onto seed surface or the vegetative reproductive organ surface in the form of mist; smearing treatment in which the Present compound or the Composition A is coated on a surface of seeds or the vegetative reproductive organs; a soaking treatment in which the seeds or vegetative reproductive organs are soaked into the solution of the Present compound or the Composition A for a certain time; and a method for coating the seeds or the vegetative reproductive organs with a carrier containing the Present compound or the Composition A (for example, film coating treatment and pellet coating treatment). Examples of the above-described vegetative reproductive organ include particularly seed potato.

When the Composition A is applied to seeds or vegetative reproductive organs, the Composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for example, a method in which the formulations comprising as an active component the Present compound only are applied, and seeds or vegetative reproductive organs are air dried, followed by applying the formulations comprising the Present ingredient: and a method in which the formulations comprising as an active component the Present compound and the Present ingredients are applied, and seeds or vegetative reproductive organs are air dried, followed by applying the formulations comprising the Present ingredients other than the already-applied Present ingredients, are included.

As used herein, seeds or vegetative reproductive organs holding the Present compound or the Composition A mean seeds or vegetative reproductive organs in the state where the Present compound or the Composition A is adhered to a surface of the seeds or the vegetative reproductive organs. The above-described seeds or vegetative reproductive organs holding the Present compound or the Composition A may be adhered by any other materials that are different from the Present compound or the Composition A before or after being adhered the Present compound or the Composition A to the seeds or vegetative reproductive organs.

Also, when the Composition A is adhered in a form of layer(s) to a surface of seeds or vegetative reproductive organs, the layer(s) is/are composed of one layer or a multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs holding the Present compound or the Composition A can be obtained, for example, by applying the formulations comprising the Present compound or the Composition A by the above-described application method to seeds or vegetative reproductive organs.

When the Present compound or the Composition A is applied for harmful arthropods control in agricultural fields, the application dose thereof is usually within a range of 1 to 10,000 g of the Present compound per 10,000 $m^2$. In the case of being applied to seeds or vegetative reproductive organs, the application dose thereof is usually within a range of 0.001 to 100 g of the Present compound per 1 Kg of seeds or vegetative reproductive organs. When the Present compound or the Composition A is formulated into an emulsifiable concentrate, a wettable powder, or a flowable, etc., they are usually applied by diluting them with water so as to make an effective concentration of the active ingredients 0.01 to 10,000 ppm, and the granular formulation, the dust formulation, or the like is usually applied as itself without diluting them.

Also, the resin preparation of the Present compound or the Composition A which is processed into a sheet or a string may be applied by winding a plant with a sheet or a string of the resin preparation, putting a string of the resin preparation around a crop so that the plant is surrounded by the string, or laying a sheet of the resin preparation on the soil surface near the root of a plant.

When the Present compound or the Composition A is used to control harmful arthropods that live inside a house, the application dose as an amount of the Present compound is usually within a range from 0.01 to 1,000 mg per 1 $m^2$ of an area to be treated, in the case of using it on a planar area. In the case of using it spatially, the application dose as an amount of the Present compound is usually within a range from 0.01 to 500 mg per 1 $m^3$ of the space to be treated. When the Present compound or the Composition A is formulated into emulsifiable concentrates, wettable powders, flowables, or the others, such formulations are usually applied after diluting them with water in such a way that a concentration of the active ingredient is within a range from 0.1 to 10,000 ppm. In the case of being formulated into oil solutions, aerosols, smoking agents, poison baits, and the others, such formulations are used as themselves without diluting them.

When the Present compound or the Composition A is used for controlling external parasites of livestock such as cows, horses, pigs, sheep, goats, and chickens and small animals such as dogs, cats, rats, and mice, the composition of the present invention may be applied to the animals by a known method in the veterinary field. Specifically, when systemic control is intended, the composition of the present invention is administered to the animals as a tablet, a mixture with feed or a suppository, or by injection (including intramuscular, subcutaneous, intravenous, and intraperitoneal injections). On the other hand, when non-systemic control is intended, the composition of the present invention is applied to the animals by means of spraying of the oil solution or aqueous solution, pour-on or spot-on treatment, washing of the animals with a shampoo formulation, or by putting a collar or ear tag made of the resin formulations to the animals. In the case of being administered to an animal body, the dose of the Present compound or the Composition A is usually within a range from 0.1 to 1,000 mg per 1 kg of an animal body weight.

Also, the Present compound or the Composition A may be used as an agent for controlling harmful arthropods in agricultural lands such as fields, paddy fields, turfs, and orchards. Examples of the plants include the followings.

corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

## EXAMPLES

Hereinafter, the present invention is illustrated more in detail by Preparation Examples, Formulation Examples, Test Examples, and the like, but the present invention is not limited to these Examples only.

In the present description, Me represents a methyl group, Et represents an ethyl group, Pr represents a propyl group, i-Pr represents an isopropyl group, Bu represents a butyl group, t-Bu represents a tert-butyl group, Hex represents a hexyl group, c-Pr represents a cyclopropyl group, c-Bu represents a cyclobutyl group, Ph represents a phenyl group, Boc represents a tert-butoxycarbonyl group, Bn represents a benzyl group, Py2 represents a 2-pyridyl group, Py3 represents a 3-pyridyl group, and Py4 represents a 4-pyridyl group. When c-Pr, Ph, Py2, Py3, and Py4 have substituent(s), the substituent(s) is/are indicated before the symbols with the substitution position(s). For example, 1-CN-c-Pr represents a 1-cyanocyclopropyl group, 3,4-$F_2$-Ph represents a 3,4-difluorophenyl group, and 4-$CF_3$-Py2 represents a 4-(trifluoromethyl)-2-pyridyl group.

First, Preparation Examples of the Present compounds and the production intermediates thereof are shown below.

When a physical property of a compound is measured by liquid chromatography / mass spectrometry (hereinafter referred to as "LCMS"), the measured molecular ion value $[M+H]^+$ or $[M-H]^-$, and retention time (hereinafter referred to as "RT") are described. The conditions of liquid chromatography (hereinafter referred to as "LC") are as follows.

[LC conditions]

Column: L-column2 ODS, inner diameter: 4.6 mm, length: 30 mm, particle size: 3 μm (Chemicals Evaluation and Research Institute, Japan)
UV measurement wavelength: 254 nm
Mobile phase: Solution A: 0.1% formic acid in water, Solution B: 0.1% formic acid in acetonitrile
Flow rate: 2.0 mL/min
Pump: LC-20 AD (manufactured by Shimadzu Corporation) two machines (high pressure gradient)
Gradient conditions: sending a solution with the concentration gradient described in Table LC1.

Table LC1

| Time (min) | Solution A (%) | Solution B (%) |
|---|---|---|
| 0.01 | 90 | 10 |
| 2.00 | 0 | 100 |
| 4.00 | 0 | 100 |
| 4.01 | 90 | 10 |

[MS condtion]

Detector: LCMS-2020 (manufactured by Shimadzu Corporation)
Ionization Method: DUIS

Reference Preparation Example 1

To a mixture of ethyl 1-(2-((tert-butoxycarbonyl)amino)ethyl)-3-hydroxy-1H-pyrazole-5-carboxylate prepared according to the method described in WO 2013/192346 pamphlet (0.5 g) and N,N-dimethylformamide (3 mL) were added cesium carbonate (1.1 g) and 2-iodopropane (0.25 mL), the resulting mixture was stirred at room temperature for 4 hours, water was added thereto, and the resulting mixture was extracted with ethyl acetate. The resulting organic layer was dried over sodium sulfate, and concentrated under reduced pressure. The resulting residue was subjected to silica gel column chromatography to give the Intermediate 1 (0.51 g).

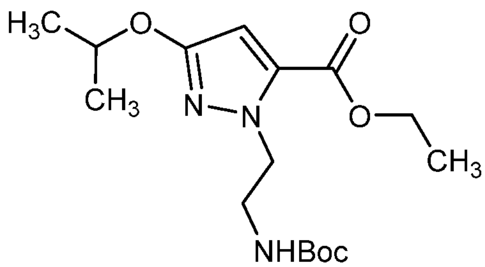

Intermediate 1: $^1$H-NMR ($CDCl_3$) δ: 6.19 (1H, s), 4.98 (1H, br s), 4.73-4.64 (1H, m), 4.54-4.51 (2H, m), 4.32 (2H, q), 3.55-3.51 (2H, m), 1.41 (9H, s), 1.36 (3H, t), 1.35-1.34 (6H, m).

Reference Preparation Example 2

To a mixture of the Intermediate 1 (0.51 g) and cyclopentyl methyl ether (5 mL) was added hydrogen chloride (an about 4 mol/L solution in cyclopentyl methyl ether) (6 mL) at 0°C, the resulting mixture was stirred at room temperature for 4 hours, and concentrated under reduced pressure. To the resulting residue was added a saturated aqueous solution of sodium carbonate, the resulting mixture was stirred at room temperature for 3 hours, and extracted with chloroform containing 2-propanol at 25%. The resulting organic layer was dried over sodium sulfate, and concentrated under reduced pressure. The resulting residue was subjected to silica gel column chromatography to give the Intermediate 2 (0.26 g).

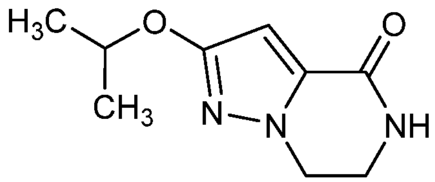

Intermediate 2: $^1$H-NMR ($CDCl_3$) δ: 6.24 (1H, s), 6.17 (1H, br s), 4.73-4.64 (1H, m), 4.23-4.20 (2H, m), 3.78-3.74 (2H, m), 1.37-1.36 (3H, m), 1.36-1.34 (3H, m).

Reference Preparation Example 3

To a mixture of ethyl 3-(trifluoromethyl)-1H-pyrazole-5-carboxylate (5.9 g) and THF (70 mL) were added triphenylphosphine (9.0 g), 2-(tert-butoxycarbonylamino)-1-ethanol (5.3 mL), and bis(2-methoxyethyl) azodicarboxylate (10 g) at 0°C, the resulting mixture was stirred at room temperature for 6 hours, water was added thereto, and the resulting mixture was extracted with ethyl acetate. The resulting organic layer was dried over sodium sulfate, and concentrated under reduced pressure. The resulting residue was subjected to silica gel column chromatography to give the Intermediate 3 (7.8 g).

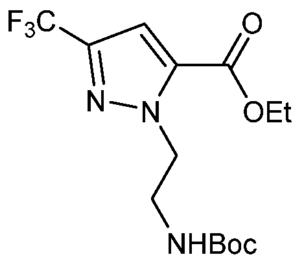

Intermediate 3: $^1$H-NMR ($CDCl_3$) δ: 7.09 (1H, s), 4.79 (1H, br s), 4.74-4.71 (2H, m), 4.37 (2H, q), 3.63 (2H, q), 1.40-1.37 (12H, m).

Reference Preparation Example 4

A compound prepared according to the Reference Preparation Example 2 by using the Intermediate 3 instead of the Intermediate 1 and a physical property thereof are shown below.

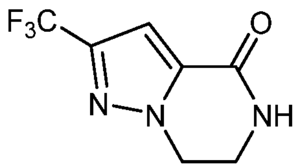

Intermediate 4: $^1$H-NMR ($CDCl_3$) δ: 7.12 (1H, s), 6.16 (1H, brs), 4.49-4.46 (2H, m), 3.87-3.83 (2H, m).

Reference Preparation Example 5

To a mixture of ethyl 1-(2-((tert-butoxycarbonyl)amino)ethyl)-3-hydroxy-1H-pyrazole-5-carboxylate (23.4 g) prepared according to the method described in WO 2013/192346 pamphlet, chloroform (150 mL), and water (170 mL) were added potassium carbonate (86.2 g) and (bromodifluoromethyl)trimethylsilane (30.2 mL) at 0°C, the resulting

mixture was stirred at room temperature for 4 hours, and extracted with chloroform. The resulting organic layer was dried over sodium sulfate, and concentrated under reduced pressure. The resulting residue was subjected to silica gel column chromatography to give the Intermediate 5 (5.5 g).

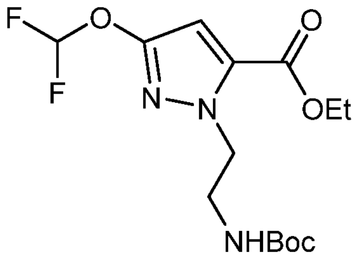

Intermediate 5: $^1$H-NMR ($CDCl_3$) δ:6.79 (1H, t), 6.44 (1H, s), 4.80 (1H, br s), 4.59-4.57 (2H, m), 4.35 (2H, q), 3.56-3.55 (2H, m), 1.41-1.36 (12H, m).

Reference Preparation Example 6

A compound prepared according to the Reference Preparation Example 2 by using the Intermediate 5 instead of the Intermediate 1 and a physical property thereof are shown below.

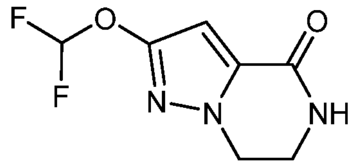

Intermediate 6: $^1$H-NMR ($CDCl_3$) δ: 6.79 (1H, t), 6.49 (1H, s), 6.00-5.91 (1H, m), 4.31-4.27 (2H, m), 3.82-3.78 (2H, m).

Reference Preparation Example 7

A compound prepared according to the Reference Preparation Example 3 by using methyl 5-bromo-1H-pyrazole-3-carboxylate instead of ethyl 3-(trifluoromethyl)-1H-pyrazole-5-carboxylate and a physical property thereof are shown below.

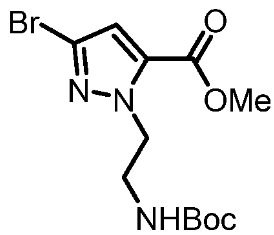

Intermediate 7: $^1$H-NMR ($CDCl_3$) δ: 6.83 (1H, s), 4.82 (1H, s), 4.66-4.63 (2H, m), 3.89 (3H, s), 3.61-3.56 (2H, m), 1.41 (9H, s).

Reference Preparation Example 8

A compound prepared according to the Reference Preparation Example 2 by using the Intermediate 7 instead of the Intermediate 1 and a physical property thereof are shown below.

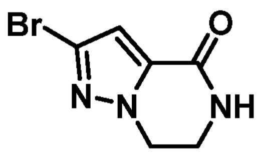

Intermediate 8: $^1$H-NMR ($CDCl_3$) δ: 6.87 (1H, s), 6.03 (1H, br s), 4.40-4.37 (2H, m), 3.81-3.77 (2H, m).

Reference Preparation Example 9

A mixture of 3-bromo-2-chloro-5-(phenylmethoxy)pyridine (7.17 g), tris(dibenzylideneaceton)dipalladium(0) (0.55 g), Xantphos (0.69 g), diisopropylamine (6.84 mL), ethanethiol (1.95 mL), and 1,4-dioxane (70 mL) was stirred under argon atmosphere at 80°C for 3.5 hours, water was added thereto, and the resulting mixture was extracted with ethyl acetate. The resulting organic layer was dried over sodium sulfate, and concentrated under reduced pressure. The resulting residue was subjected to silica gel column chromatography to give the Intermediate 9 (6.47 g).

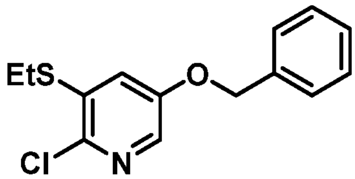

Intermediate 9: $^1$H-NMR ($CDCl_3$) δ: 7.89 (1H, d), 7.42-7.35 (5H, m), 7.10 (1H, d), 5.11 (2H, s), 2.90 (2H, q), 1.35 (3H, t).

Reference Preparation Example 10

To a mixture of the Intermediate 9 (6.5 g) and chloroform (100 mL) was added meta-chloroperbenzoic acid (13.5 g) little by little at 0°C. The resulting mixture was stirred at room temperature for 3 hours, then added to water in which sodium thiosulfate (18.6 g) and sodium hydrogen carbonate (9.9 g) were dissolved, and the resulting mixture was stirred for 0.5 hour. The resulting mixture was extracted with chloroform, the resulting organic layer was dried over sodium sulfate, and concentrated under reduced pressure. The resulting residue was subjected to silica gel column chromatography to give the Intermediate 10 (7.7 g).

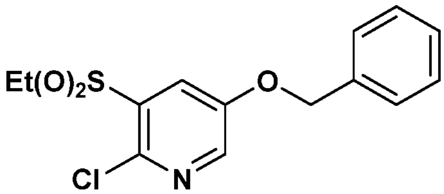

Intermediate 10: $^1$H-NMR ($CDCl_3$) δ: 8.35 (1H, d), 8.01 (1H, d), 7.44-7.35 (5H, m), 5.18 (2H, s), 3.49 (2H, q), 1.27 (3H, t).

Reference Preparation Example 11

To a mixture of the Intermediate 10 (6.86 g) and sulfolane (65 mL) was added cesium fluoride (7.35 g), the resulting mixture was stirred at 160°C for 8 hours, water was added thereto, and the resulting mixture was extracted with ethyl acetate. The resulting organic layer was dried over sodium sulfate, and concentrated under reduced pressure. The resulting residue was subjected to silica gel column chromatography to give the Intermediate 11 (3.13 g).

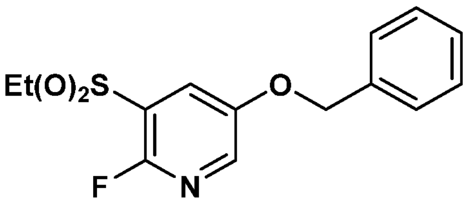

Intermediate 11: LCMS: 296 $[M+H]^+$, RT = 1.85 min

Reference Preparation Example 12

A compound prepared according to the Reference Preparation Example 11 by using 3-bromo-2-chloro-5-(difluoromethoxy)pyridine instead of the Intermediate 10 and a physical property thereof are shown below.

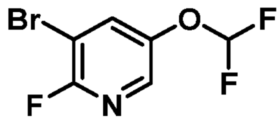

Intermediate 12: $^1$H-NMR ($CDCl_3$) δ: 8.04-8.03 (1H, m), 7.85-7.83 (1H, m), 6.53 (1H, t).

Reference Preparation Example 13

To a mixture of the Intermediate 6 (3.9 g), the Intermediate 12 (4.6 g), and dimethyl sulfoxide (25 mL) was added cesium carbonate (12.5 g), the resulting mixture was stirred at 40°C for 8 hours, water was added thereto, and the resulting mixture was extracted with ethyl acetate. The resulting organic layer was dried over sodium sulfate, and concentrated under reduced pressure. The resulting residue was subjected to silica gel column chromatography to give the Intermediate 13 (5.3 g).

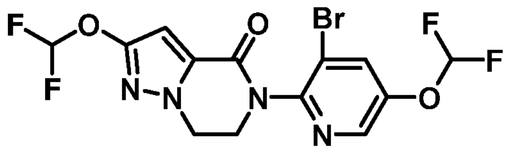

Intermediate 13: $^1$H-NMR ($CDCl_3$) δ: 8.37 (1H, d), 7.86 (1H, d), 7.00-6.41 (3H, m), 4.51-4.41 (3H, m), 4.00-3.96 (1H, m).

Reference Preparation Example 14

A compound prepared according to the Reference Preparation Example 13 by using the Intermediate 4 instead of the Intermediate 6 and by using the Intermediate 12 instead of 3-bromo-6-chloro-2-fluoropyridine, and a physical property thereof are shown below.

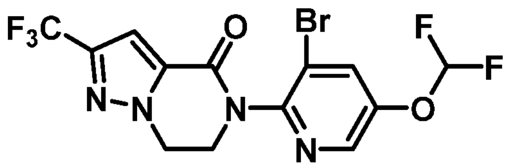

Intermediate 14: $^1$H-NMR ($CDCl_3$) δ: 8.38 (1H, d), 7.87 (1H, d), 7.24 (1H, s), 6.60 (1H, t), 4.68-4.65 (2H, m), 4.52-4.45 (1H, m), 4.07-4.01 (1H, m).

Preparation Example 1

To a mixture the Intermediate 2 (0.26 g) and N,N-dimethylformamide (5 mL) were added cesium carbonate (0.87 g) and 6-iodo-3-(ethylsulfonyl)imidazo[1,2-a]pyridine-2-fluoride prepared according to the method described in WO 2020/158889 pamphlet (1.1 g), the resulting mixture was stirred at 90°C for 7.5 hours, water was added thereto, and the resulting mixture was extracted with ethyl acetate. The resulting organic layer was dried over sodium sulfate, and concentrated under reduced pressure. The resulting residue was subjected to silica gel column chromatography to give the Present compound 1 (0.35 g).

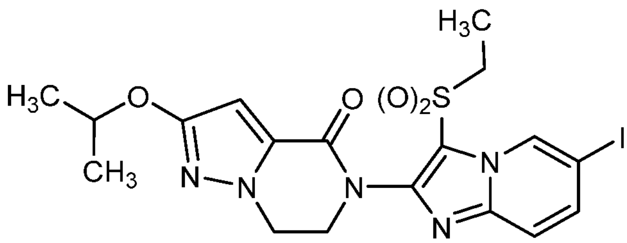

Present compound 1: $^1$H-NMR ($CDCl_3$) δ: 9.09-9.09 (1H, m), 7.70 (1H, dd), 7.49 (1H, dd), 6.31 (1H, s), 4.78-4.69 (1H, m), 4.43-4.40 (2H, m), 4.29-4.26 (2H, m), 3.58 (2H, q), 1.45 (3H, t), 1.39-1.39 (3H, m), 1.38-1.37 (3H, m).

Preparation Example 1-1

The compounds prepared according to the Preparation Example 1 and physical properties thereof are shown below. A compound represented by formula (B-1)

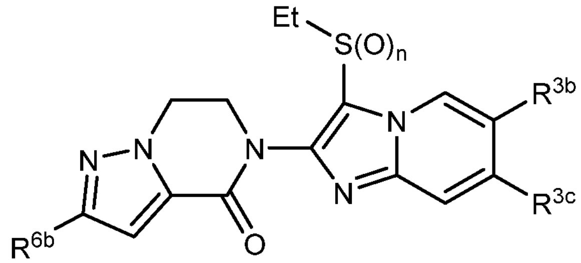

( B-1 )

wherein the combination of n, $R^{3b}$, $R^{3c}$, and $R^{6b}$ represents any one combination indicated in Table B-1.

Table B-1

| Present compound | n | $R^{3b}$ | $R^{3c}$ | $R^{6b}$ |
|---|---|---|---|---|
| 2 | 2 | I | H | $CF_3$ |
| 3 | 2 | I | H | $OCHF_2$ |
| 46 | 2 | I | H | Br |

Present compound 2: 1H-NMR (CDCl3) δ: 9.08-9.07 (1H, m), 7.72 (1H, dd), 7.50 (1H, dd), 7.21-7.21 (1H, m), 4.67 (2H, t), 4.35 (2H, t), 3.56 (2H, q), 1.45 (3H, t).
Present compound 3: 1H-NMR (CDCl3) δ: 9.07-9.06 (1H, m), 7.71 (1H, dd), 7.51-7.49 (1H, m), 6.83 (1H, t), 6.56 (1H, s), 4.50-4.47 (2H, m), 4.32-4.31 (2H, m), 3.56 (2H, q), 1.44 (3H, t).
Present compound 46: 1H-NMR (CDCl3) δ: 9.08-9.07 (1H, m), 7.71 (1H, dd), 7.49 (1H, dd), 6.95 (1H, s), 4.59-4.56 (2H, m), 4.31-4.28 (2H, m), 3.55 (2H, q), 1.44 (3H, t).

Preparation Example 1-2

A compound prepared according to the Preparation Example 1 by using 7,8-dihydroimidazo-[1,5-c]-pyrimidin-5(6H)-one instead of the Intermediate 2 and a physical property thereof are shown below.

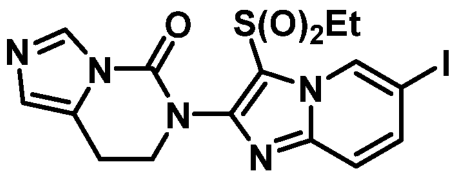

Present compound 47: 1H-NMR (CDCl3) δ: 9.07 (1H, s), 8.20 (1H, s), 7.71 (1H, dd), 7.50 (1H, d), 6.90 (1H, s), 4.12-4.05 (2H, m), 3.55 (2H, q), 3.23-3.20 (2H, m), 1.46 (3H, t).

Preparation Example 2

To a mixture of the Intermediate 4 (0.5 g) and dimethyl sulfoxide (3 mL) were added cesium carbonate (1.6 g) and 3-(ethylsulfonyl)pyridine-2-fluoride (0.46 g), the resulting mixture was stirred at 40°C for 29 hours, water was added thereto, the resulting mixture was filtered, and the resulting residue was subjected to silica gel column chromatography to give the Present compound 4 (0.1 g).

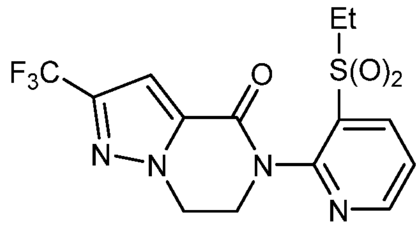

Present compound 4: $^1$H-NMR ($CDCl_3$) δ: 8.84 (1H, dd), 8.44 (1H, dd), 7.62 (1H, dd), 7.19 (1H, s), 4.83-4.75 (1H, m), 4.64-4.60 (1H, m), 4.48-4.41 (1H, m), 4.09-4.04 (1H, m), 3.41-3.27 (2H, m), 1.32 (3H, t).

Preparation Example 2-1

The compounds prepared according to the Preparation Example 2 and physical properties thereof are shown below. A compound represented by formula (B-2)

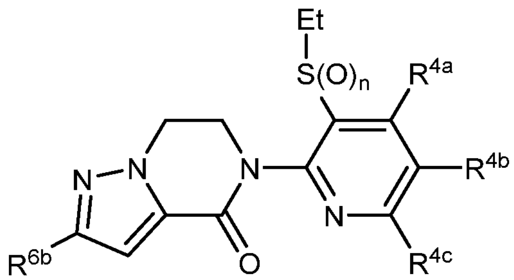

( B-2 )

wherein the combination of n, $R^{4a}$, $R^{4b}$, $R^{4c}$, and $R^{6b}$ represents any one combination indicated in Table B-2.

Table B-2

| Present compound | n | $R^{4a}$ | $R^{4b}$ | $R^{4c}$ | $R^{6b}$ |
|---|---|---|---|---|---|
| 5 | 2 | H | c-Pr | H | $CF_3$ |
| 6 | 2 | H | c-Pr | H | $OCHF_2$ |
| 7 | 2 | H | Br | H | $CF_3$ |
| 8 | 2 | H | Br | H | $OCHF_2$ |
| 33 | 2 | H | H | H | $OCHF_2$ |
| 48 | 2 | H | OBn | H | $OCHF_2$ |
| 51 | 2 | H | OBn | H | $CF_3$ |

Present compound 5: $^1$H-NMR ($CDCl_3$) δ: 8.58 (1H, d), 7.98 (1H, d), 7.18 (1H, s), 4.81-4.74 (1H, m), 4.62-4.56 (1H, m), 4.42-4.35 (1H, m), 4.04-4.00 (1H, m), 3.28 (2H, q), 2.09-2.03 (1H, m), 1.30 (3H, t), 1.26-1.18 (2H, m), 0.93-0.84 (2H, m).
Present compound 6: LCMS: 413 $[M+H]^+$, RT = 1.70 min
Present compound 7: LCMS: 453 $[M+H]^+$, RT = 1.76 min Present compound 8: $^1$H-NMR ($CDCl_3$) δ: 8.86 (1H, d), 8.53 (1H, d), 6.83 (1H, t), 6.56 (1H, s), 4.65-4.57 (1H, m), 4.45-4.36 (2H, m), 4.02-3.97 (1H, m), 3.41-3.32 (2H, m), 1.35 (3H, t).
Present compound 33: $^1$H-NMR ($CDCl_3$) δ: 8.83 (1H, dd), 8.44 (1H, dd), 7.61 (1H, dd), 6.83 (1H, t), 6.55 (1H, s), 4.66-4.59 (1H, m), 4.45-4.37 (2H, m), 4.04-4.00 (1H, m), 3.42-3.26 (2H, m), 1.31 (3H, t).
Present compound 48: $^1$H-NMR ($CDCl_3$) δ: 8.52 (1H, d), 7.93 (1H, d), 7.46-7.37 (5H, m), 6.82 (1H, t), 6.52 (1H, s), 5.27-5.18 (2H, m), 4.63-4.56 (1H, m), 4.41-4.29 (2H, m), 3.97-3.92 (1H, m), 3.34-3.21 (2H, m), 1.24 (3H, t). Present compound 51: LCMS: 481 $[M+H]^+$, RT = 1.92 min

Preparation Example 3

To a mixture of the Present compound 7 (0.5 g), 4-fluorophenylboronic acid (0.23 g), tripotassium phosphate

(0.58 g), and tetrakis(triphenylphosphine)palladium(0) (0.12 g) were added 1,2-dimethoxyethane (7 mL) and water (0.7 mL), the resulting mixture was stirred at 90°C for 3 hours, water was added thereto, and the resulting mixture was extracted with ethyl acetate. The resulting organic layer was dried over sodium sulfate, and concentrated under reduced pressure. The resulting residue was subjected to silica gel column chromatography to give the Present compound 9 (0.28 g).

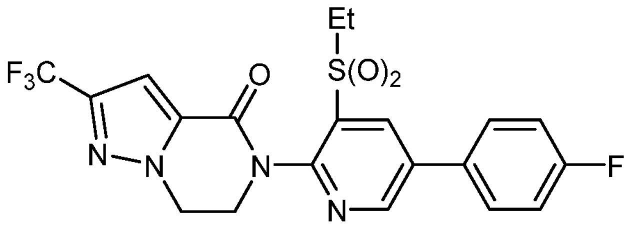

Present compound 9: $^1$H-NMR ($CDCl_3$) $\delta$: 8.97 (1H, d), 8.53 (1H, d), 7.65-7.62 (2H, m), 7.28-7.23 (2H, m), 7.21 (1H, s), 4.85-4.77 (1H, m), 4.66-4.61 (1H, m), 4.51-4.43 (1H, m), 4.13-4.07 (1H, m), 3.43-3.30 (2H, m), 1.35 (3H, t).

Preparation Example 4

To a mixture of the Present compound 7 (0.5 g), bis(pinacolato)diboron (0.56 g), [1,1'-bis(diphenylphosphino) ferrocene]dichloropalladium(II) (0.07 g), and potassium acetate (0.32 g) was added toluene (2 mL), the resulting mixture was stirred at 90°C for 3 hours, water was added thereto, and the resulting mixture was extracted with ethyl acetate. The resulting organic layer was dried over sodium sulfate, and concentrated under reduced pressure. To the resulting residue were added 2-bromo-5-cyanopyridine (0.3 g), tripotassium phosphate (0.58 g), tetrakis(triphenylphosphine)palladium(0) (0.12 g), 1,2-dimethoxyethane (7 mL), and water (0.7 mL), and the resulting mixture was stirred at 90°C for 3 hours. Water was added thereto, and the resulting mixture was extracted with ethyl acetate. The resulting organic layer was dried over sodium sulfate, and concentrated under reduced pressure. The resulting residue was subjected to silica gel column chromatography to give the Present compound 10 (0.12 g).

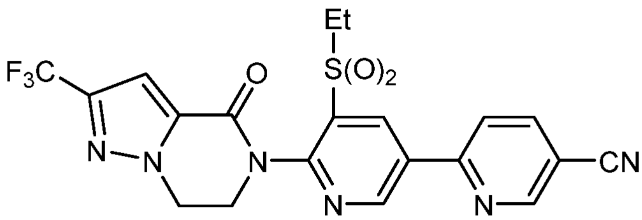

Present compound 10: $^1$H-NMR ($CDCl_3$) $\delta$: 9.45 (1H, d), 9.04-9.03 (2H, m), 8.16 (1H, dd), 8.01-7.99 (1H, m), 7.21 (1H, s), 4.83-4.76 (1H, m), 4.67-4.61 (1H, m), 4.54-4.47 (1H, m), 4.14-4.08 (1H, m), 3.44-3.39 (2H, m), 1.37 (3H, t).

Preparation Example 3-1

The compounds prepared according to the Preparation Example 3 or Preparation Example 4 and physical properties thereof are shown below.

A compound represented by the above formula (B-2), wherein the combination of n, $R^{4a}$, $R^{4b}$, $R^{4c}$, and $R^{6b}$ represents any one combination indicated in Table B-3.

Table B-3

| Present compound | n | $R^{4a}$ | $R^{4b}$ | $R^{4c}$ | $R^{6b}$ |
|---|---|---|---|---|---|
| 11 | 2 | H | 4-Cl-Ph | H | $CF_3$ |
| 12 | 2 | H | 3-Cl-Ph | H | $CF_3$ |
| 13 | 2 | H | 3, 5-$Cl_2$-Ph | H | $CF_3$ |
| 14 | 2 | H | 3-F-Ph | H | $CF_3$ |
| 15 | 2 | H | 3, 5-$F_2$-Ph | H | $CF_3$ |
| 16 | 2 | H | 4-Cl-Ph | H | $OCHF_2$ |

(continued)

| Present compound | n | $R^{4a}$ | $R^{4b}$ | $R^{4c}$ | $R^{6b}$ |
|---|---|---|---|---|---|
| 17 | 2 | H | 3-Cl-Ph | H | $OCHF_2$ |
| 18 | 2 | H | 3, 5-$Cl_2$-Ph | H | $OCHF_2$ |
| 19 | 2 | H | 4-F-Ph | H | $OCHF_2$ |
| 20 | 2 | H | 3-F-Ph | H | $OCHF_2$ |
| 21 | 2 | H | 3,5-$F_2$-Ph | H | $OCHF_2$ |
| 22 | 2 | H | 4-CN-Py2 | H | $CF_3$ |
| 23 | 2 | H | 6-CN-Py2 | H | $CF_3$ |
| 24 | 2 | H | 4-CN-Py2 | H | $OCHF_2$ |
| 25 | 2 | H | 5-CN-Py2 | H | $OCHF_2$ |
| 26 | 2 | H | 6-CN-Py2 | H | $OCHF_2$ |
| 27 | 2 | H | 4-Cl-Py2 | H | $CF_3$ |
| 28 | 2 | H | 5-Cl-Py2 | H | $CF_3$ |
| 29 | 2 | H | 6-Cl-Py2 | H | $CF_3$ |
| 30 | 2 | H | 4-Cl-Py2 | H | $OCHF_2$ |
| 31 | 2 | H | 5-Cl-Py2 | H | $OCHF_2$ |
| 32 | 2 | H | 6-Cl-Py2 | H | $OCHF_2$ |

Present compound 11: $^1$H-NMR ($CDCl_3$) δ: 8.98 (1H, d), 8.55 (1H, d), 7.60 (2H, d), 7.54 (2H, d), 7.21 (1H, s), 4.85-4.77 (1H, m), 4.67-4.62 (1H, m), 4.51-4.44 (1H, m), 4.12-4.08 (1H, m), 3.40-3.34 (2H, m), 1.35 (3H, t).

Present compound 12: $^1$H-NMR ($CDCl_3$) δ: 8.99 (1H, d), 8.55 (1H, d), 7.64-7.63 (1H, m), 7.55-7.49 (3H, m), 7.22 (1H, s), 4.85-4.77 (1H, m), 4.67-4.61 (1H, m), 4.52-4.44 (1H, m), 4.13-4.08 (1H, m), 3.41-3.35 (2H, m), 1.36 (3H, t).

Present compound 13: $^1$H-NMR ($CDCl_3$) δ: 8.96 (1H, d), 8.52 (1H, d), 7.53-7.51 (3H, m), 7.22 (1H, s), 4.84-4.77 (1H, m), 4.67-4.61 (1H, m), 4.52-4.45 (1H, m), 4.13-4.07 (1H, m), 3.39 (2H, q), 1.36 (3H, t).

Present compound 14: $^1$H-NMR ($CDCl_3$) δ: 8.99 (1H, d), 8.56 (1H, d), 7.56-7.51 (1H, m), 7.45-7.43 (1H, m), 7.38-7.34 (1H, m), 7.25-7.20 (1H, m),7.22 (1H, s), 4.85-4.77 (1H, m), 4.67-4.61 (1H, m), 4.52-4.44 (1H, m), 4.13-4.08 (1H, m), 3.43-3.33 (2H, m), 1.36 (3H, t).

Present compound 15: $^1$H-NMR ($CDCl_3$) δ: 8.97 (1H, d), 8.53 (1H, d), 7.22 (1H, s), 7.20-7.17 (2H, m), 7.00-6.95 (1H, m), 4.84-4.77 (1H, m), 4.67-4.61 (1H, m), 4.52-4.45 (1H, m), 4.13-4.07 (1H, m), 3.42-3.36 (2H, m), 1.36 (3H, t).

Present compound 16: $^1$H-NMR ($CDCl_3$) δ: 8.97 (1H, d), 8.54 (1H, d), 7.61-7.58 (2H, m), 7.55-7.52 (2H, m), 6.84 (1H, t), 6.57 (1H, s), 4.68-4.60 (1H, m), 4.47-4.40 (2H, m), 4.07-4.02 (1H, m), 3.44-3.30 (2H, m), 1.34 (3H, t).

Present compound 17: LCMS: 483 $[M+H]^+$, RT = 1.88 min

Present compound 18: $^1$H-NMR ($CDCl_3$) δ: 8.96 (1H, d), 8.52 (1H, d), 7.53-7.50 (2H, m),7.52(1H, s), 6.84 (1H, t), 6.57 (1H, s), 4.68-4.60 (1H, m), 4.48-4.41 (2H, m), 4.07-4.02 (1H, m), 3.42-3.36 (2H, m), 1.35 (3H, t).

Present compound 19: $^1$H-NMR ($CDCl_3$) δ: 8.96 (1H, d), 8.53 (1H, d), 7.65-7.62 (2H, m), 7.27-7.23 (2H, m), 6.84 (1H, t), 6.56 (1H, s), 4.69-4.64 (1H, m), 4.47-4.39 (2H, m), 4.07-4.02 (1H, m), 3.44-3.32 (2H, m), 1.34 (3H, t).

Present compound 20: $^1$H-NMR ($CDCl_3$) δ: 8.99 (1H, d), 8.55 (1H, d), 7.56-7.51 (1H, m), 7.45-7.43 (1H, m), 7.38-7.35 (1H, m), 7.24-7.20 (1H, m), 6.84 (1H, t), 6.57 (1H, s), 4.68-4.60 (1H, m), 4.48-4.41 (2H, m), 4.08-4.03 (1H, m), 3.45-3.31 (2H, m), 1.35 (3H, t).

Present compound 21: $^1$H-NMR ($CDCl_3$) δ: 8.97 (1H, d), 8.53 (1H, d), 7.21-7.15 (2H, m), 7.00-6.94 (1H, m), 6.84 (1H, t), 6.57 (1H, s), 4.68-4.60 (1H, m), 4.48-4.41 (2H, m), 4.07-4.01 (1H, m), 3.46-3.32 (2H, m), 1.35 (3H, t).

Present compound 22: $^1$H-NMR ($CDCl_3$) δ: 9.42 (1H, d), 9.03 (1H, d), 8.98-8.97 (1H, m), 8.07 (1H, s), 7.64 (1H, dd), 7.22 (1H, s), 4.83-4.77 (1H, m), 4.67-4.62 (1H, m), 4.55-4.48 (1H, m), 4.15-4.10 (1H, m), 3.46-3.40 (2H, m), 1.39 (3H, t).

Present compound 23: $^1$H-NMR ($CDCl_3$) δ: 9.44 (1H, d), 9.00 (1H, d), 8.10-8.04 (2H, m), 7.80 (1H, dd), 7.22 (1H, s), 4.84-4.77 (1H, m), 4.69-4.62 (1H, m), 4.57-4.48 (1H, m), 4.15-4.10 (1H, m), 3.43 (2H, q), 1.39 (3H, t).

Present compound 24: $^1$H-NMR ($CDCl_3$) δ: 9.41 (1H, d), 9.02 (1H, d), 8.97 (1H, dd), 8.07-8.07 (1H, m), 7.63 (1H, dd), 6.84 (1H, t), 6.57 (1H, s), 4.68-4.61 (1H, m), 4.52-4.42 (2H, m), 4.09-4.04 (1H, m), 3.47-3.38 (2H, m), 1.38 (3H, t).

Present compound 25: $^1$H-NMR ($CDCl_3$) δ: 9.45 (1H, d), 9.04-9.03 (2H, m), 8.16 (1H, dd), 8.00 (1H, dd), 6.84 (1H, t),

6.57 (1H, s), 4.68-4.61 (1H, m), 4.51-4.42 (2H, m), 4.09-4.04 (1H, m), 3.46-3.40 (2H, m), 1.38 (3H, t).

Present compound 26: $^1$H-NMR ($CDCl_3$) δ: 9.44 (1H, d), 8.99 (1H, d), 8.11-8.04 (2H, m), 7.80 (1H, dd), 6.84 (1H, t), 6.57 (1H, s), 4.68-4.61 (1H, m), 4.51-4.42 (2H, m), 4.07-4.04 (1H, m), 3.46-3.40 (2H, m), 1.38 (3H, t).

Present compound 27: LCMS: 486 $[M+H]^+$, RT = 1.86 min

Present compound 28: $^1$H-NMR ($CDCl_3$) δ: 9.39 (1H, d), 8.98 (1H, d), 8.73 (1H, d), 7.88-7.80 (2H, m), 7.21 (1H, s), 4.84-4.77 (1H, m), 4.66-4.61 (1H, m), 4.53-4.45 (1H, m), 4.13-4.08 (1H, m), 3.45-3.36 (2H, m), 1.37 (3H, t).

Present compound 29: $^1$H-NMR ($CDCl_3$) δ: 9.40 (1H, d), 8.97 (1H, d), 7.87-7.83 (1H, m), 7.79-7.77 (1H, m), 7.44 (1H, d), 7.21 (1H, s), 4.84-4.77 (1H, m), 4.67-4.62 (1H, m), 4.53-4.46 (1H, m), 4.13-4.07 (1H, m), 3.41 (2H, q), 1.38 (3H, t).

Present compound 30: $^1$H-NMR ($CDCl_3$) δ: 9.40 (1H, d), 8.97 (1H, d), 8.68 (1H, d), 7.85-7.85 (1H, m), 7.41 (1H, dd), 6.84 (1H, t), 6.56 (1H, s), 4.68-4.61 (1H, m), 4.50-4.41 (2H, m), 4.08-4.03 (1H, m), 3.48-3.35 (2H, m), 1.37 (3H, t).

Present compound 31: $^1$H-NMR ($CDCl_3$) δ: 9.39 (1H, d), 8.97 (1H, d), 8.73 (1H, dd), 7.87-7.80 (2H, m), 6.84 (1H, t), 6.56 (1H, s), 4.68-4.59 (1H, m), 4.49-4.41 (2H, m), 4.08-4.02 (1H, m), 3.45-3.35 (2H, m), 1.36 (3H, t).

Present compound 32: $^1$H-NMR ($CDCl_3$) δ: 9.40 (1H, d), 8.96 (1H, d), 7.86-7.83 (1H, m), 7.79-7.77 (1H, m), 7.46-7.42 (1H, m), 6.84 (1H, t), 6.56 (1H, s), 4.68-4.59 (1H, m), 4.49-4.41 (2H, m), 4.08-4.02 (1H, m), 3.41 (2H, q), 1.37 (3H, t).

Preparation Example 5

To a mixture of the Present compound 7 (3.00 g), tert-butyl carbamate (1.16 g), tris(dibenzylideneaceton) dipalladium(0) (0.30 g), Xantphos (0.32 g), and cesium carbonate (3.02 g) was added 1,4-dioxane (10 mL), the resulting mixture was stirred at 90°C for 3 hours, water was added thereto, and the resulting mixture was extracted with chloroform. The resulting organic layer was dried over sodium sulfate, and concentrated under reduced pressure. The resulting residue was subjected to silica gel column chromatography to give the Present compound 34 (1.44 g).

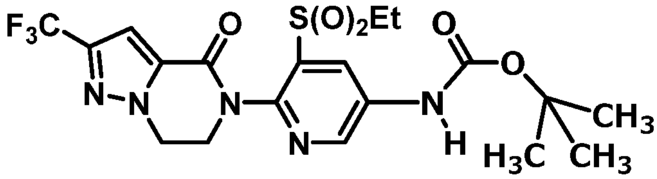

Present compound 34: $^1$H-NMR ($CDCl_3$) δ: 8.75 (1H, d), 8.57 (1H, d), 7.17 (1H, s), 6.83 (1H, s), 4.81-4.73 (1H, m), 4.61-4.56 (1H, m), 4.42-4.34 (1H, m), 4.05-4.00 (1H, m), 3.38-3.24 (2H, m), 1.55-1.54 (9H, m), 1.33 (3H, t).

Preparation Example 5-1

A compound prepared according to the Preparation Example 5 by using the Present compound 8 instead of the Present compound 7 and a physical property thereof are shown below.

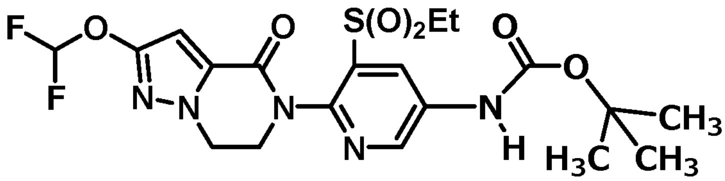

Present compound 35: $^1$H-NMR ($CDCl_3$) δ: 8.75 (1H, d), 8.56 (1H, d), 6.82 (1H, t), 6.80 (1H, s), 6.53 (1H, s), 4.64-4.56 (1H, m), 4.42-4.31 (2H, m), 4.01-3.95 (1H, m), 3.38-3.24 (2H, m), 1.55 (9H, s), 1.32 (3H, t).

Preparation Example 6

A mixture of the Present compound 34 (2.1 g) and chloroform (1 mL) was cooled to 0°C, trifluoroacetic acid (7.4 mL) was added thereto, the resulting mixture was warmed to room temperature, and then stirred for 2 hours. The resulting mixture was concentrated under reduced pressure, to the resulting residue was added water, the resulting mixture was extracted with chloroform. The resulting organic layer was dried over sodium sulfate, and concentrated under reduced pressure. The resulting residue was subjected to silica gel column chromatography to give the Present compound 36 (1.2 g).

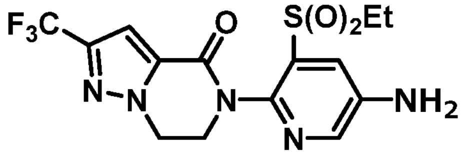

Present compound 36: $^1$H-NMR ($CDCl_3$) δ: 8.19 (1H, d), 7.62 (1H, d), 7.16 (1H, s), 4.80-4.72 (1H, m), 4.60-4.54 (1H, m), 4.36-4.29 (1H, m), 4.24 (2H, s), 4.02-3.97 (1H, m), 3.31-3.18 (2H, m), 1.29 (3H, t).

Preparation Example 6-1

A compound prepared according to the Preparation Example 6 by using the Present compound 35 instead of the Present compound 34 and a physical property thereof are shown below.

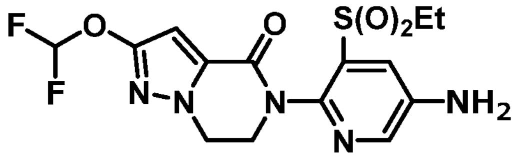

Present compound 37: LCMS: 388 $[M+H]^+$, RT = 1.30 min

Preparation Example 7

A mixture of the Present compound 36 (0.50 g), 4-(dimethylamino)pyridine (0.03 g), and pyridine (2.5 mL) was cooled to 0°C, acetyl chloride (0.11 mL) was added thereto, the resulting mixture was warmed to room temperature, and then stirred for 1.5 hours. The resulting mixture was concentrated under reduced pressure, to the resulting residue was added a saturated aqueous solution of sodium hydrogen carbonate, and the resulting mixture was extracted with chloroform. The resulting organic layer was washed with water, then dried over sodium sulfate, and concentrated under reduced pressure. The resulting residue was subjected to silica gel column chromatography to give the Present compound 38 (0.47 g).

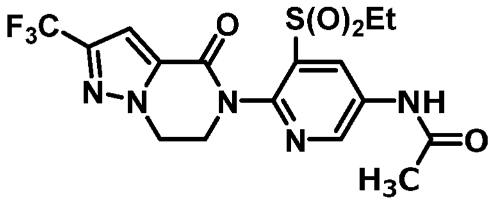

Present compound 38: $^1$H-NMR ($CDCl_3$) δ: 9.00 (1H, d), 8.60 (1H, d), 7.65 (1H, s), 7.18 (1H, s), 4.81-4.73 (1H, m), 4.63-4.57 (1H, m), 4.44-4.37 (1H, m), 4.06-4.01 (1H, m), 3.41-3.25 (2H, m), 2.28 (3H, s), 1.33 (3H, t).

Preparation Example 7-1

A compound prepared according to the Preparation Example 7 by using cyclopropanecarbonyl chloride instead of acetyl chloride and a physical property thereof are shown below.

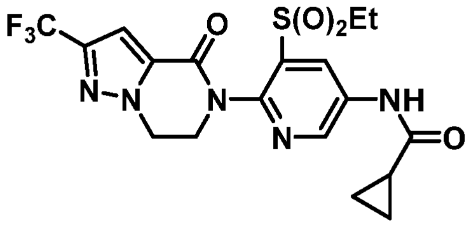

Present compound 39: $^1$H-NMR ($CDCl_3$) δ: 8.97 (1H, d), 8.66 (1H, d), 7.73 (1H, s), 7.18 (1H, s), 4.81-4.73 (1H, m), 4.62-4.57 (1H, m), 4.43-4.36 (1H, m), 4.06-4.01 (1H, m), 3.40-3.25 (2H, m), 1.55-1.53 (1H, m), 1.33 (3H, t), 1.20-1.16 (2H,

m), 1.02-0.96 (2H, m).

Preparation Example 7-2

A compound prepared according to the Preparation Example 7 by using the Present compound 37 instead of the Present compound 36 and a physical property thereof are shown below.

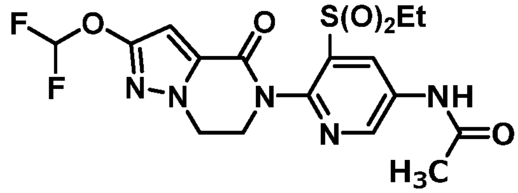

Present compound 40: $^1$H-NMR ($CDCl_3$) δ: 8.99 (1H, d), 8.59 (1H, d), 7.53 (1H, s), 6.82 (1H, t), 6.53 (1H, s), 4.64-4.56 (1H, m), 4.42-4.33 (2H, m), 4.01-3.96 (1H, m), 3.41-3.25 (2H, m), 2.28 (3H, s), 1.32 (3H, t).

Preparation Example 7-3

A compound prepared according to the Preparation Example 7-1 by using the Present compound 37 instead of the Present compound 36 and a physical property thereof are shown below.

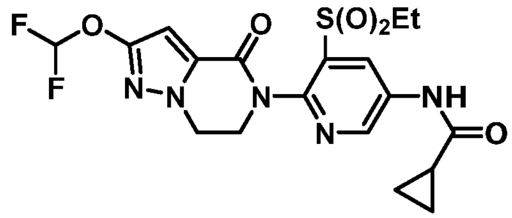

Present compound 41: $^1$H-NMR ($CDCl_3$) δ: 8.96 (1H, d), 8.63 (1H, d), 7.85 (1H, s), 6.82 (1H, t), 6.52 (1H, s), 4.63-4.56 (1H, m), 4.42-4.32 (2H, m), 4.01-3.95 (1H, m), 3.40-3.24 (2H, m), 1.64-1.55 (1H, m), 1.31 (3H, t), 1.18-1.14 (2H, m), 0.99-0.93 (2H, m).

Preparation Example 8

To a mixture of the Present compound 38 (0.37 g) and DMF (6 mL) were added cesium carbonate (0.56 g) and iodomethane (0.08 mL), and the resulting mixture was stirred at room temperature for 4.5 hours. Water was added thereto, the resulting mixture was extracted with ethyl acetate, the resulting organic layer was dried over sodium sulfate, and concentrated under reduced pressure. The resulting residue was subjected to silica gel column chromatography to give the Present compound 42 (0.31 g).

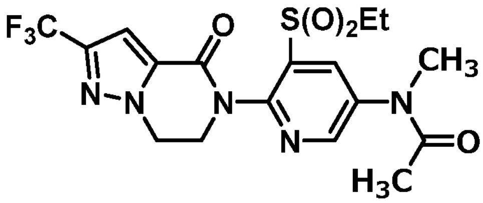

Present compound 42: $^1$H-NMR ($CDCl_3$) δ: 8.77-8.75 (1H, m), 8.31-8.29 (1H, m), 7.20 (1H, s), 4.81-4.74 (1H, m), 4.65-4.59 (1H, m), 4.48-4.41 (1H, m), 4.10-4.04 (1H, m), 3.45 (3H, s), 3.42-3.29 (2H, m), 2.19 (3H, br s), 1.34 (3H, t).

Preparation Example 8-1

A compound prepared according to the Preparation Example 8 by using the Present compound 39 instead of the Present compound 38 and a physical property thereof are shown below.

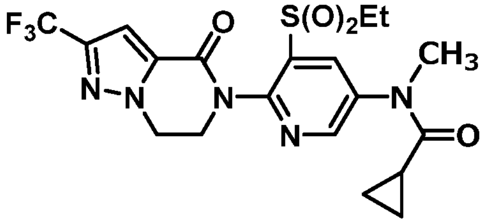

Present compound 43: $^1$H-NMR ($CDCl_3$) δ: 8.79 (1H, d), 8.35 (1H, d), 7.20 (1H, s), 4.82-4.75 (1H, m), 4.65-4.60 (1H, m), 4.49-4.42 (1H, m), 4.10-4.05 (1H, m), 3.48 (3H, s), 3.42-3.32 (2H, m), 1.54-1.52 (1H, m), 1.34 (3H, t), 1.17-1.16 (2H, m), 0.92-0.85 (2H, m).

Preparation Example 8-2

A compound prepared according to the Preparation Example 8 by using the Present compound 40 instead of the Present compound 38 and a physical property thereof are shown below.

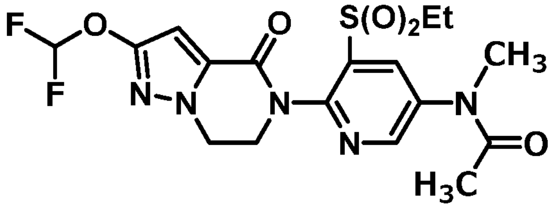

Present compound 44: $^1$H-NMR ($CDCl_3$) δ: 8.76-8.74 (1H, m), 8.29-8.28 (1H, m), 6.83 (1H, t), 6.55 (1H, s), 4.64-4.57 (1H, m), 4.44-4.37 (2H, m), 4.03-3.99 (1H, m), 3.44 (3H, br s), 3.41-3.31 (2H, m), 2.17 (3H, br s), 1.34 (3H, t).

Preparation Example 8-3

A compound prepared according to the Preparation Example 8 by using the Present compound 41 instead of the Present compound 38 and a physical property thereof are shown below.

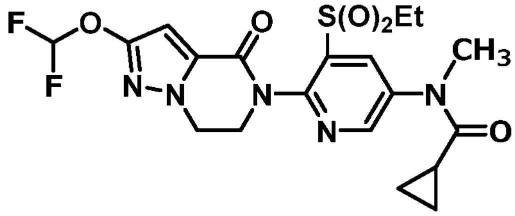

Present compound 45: $^1$H-NMR ($CDCl_3$) δ: 8.78 (1H, d), 8.34 (1H, d), 6.83 (1H, t), 6.55 (1H, s), 4.66-4.58 (1H, m), 4.45-4.38 (2H, m), 4.05-4.00 (1H, m), 3.47 (3H, s), 3.43-3.32 (2H, m), 1.59-1.49 (1H, m), 1.34 (3H, t), 1.17-1.14 (2H, m), 0.91-0.81 (2H, m).

Preparation Example 9

To a mixture of the Present compound 48 (0.92 g), methanol (8 mL), and ethyl acetate (8 mL) was added palladium 10% on carbon (wetted with ca. 55% water) (0.030 g), and the resulting mixture was stirred under hydrogen atmosphere at room temperature for 6 hours. The resulting mixture was filtered, and the resulting residue was concentrated under reduced pressure. The resulting residue was subjected to silica gel column chromatography to give the Present compound 49 (0.58 g).

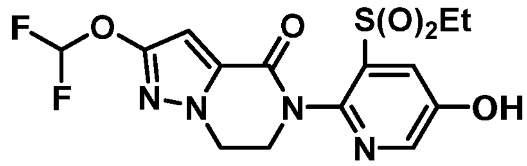

Present compound 49: LCMS: 389 $[M+H]^+$, RT = 1.36 min

Preparation Example 10

To a mixture of the Intermediate 14 (2.1 g), ethanethiol (0.45 mL), tris(dibenzylideneaceton)dipalladium(0) (0.23 g), Xantphos (0.29 g), and diisopropylamine (1.4 mL) was added 1,4-dioxane (10 mL), the resulting mixture was stirred at 80°C for 7 hours, water was added thereto, and the resulting mixture was extracted with ethyl acetate. The resulting organic layer was dried over sodium sulfate, and concentrated under reduced pressure. The resulting residue was subjected to silica gel column chromatography to give the Present compound 52 (2.0 g).

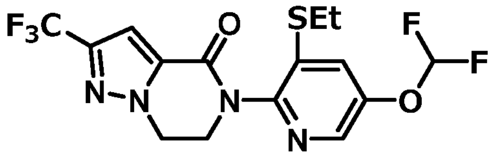

Present compound 52: $^1$H-NMR ($CDCl_3$) $\delta$: 8.16 (1H, d), 7.47 (1H, d), 7.23 (1H, s), 6.59 (1H, t), 4.67-4.64 (2H, m), 4.50-4.42 (1H, m), 4.02-3.98 (1H, m), 3.01-2.92 (2H, m), 1.35 (3H, t).

Preparation Example 10-1

A compound prepared according to the Preparation Example 10 by using the Intermediate 13 instead of the Intermediate 14 and a physical property thereof are shown below.

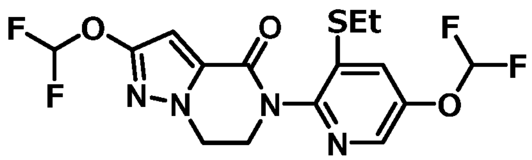

Present compound 53: $^1$H-NMR ($CDCl_3$) $\delta$: 8.15 (1H, d), 7.46 (1H, d), 6.99-6.40 (3H, m), 4.53-4.38 (3H, m), 3.96-3.92 (1H, m), 3.00-2.90 (2H, m), 1.35 (3H, t).

Preparation Example 11

A compound prepared according to the Reference Preparation Example 10 by using the Present compound 52 instead of the Intermediate 9 and a physical property thereof are shown below.

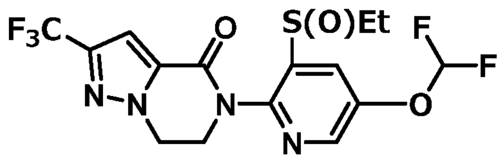

Present compound 54: LCMS: 425 $[M+H]^+$, RT = 1.70 min

Preparation Example 11-1

The compounds prepared according to the Preparation Example 11 and physical properties thereof are shown below.

A compound represented by the above formula (B-2), wherein the combination of n, $R^{4a}$, $R^{4b}$, $R^{4c}$, and $R^{6b}$ represents any one combination indicated in Table B-5.

Table B-5

| Present compound | n | $R^{4a}$ | $R^{4b}$ | $R^{4c}$ | $R^{6b}$ |
|---|---|---|---|---|---|
| 55 | 2 | H | $OCHF_2$ | H | $CF_3$ |
| 56 | 1 | H | $OCHF_2$ | H | $OCHF_2$ |
| 57 | 2 | H | $OCHF_2$ | H | $OCHF_2$ |

Present compound 55: LCMS: 441 $[M+H]^+$, RT = 1.83 min
Present compound 56: LCMS: 423 $[M+H]^+$, RT = 1.53 min
Present compound 57: $^1$H-NMR ($CDCl_3$) $\delta$: 8.67 (1H, d), 8.19 (1H, d), 7.02-6.54 (3H, m), 4.64-4.57 (1H, m), 4.45-4.35 (2H, m), 4.02-3.96 (1H, m), 3.42-3.28 (2H, m), 1.34-1.30(3H, m).

Next, examples of the Present compounds prepared according to any one of the Preparation Examples described in EXAMPLES and the Production methods disclosed in the present description are shown below.
A compound represented by formula (L-1)

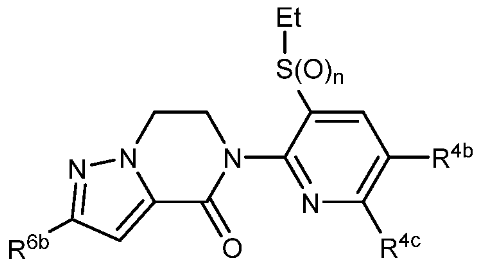

( L-1 )

(hereinafter referred to as "Compound (L-1)"), wherein n represents 0, $R^{4b}$ represents a hydrogen atom, $R^{4c}$ represents a hydrogen atom, and $R^{6b}$ represents any one substituent described in Table 1A to Table 3A (hereinafter referred to as "Compound group SX1").
The Compound (L-1), wherein n represents 1, $R^{4b}$ represents a hydrogen atom, $R^{4c}$ represents a hydrogen atom, and $R^{6b}$ represents any one substituent described in Table 1A to Table 3A (hereinafter referred to as "Compound group SX2") .
The Compound (L-1), wherein n represents 2, $R^{4b}$ represents a hydrogen atom, $R^{4c}$ represents a hydrogen atom, and $R^{6b}$ represents any one substituent described in Table 1A to Table 3A (hereinafter referred to as "Compound group SX3").
The Compound (L-1), wherein n represents 0, $R^{4b}$ represents a chlorine atom, $R^{4c}$ represents a hydrogen atom, and $R^{6b}$ represents any one substituent described in Table 1A to Table 3A (hereinafter referred to as "Compound group SX4").
The Compound (L-1), wherein n represents 1, $R^{4b}$ represents a chlorine atom, $R^{4c}$ represents a hydrogen atom, and $R^{6b}$ represents any one substituent described in Table 1A to Table 3A (hereinafter referred to as "Compound group SX5").
The Compound (L-1), wherein n represents 2, $R^{4b}$ represents a chlorine atom, $R^{4c}$ represents a hydrogen atom, and $R^{6b}$ represents any one substituent described in Table 1A to Table 3A (hereinafter referred to as "Compound group SX6").
The Compound (L-1), wherein n represents 0, $R^{4b}$ represents a bromine atom, $R^{4c}$ represents a hydrogen atom, and $R^{6b}$ represents any one substituent described in Table 1A to Table 3A (hereinafter referred to as "Compound group SX7").
The Compound (L-1), wherein n represents 1, $R^{4b}$ represents a bromine atom, $R^{4c}$ represents a hydrogen atom, and $R^{6b}$ represents any one substituent described in Table 1A to Table 3A (hereinafter referred to as "Compound group SX8").
The Compound (L-1), wherein n represents 2, $R^{4b}$ represents a bromine atom, $R^{4c}$ represents a hydrogen atom, and $R^{6b}$ represents any one substituent described in Table 1A to Table 3A (hereinafter referred to as "Compound group SX9") .
The Compound (L-1), wherein n represents 0, $R^{4b}$ represents a trifluoromethyl group, $R^{4c}$ represents a hydrogen

atom, and $R^{6b}$ represents any one substituent described in Table 1A to Table 3A (hereinafter referred to as "Compound group SX10").

The Compound (L-1), wherein n represents 1, $R^{4b}$ represents a trifluoromethyl group, $R^{4c}$ represents a hydrogen atom, and $R^{6b}$ represents any one substituent described in Table 1A to Table 3A (hereinafter referred to as "Compound group SX11").

The Compound (L-1), wherein n represents 2, $R^{4b}$ represents a trifluoromethyl group, $R^{4c}$ represents a hydrogen atom, and $R^{6b}$ represents any one substituent described in Table 1A to Table 3A (hereinafter referred to as "Compound group SX12").

The Compound (L-1), wherein n represents 0, $R^{4b}$ represents a difluoromethyl group, $R^{4c}$ represents a hydrogen atom, and $R^{6b}$ represents any one substituent described in Table 1A to Table 3A (hereinafter referred to as "Compound group SX13").

The Compound (L-1), wherein n represents 1, $R^{4b}$ represents a difluoromethyl group, $R^{4c}$ represents a hydrogen atom, and $R^{6b}$ represents any one substituent described in Table 1A to Table 3A (hereinafter referred to as "Compound group SX14").

The Compound (L-1), wherein n represents 2, $R^{4b}$ represents a difluoromethyl group, $R^{4c}$ represents a hydrogen atom, and $R^{6b}$ represents any one substituent described in Table 1A to Table 3A (hereinafter referred to as "Compound group SX15").

The Compound (L-1), wherein n represents 0, $R^{4b}$ represents a trifluoromethoxy group, $R^{4c}$ represents a hydrogen atom, and $R^{6b}$ represents any one substituent described in Table 1A to Table 3A (hereinafter referred to as "Compound group SX16").

The Compound (L-1), wherein n represents 1, $R^{4b}$ represents a trifluoromethoxy group, $R^{4c}$ represents a hydrogen atom, and $R^{6b}$ represents any one substituent described in Table 1A to Table 3A (hereinafter referred to as "Compound group SX17").

The Compound (L-1), wherein n represents 2, $R^{4b}$ represents a trifluoromethoxy group, $R^{4c}$ represents a hydrogen atom, and $R^{6b}$ represents any one substituent described in Table 1A to Table 3A (hereinafter referred to as "Compound group SX18").

The Compound (L-1), wherein n represents 0, $R^{4b}$ represents a difluoromethoxy group, $R^{4c}$ represents a hydrogen atom, and $R^{6b}$ represents any one substituent described in Table 1A to Table 3A (hereinafter referred to as "Compound group SX19").

The Compound (L-1), wherein n represents 1, $R^{4b}$ represents a difluoromethoxy group, $R^{4c}$ represents a hydrogen atom, and $R^{6b}$ represents any one substituent described in Table 1A to Table 3A (hereinafter referred to as "Compound group SX20").

The Compound (L-1), wherein n represents 2, $R^{4b}$ represents a difluoromethoxy group, $R^{4c}$ represents a hydrogen atom, and $R^{6b}$ represents any one substituent described in Table 1A to Table 3A (hereinafter referred to as "Compound group SX21").

The Compound (L-1), wherein n represents 0, $R^{4b}$ represents a cyclopropyl group, $R^{4c}$ represents a hydrogen atom, and $R^{6b}$ represents any one substituent described in Table 1A to Table 3A (hereinafter referred to as "Compound group SX22").

The Compound (L-1), wherein n represents 1, $R^{4b}$ represents a cyclopropyl group, $R^{4c}$ represents a hydrogen atom, and $R^{6b}$ represents any one substituent described in Table 1A to Table 3A (hereinafter referred to as "Compound group SX23").

The Compound (L-1), wherein n represents 2, $R^{4b}$ represents a cyclopropyl group, $R^{4c}$ represents a hydrogen atom, and $R^{6b}$ represents any one substituent described in Table 1A to Table 3A (hereinafter referred to as "Compound group SX24").

The Compound (L-1), wherein n represents 0, $R^{4b}$ represents a 1-cyanocyclopropyl group, $R^{4c}$ represents a hydrogen atom, and $R^{6b}$ represents any one substituent described in Table 1A to Table 3A (hereinafter referred to as "Compound group SX25").

The Compound (L-1), wherein n represents 1, $R^{4b}$ represents a 1-cyanocyclopropyl group, $R^{4c}$ represents a hydrogen atom, and $R^{6b}$ represents any one substituent described in Table 1A to Table 3A (hereinafter referred to as "Compound group SX26").

The Compound (L-1), wherein n represents 2, $R^{4b}$ represents a 1-cyanocyclopropyl group, $R^{4c}$ represents a hydrogen atom, and $R^{6b}$ represents any one substituent described in Table 1A to Table 3A (hereinafter referred to as "Compound group SX27").

The Compound (L-1), wherein n represents 0, $R^{4b}$ represents a hydrogen atom, $R^{4c}$ represents a chlorine atom, and $R^{6b}$ represents any one substituent described in Table 1A to Table 3A (hereinafter referred to as "Compound group SX28").

The Compound (L-1), wherein n represents 1, $R^{4b}$ represents a hydrogen atom, $R^{4c}$ represents a chlorine atom, and $R^{6b}$ represents any one substituent described in Table 1A to Table 3A (hereinafter referred to as "Compound group

SX29").

The Compound (L-1), wherein n represents 2, $R^{4b}$ represents a hydrogen atom, $R^{4c}$ represents a chlorine atom, and $R^{6b}$ represents any one substituent described in Table 1A to Table 3A (hereinafter referred to as "Compound group SX30").

The Compound (L-1), wherein n represents 0, $R^{4b}$ represents a hydrogen atom, $R^{4c}$ represents a bromine atom, and $R^{6b}$ represents any one substituent described in Table 1A to Table 3A (hereinafter referred to as "Compound group SX31").

The Compound (L-1), wherein n represents 1, $R^{4b}$ represents a hydrogen atom, $R^{4c}$ represents a bromine atom, and $R^{6b}$ represents any one substituent described in Table 1A to Table 3A (hereinafter referred to as "Compound group SX32").

The Compound (L-1), wherein n represents 2, $R^{4b}$ represents a hydrogen atom, $R^{4c}$ represents a bromine atom, and $R^{6b}$ represents any one substituent described in Table 1A to Table 3A (hereinafter referred to as "Compound group SX33").

The Compound (L-1), wherein n represents 0, $R^{4b}$ represents a hydrogen atom, $R^{4c}$ represents a trifluoromethyl group, and $R^{6b}$ represents any one substituent described in Table 1A to Table 3A (hereinafter referred to as "Compound group SX34").

The Compound (L-1), wherein n represents 1, $R^{4b}$ represents a hydrogen atom, $R^{4c}$ represents a trifluoromethyl group, and $R^{6b}$ represents any one substituent described in Table 1A to Table 3A (hereinafter referred to as "Compound group SX35").

The Compound (L-1), wherein n represents 2, $R^{4b}$ represents a hydrogen atom, $R^{4c}$ represents a trifluoromethyl group, and $R^{6b}$ represents any one substituent described in Table 1A to Table 3A (hereinafter referred to as "Compound group SX36").

The Compound (L-1), wherein n represents 0, $R^{4b}$ represents a hydrogen atom, $R^{4c}$ represents a difluoromethyl group, and $R^{6b}$ represents any one substituent described in Table 1A to Table 3A (hereinafter referred to as "Compound group SX37").

The Compound (L-1), wherein n represents 1, $R^{4b}$ represents a hydrogen atom, $R^{4c}$ represents a difluoromethyl group, and $R^{6b}$ represents any one substituent described in Table 1A to Table 3A (hereinafter referred to as "Compound group SX38").

The Compound (L-1), wherein n represents 2, $R^{4b}$ represents a hydrogen atom, $R^{4c}$ represents a difluoromethyl group, and $R^{6b}$ represents any one substituent described in Table 1A to Table 3A (hereinafter referred to as "Compound group SX39").

The Compound (L-1), wherein n represents 0, $R^{4b}$ represents a hydrogen atom, $R^{4c}$ represents a trifluoromethoxy group, and $R^{6b}$ represents any one substituent described in Table 1A to Table 3A (hereinafter referred to as "Compound group SX40").

The Compound (L-1), wherein n represents 1, $R^{4b}$ represents a hydrogen atom, $R^{4c}$ represents a trifluoromethoxy group, and $R^{6b}$ represents any one substituent described in Table 1A to Table 3A (hereinafter referred to as "Compound group SX41").

The Compound (L-1), wherein n represents 2, $R^{4b}$ represents a hydrogen atom, $R^{4c}$ represents a trifluoromethoxy group, and $R^{6b}$ represents any one substituent described in Table 1A to Table 3A (hereinafter referred to as "Compound group SX42").

The Compound (L-1), wherein n represents 0, $R^{4b}$ represents a hydrogen atom, $R^{4c}$ represents a difluoromethoxy group, and $R^{6b}$ represents any one substituent described in Table 1A to Table 3A (hereinafter referred to as "Compound group SX43").

The Compound (L-1), wherein n represents 1, $R^{4b}$ represents a hydrogen atom, $R^{4c}$ represents a difluoromethoxy group, and $R^{6b}$ represents any one substituent described in Table 1A to Table 3A (hereinafter referred to as "Compound group SX44").

The Compound (L-1), wherein n represents 2, $R^{4b}$ represents a hydrogen atom, $R^{4c}$ represents a difluoromethoxy group, and $R^{6b}$ represents any one substituent described in Table 1A to Table 3A (hereinafter referred to as "Compound group SX45").

The Compound (L-1), wherein n represents 0, $R^{4b}$ represents a hydrogen atom, $R^{4c}$ represents a cyclopropyl group, and $R^{6b}$ represents any one substituent described in Table 1A to Table 3A (hereinafter referred to as "Compound group SX46").

The Compound (L-1), wherein n represents 1, $R^{4b}$ represents a hydrogen atom, $R^{4c}$ represents a cyclopropyl group, and $R^{6b}$ represents any one substituent described in Table 1A to Table 3A (hereinafter referred to as "Compound group SX47").

The Compound (L-1), wherein n represents 2, $R^{4b}$ represents a hydrogen atom, $R^{4c}$ represents a cyclopropyl group, and $R^{6b}$ represents any one substituent described in Table 1A to Table 3A (hereinafter referred to as "Compound group SX48").

The Compound (L-1), wherein n represents 0, $R^{4b}$ represents a hydrogen atom, $R^{4c}$ represents a 1-cyanocyclopropyl group, and $R^{6b}$ represents any one substituent described in Table 1A to Table 3A (hereinafter referred to as "Compound group SX49").

The Compound (L-1), wherein n represents 1, $R^{4b}$ represents a hydrogen atom, $R^{4c}$ represents a 1-cyanocyclopropyl group, and $R^{6b}$ represents any one substituent described in Table 1A to Table 3A (hereinafter referred to as "Compound group SX50").

The Compound (L-1), wherein n represents 2, $R^{4b}$ represents a hydrogen atom, $R^{4c}$ represents a 1-cyanocyclopropyl group, and $R^{6b}$ represents any one substituent described in Table 1A to Table 3A (hereinafter referred to as "Compound group SX51").

[Table 1A]

| |
|---|
| $CF_3$ |
| $CHF_2$ |
| $CH_2CF_3$ |
| $CF_2CF_3$ |
| $CH_2CF_2CF_3$ |
| $CF_2CF_2CF_3$ |
| $CF_2CF_2CF_2CF_3$ |
| $CF_2CF_2CF_2CF_2CF_3$ |
| $OCF_3$ |
| $OCHF_2$ |
| $OCH_2CF_3$ |
| $OCH_2CHF_2$ |
| $OCF_2CF_3$ |
| $OCH(CH_3)CF_3$ |
| $OCH_2CF_2CHF_2$ |
| $OCH_2CF_2CF_3$ |
| $OCF_2CF_2CF_3$ |
| $OCH_2CF_2CHFCF_3$ |
| $OCH_2CF_2CF_2CF_{\cdot 3}$ |
| $OCF_2CF_2CF_2CF_3$ |
| $OCH_2CF_2CF_2CF_2CF_{\cdot 3}$ |
| $OCH_2C(Me)_2CN$ |
| $OCH_2$(1-CN-c-Pr) |
| $OCH_2$(2,2-$F_2$-c-Pr) |
| $OCH_2$(1-$CF_3$-c-Pr) |
| $OS(O)_2CF_3$ |
| $OS(O)_2CF_2CF_3$ |
| $OS(O)_2CF_2CF_2CF_3$ |

[Table 2A]

| |
|---|
| $SCF_3$ |
| $SCH_2CF_3$ |
| $SCF_2CF_3$ |

(continued)

| |
|---|
| $SCH_2CF_2CF_3$ |
| $SCF_2CF_2CF_3$ |
| $SCH_2CF_2CF_2CF_3$ |
| $SCF_2CF_2CF_2CF_3$ |
| $S(O)CF_3$ |
| $S(O)CH_2CF_3$ |
| $S(O)CF_2CF_3$ |
| $S(O)CH_2CF_2CF_3$ |
| $S(O)CF_2CF_2CF_3$ |
| $S(O)CH_2CF_2CF_2CF_3$ |
| $S(O)CF_2CF_2CF_2CF_3$ |
| $S(O)_2CF_3$ |
| $S(O)_2CH_2CF_3$ |
| $S(O)_2CF_2CF_3$ |
| $S(O)_2CH_2CF_2CF_3)$ |
| $S(O)_2CF_2CF_2CF_3)$ |
| $S(O)_2CH_2CF_2CF_2CF_3$ |
| $S(O)_2CF_2CF_2CF_2CF_3)$ |
| $OS(O)_2CF_3$ |
| $OS(O)_2CH_2CF_3)$ |
| $OS(O)_2CF_2CF_3)$ |
| $OS(O)_2CH_2CF_2CF_3$ |
| $OS(O)_2CF_2CF_2CF_3$ |
| $OS(O)_2CH_2CF_2CF_2CF_3$ |
| $OS(O)_2CF_2CF_2CF_2CF_3)$ |

[Table 3A]

| |
|---|
| F |
| Cl |
| Br |
| I |
| Me |
| Et |
| Pr |
| i-Pr |
| Bu |
| Hex |
| $CH=CH_2$ |
| $CMe=CH_2$ |
| 1-F-c-Pr |

(continued)

| |
|---|
| 2,2-$F_2$-c-Pr |
| c-Pr |
| c-Bu |
| 1-CN-c-Pr |
| 1-CN-c-Bu |

A compound represented by formula (L-2)

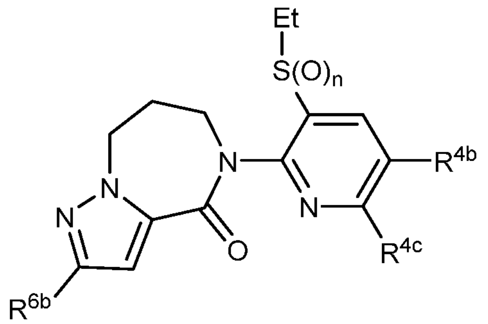

( L-2 )

(hereinafter referred to as "Compound (L-2)"), wherein n represents 0, $R^{4b}$ represents a hydrogen atom, $R^{4c}$ represents a hydrogen atom, and $R^{6b}$ represents any one substituent described in Table 1A to Table 3A (hereinafter referred to as "Compound group SX52").

The Compound (L-2), wherein n represents 1, $R^{4b}$ represents a hydrogen atom, $R^{4c}$ represents a hydrogen atom, and $R^{6b}$ represents any one substituent described in Table 1A to Table 3A (hereinafter referred to as "Compound group SX53").

The Compound (L-2), wherein n represents 2, $R^{4b}$ represents a hydrogen atom, $R^{4c}$ represents a hydrogen atom, and $R^{6b}$ represents any one substituent described in Table 1A to Table 3A (hereinafter referred to as "Compound group SX54").

The Compound (L-2), wherein n represents 0, $R^{4b}$ represents a chlorine atom, $R^{4c}$ represents a hydrogen atom, and $R^{6b}$ represents any one substituent described in Table 1A to Table 3A (hereinafter referred to as "Compound group SX55").

The Compound (L-2), wherein n represents 1, $R^{4b}$ represents a chlorine atom, $R^{4c}$ represents a hydrogen atom, and $R^{6b}$ represents any one substituent described in Table 1A to Table 3A (hereinafter referred to as "Compound group SX56").

The Compound (L-2), wherein n represents 2, $R^{4b}$ represents a chlorine atom, $R^{4c}$ represents a hydrogen atom, and $R^{6b}$ represents any one substituent described in Table 1A to Table 3A (hereinafter referred to as "Compound group SX57").

The Compound (L-2), wherein n represents 0, $R^{4b}$ represents a bromine atom, $R^{4c}$ represents a hydrogen atom, and $R^{6b}$ represents any one substituent described in Table 1A to Table 3A (hereinafter referred to as "Compound group SX58").

The Compound (L-2), wherein n represents 1, $R^{4b}$ represents a bromine atom, $R^{4c}$ represents a hydrogen atom, and $R^{6b}$ represents any one substituent described in Table 1A to Table 3A (hereinafter referred to as "Compound group SX59").

The Compound (L-2), wherein n represents 2, $R^{4b}$ represents a bromine atom, $R^{4c}$ represents a hydrogen atom, and $R^{6b}$ represents any one substituent described in Table 1A to Table 3A (hereinafter referred to as "Compound group SX60").

The Compound (L-2), wherein n represents 0, $R^{4b}$ represents a trifluoromethyl group, $R^{4c}$ represents a hydrogen atom, and $R^{6b}$ represents any one substituent described in Table 1A to Table 3A (hereinafter referred to as "Compound group SX61").

The Compound (L-2), wherein n represents 1, $R^{4b}$ represents a trifluoromethyl group, $R^{4c}$ represents a hydrogen atom, and $R^{6b}$ represents any one substituent described in Table 1A to Table 3A (hereinafter referred to as "Compound group SX62").

The Compound (L-2), wherein n represents 2, $R^{4b}$ represents a trifluoromethyl group, $R^{4c}$ represents a hydrogen atom, and $R^{6b}$ represents any one substituent described in Table 1A to Table 3A (hereinafter referred to as "Compound

group SX63").

The Compound (L-2), wherein n represents 0, $R^{4b}$ represents a difluoromethyl group, $R^{4c}$ represents a hydrogen atom, and $R^{6b}$ represents any one substituent described in Table 1A to Table 3A (hereinafter referred to as "Compound group SX64").

The Compound (L-2), wherein n represents 1, $R^{4b}$ represents a difluoromethyl group, $R^{4c}$ represents a hydrogen atom, and $R^{6b}$ represents any one substituent described in Table 1A to Table 3A (hereinafter referred to as "Compound group SX65").

The Compound (L-2), wherein n represents 2, $R^{4b}$ represents a difluoromethyl group, $R^{4c}$ represents a hydrogen atom, and $R^{6b}$ represents any one substituent described in Table 1A to Table 3A (hereinafter referred to as "Compound group SX66").

The Compound (L-2), wherein n represents 0, $R^{4b}$ represents a trifluoromethoxy group, $R^{4c}$ represents a hydrogen atom, and $R^{6b}$ represents any one substituent described in Table 1A to Table 3A (hereinafter referred to as "Compound group SX67").

The Compound (L-2), wherein n represents 1, $R^{4b}$ represents a trifluoromethoxy group, $R^{4c}$ represents a hydrogen atom, and $R^{6b}$ represents any one substituent described in Table 1A to Table 3A (hereinafter referred to as "Compound group SX68").

The Compound (L-2), wherein n represents 2, $R^{4b}$ represents a trifluoromethoxy group, $R^{4c}$ represents a hydrogen atom, and $R^{6b}$ represents any one substituent described in Table 1A to Table 3A (hereinafter referred to as "Compound group SX69").

The Compound (L-2), wherein n represents 0, $R^{4b}$ represents a difluoromethoxy group, $R^{4c}$ represents a hydrogen atom, and $R^{6b}$ represents any one substituent described in Table 1A to Table 3A (hereinafter referred to as "Compound group SX70").

The Compound (L-2), wherein n represents 1, $R^{4b}$ represents a difluoromethoxy group, $R^{4c}$ represents a hydrogen atom, and $R^{6b}$ represents any one substituent described in Table 1A to Table 3A (hereinafter referred to as "Compound group SX71").

The Compound (L-2), wherein n represents 2, $R^{4b}$ represents a difluoromethoxy group, $R^{4c}$ represents a hydrogen atom, and $R^{6b}$ represents any one substituent described in Table 1A to Table 3A (hereinafter referred to as "Compound group SX72").

The Compound (L-2), wherein n represents 0, $R^{4b}$ represents a cyclopropyl group, $R^{4c}$ represents a hydrogen atom, and $R^{6b}$ represents any one substituent described in Table 1A to Table 3A (hereinafter referred to as "Compound group SX73").

The Compound (L-2), wherein n represents 1, $R^{4b}$ represents a cyclopropyl group, $R^{4c}$ represents a hydrogen atom, and $R^{6b}$ represents any one substituent described in Table 1A to Table 3A (hereinafter referred to as "Compound group SX74").

The Compound (L-2), wherein n represents 2, $R^{4b}$ represents a cyclopropyl group, $R^{4c}$ represents a hydrogen atom, and $R^{6b}$ represents any one substituent described in Table 1A to Table 3A (hereinafter referred to as "Compound group SX75").

The Compound (L-2), wherein n represents 0, $R^{4b}$ represents a 1-cyanocyclopropyl group, $R^{4c}$ represents a hydrogen atom, and $R^{6b}$ represents any one substituent described in Table 1A to Table 3A (hereinafter referred to as "Compound group SX76").

The Compound (L-2), wherein n represents 1, $R^{4b}$ represents a 1-cyanocyclopropyl group, $R^{4c}$ represents a hydrogen atom, and $R^{6b}$ represents any one substituent described in Table 1A to Table 3A (hereinafter referred to as "Compound group SX77").

The Compound (L-2), wherein n represents 2, $R^{4b}$ represents a 1-cyanocyclopropyl group, $R^{4c}$ represents a hydrogen atom, and $R^{6b}$ represents any one substituent described in Table 1A to Table 3A (hereinafter referred to as "Compound group SX78").

The Compound (L-2), wherein n represents 0, $R^{4b}$ represents a hydrogen atom, $R^{4c}$ represents a chlorine atom, and $R^{6b}$ represents any one substituent described in Table 1A to Table 3A (hereinafter referred to as "Compound group SX79").

The Compound (L-2), wherein n represents 1, $R^{4b}$ represents a hydrogen atom, $R^{4c}$ represents a chlorine atom, and $R^{6b}$ represents any one substituent described in Table 1A to Table 3A (hereinafter referred to as "Compound group SX80").

The Compound (L-2), wherein n represents 2, $R^{4b}$ represents a hydrogen atom, $R^{4c}$ represents a chlorine atom, and $R^{6b}$ represents any one substituent described in Table 1A to Table 3A (hereinafter referred to as "Compound group SX81").

The Compound (L-2), wherein n represents 0, $R^{4b}$ represents a hydrogen atom, $R^{4c}$ represents a bromine atom, and $R^{6b}$ represents any one substituent described in Table 1A to Table 3A (hereinafter referred to as "Compound group SX82").

The Compound (L-2), wherein n represents 1, $R^{4b}$ represents a hydrogen atom, $R^{4c}$ represents a bromine atom, and $R^{6b}$ represents any one substituent described in Table 1A to Table 3A (hereinafter referred to as "Compound group SX83").

The Compound (L-2), wherein n represents 2, $R^{4b}$ represents a hydrogen atom, $R^{4c}$ represents a bromine atom, and $R^{6b}$ represents any one substituent described in Table 1A to Table 3A (hereinafter referred to as "Compound group SX84").

The Compound (L-2), wherein n represents 0, $R^{4b}$ represents a hydrogen atom, $R^{4c}$ represents a trifluoromethyl group, and $R^{6b}$ represents any one substituent described in Table 1A to Table 3A (hereinafter referred to as "Compound group SX85").

The Compound (L-2), wherein n represents 1, $R^{4b}$ represents a hydrogen atom, $R^{4c}$ represents a trifluoromethyl group, and $R^{6b}$ represents any one substituent described in Table 1A to Table 3A (hereinafter referred to as "Compound group SX86").

The Compound (L-2), wherein n represents 2, $R^{4b}$ represents a hydrogen atom, $R^{4c}$ represents a trifluoromethyl group, and $R^{6b}$ represents any one substituent described in Table 1A to Table 3A (hereinafter referred to as "Compound group SX87").

The Compound (L-2), wherein n represents 0, $R^{4b}$ represents a hydrogen atom, $R^{4c}$ represents a difluoromethyl group, and $R^{6b}$ represents any one substituent described in Table 1A to Table 3A (hereinafter referred to as "Compound group SX88").

The Compound (L-2), wherein n represents 1, $R^{4b}$ represents a hydrogen atom, $R^{4c}$ represents a difluoromethyl group, and $R^{6b}$ represents any one substituent described in Table 1A to Table 3A (hereinafter referred to as "Compound group SX89").

The Compound (L-2), wherein n represents 2, $R^{4b}$ represents a hydrogen atom, $R^{4c}$ represents a difluoromethyl group, and $R^{6b}$ represents any one substituent described in Table 1A to Table 3A (hereinafter referred to as "Compound group SX90").

The Compound (L-2), wherein n represents 0, $R^{4b}$ represents a hydrogen atom, $R^{4c}$ represents a trifluoromethoxy group, and $R^{6b}$ represents any one substituent described in Table 1A to Table 3A (hereinafter referred to as "Compound group SX91").

The Compound (L-2), wherein n represents 1, $R^{4b}$ represents a hydrogen atom, $R^{4c}$ represents a trifluoromethoxy group, and $R^{6b}$ represents any one substituent described in Table 1A to Table 3A (hereinafter referred to as "Compound group SX92").

The Compound (L-2), wherein n represents 2, $R^{4b}$ represents a hydrogen atom, $R^{4c}$ represents a trifluoromethoxy group, and $R^{6b}$ represents any one substituent described in Table 1A to Table 3A (hereinafter referred to as "Compound group SX93").

The Compound (L-2), wherein n represents 0, $R^{4b}$ represents a hydrogen atom, $R^{4c}$ represents a difluoromethoxy group, and $R^{6b}$ represents any one substituent described in Table 1A to Table 3A (hereinafter referred to as "Compound group SX94").

The Compound (L-2), wherein n represents 1, $R^{4b}$ represents a hydrogen atom, $R^{4c}$ represents a difluoromethoxy group, and $R^{6b}$ represents any one substituent described in Table 1A to Table 3A (hereinafter referred to as "Compound group SX95").

The Compound (L-2), wherein n represents 2, $R^{4b}$ represents a hydrogen atom, $R^{4c}$ represents a difluoromethoxy group, and $R^{6b}$ represents any one substituent described in Table 1A to Table 3A (hereinafter referred to as "Compound group SX96").

The Compound (L-2), wherein n represents 0, $R^{4b}$ represents a hydrogen atom, $R^{4c}$ represents a cyclopropyl group, and $R^{6b}$ represents any one substituent described in Table 1A to Table 3A (hereinafter referred to as "Compound group SX97").

The Compound (L-2), wherein n represents 1, $R^{4b}$ represents a hydrogen atom, $R^{4c}$ represents a cyclopropyl group, and $R^{6b}$ represents any one substituent described in Table 1A to Table 3A (hereinafter referred to as "Compound group SX98").

The Compound (L-2), wherein n represents 2, $R^{4b}$ represents a hydrogen atom, $R^{4c}$ represents a cyclopropyl group, and $R^{6b}$ represents any one substituent described in Table 1A to Table 3A (hereinafter referred to as "Compound group SX99").

The Compound (L-2), wherein n represents 0, $R^{4b}$ represents a hydrogen atom, $R^{4c}$ represents a 1-cyanocyclopropyl group, and $R^{6b}$ represents any one substituent described in Table 1A to Table 3A (hereinafter referred to as "Compound group SX100").

The Compound (L-2), wherein n represents 1, $R^{4b}$ represents a hydrogen atom, $R^{4c}$ represents a 1-cyanocyclopropyl group, and $R^{6b}$ represents any one substituent described in Table 1A to Table 3A (hereinafter referred to as "Compound group SX101").

The Compound (L-2), wherein n represents 2, $R^{4b}$ represents a hydrogen atom, $R^{4c}$ represents a 1-cyanocyclopropyl group, and $R^{6b}$ represents any one substituent described in Table 1A to Table 3A (hereinafter referred to as "Compound group SX102").

A compound represented by formula (L-3)

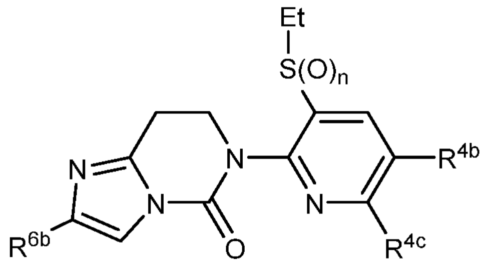

( L-3 )

(hereinafter referred to as "Compound (L-3)"), wherein n represents 0, $R^{4b}$ represents a hydrogen atom, $R^{4c}$ represents a hydrogen atom, and $R^{6b}$ represents any one substituent described in Table 1A to Table 3A (hereinafter referred to as "Compound group SX103").

The Compound (L-3), wherein n represents 1, $R^{4b}$ represents a hydrogen atom, $R^{4c}$ represents a hydrogen atom, and $R^{6b}$ represents any one substituent described in Table 1A to Table 3A (hereinafter referred to as "Compound group SX104").

The Compound (L-3), wherein n represents 2, $R^{4b}$ represents a hydrogen atom, $R^{4c}$ represents a hydrogen atom, and $R^{6b}$ represents any one substituent described in Table 1A to Table 3A (hereinafter referred to as "Compound group SX105").

The Compound (L-3), wherein n represents 0, $R^{4b}$ represents a chlorine atom, $R^{4c}$ represents a hydrogen atom, and $R^{6b}$ represents any one substituent described in Table 1A to Table 3A (hereinafter referred to as "Compound group SX106").

The Compound (L-3), wherein n represents 1, $R^{4b}$ represents a chlorine atom, $R^{4c}$ represents a hydrogen atom, and $R^{6b}$ represents any one substituent described in Table 1A to Table 3A (hereinafter referred to as "Compound group SX107").

The Compound (L-3), wherein n represents 2, $R^{4b}$ represents a chlorine atom, $R^{4c}$ represents a hydrogen atom, and $R^{6b}$ represents any one substituent described in Table 1A to Table 3A (hereinafter referred to as "Compound group SX108").

The Compound (L-3), wherein n represents 0, $R^{4b}$ represents a bromine atom, $R^{4c}$ represents a hydrogen atom, and $R^{6b}$ represents any one substituent described in Table 1A to Table 3A (hereinafter referred to as "Compound group SX109").

The Compound (L-3), wherein n represents 1, $R^{4b}$ represents a bromine atom, $R^{4c}$ represents a hydrogen atom, and $R^{6b}$ represents any one substituent described in Table 1A to Table 3A (hereinafter referred to as "Compound group SX110").

The Compound (L-3), wherein n represents 2, $R^{4b}$ represents a bromine atom, $R^{4c}$ represents a hydrogen atom, and $R^{6b}$ represents any one substituent described in Table 1A to Table 3A (hereinafter referred to as "Compound group SX111").

The Compound (L-3), wherein n represents 0, $R^{4b}$ represents a trifluoromethyl group, $R^{4c}$ represents a hydrogen atom, and $R^{6b}$ represents any one substituent described in Table 1A to Table 3A (hereinafter referred to as "Compound group SX112").

The Compound (L-3), wherein n represents 1, $R^{4b}$ represents a trifluoromethyl group, $R^{4c}$ represents a hydrogen atom, and $R^{6b}$ represents any one substituent described in Table 1A to Table 3A (hereinafter referred to as "Compound group SX113").

The Compound (L-3), wherein n represents 2, $R^{4b}$ represents a trifluoromethyl group, $R^{4c}$ represents a hydrogen atom, and $R^{6b}$ represents any one substituent described in Table 1A to Table 3A (hereinafter referred to as "Compound group SX114").

The Compound (L-3), wherein n represents 0, $R^{4b}$ represents a difluoromethyl group, $R^{4c}$ represents a hydrogen atom, and $R^{6b}$ represents any one substituent described in Table 1A to Table 3A (hereinafter referred to as "Compound group SX115").

The Compound (L-3), wherein n represents 1, $R^{4b}$ represents a difluoromethyl group, $R^{4c}$ represents a hydrogen atom, and $R^{6b}$ represents any one substituent described in Table 1A to Table 3A (hereinafter referred to as "Compound group SX116").

The Compound (L-3), wherein n represents 2, $R^{4b}$ represents a difluoromethyl group, $R^{4c}$ represents a hydrogen atom, and $R^{6b}$ represents any one substituent described in Table 1A to Table 3A (hereinafter referred to as "Compound

group SX117").

The Compound (L-3), wherein n represents 0, $R^{4b}$ represents a trifluoromethoxy group, $R^{4c}$ represents a hydrogen atom, and $R^{6b}$ represents any one substituent described in Table 1A to Table 3A (hereinafter referred to as "Compound group SX118").

The Compound (L-3), wherein n represents 1, $R^{4b}$ represents a trifluoromethoxy group, $R^{4c}$ represents a hydrogen atom, and $R^{6b}$ represents any one substituent described in Table 1A to Table 3A (hereinafter referred to as "Compound group SX119").

The Compound (L-3), wherein n represents 2, $R^{4b}$ represents a trifluoromethoxy group, $R^{4c}$ represents a hydrogen atom, and $R^{6b}$ represents any one substituent described in Table 1A to Table 3A (hereinafter referred to as "Compound group SX120").

The Compound (L-3), wherein n represents 0, $R^{4b}$ represents a difluoromethoxy group, $R^{4c}$ represents a hydrogen atom, and $R^{6b}$ represents any one substituent described in Table 1A to Table 3A (hereinafter referred to as "Compound group SX121").

The Compound (L-3), wherein n represents 1, $R^{4b}$ represents a difluoromethoxy group, $R^{4c}$ represents a hydrogen atom, and $R^{6b}$ represents any one substituent described in Table 1A to Table 3A (hereinafter referred to as "Compound group SX122").

The Compound (L-3), wherein n represents 2, $R^{4b}$ represents a difluoromethoxy group, $R^{4c}$ represents a hydrogen atom, and $R^{6b}$ represents any one substituent described in Table 1A to Table 3A (hereinafter referred to as "Compound group SX123").

The Compound (L-3), wherein n represents 0, $R^{4b}$ represents a cyclopropyl group, $R^{4c}$ represents a hydrogen atom, and $R^{6b}$ represents any one substituent described in Table 1A to Table 3A (hereinafter referred to as "Compound group SX124").

The Compound (L-3), wherein n represents 1, $R^{4b}$ represents a cyclopropyl group, $R^{4c}$ represents a hydrogen atom, and $R^{6b}$ represents any one substituent described in Table 1A to Table 3A (hereinafter referred to as "Compound group SX125").

The Compound (L-3), wherein n represents 2, $R^{4b}$ represents a cyclopropyl group, $R^{4c}$ represents a hydrogen atom, and $R^{6b}$ represents any one substituent described in Table 1A to Table 3A (hereinafter referred to as "Compound group SX126").

The Compound (L-3), wherein n represents 0, $R^{4b}$ represents a 1-cyanocyclopropyl group, $R^{4c}$ represents a hydrogen atom, and $R^{6b}$ represents any one substituent described in Table 1A to Table 3A (hereinafter referred to as "Compound group SX127").

The Compound (L-3), wherein n represents 1, $R^{4b}$ represents a 1-cyanocyclopropyl group, $R^{4c}$ represents a hydrogen atom, and $R^{6b}$ represents any one substituent described in Table 1A to Table 3A (hereinafter referred to as "Compound group SX128").

The Compound (L-3), wherein n represents 2, $R^{4b}$ represents a 1-cyanocyclopropyl group, $R^{4c}$ represents a hydrogen atom, and $R^{6b}$ represents any one substituent described in Table 1A to Table 3A (hereinafter referred to as "Compound group SX129").

The Compound (L-3), wherein n represents 0, $R^{4b}$ represents a hydrogen atom, $R^{4c}$ represents a chlorine atom, and $R^{6b}$ represents any one substituent described in Table 1A to Table 3A (hereinafter referred to as "Compound group SX130").

The Compound (L-3), wherein n represents 1, $R^{4b}$ represents a hydrogen atom, $R^{4c}$ represents a chlorine atom, and $R^{6b}$ represents any one substituent described in Table 1A to Table 3A (hereinafter referred to as "Compound group SX131").

The Compound (L-3), wherein n represents 2, $R^{4b}$ represents a hydrogen atom, $R^{4c}$ represents a chlorine atom, and $R^{6b}$ represents any one substituent described in Table 1A to Table 3A (hereinafter referred to as "Compound group SX132").

The Compound (L-3), wherein n represents 0, $R^{4b}$ represents a hydrogen atom, $R^{4c}$ represents a bromine atom, and $R^{6b}$ represents any one substituent described in Table 1A to Table 3A (hereinafter referred to as "Compound group SX133").

The Compound (L-3), wherein n represents 1, $R^{4b}$ represents a hydrogen atom, $R^{4c}$ represents a bromine atom, and $R^{6b}$ represents any one substituent described in Table 1A to Table 3A (hereinafter referred to as "Compound group SX134").

The Compound (L-3), wherein n represents 2, $R^{4b}$ represents a hydrogen atom, $R^{4c}$ represents a bromine atom, and $R^{6b}$ represents any one substituent described in Table 1A to Table 3A (hereinafter referred to as "Compound group SX135").

The Compound (L-3), wherein n represents 0, $R^{4b}$ represents a hydrogen atom, $R^{4c}$ represents a trifluoromethyl group, and $R^{6b}$ represents any one substituent described in Table 1A to Table 3A (hereinafter referred to as "Compound group SX136").

The Compound (L-3), wherein n represents 1, $R^{4b}$ represents a hydrogen atom, $R^{4c}$ represents a trifluoromethyl group, and $R^{6b}$ represents any one substituent described in Table 1A to Table 3A (hereinafter referred to as "Compound group SX137").

The Compound (L-3), wherein n represents 2, $R^{4b}$ represents a hydrogen atom, $R^{4c}$ represents a trifluoromethyl group, and $R^{6b}$ represents any one substituent described in Table 1A to Table 3A (hereinafter referred to as "Compound group SX138").

The Compound (L-3), wherein n represents 0, $R^{4b}$ represents a hydrogen atom, $R^{4c}$ represents a difluoromethyl group, and $R^{6b}$ represents any one substituent described in Table 1A to Table 3A (hereinafter referred to as "Compound group SX139").

The Compound (L-3), wherein n represents 1, $R^{4b}$ represents a hydrogen atom, $R^{4c}$ represents a difluoromethyl group, and $R^{6b}$ represents any one substituent described in Table 1A to Table 3A (hereinafter referred to as "Compound group SX140").

The Compound (L-3), wherein n represents 2, $R^{4b}$ represents a hydrogen atom, $R^{4c}$ represents a difluoromethyl group, and $R^{6b}$ represents any one substituent described in Table 1A to Table 3A (hereinafter referred to as "Compound group SX141").

The Compound (L-3), wherein n represents 0, $R^{4b}$ represents a hydrogen atom, $R^{4c}$ represents a trifluoromethoxy group, and $R^{6b}$ represents any one substituent described in Table 1A to Table 3A (hereinafter referred to as "Compound group SX142").

The Compound (L-3), wherein n represents 1, $R^{4b}$ represents a hydrogen atom, $R^{4c}$ represents a trifluoromethoxy group, and $R^{6b}$ represents any one substituent described in Table 1A to Table 3A (hereinafter referred to as "Compound group SX143").

The Compound (L-3), wherein n represents 2, $R^{4b}$ represents a hydrogen atom, $R^{4c}$ represents a trifluoromethoxy group, and $R^{6b}$ represents any one substituent described in Table 1A to Table 3A (hereinafter referred to as "Compound group SX144").

The Compound (L-3), wherein n represents 0, $R^{4b}$ represents a hydrogen atom, $R^{4c}$ represents a difluoromethoxy group, and $R^{6b}$ represents any one substituent described in Table 1A to Table 3A (hereinafter referred to as "Compound group SX145").

The Compound (L-3), wherein n represents 1, $R^{4b}$ represents a hydrogen atom, $R^{4c}$ represents a difluoromethoxy group, and $R^{6b}$ represents any one substituent described in Table 1A to Table 3A (hereinafter referred to as "Compound group SX146").

The Compound (L-3), wherein n represents 2, $R^{4b}$ represents a hydrogen atom, $R^{4c}$ represents a difluoromethoxy group, and $R^{6b}$ represents any one substituent described in Table 1A to Table 3A (hereinafter referred to as "Compound group SX147").

The Compound (L-3), wherein n represents 0, $R^{4b}$ represents a hydrogen atom, $R^{4c}$ represents a cyclopropyl group, and $R^{6b}$ represents any one substituent described in Table 1A to Table 3A (hereinafter referred to as "Compound group SX148").

The Compound (L-3), wherein n represents 1, $R^{4b}$ represents a hydrogen atom, $R^{4c}$ represents a cyclopropyl group, and $R^{6b}$ represents any one substituent described in Table 1A to Table 3A (hereinafter referred to as "Compound group SX149").

The Compound (L-3), wherein n represents 2, $R^{4b}$ represents a hydrogen atom, $R^{4c}$ represents a cyclopropyl group, and $R^{6b}$ represents any one substituent described in Table 1A to Table 3A (hereinafter referred to as "Compound group SX150").

The Compound (L-3), wherein n represents 0, $R^{4b}$ represents a hydrogen atom, $R^{4c}$ represents a 1-cyanocyclopropyl group, and $R^{6b}$ represents any one substituent described in Table 1A to Table 3A (hereinafter referred to as "Compound group SX151").

The Compound (L-3), wherein n represents 1, $R^{4b}$ represents a hydrogen atom, $R^{4c}$ represents a 1-cyanocyclopropyl group, and $R^{6b}$ represents any one substituent described in Table 1A to Table 3A (hereinafter referred to as "Compound group SX152").

The Compound (L-3), wherein n represents 2, $R^{4b}$ represents a hydrogen atom, $R^{4c}$ represents a 1-cyanocyclopropyl group, and $R^{6b}$ represents any one substituent described in Table 1A to Table 3A (hereinafter referred to as "Compound group SX153").

A compound represented by formula (L-4)

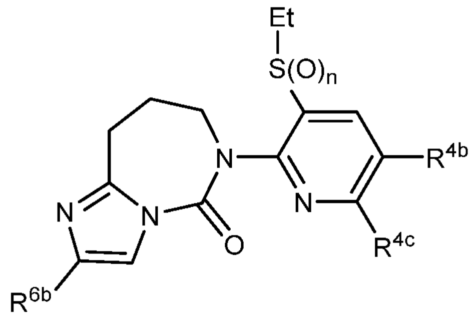

( L-4 )

(hereinafter referred to as "Compound (L-4)"), wherein n represents 0, $R^{4b}$ represents a hydrogen atom, $R^{4c}$ represents a hydrogen atom, and $R^{6b}$ represents any one substituent described in Table 1A to Table 3A (hereinafter referred to as "Compound group SX154").

The Compound (L-4), wherein n represents 1, $R^{4b}$ represents a hydrogen atom, $R^{4c}$ represents a hydrogen atom, and $R^{6b}$ represents any one substituent described in Table 1A to Table 3A (hereinafter referred to as "Compound group SX155").

The Compound (L-4), wherein n represents 2, $R^{4b}$ represents a hydrogen atom, $R^{4c}$ represents a hydrogen atom, and $R^{6b}$ represents any one substituent described in Table 1A to Table 3A (hereinafter referred to as "Compound group SX156").

The Compound (L-4), wherein n represents 0, $R^{4b}$ represents a chlorine atom, $R^{4c}$ represents a hydrogen atom, and $R^{6b}$ represents any one substituent described in Table 1A to Table 3A (hereinafter referred to as "Compound group SX157").

The Compound (L-4), wherein n represents 1, $R^{4b}$ represents a chlorine atom, $R^{4c}$ represents a hydrogen atom, and $R^{6b}$ represents any one substituent described in Table 1A to Table 3A (hereinafter referred to as "Compound group SX158").

The Compound (L-4), wherein n represents 2, $R^{4b}$ represents a chlorine atom, $R^{4c}$ represents a hydrogen atom, and $R^{6b}$ represents any one substituent described in Table 1A to Table 3A (hereinafter referred to as "Compound group SX159").

The Compound (L-4), wherein n represents 0, $R^{4b}$ represents a bromine atom, $R^{4c}$ represents a hydrogen atom, and $R^{6b}$ represents any one substituent described in Table 1A to Table 3A (hereinafter referred to as "Compound group SX160") .

The Compound (L-4), wherein n represents 1, $R^{4b}$ represents a bromine atom, $R^{4c}$ represents a hydrogen atom, and $R^{6b}$ represents any one substituent described in Table 1A to Table 3A (hereinafter referred to as "Compound group SX161").

The Compound (L-4), wherein n represents 2, $R^{4b}$ represents a bromine atom, $R^{4c}$ represents a hydrogen atom, and $R^{6b}$ represents any one substituent described in Table 1A to Table 3A (hereinafter referred to as "Compound group SX162") .

The Compound (L-4), wherein n represents 0, $R^{4b}$ represents a trifluoromethyl group, $R^{4c}$ represents a hydrogen atom, and $R^{6b}$ represents any one substituent described in Table 1A to Table 3A (hereinafter referred to as "Compound group SX163").

The Compound (L-4), wherein n represents 1, $R^{4b}$ represents a trifluoromethyl group, $R^{4c}$ represents a hydrogen atom, and $R^{6b}$ represents any one substituent described in Table 1A to Table 3A (hereinafter referred to as "Compound group SX164").

The Compound (L-4), wherein n represents 2, $R^{4b}$ represents a trifluoromethyl group, $R^{4c}$ represents a hydrogen atom, and $R^{6b}$ represents any one substituent described in Table 1A to Table 3A (hereinafter referred to as "Compound group SX165").

The Compound (L-4), wherein n represents 0, $R^{4b}$ represents a difluoromethyl group, $R^{4c}$ represents a hydrogen atom, and $R^{6b}$ represents any one substituent described in Table 1A to Table 3A (hereinafter referred to as "Compound group SX166").

The Compound (L-4), wherein n represents 1, $R^{4b}$ represents a difluoromethyl group, $R^{4c}$ represents a hydrogen atom, and $R^{6b}$ represents any one substituent described in Table 1A to Table 3A (hereinafter referred to as "Compound group SX167").

The Compound (L-4), wherein n represents 2, $R^{4b}$ represents a difluoromethyl group, $R^{4c}$ represents a hydrogen atom, and $R^{6b}$ represents any one substituent described in Table 1A to Table 3A (hereinafter referred to as "Compound group SX168").

The Compound (L-4), wherein n represents 0, $R^{4b}$ represents a trifluoromethoxy group, $R^{4c}$ represents a hydrogen atom, and $R^{6b}$ represents any one substituent described in Table 1A to Table 3A (hereinafter referred to as

"Compound group SX169").

The Compound (L-4), wherein n represents 1, $R^{4b}$ represents a trifluoromethoxy group, $R^{4c}$ represents a hydrogen atom, and $R^{6b}$ represents any one substituent described in Table 1A to Table 3A (hereinafter referred to as "Compound group SX170").

The Compound (L-4), wherein n represents 2, $R^{4b}$ represents a trifluoromethoxy group, $R^{4c}$ represents a hydrogen atom, and $R^{6b}$ represents any one substituent described in Table 1A to Table 3A (hereinafter referred to as "Compound group SX171").

The Compound (L-4), wherein n represents 0, $R^{4b}$ represents a difluoromethoxy group, $R^{4c}$ represents a hydrogen atom, and $R^{6b}$ represents any one substituent described in Table 1A to Table 3A (hereinafter referred to as "Compound group SX172").

The Compound (L-4), wherein n represents 1, $R^{4b}$ represents a difluoromethoxy group, $R^{4c}$ represents a hydrogen atom, and $R^{6b}$ represents any one substituent described in Table 1A to Table 3A (hereinafter referred to as "Compound group SX173").

The Compound (L-4), wherein n represents 2, $R^{4b}$ represents a difluoromethoxy group, $R^{4c}$ represents a hydrogen atom, and $R^{6b}$ represents any one substituent described in Table 1A to Table 3A (hereinafter referred to as "Compound group SX174").

The Compound (L-4), wherein n represents 0, $R^{4b}$ represents a cyclopropyl group, $R^{4c}$ represents a hydrogen atom, and $R^{6b}$ represents any one substituent described in Table 1A to Table 3A (hereinafter referred to as "Compound group SX175").

The Compound (L-4), wherein n represents 1, $R^{4b}$ represents a cyclopropyl group, $R^{4c}$ represents a hydrogen atom, and $R^{6b}$ represents any one substituent described in Table 1A to Table 3A (hereinafter referred to as "Compound group SX176").

The Compound (L-4), wherein n represents 2, $R^{4b}$ represents a cyclopropyl group, $R^{4c}$ represents a hydrogen atom, and $R^{6b}$ represents any one substituent described in Table 1A to Table 3A (hereinafter referred to as "Compound group SX177").

The Compound (L-4), wherein n represents 0, $R^{4b}$ represents a 1-cyanocyclopropyl group, $R^{4c}$ represents a hydrogen atom, and $R^{6b}$ represents any one substituent described in Table 1A to Table 3A (hereinafter referred to as "Compound group SX178").

The Compound (L-4), wherein n represents 1, $R^{4b}$ represents a 1-cyanocyclopropyl group, $R^{4c}$ represents a hydrogen atom, and $R^{6b}$ represents any one substituent described in Table 1A to Table 3A (hereinafter referred to as "Compound group SX179").

The Compound (L-4), wherein n represents 2, $R^{4b}$ represents a 1-cyanocyclopropyl group, $R^{4c}$ represents a hydrogen atom, and $R^{6b}$ represents any one substituent described in Table 1A to Table 3A (hereinafter referred to as "Compound group SX180").

The Compound (L-4), wherein n represents 0, $R^{4b}$ represents a hydrogen atom, $R^{4c}$ represents a chlorine atom, and $R^{6b}$ represents any one substituent described in Table 1A to Table 3A (hereinafter referred to as "Compound group SX181").

The Compound (L-4), wherein n represents 1, $R^{4b}$ represents a hydrogen atom, $R^{4c}$ represents a chlorine atom, and $R^{6b}$ represents any one substituent described in Table 1A to Table 3A (hereinafter referred to as "Compound group SX182").

The Compound (L-4), wherein n represents 2, $R^{4b}$ represents a hydrogen atom, $R^{4c}$ represents a chlorine atom, and $R^{6b}$ represents any one substituent described in Table 1A to Table 3A (hereinafter referred to as "Compound group SX183").

The Compound (L-4), wherein n represents 0, $R^{4b}$ represents a hydrogen atom, $R^{4c}$ represents a bromine atom, and $R^{6b}$ represents any one substituent described in Table 1A to Table 3A (hereinafter referred to as "Compound group SX184").

The Compound (L-4), wherein n represents 1, $R^{4b}$ represents a hydrogen atom, $R^{4c}$ represents a bromine atom, and $R^{6b}$ represents any one substituent described in Table 1A to Table 3A (hereinafter referred to as "Compound group SX185").

The Compound (L-4), wherein n represents 2, $R^{4b}$ represents a hydrogen atom, $R^{4c}$ represents a bromine atom, and $R^{6b}$ represents any one substituent described in Table 1A to Table 3A (hereinafter referred to as "Compound group SX186") .

The Compound (L-4), wherein n represents 0, $R^{4b}$ represents a hydrogen atom, $R^{4c}$ represents a trifluoromethyl group, and $R^{6b}$ represents any one substituent described in Table 1A to Table 3A (hereinafter referred to as "Compound group SX187").

The Compound (L-4), wherein n represents 1, $R^{4b}$ represents a hydrogen atom, $R^{4c}$ represents a trifluoromethyl group, and $R^{6b}$ represents any one substituent described in Table 1A to Table 3A (hereinafter referred to as "Compound group SX188").

The Compound (L-4), wherein n represents 2, $R^{4b}$ represents a hydrogen atom, $R^{4c}$ represents a trifluoromethyl group, and $R^{6b}$ represents any one substituent described in Table 1A to Table 3A (hereinafter referred to as "Compound group SX189").

The Compound (L-4), wherein n represents 0, $R^{4b}$ represents a hydrogen atom, $R^{4c}$ represents a difluoromethyl group, and $R^{6b}$ represents any one substituent described in Table 1A to Table 3A (hereinafter referred to as "Compound group SX190").

The Compound (L-4), wherein n represents 1, $R^{4b}$ represents a hydrogen atom, $R^{4c}$ represents a difluoromethyl group, and $R^{6b}$ represents any one substituent described in Table 1A to Table 3A (hereinafter referred to as "Compound group SX191").

The Compound (L-4), wherein n represents 2, $R^{4b}$ represents a hydrogen atom, $R^{4c}$ represents a difluoromethyl group, and $R^{6b}$ represents any one substituent described in Table 1A to Table 3A (hereinafter referred to as "Compound group SX192").

The Compound (L-4), wherein n represents 0, $R^{4b}$ represents a hydrogen atom, $R^{4c}$ represents a trifluoromethoxy group, and $R^{6b}$ represents any one substituent described in Table 1A to Table 3A (hereinafter referred to as "Compound group SX193").

The Compound (L-4), wherein n represents 1, $R^{4b}$ represents a hydrogen atom, $R^{4c}$ represents a trifluoromethoxy group, and $R^{6b}$ represents any one substituent described in Table 1A to Table 3A (hereinafter referred to as "Compound group SX194").

The Compound (L-4), wherein n represents 2, $R^{4b}$ represents a hydrogen atom, $R^{4c}$ represents a trifluoromethoxy group, and $R^{6b}$ represents any one substituent described in Table 1A to Table 3A (hereinafter referred to as "Compound group SX195").

The Compound (L-4), wherein n represents 0, $R^{4b}$ represents a hydrogen atom, $R^{4c}$ represents a difluoromethoxy group, and $R^{6b}$ represents any one substituent described in Table 1A to Table 3A (hereinafter referred to as "Compound group SX196").

The Compound (L-4), wherein n represents 1, $R^{4b}$ represents a hydrogen atom, $R^{4c}$ represents a difluoromethoxy group, and $R^{6b}$ represents any one substituent described in Table 1A to Table 3A (hereinafter referred to as "Compound group SX197").

The Compound (L-4), wherein n represents 2, $R^{4b}$ represents a hydrogen atom, $R^{4c}$ represents a difluoromethoxy group, and $R^{6b}$ represents any one substituent described in Table 1A to Table 3A (hereinafter referred to as "Compound group SX198").

The Compound (L-4), wherein n represents 0, $R^{4b}$ represents a hydrogen atom, $R^{4c}$ represents a cyclopropyl group, and $R^{6b}$ represents any one substituent described in Table 1A to Table 3A (hereinafter referred to as "Compound group SX199").

The Compound (L-4), wherein n represents 1, $R^{4b}$ represents a hydrogen atom, $R^{4c}$ represents a cyclopropyl group, and $R^{6b}$ represents any one substituent described in Table 1A to Table 3A (hereinafter referred to as "Compound group SX200").

The Compound (L-4), wherein n represents 2, $R^{4b}$ represents a hydrogen atom, $R^{4c}$ represents a cyclopropyl group, and $R^{6b}$ represents any one substituent described in Table 1A to Table 3A (hereinafter referred to as "Compound group SX201").

The Compound (L-4), wherein n represents 0, $R^{4b}$ represents a hydrogen atom, $R^{4c}$ represents a 1-cyanocyclopropyl group, and $R^{6b}$ represents any one substituent described in Table 1A to Table 3A (hereinafter referred to as "Compound group SX202").

The Compound (L-4), wherein n represents 1, $R^{4b}$ represents a hydrogen atom, $R^{4c}$ represents a 1-cyanocyclopropyl group, and $R^{6b}$ represents any one substituent described in Table 1A to Table 3A (hereinafter referred to as "Compound group SX203").

The Compound (L-4), wherein n represents 2, $R^{4b}$ represents a hydrogen atom, $R^{4c}$ represents a 1-cyanocyclopropyl group, and $R^{6b}$ represents any one substituent described in Table 1A to Table 3A (hereinafter referred to as "Compound group SX204").

A compound represented by formula (L-5)

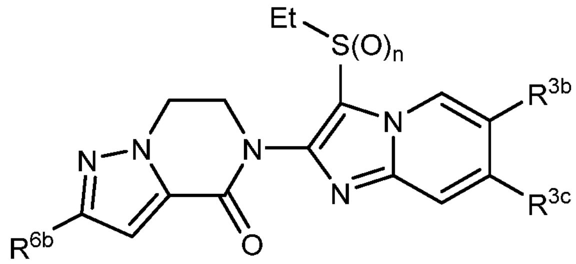

( L-5 )

(hereinafter referred to as "Compound (L-5)"), wherein n represents 0, $R^{3b}$ represents a hydrogen atom, $R^{3c}$ represents a hydrogen atom, and $R^{6b}$ represents any one substituent described in Table 1A to Table 3A (hereinafter referred to as "Compound group SX205").

The Compound (L-5), wherein n represents 1, $R^{3b}$ represents a hydrogen atom, $R^{3c}$ represents a hydrogen atom, and $R^{6b}$ represents any one substituent described in Table 1A to Table 3A (hereinafter referred to as "Compound group SX206") .

The Compound (L-5), wherein n represents 2, $R^{3b}$ represents a hydrogen atom, $R^{3c}$ represents a hydrogen atom, and $R^{6b}$ represents any one substituent described in Table 1A to Table 3A (hereinafter referred to as "Compound group SX207").

The Compound (L-5), wherein n represents 0, $R^{3b}$ represents a fluorine atom, $R^{3c}$ represents a hydrogen atom, and $R^{6b}$ represents any one substituent described in Table 1A to Table 3A (hereinafter referred to as "Compound group SX208").

The Compound (L-5), wherein n represents 1, $R^{3b}$ represents a fluorine atom, $R^{3c}$ represents a hydrogen atom, and $R^{6b}$ represents any one substituent described in Table 1A to Table 3A (hereinafter referred to as "Compound group SX209").

The Compound (L-5), wherein n represents 2, $R^{3b}$ represents a fluorine atom, $R^{3c}$ represents a hydrogen atom, and $R^{6b}$ represents any one substituent described in Table 1A to Table 3A (hereinafter referred to as "Compound group SX210").

The Compound (L-5), wherein n represents 0, $R^{3b}$ represents a chlorine atom, $R^{3c}$ represents a hydrogen atom, and $R^{6b}$ represents any one substituent described in Table 1A to Table 3A (hereinafter referred to as "Compound group SX211").

The Compound (L-5), wherein n represents 1, $R^{3b}$ represents a chlorine atom, $R^{3c}$ represents a hydrogen atom, and $R^{6b}$ represents any one substituent described in Table 1A to Table 3A (hereinafter referred to as "Compound group SX212").

The Compound (L-5), wherein n represents 2, $R^{3b}$ represents a chlorine atom, $R^{3c}$ represents a hydrogen atom, and $R^{6b}$ represents any one substituent described in Table 1A to Table 3A (hereinafter referred to as "Compound group SX213").

The Compound (L-5), wherein n represents 0, $R^{3b}$ represents a bromine atom, $R^{3c}$ represents a hydrogen atom, and $R^{6b}$ represents any one substituent described in Table 1A to Table 3A (hereinafter referred to as "Compound group SX214").

The Compound (L-5), wherein n represents 1, $R^{3b}$ represents a bromine atom, $R^{3c}$ represents a hydrogen atom, and $R^{6b}$ represents any one substituent described in Table 1A to Table 3A (hereinafter referred to as "Compound group SX215").

The Compound (L-5), wherein n represents 2, $R^{3b}$ represents a bromine atom, $R^{3c}$ represents a hydrogen atom, and $R^{6b}$ represents any one substituent described in Table 1A to Table 3A (hereinafter referred to as "Compound group SX216").

The Compound (L-5), wherein n represents 0, $R^{3b}$ represents an iodine atom, $R^{3c}$ represents a hydrogen atom, and $R^{6b}$ represents any one substituent described in Table 1A to Table 3A (hereinafter referred to as "Compound group SX217").

The Compound (L-5), wherein n represents 1, $R^{3b}$ represents an iodine atom, $R^{3c}$ represents a hydrogen atom, and $R^{6b}$ represents any one substituent described in Table 1A to Table 3A (hereinafter referred to as "Compound group SX218").

The Compound (L-5), wherein n represents 2, $R^{3b}$ represents an iodine atom, $R^{3c}$ represents a hydrogen atom, and $R^{6b}$ represents any one substituent described in Table 1A to Table 3A (hereinafter referred to as "Compound group SX219").

The Compound (L-5), wherein n represents 0, $R^{3b}$ represents a trifluoromethyl group, $R^{3c}$ represents a hydrogen atom, and $R^{6b}$ represents any one substituent described in Table 1A to Table 3A (hereinafter referred to as "Compound group SX220").

The Compound (L-5), wherein n represents 1, $R^{3b}$ represents a trifluoromethyl group, $R^{3c}$ represents a hydrogen

atom, and $R^{6b}$ represents any one substituent described in Table 1A to Table 3A (hereinafter referred to as "Compound group SX221").

The Compound (L-5), wherein n represents 2, $R^{3b}$ represents a trifluoromethyl group, $R^{3c}$ represents a hydrogen atom, and $R^{6b}$ represents any one substituent described in Table 1A to Table 3A (hereinafter referred to as "Compound group SX222").

The Compound (L-5), wherein n represents 0, $R^{3b}$ represents a difluoromethyl group, $R^{3c}$ represents a hydrogen atom, and $R^{6b}$ represents any one substituent described in Table 1A to Table 3A (hereinafter referred to as "Compound group SX223").

The Compound (L-5), wherein n represents 1, $R^{3b}$ represents a difluoromethyl group, $R^{3c}$ represents a hydrogen atom, and $R^{6b}$ represents any one substituent described in Table 1A to Table 3A (hereinafter referred to as "Compound group SX224").

The Compound (L-5), wherein n represents 2, $R^{3b}$ represents a difluoromethyl group, $R^{3c}$ represents a hydrogen atom, and $R^{6b}$ represents any one substituent described in Table 1A to Table 3A (hereinafter referred to as "Compound group SX225").

The Compound (L-5), wherein n represents 0, $R^{3b}$ represents a trifluoromethoxy group, $R^{3c}$ represents a hydrogen atom, and $R^{6b}$ represents any one substituent described in Table 1A to Table 3A (hereinafter referred to as "Compound group SX226").

The Compound (L-5), wherein n represents 1, $R^{3b}$ represents a trifluoromethoxy group, $R^{3c}$ represents a hydrogen atom, and $R^{6b}$ represents any one substituent described in Table 1A to Table 3A (hereinafter referred to as "Compound group SX227").

The Compound (L-5), wherein n represents 2, $R^{3b}$ represents a trifluoromethoxy group, $R^{3c}$ represents a hydrogen atom, and $R^{6b}$ represents any one substituent described in Table 1A to Table 3A (hereinafter referred to as "Compound group SX228").

The Compound (L-5), wherein n represents 0, $R^{3b}$ represents a difluoromethoxy group, $R^{3c}$ represents a hydrogen atom, and $R^{6b}$ represents any one substituent described in Table 1A to Table 3A (hereinafter referred to as "Compound group SX229").

The Compound (L-5), wherein n represents 1, $R^{3b}$ represents a difluoromethoxy group, $R^{3c}$ represents a hydrogen atom, and $R^{6b}$ represents any one substituent described in Table 1A to Table 3A (hereinafter referred to as "Compound group SX230").

The Compound (L-5), wherein n represents 2, $R^{3b}$ represents a difluoromethoxy group, $R^{3c}$ represents a hydrogen atom, and $R^{6b}$ represents any one substituent described in Table 1A to Table 3A (hereinafter referred to as "Compound group SX231").

The Compound (L-5), wherein n represents 0, $R^{3b}$ represents a cyclopropyl group, $R^{3c}$ represents a hydrogen atom, and $R^{6b}$ represents any one substituent described in Table 1A to Table 3A (hereinafter referred to as "Compound group SX232").

The Compound (L-5), wherein n represents 1, $R^{3b}$ represents a cyclopropyl group, $R^{3c}$ represents a hydrogen atom, and $R^{6b}$ represents any one substituent described in Table 1A to Table 3A (hereinafter referred to as "Compound group SX233").

The Compound (L-5), wherein n represents 2, $R^{3b}$ represents a cyclopropyl group, $R^{3c}$ represents a hydrogen atom, and $R^{6b}$ represents any one substituent described in Table 1A to Table 3A (hereinafter referred to as "Compound group SX234").

The Compound (L-5), wherein n represents 0, $R^{3b}$ represents a 1-cyanocyclopropyl group, $R^{3c}$ represents a hydrogen atom, and $R^{6b}$ represents any one substituent described in Table 1A to Table 3A (hereinafter referred to as "Compound group SX235").

The Compound (L-5), wherein n represents 1, $R^{3b}$ represents a 1-cyanocyclopropyl group, $R^{3c}$ represents a hydrogen atom, and $R^{6b}$ represents any one substituent described in Table 1A to Table 3A (hereinafter referred to as "Compound group SX236").

The Compound (L-5), wherein n represents 2, $R^{3b}$ represents a 1-cyanocyclopropyl group, $R^{3c}$ represents a hydrogen atom, and $R^{6b}$ represents any one substituent described in Table 1A to Table 3A (hereinafter referred to as "Compound group SX237").

The Compound (L-5), wherein n represents 0, $R^{3b}$ represents a hydrogen atom, $R^{3c}$ represents a fluorine atom, and $R^{6b}$ represents any one substituent described in Table 1A to Table 3A (hereinafter referred to as "Compound group SX238").

The Compound (L-5), wherein n represents 1, $R^{3b}$ represents a hydrogen atom, $R^{3c}$ represents a fluorine atom, and $R^{6b}$ represents any one substituent described in Table 1A to Table 3A (hereinafter referred to as "Compound group SX239").

The Compound (L-5), wherein n represents 2, $R^{3b}$ represents a hydrogen atom, $R^{3c}$ represents a fluorine atom, and $R^{6b}$ represents any one substituent described in Table 1A to Table 3A (hereinafter referred to as "Compound group

SX240").

The Compound (L-5), wherein n represents 0, $R^{3b}$ represents a hydrogen atom, $R^{3c}$ represents a chlorine atom, and $R^{6b}$ represents any one substituent described in Table 1A to Table 3A (hereinafter referred to as "Compound group SX241").

The Compound (L-5), wherein n represents 1, $R^{3b}$ represents a hydrogen atom, $R^{3c}$ represents a chlorine atom, and $R^{6b}$ represents any one substituent described in Table 1A to Table 3A (hereinafter referred to as "Compound group SX242").

The Compound (L-5), wherein n represents 2, $R^{3b}$ represents a hydrogen atom, $R^{3c}$ represents a chlorine atom, and $R^{6b}$ represents any one substituent described in Table 1A to Table 3A (hereinafter referred to as "Compound group SX243").

The Compound (L-5), wherein n represents 0, $R^{3b}$ represents a hydrogen atom, $R^{3c}$ represents a bromine atom, and $R^{6b}$ represents any one substituent described in Table 1A to Table 3A (hereinafter referred to as "Compound group SX244").

The Compound (L-5), wherein n represents 1, $R^{3b}$ represents a hydrogen atom, $R^{3c}$ represents a bromine atom, and $R^{6b}$ represents any one substituent described in Table 1A to Table 3A (hereinafter referred to as "Compound group SX245").

The Compound (L-5), wherein n represents 2, $R^{3b}$ represents a hydrogen atom, $R^{3c}$ represents a bromine atom, and $R^{6b}$ represents any one substituent described in Table 1A to Table 3A (hereinafter referred to as "Compound group SX246").

The Compound (L-5), wherein n represents 0, $R^{3b}$ represents a hydrogen atom, $R^{3c}$ represents an iodine atom, and $R^{6b}$ represents any one substituent described in Table 1A to Table 3A (hereinafter referred to as "Compound group SX247").

The Compound (L-5), wherein n represents 1, $R^{3b}$ represents a hydrogen atom, $R^{3c}$ represents an iodine atom, and $R^{6b}$ represents any one substituent described in Table 1A to Table 3A (hereinafter referred to as "Compound group SX248") .

The Compound (L-5), wherein n represents 2, $R^{3b}$ represents a hydrogen atom, $R^{3c}$ represents an iodine atom, and $R^{6b}$ represents any one substituent described in Table 1A to Table 3A (hereinafter referred to as "Compound group SX249").

The Compound (L-5), wherein n represents 0, $R^{3b}$ represents a hydrogen atom, $R^{3c}$ represents a trifluoromethyl group, and $R^{6b}$ represents any one substituent described in Table 1A to Table 3A (hereinafter referred to as "Compound group SX250").

The Compound (L-5), wherein n represents 1, $R^{3b}$ represents a hydrogen atom, $R^{3c}$ represents a trifluoromethyl group, and $R^{6b}$ represents any one substituent described in Table 1A to Table 3A (hereinafter referred to as "Compound group SX251").

The Compound (L-5), wherein n represents 2, $R^{3b}$ represents a hydrogen atom, $R^{3c}$ represents a trifluoromethyl group, and $R^{6b}$ represents any one substituent described in Table 1A to Table 3A (hereinafter referred to as "Compound group SX252").

The Compound (L-5), wherein n represents 0, $R^{3b}$ represents a hydrogen atom, $R^{3c}$ represents a difluoromethyl group, and $R^{6b}$ represents any one substituent described in Table 1A to Table 3A (hereinafter referred to as "Compound group SX253").

The Compound (L-5), wherein n represents 1, $R^{3b}$ represents a hydrogen atom, $R^{3c}$ represents a difluoromethyl group, and $R^{6b}$ represents any one substituent described in Table 1A to Table 3A (hereinafter referred to as "Compound group SX254").

The Compound (L-5), wherein n represents 2, $R^{3b}$ represents a hydrogen atom, $R^{3c}$ represents a difluoromethyl group, and $R^{6b}$ represents any one substituent described in Table 1A to Table 3A (hereinafter referred to as "Compound group SX255").

The Compound (L-5), wherein n represents 0, $R^{3b}$ represents a hydrogen atom, $R^{3c}$ represents a trifluoromethoxy group, and $R^{6b}$ represents any one substituent described in Table 1A to Table 3A (hereinafter referred to as "Compound group SX256").

The Compound (L-5), wherein n represents 1, $R^{3b}$ represents a hydrogen atom, $R^{3c}$ represents a trifluoromethoxy group, and $R^{6b}$ represents any one substituent described in Table 1A to Table 3A (hereinafter referred to as "Compound group SX257").

The Compound (L-5), wherein n represents 2, $R^{3b}$ represents a hydrogen atom, $R^{3c}$ represents a trifluoromethoxy group, and $R^{6b}$ represents any one substituent described in Table 1A to Table 3A (hereinafter referred to as "Compound group SX258").

The Compound (L-5), wherein n represents 0, $R^{3b}$ represents a hydrogen atom, $R^{3c}$ represents a difluoromethoxy group, and $R^{6b}$ represents any one substituent described in Table 1A to Table 3A (hereinafter referred to as "Compound group SX259").

The Compound (L-5), wherein n represents 1, $R^{3b}$ represents a hydrogen atom, $R^{3c}$ represents a difluoromethoxy group, and $R^{6b}$ represents any one substituent described in Table 1A to Table 3A (hereinafter referred to as "Compound group SX260").

The Compound (L-5), wherein n represents 2, $R^{3b}$ represents a hydrogen atom, $R^{3c}$ represents a difluoromethoxy group, and $R^{6b}$ represents any one substituent described in Table 1A to Table 3A (hereinafter referred to as "Compound group SX261").

The Compound (L-5), wherein n represents 0, $R^{3b}$ represents a hydrogen atom, $R^{3c}$ represents a cyclopropyl group, and $R^{6b}$ represents any one substituent described in Table 1A to Table 3A (hereinafter referred to as "Compound group SX262").

The Compound (L-5), wherein n represents 1, $R^{3b}$ represents a hydrogen atom, $R^{3c}$ represents a cyclopropyl group, and $R^{6b}$ represents any one substituent described in Table 1A to Table 3A (hereinafter referred to as "Compound group SX263").

The Compound (L-5), wherein n represents 2, $R^{3b}$ represents a hydrogen atom, $R^{3c}$ represents a cyclopropyl group, and $R^{6b}$ represents any one substituent described in Table 1A to Table 3A (hereinafter referred to as "Compound group SX264").

The Compound (L-5), wherein n represents 0, $R^{3b}$ represents a hydrogen atom, $R^{3c}$ represents a 1-cyanocyclopropyl group, and $R^{6b}$ represents any one substituent described in Table 1A to Table 3A (hereinafter referred to as "Compound group SX265").

The Compound (L-5), wherein n represents 1, $R^{3b}$ represents a hydrogen atom, $R^{3c}$ represents a 1-cyanocyclopropyl group, and $R^{6b}$ represents any one substituent described in Table 1A to Table 3A (hereinafter referred to as "Compound group SX266").

The Compound (L-5), wherein n represents 2, $R^{3b}$ represents a hydrogen atom, $R^{3c}$ represents a 1-cyanocyclopropyl group, and $R^{6b}$ represents any one substituent described in Table 1A to Table 3A (hereinafter referred to as "Compound group SX267").

A compound represented by formula (L-6)

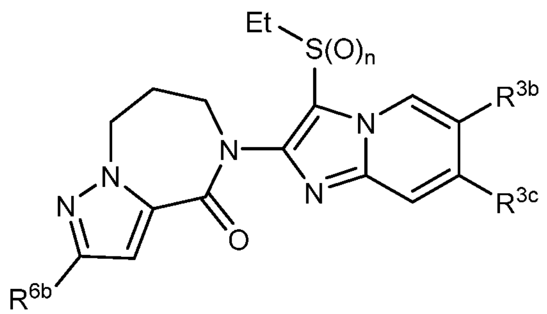

( L-6 )

(hereinafter referred to as "Compound (L-6)"), wherein n represents 0, $R^{3b}$ represents a hydrogen atom, $R^{3c}$ represents a hydrogen atom, and $R^{6b}$ represents any one substituent described in Table 1A to Table 3A (hereinafter referred to as "Compound group SX268").

The Compound (L-6), wherein n represents 1, $R^{3b}$ represents a hydrogen atom, $R^{3c}$ represents a hydrogen atom, and $R^{6b}$ represents any one substituent described in Table 1A to Table 3A (hereinafter referred to as "Compound group SX269") .

The Compound (L-6), wherein n represents 2, $R^{3b}$ represents a hydrogen atom, $R^{3c}$ represents a hydrogen atom, and $R^{6b}$ represents any one substituent described in Table 1A to Table 3A (hereinafter referred to as "Compound group SX270").

The Compound (L-6), wherein n represents 0, $R^{3b}$ represents a fluorine atom, $R^{3c}$ represents a hydrogen atom, and $R^{6b}$ represents any one substituent described in Table 1A to Table 3A (hereinafter referred to as "Compound group SX271").

The Compound (L-6), wherein n represents 1, $R^{3b}$ represents a fluorine atom, $R^{3c}$ represents a hydrogen atom, and $R^{6b}$ represents any one substituent described in Table 1A to Table 3A (hereinafter referred to as "Compound group SX272").

The Compound (L-6), wherein n represents 2, $R^{3b}$ represents a fluorine atom, $R^{3c}$ represents a hydrogen atom, and $R^{6b}$ represents any one substituent described in Table 1A to Table 3A (hereinafter referred to as "Compound group SX273").

The Compound (L-6), wherein n represents 0, $R^{3b}$ represents a chlorine atom, $R^{3c}$ represents a hydrogen atom, and $R^{6b}$ represents any one substituent described in Table 1A to Table 3A (hereinafter referred to as "Compound group SX274").

The Compound (L-6), wherein n represents 1, $R^{3b}$ represents a chlorine atom, $R^{3c}$ represents a hydrogen atom, and $R^{6b}$ represents any one substituent described in Table 1A to Table 3A (hereinafter referred to as "Compound group SX275").

The Compound (L-6), wherein n represents 2, $R^{3b}$ represents a chlorine atom, $R^{3c}$ represents a hydrogen atom, and $R^{6b}$ represents any one substituent described in Table 1A to Table 3A (hereinafter referred to as "Compound group SX276") .

The Compound (L-6), wherein n represents 0, $R^{3b}$ represents a bromine atom, $R^{3c}$ represents a hydrogen atom, and $R^{6b}$ represents any one substituent described in Table 1A to Table 3A (hereinafter referred to as "Compound group SX277") .

The Compound (L-6), wherein n represents 1, $R^{3b}$ represents a bromine atom, $R^{3c}$ represents a hydrogen atom, and $R^{6b}$ represents any one substituent described in Table 1A to Table 3A (hereinafter referred to as "Compound group SX278").

The Compound (L-6), wherein n represents 2, $R^{3b}$ represents a bromine atom, $R^{3c}$ represents a hydrogen atom, and $R^{6b}$ represents any one substituent described in Table 1A to Table 3A (hereinafter referred to as "Compound group SX279").

The Compound (L-6), wherein n represents 0, $R^{3b}$ represents an iodine atom, $R^{3c}$ represents a hydrogen atom, and $R^{6b}$ represents any one substituent described in Table 1A to Table 3A (hereinafter referred to as "Compound group SX280") .

The Compound (L-6), wherein n represents 1, $R^{3b}$ represents an iodine atom, $R^{3c}$ represents a hydrogen atom, and $R^{6b}$ represents any one substituent described in Table 1A to Table 3A (hereinafter referred to as "Compound group SX281").

The Compound (L-6), wherein n represents 2, $R^{3b}$ represents an iodine atom, $R^{3c}$ represents a hydrogen atom, and $R^{6b}$ represents any one substituent described in Table 1A to Table 3A (hereinafter referred to as "Compound group SX282").

The Compound (L-6), wherein n represents 0, $R^{3b}$ represents a trifluoromethyl group, $R^{3c}$ represents a hydrogen atom, and $R^{6b}$ represents any one substituent described in Table 1A to Table 3A (hereinafter referred to as "Compound group SX283").

The Compound (L-6), wherein n represents 1, $R^{3b}$ represents a trifluoromethyl group, $R^{3c}$ represents a hydrogen atom, and $R^{6b}$ represents any one substituent described in Table 1A to Table 3A (hereinafter referred to as "Compound group SX284").

The Compound (L-6), wherein n represents 2, $R^{3b}$ represents a trifluoromethyl group, $R^{3c}$ represents a hydrogen atom, and $R^{6b}$ represents any one substituent described in Table 1A to Table 3A (hereinafter referred to as "Compound group SX285").

The Compound (L-6), wherein n represents 0, $R^{3b}$ represents a difluoromethyl group, $R^{3c}$ represents a hydrogen atom, and $R^{6b}$ represents any one substituent described in Table 1A to Table 3A (hereinafter referred to as "Compound group SX286").

The Compound (L-6), wherein n represents 1, $R^{3b}$ represents a difluoromethyl group, $R^{3c}$ represents a hydrogen atom, and $R^{6b}$ represents any one substituent described in Table 1A to Table 3A (hereinafter referred to as "Compound group SX287").

The Compound (L-6), wherein n represents 2, $R^{3b}$ represents a difluoromethyl group, $R^{3c}$ represents a hydrogen atom, and $R^{6b}$ represents any one substituent described in Table 1A to Table 3A (hereinafter referred to as "Compound group SX288").

The Compound (L-6), wherein n represents 0, $R^{3b}$ represents a trifluoromethoxy group, $R^{3c}$ represents a hydrogen atom, and $R^{6b}$ represents any one substituent described in Table 1A to Table 3A (hereinafter referred to as "Compound group SX289").

The Compound (L-6), wherein n represents 1, $R^{3b}$ represents a trifluoromethoxy group, $R^{3c}$ represents a hydrogen atom, and $R^{6b}$ represents any one substituent described in Table 1A to Table 3A (hereinafter referred to as "Compound group SX290").

The Compound (L-6), wherein n represents 2, $R^{3b}$ represents a trifluoromethoxy group, $R^{3c}$ represents a hydrogen atom, and $R^{6b}$ represents any one substituent described in Table 1A to Table 3A (hereinafter referred to as "Compound group SX291").

The Compound (L-6), wherein n represents 0, $R^{3b}$ represents a difluoromethoxy group, $R^{3c}$ represents a hydrogen atom, and $R^{6b}$ represents any one substituent described in Table 1A to Table 3A (hereinafter referred to as "Compound group SX292").

The Compound (L-6), wherein n represents 1, $R^{3b}$ represents a difluoromethoxy group, $R^{3c}$ represents a hydrogen atom, and $R^{6b}$ represents any one substituent described in Table 1A to Table 3A (hereinafter referred to as "Compound group SX293").

The Compound (L-6), wherein n represents 2, $R^{3b}$ represents a difluoromethoxy group, $R^{3c}$ represents a

hydrogen atom, and $R^{6b}$ represents any one substituent described in Table 1A to Table 3A (hereinafter referred to as "Compound group SX294").

The Compound (L-6), wherein n represents 0, $R^{3b}$ represents a cyclopropyl group, $R^{3c}$ represents a hydrogen atom, and $R^{6b}$ represents any one substituent described in Table 1A to Table 3A (hereinafter referred to as "Compound group SX295").

The Compound (L-6), wherein n represents 1, $R^{3b}$ represents a cyclopropyl group, $R^{3c}$ represents a hydrogen atom, and $R^{6b}$ represents any one substituent described in Table 1A to Table 3A (hereinafter referred to as "Compound group SX296").

The Compound (L-6), wherein n represents 2, $R^{3b}$ represents a cyclopropyl group, $R^{3c}$ represents a hydrogen atom, and $R^{6b}$ represents any one substituent described in Table 1A to Table 3A (hereinafter referred to as "Compound group SX297").

The Compound (L-6), wherein n represents 0, $R^{3b}$ represents a 1-cyanocyclopropyl group, $R^{3c}$ represents a hydrogen atom, and $R^{6b}$ represents any one substituent described in Table 1A to Table 3A (hereinafter referred to as "Compound group SX298").

The Compound (L-6), wherein n represents 1, $R^{3b}$ represents a 1-cyanocyclopropyl group, $R^{3c}$ represents a hydrogen atom, and $R^{6b}$ represents any one substituent described in Table 1A to Table 3A (hereinafter referred to as "Compound group SX299").

The Compound (L-6), wherein n represents 2, $R^{3b}$ represents a 1-cyanocyclopropyl group, $R^{3c}$ represents a hydrogen atom, and $R^{6b}$ represents any one substituent described in Table 1A to Table 3A (hereinafter referred to as "Compound group SX300").

The Compound (L-6), wherein n represents 0, $R^{3b}$ represents a hydrogen atom, $R^{3c}$ represents a fluorine atom, and $R^{6b}$ represents any one substituent described in Table 1A to Table 3A (hereinafter referred to as "Compound group SX301").

The Compound (L-6), wherein n represents 1, $R^{3b}$ represents a hydrogen atom, $R^{3c}$ represents a fluorine atom, and $R^{6b}$ represents any one substituent described in Table 1A to Table 3A (hereinafter referred to as "Compound group SX302").

The Compound (L-6), wherein n represents 2, $R^{3b}$ represents a hydrogen atom, $R^{3c}$ represents a fluorine atom, and $R^{6b}$ represents any one substituent described in Table 1A to Table 3A (hereinafter referred to as "Compound group SX303").

The Compound (L-6), wherein n represents 0, $R^{3b}$ represents a hydrogen atom, $R^{3c}$ represents a chlorine atom, and $R^{6b}$ represents any one substituent described in Table 1A to Table 3A (hereinafter referred to as "Compound group SX304").

The Compound (L-6), wherein n represents 1, $R^{3b}$ represents a hydrogen atom, $R^{3c}$ represents a chlorine atom, and $R^{6b}$ represents any one substituent described in Table 1A to Table 3A (hereinafter referred to as "Compound group SX305").

The Compound (L-6), wherein n represents 2, $R^{3b}$ represents a hydrogen atom, $R^{3c}$ represents a chlorine atom, and $R^{6b}$ represents any one substituent described in Table 1A to Table 3A (hereinafter referred to as "Compound group SX306") .

The Compound (L-6), wherein n represents 0, $R^{3b}$ represents a hydrogen atom, $R^{3c}$ represents a bromine atom, and $R^{6b}$ represents any one substituent described in Table 1A to Table 3A (hereinafter referred to as "Compound group SX307").

The Compound (L-6), wherein n represents 1, $R^{3b}$ represents a hydrogen atom, $R^{3c}$ represents a bromine atom, and $R^{6b}$ represents any one substituent described in Table 1A to Table 3A (hereinafter referred to as "Compound group SX308").

The Compound (L-6), wherein n represents 2, $R^{3b}$ represents a hydrogen atom, $R^{3c}$ represents a bromine atom, and $R^{6b}$ represents any one substituent described in Table 1A to Table 3A (hereinafter referred to as "Compound group SX309").

The Compound (L-6), wherein n represents 0, $R^{3b}$ represents a hydrogen atom, $R^{3c}$ represents an iodine atom, and $R^{6b}$ represents any one substituent described in Table 1A to Table 3A (hereinafter referred to as "Compound group SX310").

The Compound (L-6), wherein n represents 1, $R^{3b}$ represents a hydrogen atom, $R^{3c}$ represents an iodine atom, and $R^{6b}$ represents any one substituent described in Table 1A to Table 3A (hereinafter referred to as "Compound group SX311").

The Compound (L-6), wherein n represents 2, $R^{3b}$ represents a hydrogen atom, $R^{3c}$ represents an iodine atom, and $R^{6b}$ represents any one substituent described in Table 1A to Table 3A (hereinafter referred to as "Compound group SX312").

The Compound (L-6), wherein n represents 0, $R^{3b}$ represents a hydrogen atom, $R^{3c}$ represents a trifluoromethyl group, and $R^{6b}$ represents any one substituent described in Table 1A to Table 3A (hereinafter referred to as "Compound

group SX313").

The Compound (L-6), wherein n represents 1, $R^{3b}$ represents a hydrogen atom, $R^{3c}$ represents a trifluoromethyl group, and $R^{6b}$ represents any one substituent described in Table 1A to Table 3A (hereinafter referred to as "Compound group SX314").

The Compound (L-6), wherein n represents 2, $R^{3b}$ represents a hydrogen atom, $R^{3c}$ represents a trifluoromethyl group, and $R^{6b}$ represents any one substituent described in Table 1A to Table 3A (hereinafter referred to as "Compound group SX315").

The Compound (L-6), wherein n represents 0, $R^{3b}$ represents a hydrogen atom, $R^{3c}$ represents a difluoromethyl group, and $R^{6b}$ represents any one substituent described in Table 1A to Table 3A (hereinafter referred to as "Compound group SX316").

The Compound (L-6), wherein n represents 1, $R^{3b}$ represents a hydrogen atom, $R^{3c}$ represents a difluoromethyl group, and $R^{6b}$ represents any one substituent described in Table 1A to Table 3A (hereinafter referred to as "Compound group SX317").

The Compound (L-6), wherein n represents 2, $R^{3b}$ represents a hydrogen atom, $R^{3c}$ represents a difluoromethyl group, and $R^{6b}$ represents any one substituent described in Table 1A to Table 3A (hereinafter referred to as "Compound group SX318").

The Compound (L-6), wherein n represents 0, $R^{3b}$ represents a hydrogen atom, $R^{3c}$ represents a trifluoromethoxy group, and $R^{6b}$ represents any one substituent described in Table 1A to Table 3A (hereinafter referred to as "Compound group SX319").

The Compound (L-6), wherein n represents 1, $R^{3b}$ represents a hydrogen atom, $R^{3c}$ represents a trifluoromethoxy group, and $R^{6b}$ represents any one substituent described in Table 1A to Table 3A (hereinafter referred to as "Compound group SX320").

The Compound (L-6), wherein n represents 2, $R^{3b}$ represents a hydrogen atom, $R^{3c}$ represents a trifluoromethoxy group, and $R^{6b}$ represents any one substituent described in Table 1A to Table 3A (hereinafter referred to as "Compound group SX321").

The Compound (L-6), wherein n represents 0, $R^{3b}$ represents a hydrogen atom, $R^{3c}$ represents a difluoromethoxy group, and $R^{6b}$ represents any one substituent described in Table 1A to Table 3A (hereinafter referred to as "Compound group SX322").

The Compound (L-6), wherein n represents 1, $R^{3b}$ represents a hydrogen atom, $R^{3c}$ represents a difluoromethoxy group, and $R^{6b}$ represents any one substituent described in Table 1A to Table 3A (hereinafter referred to as "Compound group SX323").

The Compound (L-6), wherein n represents 2, $R^{3b}$ represents a hydrogen atom, $R^{3c}$ represents a difluoromethoxy group, and $R^{6b}$ represents any one substituent described in Table 1A to Table 3A (hereinafter referred to as "Compound group SX324").

The Compound (L-6), wherein n represents 0, $R^{3b}$ represents a hydrogen atom, $R^{3c}$ represents a cyclopropyl group, and $R^{6b}$ represents any one substituent described in Table 1A to Table 3A (hereinafter referred to as "Compound group SX325").

The Compound (L-6), wherein n represents 1, $R^{3b}$ represents a hydrogen atom, $R^{3c}$ represents a cyclopropyl group, and $R^{6b}$ represents any one substituent described in Table 1A to Table 3A (hereinafter referred to as "Compound group SX326").

The Compound (L-6), wherein n represents 2, $R^{3b}$ represents a hydrogen atom, $R^{3c}$ represents a cyclopropyl group, and $R^{6b}$ represents any one substituent described in Table 1A to Table 3A (hereinafter referred to as "Compound group SX327").

The Compound (L-6), wherein n represents 0, $R^{3b}$ represents a hydrogen atom, $R^{3c}$ represents a 1-cyanocyclopropyl group, and $R^{6b}$ represents any one substituent described in Table 1A to Table 3A (hereinafter referred to as "Compound group SX328").

The Compound (L-6), wherein n represents 1, $R^{3b}$ represents a hydrogen atom, $R^{3c}$ represents a 1-cyanocyclopropyl group, and $R^{6b}$ represents any one substituent described in Table 1A to Table 3A (hereinafter referred to as "Compound group SX329").

The Compound (L-6), wherein n represents 2, $R^{3b}$ represents a hydrogen atom, $R^{3c}$ represents a 1-cyanocyclopropyl group, and $R^{6b}$ represents any one substituent described in Table 1A to Table 3A (hereinafter referred to as "Compound group SX330").

A compound represented by formula (L-7)

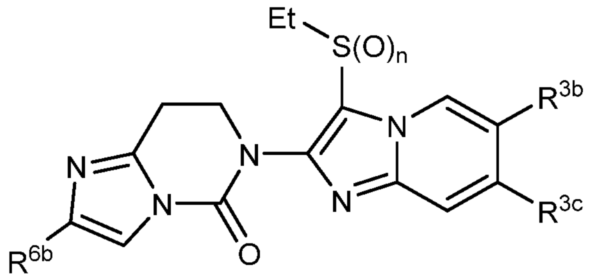

( L-7 )

(hereinafter referred to as "Compound (L-7)"), wherein n represents 0, $R^{3b}$ represents a hydrogen atom, $R^{3c}$ represents a hydrogen atom, and $R^{6b}$ represents any one substituent described in Table 1A to Table 3A (hereinafter referred to as "Compound group SX331").

The Compound (L-7), wherein n represents 1, $R^{3b}$ represents a hydrogen atom, $R^{3c}$ represents a hydrogen atom, and $R^{6b}$ represents any one substituent described in Table 1A to Table 3A (hereinafter referred to as "Compound group SX332").

The Compound (L-7), wherein n represents 2, $R^{3b}$ represents a hydrogen atom, $R^{3c}$ represents a hydrogen atom, and $R^{6b}$ represents any one substituent described in Table 1A to Table 3A (hereinafter referred to as "Compound group SX333").

The Compound (L-7), wherein n represents 0, $R^{3b}$ represents a fluorine atom, $R^{3c}$ represents a hydrogen atom, and $R^{6b}$ represents any one substituent described in Table 1A to Table 3A (hereinafter referred to as "Compound group SX334").

The Compound (L-7), wherein n represents 1, $R^{3b}$ represents a fluorine atom, $R^{3c}$ represents a hydrogen atom, and $R^{6b}$ represents any one substituent described in Table 1A to Table 3A (hereinafter referred to as "Compound group SX335").

The Compound (L-7), wherein n represents 2, $R^{3b}$ represents a fluorine atom, $R^{3c}$ represents a hydrogen atom, and $R^{6b}$ represents any one substituent described in Table 1A to Table 3A (hereinafter referred to as "Compound group SX336").

The Compound (L-7), wherein n represents 0, $R^{3b}$ represents a chlorine atom, $R^{3c}$ represents a hydrogen atom, and $R^{6b}$ represents any one substituent described in Table 1A to Table 3A (hereinafter referred to as "Compound group SX337").

The Compound (L-7), wherein n represents 1, $R^{3b}$ represents a chlorine atom, $R^{3c}$ represents a hydrogen atom, and $R^{6b}$ represents any one substituent described in Table 1A to Table 3A (hereinafter referred to as "Compound group SX338").

The Compound (L-7), wherein n represents 2, $R^{3b}$ represents a chlorine atom, $R^{3c}$ represents a hydrogen atom, and $R^{6b}$ represents any one substituent described in Table 1A to Table 3A (hereinafter referred to as "Compound group SX339").

The Compound (L-7), wherein n represents 0, $R^{3b}$ represents a bromine atom, $R^{3c}$ represents a hydrogen atom, and $R^{6b}$ represents any one substituent described in Table 1A to Table 3A (hereinafter referred to as "Compound group SX340").

The Compound (L-7), wherein n represents 1, $R^{3b}$ represents a bromine atom, $R^{3c}$ represents a hydrogen atom, and $R^{6b}$ represents any one substituent described in Table 1A to Table 3A (hereinafter referred to as "Compound group SX341").

The Compound (L-7), wherein n represents 2, $R^{3b}$ represents a bromine atom, $R^{3c}$ represents a hydrogen atom, and $R^{6b}$ represents any one substituent described in Table 1A to Table 3A (hereinafter referred to as "Compound group SX342").

The Compound (L-7), wherein n represents 0, $R^{3b}$ represents an iodine atom, $R^{3c}$ represents a hydrogen atom, and $R^{6b}$ represents any one substituent described in Table 1A to Table 3A (hereinafter referred to as "Compound group SX343").

The Compound (L-7), wherein n represents 1, $R^{3b}$ represents an iodine atom, $R^{3c}$ represents a hydrogen atom, and $R^{6b}$ represents any one substituent described in Table 1A to Table 3A (hereinafter referred to as "Compound group SX344").

The Compound (L-7), wherein n represents 2, $R^{3b}$ represents an iodine atom, $R^{3c}$ represents a hydrogen atom, and $R^{6b}$ represents any one substituent described in Table 1A to Table 3A (hereinafter referred to as "Compound group SX345").

The Compound (L-7), wherein n represents 0, $R^{3b}$ represents a trifluoromethyl group, $R^{3c}$ represents a hydrogen atom, and $R^{6b}$ represents any one substituent described in Table 1A to Table 3A (hereinafter referred to as "Compound group SX346").

The Compound (L-7), wherein n represents 1, $R^{3b}$ represents a trifluoromethyl group, $R^{3c}$ represents a hydrogen atom, and $R^{6b}$ represents any one substituent described in Table 1A to Table 3A (hereinafter referred to as "Compound group SX347").

The Compound (L-7), wherein n represents 2, $R^{3b}$ represents a trifluoromethyl group, $R^{3c}$ represents a hydrogen atom, and $R^{6b}$ represents any one substituent described in Table 1A to Table 3A (hereinafter referred to as "Compound group SX348").

The Compound (L-7), wherein n represents 0, $R^{3b}$ represents a difluoromethyl group, $R^{3c}$ represents a hydrogen atom, and $R^{6b}$ represents any one substituent described in Table 1A to Table 3A (hereinafter referred to as "Compound group SX349").

The Compound (L-7), wherein n represents 1, $R^{3b}$ represents a difluoromethyl group, $R^{3c}$ represents a hydrogen atom, and $R^{6b}$ represents any one substituent described in Table 1A to Table 3A (hereinafter referred to as "Compound group SX350").

The Compound (L-7), wherein n represents 2, $R^{3b}$ represents a difluoromethyl group, $R^{3c}$ represents a hydrogen atom, and $R^{6b}$ represents any one substituent described in Table 1A to Table 3A (hereinafter referred to as "Compound group SX351").

The Compound (L-7), wherein n represents 0, $R^{3b}$ represents a trifluoromethoxy group, $R^{3c}$ represents a hydrogen atom, and $R^{6b}$ represents any one substituent described in Table 1A to Table 3A (hereinafter referred to as "Compound group SX352").

The Compound (L-7), wherein n represents 1, $R^{3b}$ represents a trifluoromethoxy group, $R^{3c}$ represents a hydrogen atom, and $R^{6b}$ represents any one substituent described in Table 1A to Table 3A (hereinafter referred to as "Compound group SX353").

The Compound (L-7), wherein n represents 2, $R^{3b}$ represents a trifluoromethoxy group, $R^{3c}$ represents a hydrogen atom, and $R^{6b}$ represents any one substituent described in Table 1A to Table 3A (hereinafter referred to as "Compound group SX354").

The Compound (L-7), wherein n represents 0, $R^{3b}$ represents a difluoromethoxy group, $R^{3c}$ represents a hydrogen atom, and $R^{6b}$ represents any one substituent described in Table 1A to Table 3A (hereinafter referred to as "Compound group SX355").

The Compound (L-7), wherein n represents 1, $R^{3b}$ represents a difluoromethoxy group, $R^{3c}$ represents a hydrogen atom, and $R^{6b}$ represents any one substituent described in Table 1A to Table 3A (hereinafter referred to as "Compound group SX356").

The Compound (L-7), wherein n represents 2, $R^{3b}$ represents a difluoromethoxy group, $R^{3c}$ represents a hydrogen atom, and $R^{6b}$ represents any one substituent described in Table 1A to Table 3A (hereinafter referred to as "Compound group SX357").

The Compound (L-7), wherein n represents 0, $R^{3b}$ represents a cyclopropyl group, $R^{3c}$ represents a hydrogen atom, and $R^{6b}$ represents any one substituent described in Table 1A to Table 3A (hereinafter referred to as "Compound group SX358").

The Compound (L-7), wherein n represents 1, $R^{3b}$ represents a cyclopropyl group, $R^{3c}$ represents a hydrogen atom, and $R^{6b}$ represents any one substituent described in Table 1A to Table 3A (hereinafter referred to as "Compound group SX359").

The Compound (L-7), wherein n represents 2, $R^{3b}$ represents a cyclopropyl group, $R^{3c}$ represents a hydrogen atom, and $R^{6b}$ represents any one substituent described in Table 1A to Table 3A (hereinafter referred to as "Compound group SX360").

The Compound (L-7), wherein n represents 0, $R^{3b}$ represents a 1-cyanocyclopropyl group, $R^{3c}$ represents a hydrogen atom, and $R^{6b}$ represents any one substituent described in Table 1A to Table 3A (hereinafter referred to as "Compound group SX361").

The Compound (L-7), wherein n represents 1, $R^{3b}$ represents a 1-cyanocyclopropyl group, $R^{3c}$ represents a hydrogen atom, and $R^{6b}$ represents any one substituent described in Table 1A to Table 3A (hereinafter referred to as "Compound group SX362").

The Compound (L-7), wherein n represents 2, $R^{3b}$ represents a 1-cyanocyclopropyl group, $R^{3c}$ represents a hydrogen atom, and $R^{6b}$ represents any one substituent described in Table 1A to Table 3A (hereinafter referred to as "Compound group SX363").

The Compound (L-7), wherein n represents 0, $R^{3b}$ represents a hydrogen atom, $R^{3c}$ represents a fluorine atom, and $R^{6b}$ represents any one substituent described in Table 1A to Table 3A (hereinafter referred to as "Compound group SX364").

The Compound (L-7), wherein n represents 1, $R^{3b}$ represents a hydrogen atom, $R^{3c}$ represents a fluorine atom, and $R^{6b}$ represents any one substituent described in Table 1A to Table 3A (hereinafter referred to as "Compound group SX365").

The Compound (L-7), wherein n represents 2, $R^{3b}$ represents a hydrogen atom, $R^{3c}$ represents a fluorine atom,

and $R^{6b}$ represents any one substituent described in Table 1A to Table 3A (hereinafter referred to as "Compound group SX366") .

The Compound (L-7), wherein n represents 0, $R^{3b}$ represents a hydrogen atom, $R^{3c}$ represents a chlorine atom, and $R^{6b}$ represents any one substituent described in Table 1A to Table 3A (hereinafter referred to as "Compound group SX367").

The Compound (L-7), wherein n represents 1, $R^{3b}$ represents a hydrogen atom, $R^{3c}$ represents a chlorine atom, and $R^{6b}$ represents any one substituent described in Table 1A to Table 3A (hereinafter referred to as "Compound group SX368") .

The Compound (L-7), wherein n represents 2, $R^{3b}$ represents a hydrogen atom, $R^{3c}$ represents a chlorine atom, and $R^{6b}$ represents any one substituent described in Table 1A to Table 3A (hereinafter referred to as "Compound group SX369").

The Compound (L-7), wherein n represents 0, $R^{3b}$ represents a hydrogen atom, $R^{3c}$ represents a bromine atom, and $R^{6b}$ represents any one substituent described in Table 1A to Table 3A (hereinafter referred to as "Compound group SX370").

The Compound (L-7), wherein n represents 1, $R^{3b}$ represents a hydrogen atom, $R^{3c}$ represents a bromine atom, and $R^{6b}$ represents any one substituent described in Table 1A to Table 3A (hereinafter referred to as "Compound group SX371").

The Compound (L-7), wherein n represents 2, $R^{3b}$ represents a hydrogen atom, $R^{3c}$ represents a bromine atom, and $R^{6b}$ represents any one substituent described in Table 1A to Table 3A (hereinafter referred to as "Compound group SX372").

The Compound (L-7), wherein n represents 0, $R^{3b}$ represents a hydrogen atom, $R^{3c}$ represents an iodine atom, and $R^{6b}$ represents any one substituent described in Table 1A to Table 3A (hereinafter referred to as "Compound group SX373").

The Compound (L-7), wherein n represents 1, $R^{3b}$ represents a hydrogen atom, $R^{3c}$ represents an iodine atom, and $R^{6b}$ represents any one substituent described in Table 1A to Table 3A (hereinafter referred to as "Compound group SX374").

The Compound (L-7), wherein n represents 2, $R^{3b}$ represents a hydrogen atom, $R^{3c}$ represents an iodine atom, and $R^{6b}$ represents any one substituent described in Table 1A to Table 3A (hereinafter referred to as "Compound group SX375").

The Compound (L-7), wherein n represents 0, $R^{3b}$ represents a hydrogen atom, $R^{3c}$ represents a trifluoromethyl group, and $R^{6b}$ represents any one substituent described in Table 1A to Table 3A (hereinafter referred to as "Compound group SX376").

The Compound (L-7), wherein n represents 1, $R^{3b}$ represents a hydrogen atom, $R^{3c}$ represents a trifluoromethyl group, and $R^{6b}$ represents any one substituent described in Table 1A to Table 3A (hereinafter referred to as "Compound group SX377").

The Compound (L-7), wherein n represents 2, $R^{3b}$ represents a hydrogen atom, $R^{3c}$ represents a trifluoromethyl group, and $R^{6b}$ represents any one substituent described in Table 1A to Table 3A (hereinafter referred to as "Compound group SX378").

The Compound (L-7), wherein n represents 0, $R^{3b}$ represents a hydrogen atom, $R^{3c}$ represents a difluoromethyl group, and $R^{6b}$ represents any one substituent described in Table 1A to Table 3A (hereinafter referred to as "Compound group SX379").

The Compound (L-7), wherein n represents 1, $R^{3b}$ represents a hydrogen atom, $R^{3c}$ represents a difluoromethyl group, and $R^{6b}$ represents any one substituent described in Table 1A to Table 3A (hereinafter referred to as "Compound group SX380").

The Compound (L-7), wherein n represents 2, $R^{3b}$ represents a hydrogen atom, $R^{3c}$ represents a difluoromethyl group, and $R^{6b}$ represents any one substituent described in Table 1A to Table 3A (hereinafter referred to as "Compound group SX381").

The Compound (L-7), wherein n represents 0, $R^{3b}$ represents a hydrogen atom, $R^{3c}$ represents a trifluoromethoxy group, and $R^{6b}$ represents any one substituent described in Table 1A to Table 3A (hereinafter referred to as "Compound group SX382").

The Compound (L-7), wherein n represents 1, $R^{3b}$ represents a hydrogen atom, $R^{3c}$ represents a trifluoromethoxy group, and $R^{6b}$ represents any one substituent described in Table 1A to Table 3A (hereinafter referred to as "Compound group SX383").

The Compound (L-7), wherein n represents 2, $R^{3b}$ represents a hydrogen atom, $R^{3c}$ represents a trifluoromethoxy group, and $R^{6b}$ represents any one substituent described in Table 1A to Table 3A (hereinafter referred to as "Compound group SX384").

The Compound (L-7), wherein n represents 0, $R^{3b}$ represents a hydrogen atom, $R^{3c}$ represents a difluoromethoxy group, and $R^{6b}$ represents any one substituent described in Table 1A to Table 3A (hereinafter referred to as

"Compound group SX385").

The Compound (L-7), wherein n represents 1, $R^{3b}$ represents a hydrogen atom, $R^{3c}$ represents a difluoromethoxy group, and $R^{6b}$ represents any one substituent described in Table 1A to Table 3A (hereinafter referred to as "Compound group SX386").

The Compound (L-7), wherein n represents 2, $R^{3b}$ represents a hydrogen atom, $R^{3c}$ represents a difluoromethoxy group, and $R^{6b}$ represents any one substituent described in Table 1A to Table 3A (hereinafter referred to as "Compound group SX387").

The Compound (L-7), wherein n represents 0, $R^{3b}$ represents a hydrogen atom, $R^{3c}$ represents a cyclopropyl group, and $R^{6b}$ represents any one substituent described in Table 1A to Table 3A (hereinafter referred to as "Compound group SX388").

The Compound (L-7), wherein n represents 1, $R^{3b}$ represents a hydrogen atom, $R^{3c}$ represents a cyclopropyl group, and $R^{6b}$ represents any one substituent described in Table 1A to Table 3A (hereinafter referred to as "Compound group SX389").

The Compound (L-7), wherein n represents 2, $R^{3b}$ represents a hydrogen atom, $R^{3c}$ represents a cyclopropyl group, and $R^{6b}$ represents any one substituent described in Table 1A to Table 3A (hereinafter referred to as "Compound group SX390").

The Compound (L-7), wherein n represents 0, $R^{3b}$ represents a hydrogen atom, $R^{3c}$ represents a 1-cyanocyclopropyl group, and $R^{6b}$ represents any one substituent described in Table 1A to Table 3A (hereinafter referred to as "Compound group SX391").

The Compound (L-7), wherein n represents 1, $R^{3b}$ represents a hydrogen atom, $R^{3c}$ represents a 1-cyanocyclopropyl group, and $R^{6b}$ represents any one substituent described in Table 1A to Table 3A (hereinafter referred to as "Compound group SX392").

The Compound (L-7), wherein n represents 2, $R^{3b}$ represents a hydrogen atom, $R^{3c}$ represents a 1-cyanocyclopropyl group, and $R^{6b}$ represents any one substituent described in Table 1A to Table 3A (hereinafter referred to as "Compound group SX393").

A compound represented by formula (L-8)

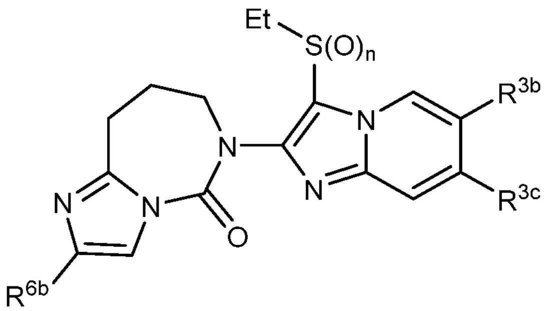

( L-8 )

(hereinafter referred to as "Compound (L-8)"), wherein n represents 0, $R^{3b}$ represents a hydrogen atom, $R^{3c}$ represents a hydrogen atom, and $R^{6b}$ represents any one substituent described in Table 1A to Table 3A (hereinafter referred to as "Compound group SX394").

The Compound (L-8), wherein n represents 1, $R^{3b}$ represents a hydrogen atom, $R^{3c}$ represents a hydrogen atom, and $R^{6b}$ represents any one substituent described in Table 1A to Table 3A (hereinafter referred to as "Compound group SX395").

The Compound (L-8), wherein n represents 2, $R^{3b}$ represents a hydrogen atom, $R^{3c}$ represents a hydrogen atom, and $R^{6b}$ represents any one substituent described in Table 1A to Table 3A (hereinafter referred to as "Compound group SX396") .

The Compound (L-8), wherein n represents 0, $R^{3b}$ represents a fluorine atom, $R^{3c}$ represents a hydrogen atom, and $R^{6b}$ represents any one substituent described in Table 1A to Table 3A (hereinafter referred to as "Compound group SX397").

The Compound (L-8), wherein n represents 1, $R^{3b}$ represents a fluorine atom, $R^{3c}$ represents a hydrogen atom, and $R^{6b}$ represents any one substituent described in Table 1A to Table 3A (hereinafter referred to as "Compound group SX398").

The Compound (L-8), wherein n represents 2, $R^{3b}$ represents a fluorine atom, $R^{3c}$ represents a hydrogen atom, and $R^{6b}$ represents any one substituent described in Table 1A to Table 3A (hereinafter referred to as "Compound group SX399").

The Compound (L-8), wherein n represents 0, $R^{3b}$ represents a chlorine atom, $R^{3c}$ represents a hydrogen atom, and $R^{6b}$ represents any one substituent described in Table 1A to Table 3A (hereinafter referred to as "Compound group

SX400").

The Compound (L-8), wherein n represents 1, $R^{3b}$ represents a chlorine atom, $R^{3c}$ represents a hydrogen atom, and $R^{6b}$ represents any one substituent described in Table 1A to Table 3A (hereinafter referred to as "Compound group SX401").

The Compound (L-8), wherein n represents 2, $R^{3b}$ represents a chlorine atom, $R^{3c}$ represents a hydrogen atom, and $R^{6b}$ represents any one substituent described in Table 1A to Table 3A (hereinafter referred to as "Compound group SX402").

The Compound (L-8), wherein n represents 0, $R^{3b}$ represents a bromine atom, $R^{3c}$ represents a hydrogen atom, and $R^{6b}$ represents any one substituent described in Table 1A to Table 3A (hereinafter referred to as "Compound group SX403").

The Compound (L-8), wherein n represents 1, $R^{3b}$ represents a bromine atom, $R^{3c}$ represents a hydrogen atom, and $R^{6b}$ represents any one substituent described in Table 1A to Table 3A (hereinafter referred to as "Compound group SX404").

The Compound (L-8), wherein n represents 2, $R^{3b}$ represents a bromine atom, $R^{3c}$ represents a hydrogen atom, and $R^{6b}$ represents any one substituent described in Table 1A to Table 3A (hereinafter referred to as "Compound group SX405").

The Compound (L-8), wherein n represents 0, $R^{3b}$ represents an iodine atom, $R^{3c}$ represents a hydrogen atom, and $R^{6b}$ represents any one substituent described in Table 1A to Table 3A (hereinafter referred to as "Compound group SX406") .

The Compound (L-8), wherein n represents 1, $R^{3b}$ represents an iodine atom, $R^{3c}$ represents a hydrogen atom, and $R^{6b}$ represents any one substituent described in Table 1A to Table 3A (hereinafter referred to as "Compound group SX407") .

The Compound (L-8), wherein n represents 2, $R^{3b}$ represents an iodine atom, $R^{3c}$ represents a hydrogen atom, and $R^{6b}$ represents any one substituent described in Table 1A to Table 3A (hereinafter referred to as "Compound group SX408") .

The Compound (L-8), wherein n represents 0, $R^{3b}$ represents a trifluoromethyl group, $R^{3c}$ represents a hydrogen atom, and $R^{6b}$ represents any one substituent described in Table 1A to Table 3A (hereinafter referred to as "Compound group SX409").

The Compound (L-8), wherein n represents 1, $R^{3b}$ represents a trifluoromethyl group, $R^{3c}$ represents a hydrogen atom, and $R^{6b}$ represents any one substituent described in Table 1A to Table 3A (hereinafter referred to as "Compound group SX410").

The Compound (L-8), wherein n represents 2, $R^{3b}$ represents a trifluoromethyl group, $R^{3c}$ represents a hydrogen atom, and $R^{6b}$ represents any one substituent described in Table 1A to Table 3A (hereinafter referred to as "Compound group SX411").

The Compound (L-8), wherein n represents 0, $R^{3b}$ represents a difluoromethyl group, $R^{3c}$ represents a hydrogen atom, and $R^{6b}$ represents any one substituent described in Table 1A to Table 3A (hereinafter referred to as "Compound group SX412").

The Compound (L-8), wherein n represents 1, $R^{3b}$ represents a difluoromethyl group, $R^{3c}$ represents a hydrogen atom, and $R^{6b}$ represents any one substituent described in Table 1A to Table 3A (hereinafter referred to as "Compound group SX413").

The Compound (L-8), wherein n represents 2, $R^{3b}$ represents a difluoromethyl group, $R^{3c}$ represents a hydrogen atom, and $R^{6b}$ represents any one substituent described in Table 1A to Table 3A (hereinafter referred to as "Compound group SX414").

The Compound (L-8), wherein n represents 0, $R^{3b}$ represents a trifluoromethoxy group, $R^{3c}$ represents a hydrogen atom, and $R^{6b}$ represents any one substituent described in Table 1A to Table 3A (hereinafter referred to as "Compound group SX415").

The Compound (L-8), wherein n represents 1, $R^{3b}$ represents a trifluoromethoxy group, $R^{3c}$ represents a hydrogen atom, and $R^{6b}$ represents any one substituent described in Table 1A to Table 3A (hereinafter referred to as "Compound group SX416").

The Compound (L-8), wherein n represents 2, $R^{3b}$ represents a trifluoromethoxy group, $R^{3c}$ represents a hydrogen atom, and $R^{6b}$ represents any one substituent described in Table 1A to Table 3A (hereinafter referred to as "Compound group SX417").

The Compound (L-8), wherein n represents 0, $R^{3b}$ represents a difluoromethoxy group, $R^{3c}$ represents a hydrogen atom, and $R^{6b}$ represents any one substituent described in Table 1A to Table 3A (hereinafter referred to as "Compound group SX418").

The Compound (L-8), wherein n represents 1, $R^{3b}$ represents a difluoromethoxy group, $R^{3c}$ represents a hydrogen atom, and $R^{6b}$ represents any one substituent described in Table 1A to Table 3A (hereinafter referred to as "Compound group SX419").

The Compound (L-8), wherein n represents 2, $R^{3b}$ represents a difluoromethoxy group, $R^{3c}$ represents a hydrogen atom, and $R^{6b}$ represents any one substituent described in Table 1A to Table 3A (hereinafter referred to as "Compound group SX420").

The Compound (L-8), wherein n represents 0, $R^{3b}$ represents a cyclopropyl group, $R^{3c}$ represents a hydrogen atom, and $R^{6b}$ represents any one substituent described in Table 1A to Table 3A (hereinafter referred to as "Compound group SX421").

The Compound (L-8), wherein n represents 1, $R^{3b}$ represents a cyclopropyl group, $R^{3c}$ represents a hydrogen atom, and $R^{6b}$ represents any one substituent described in Table 1A to Table 3A (hereinafter referred to as "Compound group SX422").

The Compound (L-8), wherein n represents 2, $R^{3b}$ represents a cyclopropyl group, $R^{3c}$ represents a hydrogen atom, and $R^{6b}$ represents any one substituent described in Table 1A to Table 3A (hereinafter referred to as "Compound group SX423").

The Compound (L-8), wherein n represents 0, $R^{3b}$ represents a 1-cyanocyclopropyl group, $R^{3c}$ represents a hydrogen atom, and $R^{6b}$ represents any one substituent described in Table 1A to Table 3A (hereinafter referred to as "Compound group SX424").

The Compound (L-8), wherein n represents 1, $R^{3b}$ represents a 1-cyanocyclopropyl group, $R^{3c}$ represents a hydrogen atom, and $R^{6b}$ represents any one substituent described in Table 1A to Table 3A (hereinafter referred to as "Compound group SX425").

The Compound (L-8), wherein n represents 2, $R^{3b}$ represents a 1-cyanocyclopropyl group, $R^{3c}$ represents a hydrogen atom, and $R^{6b}$ represents any one substituent described in Table 1A to Table 3A (hereinafter referred to as "Compound group SX426").

The Compound (L-8), wherein n represents 0, $R^{3b}$ represents a hydrogen atom, $R^{3c}$ represents a fluorine atom, and $R^{6b}$ represents any one substituent described in Table 1A to Table 3A (hereinafter referred to as "Compound group SX427") .

The Compound (L-8), wherein n represents 1, $R^{3b}$ represents a hydrogen atom, $R^{3c}$ represents a fluorine atom, and $R^{6b}$ represents any one substituent described in Table 1A to Table 3A (hereinafter referred to as "Compound group SX428") .

The Compound (L-8), wherein n represents 2, $R^{3b}$ represents a hydrogen atom, $R^{3c}$ represents a fluorine atom, and $R^{6b}$ represents any one substituent described in Table 1A to Table 3A (hereinafter referred to as "Compound group SX429").

The Compound (L-8), wherein n represents 0, $R^{3b}$ represents a hydrogen atom, $R^{3c}$ represents a chlorine atom, and $R^{6b}$ represents any one substituent described in Table 1A to Table 3A (hereinafter referred to as "Compound group SX430").

The Compound (L-8), wherein n represents 1, $R^{3b}$ represents a hydrogen atom, $R^{3c}$ represents a chlorine atom, and $R^{6b}$ represents any one substituent described in Table 1A to Table 3A (hereinafter referred to as "Compound group SX431").

The Compound (L-8), wherein n represents 2, $R^{3b}$ represents a hydrogen atom, $R^{3c}$ represents a chlorine atom, and $R^{6b}$ represents any one substituent described in Table 1A to Table 3A (hereinafter referred to as "Compound group SX432").

The Compound (L-8), wherein n represents 0, $R^{3b}$ represents a hydrogen atom, $R^{3c}$ represents a bromine atom, and $R^{6b}$ represents any one substituent described in Table 1A to Table 3A (hereinafter referred to as "Compound group SX433").

The Compound (L-8), wherein n represents 1, $R^{3b}$ represents a hydrogen atom, $R^{3c}$ represents a bromine atom, and $R^{6b}$ represents any one substituent described in Table 1A to Table 3A (hereinafter referred to as "Compound group SX434").

The Compound (L-8), wherein n represents 2, $R^{3b}$ represents a hydrogen atom, $R^{3c}$ represents a bromine atom, and $R^{6b}$ represents any one substituent described in Table 1A to Table 3A (hereinafter referred to as "Compound group SX435").

The Compound (L-8), wherein n represents 0, $R^{3b}$ represents a hydrogen atom, $R^{3c}$ represents an iodine atom, and $R^{6b}$ represents any one substituent described in Table 1A to Table 3A (hereinafter referred to as "Compound group SX436") .

The Compound (L-8), wherein n represents 1, $R^{3b}$ represents a hydrogen atom, $R^{3c}$ represents an iodine atom, and $R^{6b}$ represents any one substituent described in Table 1A to Table 3A (hereinafter referred to as "Compound group SX437") .

The Compound (L-8), wherein n represents 2, $R^{3b}$ represents a hydrogen atom, $R^{3c}$ represents an iodine atom, and $R^{6b}$ represents any one substituent described in Table 1A to Table 3A (hereinafter referred to as "Compound group SX438").

The Compound (L-8), wherein n represents 0, $R^{3b}$ represents a hydrogen atom, $R^{3c}$ represents a trifluoromethyl

group, and $R^{6b}$ represents any one substituent described in Table 1A to Table 3A (hereinafter referred to as "Compound group SX439").

The Compound (L-8), wherein n represents 1, $R^{3b}$ represents a hydrogen atom, $R^{3c}$ represents a trifluoromethyl group, and $R^{6b}$ represents any one substituent described in Table 1A to Table 3A (hereinafter referred to as "Compound group SX440").

The Compound (L-8), wherein n represents 2, $R^{3b}$ represents a hydrogen atom, $R^{3c}$ represents a trifluoromethyl group, and $R^{6b}$ represents any one substituent described in Table 1A to Table 3A (hereinafter referred to as "Compound group SX441").

The Compound (L-8), wherein n represents 0, $R^{3b}$ represents a hydrogen atom, $R^{3c}$ represents a difluoromethyl group, and $R^{6b}$ represents any one substituent described in Table 1A to Table 3A (hereinafter referred to as "Compound group SX442").

The Compound (L-8), wherein n represents 1, $R^{3b}$ represents a hydrogen atom, $R^{3c}$ represents a difluoromethyl group, and $R^{6b}$ represents any one substituent described in Table 1A to Table 3A (hereinafter referred to as "Compound group SX443").

The Compound (L-8), wherein n represents 2, $R^{3b}$ represents a hydrogen atom, $R^{3c}$ represents a difluoromethyl group, and $R^{6b}$ represents any one substituent described in Table 1A to Table 3A (hereinafter referred to as "Compound group SX444").

The Compound (L-8), wherein n represents 0, $R^{3b}$ represents a hydrogen atom, $R^{3c}$ represents a trifluoromethoxy group, and $R^{6b}$ represents any one substituent described in Table 1A to Table 3A (hereinafter referred to as "Compound group SX445").

The Compound (L-8), wherein n represents 1, $R^{3b}$ represents a hydrogen atom, $R^{3c}$ represents a trifluoromethoxy group, and $R^{6b}$ represents any one substituent described in Table 1A to Table 3A (hereinafter referred to as "Compound group SX446").

The Compound (L-8), wherein n represents 2, $R^{3b}$ represents a hydrogen atom, $R^{3c}$ represents a trifluoromethoxy group, and $R^{6b}$ represents any one substituent described in Table 1A to Table 3A (hereinafter referred to as "Compound group SX447").

The Compound (L-8), wherein n represents 0, $R^{3b}$ represents a hydrogen atom, $R^{3c}$ represents a difluoromethoxy group, and $R^{6b}$ represents any one substituent described in Table 1A to Table 3A (hereinafter referred to as "Compound group SX448").

The Compound (L-8), wherein n represents 1, $R^{3b}$ represents a hydrogen atom, $R^{3c}$ represents a difluoromethoxy group, and $R^{6b}$ represents any one substituent described in Table 1A to Table 3A (hereinafter referred to as "Compound group SX449").

The Compound (L-8), wherein n represents 2, $R^{3b}$ represents a hydrogen atom, $R^{3c}$ represents a difluoromethoxy group, and $R^{6b}$ represents any one substituent described in Table 1A to Table 3A (hereinafter referred to as "Compound group SX450").

The Compound (L-8), wherein n represents 0, $R^{3b}$ represents a hydrogen atom, $R^{3c}$ represents a cyclopropyl group, and $R^{6b}$ represents any one substituent described in Table 1A to Table 3A (hereinafter referred to as "Compound group SX451").

The Compound (L-8), wherein n represents 1, $R^{3b}$ represents a hydrogen atom, $R^{3c}$ represents a cyclopropyl group, and $R^{6b}$ represents any one substituent described in Table 1A to Table 3A (hereinafter referred to as "Compound group SX452").

The Compound (L-8), wherein n represents 2, $R^{3b}$ represents a hydrogen atom, $R^{3c}$ represents a cyclopropyl group, and $R^{6b}$ represents any one substituent described in Table 1A to Table 3A (hereinafter referred to as "Compound group SX453").

The Compound (L-8), wherein n represents 0, $R^{3b}$ represents a hydrogen atom, $R^{3c}$ represents a 1-cyanocyclopropyl group, and $R^{6b}$ represents any one substituent described in Table 1A to Table 3A (hereinafter referred to as "Compound group SX454").

The Compound (L-8), wherein n represents 1, $R^{3b}$ represents a hydrogen atom, $R^{3c}$ represents a 1-cyanocyclopropyl group, and $R^{6b}$ represents any one substituent described in Table 1A to Table 3A (hereinafter referred to as "Compound group SX455").

The Compound (L-8), wherein n represents 2, $R^{3b}$ represents a hydrogen atom, $R^{3c}$ represents a 1-cyanocyclopropyl group, and $R^{6b}$ represents any one substituent described in Table 1A to Table 3A (hereinafter referred to as "Compound group SX456").

A compound represented by formula (L-9)

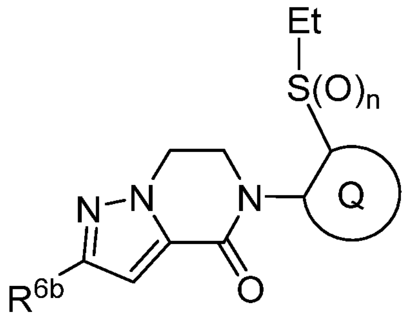

( L-9 )

(hereinafter referred to as "Compound (L-9)"), wherein n represents 0, Q represents a group represented by Q1-1, and $R^{6b}$ represents any one substituent described in Table 1A to Table 3A (hereinafter referred to as "Compound group SX457").

The Compound (L-9), wherein n represents 1, Q represents a group represented by Q1-1, and $R^{6b}$ represents any one substituent described in Table 1A to Table 3A (hereinafter referred to as "Compound group SX458").

The Compound (L-9), wherein n represents 2, Q represents a group represented by Q1-1, and $R^{6b}$ represents any one substituent described in Table 1A to Table 3A (hereinafter referred to as "Compound group SX459").

The Compound (L-9), wherein n represents 0, Q represents a group represented by Q1-2, and $R^{6b}$ represents any one substituent described in Table 1A to Table 3A (hereinafter referred to as "Compound group SX460").

The Compound (L-9), wherein n represents 1, Q represents a group represented by Q1-2, and $R^{6b}$ represents any one substituent described in Table 1A to Table 3A (hereinafter referred to as "Compound group SX461").

The Compound (L-9), wherein n represents 2, Q represents a group represented by Q1-2, and $R^{6b}$ represents any one substituent described in Table 1A to Table 3A (hereinafter referred to as "Compound group SX462").

The Compound (L-9), wherein n represents 0, Q represents a group represented by Q1-3, and $R^{6b}$ represents any one substituent described in Table 1A to Table 3A (hereinafter referred to as "Compound group SX463").

The Compound (L-9), wherein n represents 1, Q represents a group represented by Q1-3, and $R^{6b}$ represents any one substituent described in Table 1A to Table 3A (hereinafter referred to as "Compound group SX464").

The Compound (L-9), wherein n represents 2, Q represents a group represented by Q1-3, and $R^{6b}$ represents any one substituent described in Table 1A to Table 3A (hereinafter referred to as "Compound group SX465").

The Compound (L-9), wherein n represents 0, Q represents a group represented by Q1-4, and $R^{6b}$ represents any one substituent described in Table 1A to Table 3A (hereinafter referred to as "Compound group SX466").

The Compound (L-9), wherein n represents 1, Q represents a group represented by Q1-4, and $R^{6b}$ represents any one substituent described in Table 1A to Table 3A (hereinafter referred to as "Compound group SX467").

The Compound (L-9), wherein n represents 2, Q represents a group represented by Q1-4, and $R^{6b}$ represents any one substituent described in Table 1A to Table 3A (hereinafter referred to as "Compound group SX468").

The Compound (L-9), wherein n represents 0, Q represents a group represented by Q1-5, and $R^{6b}$ represents any one substituent described in Table 1A to Table 3A (hereinafter referred to as "Compound group SX469").

The Compound (L-9), wherein n represents 1, Q represents a group represented by Q1-5, and $R^{6b}$ represents any one substituent described in Table 1A to Table 3A (hereinafter referred to as "Compound group SX470").

The Compound (L-9), wherein n represents 2, Q represents a group represented by Q1-5, and $R^{6b}$ represents any one substituent described in Table 1A to Table 3A (hereinafter referred to as "Compound group SX471").

The Compound (L-9), wherein n represents 0, Q represents a group represented by Q1-6, and $R^{6b}$ represents any one substituent described in Table 1A to Table 3A (hereinafter referred to as "Compound group SX472").

The Compound (L-9), wherein n represents 1, Q represents a group represented by Q1-6, and $R^{6b}$ represents any one substituent described in Table 1A to Table 3A (hereinafter referred to as "Compound group SX473").

The Compound (L-9), wherein n represents 2, Q represents a group represented by Q1-6, and $R^{6b}$ represents any one substituent described in Table 1A to Table 3A (hereinafter referred to as "Compound group SX474").

The Compound (L-9), wherein n represents 0, Q represents a group represented by Q1-7, and $R^{6b}$ represents any one substituent described in Table 1A to Table 3A (hereinafter referred to as "Compound group SX475").

The Compound (L-9), wherein n represents 1, Q represents a group represented by Q1-7, and $R^{6b}$ represents any one substituent described in Table 1A to Table 3A (hereinafter referred to as "Compound group SX476").

The Compound (L-9), wherein n represents 2, Q represents a group represented by Q1-7, and $R^{6b}$ represents any one substituent described in Table 1A to Table 3A (hereinafter referred to as "Compound group SX477").

The Compound (L-9), wherein n represents 0, Q represents a group represented by Q1-8, and $R^{6b}$ represents any one substituent described in Table 1A to Table 3A (hereinafter referred to as "Compound group SX478").

The Compound (L-9), wherein n represents 1, Q represents a group represented by Q1-8, and $R^{6b}$ represents any one substituent described in Table 1A to Table 3A (hereinafter referred to as "Compound group SX479").

The Compound (L-9), wherein n represents 2, Q represents a group represented by Q1-8, and $R^{6b}$ represents

any one substituent described in Table 1A to Table 3A (hereinafter referred to as "Compound group SX480").

The Compound (L-9), wherein n represents 0, Q represents a group represented by Q1-9, and $R^{6b}$ represents any one substituent described in Table 1A to Table 3A (hereinafter referred to as "Compound group SX481").

The Compound (L-9), wherein n represents 1, Q represents a group represented by Q1-9, and $R^{6b}$ represents any one substituent described in Table 1A to Table 3A (hereinafter referred to as "Compound group SX482").

The Compound (L-9), wherein n represents 2, Q represents a group represented by Q1-9, and $R^{6b}$ represents any one substituent described in Table 1A to Table 3A (hereinafter referred to as "Compound group SX483").

The Compound (L-9), wherein n represents 0, Q represents a group represented by Q1-10, and $R^{6b}$ represents any one substituent described in Table 1A to Table 3A (hereinafter referred to as "Compound group SX484").

The Compound (L-9), wherein n represents 1, Q represents a group represented by Q1-10, and $R^{6b}$ represents any one substituent described in Table 1A to Table 3A (hereinafter referred to as "Compound group SX485").

The Compound (L-9), wherein n represents 2, Q represents a group represented by Q1-10, and $R^{6b}$ represents any one substituent described in Table 1A to Table 3A (hereinafter referred to as "Compound group SX486").

The Compound (L-9), wherein n represents 0, Q represents a group represented by Q1-11, and $R^{6b}$ represents any one substituent described in Table 1A to Table 3A (hereinafter referred to as "Compound group SX487").

The Compound (L-9), wherein n represents 1, Q represents a group represented by Q1-11, and $R^{6b}$ represents any one substituent described in Table 1A to Table 3A (hereinafter referred to as "Compound group SX488").

The Compound (L-9), wherein n represents 2, Q represents a group represented by Q1-11, and $R^{6b}$ represents any one substituent described in Table 1A to Table 3A (hereinafter referred to as "Compound group SX489").

The Compound (L-9), wherein n represents 0, Q represents a group represented by Q1-12, and $R^{6b}$ represents any one substituent described in Table 1A to Table 3A (hereinafter referred to as "Compound group SX490").

The Compound (L-9), wherein n represents 1, Q represents a group represented by Q1-12, and $R^{6b}$ represents any one substituent described in Table 1A to Table 3A (hereinafter referred to as "Compound group SX491").

The Compound (L-9), wherein n represents 2, Q represents a group represented by Q1-12, and $R^{6b}$ represents any one substituent described in Table 1A to Table 3A (hereinafter referred to as "Compound group SX492").

The Compound (L-9), wherein n represents 0, Q represents a group represented by Q1-13, and $R^{6b}$ represents any one substituent described in Table 1A to Table 3A (hereinafter referred to as "Compound group SX493").

The Compound (L-9), wherein n represents 1, Q represents a group represented by Q1-13, and $R^{6b}$ represents any one substituent described in Table 1A to Table 3A (hereinafter referred to as "Compound group SX494").

The Compound (L-9), wherein n represents 2, Q represents a group represented by Q1-13, and $R^{6b}$ represents any one substituent described in Table 1A to Table 3A (hereinafter referred to as "Compound group SX495").

The Compound (L-9), wherein n represents 0, Q represents a group represented by Q1-14, and $R^{6b}$ represents any one substituent described in Table 1A to Table 3A (hereinafter referred to as "Compound group SX496").

The Compound (L-9), wherein n represents 1, Q represents a group represented by Q1-14, and $R^{6b}$ represents any one substituent described in Table 1A to Table 3A (hereinafter referred to as "Compound group SX497").

The Compound (L-9), wherein n represents 2, Q represents a group represented by Q1-14, and $R^{6b}$ represents any one substituent described in Table 1A to Table 3A (hereinafter referred to as "Compound group SX498").

The Compound (L-9), wherein n represents 0, Q represents a group represented by Q1-15, and $R^{6b}$ represents any one substituent described in Table 1A to Table 3A (hereinafter referred to as "Compound group SX499").

The Compound (L-9), wherein n represents 1, Q represents a group represented by Q1-15, and $R^{6b}$ represents any one substituent described in Table 1A to Table 3A (hereinafter referred to as "Compound group SX500").

The Compound (L-9), wherein n represents 2, Q represents a group represented by Q1-15, and $R^{6b}$ represents any one substituent described in Table 1A to Table 3A (hereinafter referred to as "Compound group SX501").

The Compound (L-9), wherein n represents 0, Q represents a group represented by Q1-16, and $R^{6b}$ represents any one substituent described in Table 1A to Table 3A (hereinafter referred to as "Compound group SX502").

The Compound (L-9), wherein n represents 1, Q represents a group represented by Q1-16, and $R^{6b}$ represents any one substituent described in Table 1A to Table 3A (hereinafter referred to as "Compound group SX503").

The Compound (L-9), wherein n represents 2, Q represents a group represented by Q1-16, and $R^{6b}$ represents any one substituent described in Table 1A to Table 3A (hereinafter referred to as "Compound group SX504").

The Compound (L-9), wherein n represents 0, Q represents a group represented by Q1-17, and $R^{6b}$ represents any one substituent described in Table 1A to Table 3A (hereinafter referred to as "Compound group SX505").

The Compound (L-9), wherein n represents 1, Q represents a group represented by Q1-17, and $R^{6b}$ represents any one substituent described in Table 1A to Table 3A (hereinafter referred to as "Compound group SX506").

The Compound (L-9), wherein n represents 2, Q represents a group represented by Q1-17, and $R^{6b}$ represents any one substituent described in Table 1A to Table 3A (hereinafter referred to as "Compound group SX507").

The Compound (L-9), wherein n represents 0, Q represents a group represented by Q1-18, and $R^{6b}$ represents any one substituent described in Table 1A to Table 3A (hereinafter referred to as "Compound group SX508").

The Compound (L-9), wherein n represents 1, Q represents a group represented by Q1-18, and $R^{6b}$ represents

any one substituent described in Table 1A to Table 3A (hereinafter referred to as "Compound group SX509").

The Compound (L-9), wherein n represents 2, Q represents a group represented by Q1-18, and $R^{6b}$ represents any one substituent described in Table 1A to Table 3A (hereinafter referred to as "Compound group SX510").

The Compound (L-9), wherein n represents 0, Q represents a group represented by Q2-1, and $R^{6b}$ represents any one substituent described in Table 1A to Table 3A (hereinafter referred to as "Compound group SX511").

The Compound (L-9), wherein n represents 1, Q represents a group represented by Q2-1, and $R^{6b}$ represents any one substituent described in Table 1A to Table 3A (hereinafter referred to as "Compound group SX512").

The Compound (L-9), wherein n represents 2, Q represents a group represented by Q2-1, and $R^{6b}$ represents any one substituent described in Table 1A to Table 3A (hereinafter referred to as "Compound group SX513").

The Compound (L-9), wherein n represents 0, Q represents a group represented by Q2-2, and $R^{6b}$ represents any one substituent described in Table 1A to Table 3A (hereinafter referred to as "Compound group SX514").

The Compound (L-9), wherein n represents 1, Q represents a group represented by Q2-2, and $R^{6b}$ represents any one substituent described in Table 1A to Table 3A (hereinafter referred to as "Compound group SX515").

The Compound (L-9), wherein n represents 2, Q represents a group represented by Q2-2, and $R^{6b}$ represents any one substituent described in Table 1A to Table 3A (hereinafter referred to as "Compound group SX516").

The Compound (L-9), wherein n represents 0, Q represents a group represented by Q2-3, and $R^{6b}$ represents any one substituent described in Table 1A to Table 3A (hereinafter referred to as "Compound group SX517").

The Compound (L-9), wherein n represents 1, Q represents a group represented by Q2-3, and $R^{6b}$ represents any one substituent described in Table 1A to Table 3A (hereinafter referred to as "Compound group SX518").

The Compound (L-9), wherein n represents 2, Q represents a group represented by Q2-3, and $R^{6b}$ represents any one substituent described in Table 1A to Table 3A (hereinafter referred to as "Compound group SX519").

The Compound (L-9), wherein n represents 0, Q represents a group represented by Q2-4, and $R^{6b}$ represents any one substituent described in Table 1A to Table 3A (hereinafter referred to as "Compound group SX520").

The Compound (L-9), wherein n represents 1, Q represents a group represented by Q2-4, and $R^{6b}$ represents any one substituent described in Table 1A to Table 3A (hereinafter referred to as "Compound group SX521").

The Compound (L-9), wherein n represents 2, Q represents a group represented by Q2-4, and $R^{6b}$ represents any one substituent described in Table 1A to Table 3A (hereinafter referred to as "Compound group SX522").

The Compound (L-9), wherein n represents 0, Q represents a group represented by Q2-5, and $R^{6b}$ represents any one substituent described in Table 1A to Table 3A (hereinafter referred to as "Compound group SX523").

The Compound (L-9), wherein n represents 1, Q represents a group represented by Q2-5, and $R^{6b}$ represents any one substituent described in Table 1A to Table 3A (hereinafter referred to as "Compound group SX524").

The Compound (L-9), wherein n represents 2, Q represents a group represented by Q2-5, and $R^{6b}$ represents any one substituent described in Table 1A to Table 3A (hereinafter referred to as "Compound group SX525").

The Compound (L-9), wherein n represents 0, Q represents a group represented by Q2-6, and $R^{6b}$ represents any one substituent described in Table 1A to Table 3A (hereinafter referred to as "Compound group SX526").

The Compound (L-9), wherein n represents 1, Q represents a group represented by Q2-6, and $R^{6b}$ represents any one substituent described in Table 1A to Table 3A (hereinafter referred to as "Compound group SX527").

The Compound (L-9), wherein n represents 2, Q represents a group represented by Q2-6, and $R^{6b}$ represents any one substituent described in Table 1A to Table 3A (hereinafter referred to as "Compound group SX528").

The Compound (L-9), wherein n represents 0, Q represents a group represented by Q2-7, and $R^{6b}$ represents any one substituent described in Table 1A to Table 3A (hereinafter referred to as "Compound group SX529").

The Compound (L-9), wherein n represents 1, Q represents a group represented by Q2-7, and $R^{6b}$ represents any one substituent described in Table 1A to Table 3A (hereinafter referred to as "Compound group SX530").

The Compound (L-9), wherein n represents 2, Q represents a group represented by Q2-7, and $R^{6b}$ represents any one substituent described in Table 1A to Table 3A (hereinafter referred to as "Compound group SX531").

The Compound (L-9), wherein n represents 0, Q represents a group represented by Q2-8, and $R^{6b}$ represents any one substituent described in Table 1A to Table 3A (hereinafter referred to as "Compound group SX532").

The Compound (L-9), wherein n represents 1, Q represents a group represented by Q2-8, and $R^{6b}$ represents any one substituent described in Table 1A to Table 3A (hereinafter referred to as "Compound group SX533").

The Compound (L-9), wherein n represents 2, Q represents a group represented by Q2-8, and $R^{6b}$ represents any one substituent described in Table 1A to Table 3A (hereinafter referred to as "Compound group SX534").

The Compound (L-9), wherein n represents 0, Q represents a group represented by Q2-9, and $R^{6b}$ represents any one substituent described in Table 1A to Table 3A (hereinafter referred to as "Compound group SX535").

The Compound (L-9), wherein n represents 1, Q represents a group represented by Q2-9, and $R^{6b}$ represents any one substituent described in Table 1A to Table 3A (hereinafter referred to as "Compound group SX536").

The Compound (L-9), wherein n represents 2, Q represents a group represented by Q2-9, and $R^{6b}$ represents any one substituent described in Table 1A to Table 3A (hereinafter referred to as "Compound group SX537").

The Compound (L-9), wherein n represents 0, Q represents a group represented by Q2-10, and $R^{6b}$ represents

any one substituent described in Table 1A to Table 3A (hereinafter referred to as "Compound group SX538").

The Compound (L-9), wherein n represents 1, Q represents a group represented by Q2-10, and $R^{6b}$ represents any one substituent described in Table 1A to Table 3A (hereinafter referred to as "Compound group SX539").

The Compound (L-9), wherein n represents 2, Q represents a group represented by Q2-10, and $R^{6b}$ represents any one substituent described in Table 1A to Table 3A (hereinafter referred to as "Compound group SX540").

The Compound (L-9), wherein n represents 0, Q represents a group represented by Q2-11, and $R^{6b}$ represents any one substituent described in Table 1A to Table 3A (hereinafter referred to as "Compound group SX541").

The Compound (L-9), wherein n represents 1, Q represents a group represented by Q2-11, and $R^{6b}$ represents any one substituent described in Table 1A to Table 3A (hereinafter referred to as "Compound group SX542").

The Compound (L-9), wherein n represents 2, Q represents a group represented by Q2-11, and $R^{6b}$ represents any one substituent described in Table 1A to Table 3A (hereinafter referred to as "Compound group SX543").

The Compound (L-9), wherein n represents 0, Q represents a group represented by Q2-12, and $R^{6b}$ represents any one substituent described in Table 1A to Table 3A (hereinafter referred to as "Compound group SX544").

The Compound (L-9), wherein n represents 1, Q represents a group represented by Q2-12, and $R^{6b}$ represents any one substituent described in Table 1A to Table 3A (hereinafter referred to as "Compound group SX545").

The Compound (L-9), wherein n represents 2, Q represents a group represented by Q2-12, and $R^{6b}$ represents any one substituent described in Table 1A to Table 3A (hereinafter referred to as "Compound group SX546").

The Compound (L-9), wherein n represents 0, Q represents a group represented by Q2-13, and $R^{6b}$ represents any one substituent described in Table 1A to Table 3A (hereinafter referred to as "Compound group SX547").

The Compound (L-9), wherein n represents 1, Q represents a group represented by Q2-13, and $R^{6b}$ represents any one substituent described in Table 1A to Table 3A (hereinafter referred to as "Compound group SX548").

The Compound (L-9), wherein n represents 2, Q represents a group represented by Q2-13, and $R^{6b}$ represents any one substituent described in Table 1A to Table 3A (hereinafter referred to as "Compound group SX549").

The Compound (L-9), wherein n represents 0, Q represents a group represented by Q2-14, and $R^{6b}$ represents any one substituent described in Table 1A to Table 3A (hereinafter referred to as "Compound group SX550").

The Compound (L-9), wherein n represents 1, Q represents a group represented by Q2-14, and $R^{6b}$ represents any one substituent described in Table 1A to Table 3A (hereinafter referred to as "Compound group SX551").

The Compound (L-9), wherein n represents 2, Q represents a group represented by Q2-14, and $R^{6b}$ represents any one substituent described in Table 1A to Table 3A (hereinafter referred to as "Compound group SX552").

The Compound (L-9), wherein n represents 0, Q represents a group represented by Q2-15, and $R^{6b}$ represents any one substituent described in Table 1A to Table 3A (hereinafter referred to as "Compound group SX553").

The Compound (L-9), wherein n represents 1, Q represents a group represented by Q2-15, and $R^{6b}$ represents any one substituent described in Table 1A to Table 3A (hereinafter referred to as "Compound group SX554").

The Compound (L-9), wherein n represents 2, Q represents a group represented by Q2-15, and $R^{6b}$ represents any one substituent described in Table 1A to Table 3A (hereinafter referred to as "Compound group SX555").

The Compound (L-9), wherein n represents 0, Q represents a group represented by Q2-16, and $R^{6b}$ represents any one substituent described in Table 1A to Table 3A (hereinafter referred to as "Compound group SX556").

The Compound (L-9), wherein n represents 1, Q represents a group represented by Q2-16, and $R^{6b}$ represents any one substituent described in Table 1A to Table 3A (hereinafter referred to as "Compound group SX557").

The Compound (L-9), wherein n represents 2, Q represents a group represented by Q2-16, and $R^{6b}$ represents any one substituent described in Table 1A to Table 3A (hereinafter referred to as "Compound group SX558").

The Compound (L-9), wherein n represents 0, Q represents a group represented by Q2-17, and $R^{6b}$ represents any one substituent described in Table 1A to Table 3A (hereinafter referred to as "Compound group SX559").

The Compound (L-9), wherein n represents 1, Q represents a group represented by Q2-17, and $R^{6b}$ represents any one substituent described in Table 1A to Table 3A (hereinafter referred to as "Compound group SX560").

The Compound (L-9), wherein n represents 2, Q represents a group represented by Q2-17, and $R^{6b}$ represents any one substituent described in Table 1A to Table 3A (hereinafter referred to as "Compound group SX561").

The Compound (L-9), wherein n represents 0, Q represents a group represented by Q2-18, and $R^{6b}$ represents any one substituent described in Table 1A to Table 3A (hereinafter referred to as "Compound group SX562").

The Compound (L-9), wherein n represents 1, Q represents a group represented by Q2-18, and $R^{6b}$ represents any one substituent described in Table 1A to Table 3A (hereinafter referred to as "Compound group SX563").

The Compound (L-9), wherein n represents 2, Q represents a group represented by Q2-18, and $R^{6b}$ represents any one substituent described in Table 1A to Table 3A (hereinafter referred to as "Compound group SX564").

The Compound (L-9), wherein n represents 0, Q represents a group represented by Q2-19, and $R^{6b}$ represents any one substituent described in Table 1A to Table 3A (hereinafter referred to as "Compound group SX565").

The Compound (L-9), wherein n represents 1, Q represents a group represented by Q2-19, and $R^{6b}$ represents any one substituent described in Table 1A to Table 3A (hereinafter referred to as "Compound group SX566").

The Compound (L-9), wherein n represents 2, Q represents a group represented by Q2-19, and $R^{6b}$ represents

any one substituent described in Table 1A to Table 3A (hereinafter referred to as "Compound group SX567").

The Compound (L-9), wherein n represents 0, Q represents a group represented by Q2-20, and $R^{6b}$ represents any one substituent described in Table 1A to Table 3A (hereinafter referred to as "Compound group SX568").

The Compound (L-9), wherein n represents 1, Q represents a group represented by Q2-20, and $R^{6b}$ represents any one substituent described in Table 1A to Table 3A (hereinafter referred to as "Compound group SX569").

The Compound (L-9), wherein n represents 2, Q represents a group represented by Q2-20, and $R^{6b}$ represents any one substituent described in Table 1A to Table 3A (hereinafter referred to as "Compound group SX570").

The Compound (L-9), wherein n represents 0, Q represents a group represented by Q2-21, and $R^{6b}$ represents any one substituent described in Table 1A to Table 3A (hereinafter referred to as "Compound group SX571").

The Compound (L-9), wherein n represents 1, Q represents a group represented by Q2-21, and $R^{6b}$ represents any one substituent described in Table 1A to Table 3A (hereinafter referred to as "Compound group SX572").

The Compound (L-9), wherein n represents 2, Q represents a group represented by Q2-21, and $R^{6b}$ represents any one substituent described in Table 1A to Table 3A (hereinafter referred to as "Compound group SX573").

The Compound (L-9), wherein n represents 0, Q represents a group represented by Q2-22, and $R^{6b}$ represents any one substituent described in Table 1A to Table 3A (hereinafter referred to as "Compound group SX574").

The Compound (L-9), wherein n represents 1, Q represents a group represented by Q2-22, and $R^{6b}$ represents any one substituent described in Table 1A to Table 3A (hereinafter referred to as "Compound group SX575").

The Compound (L-9), wherein n represents 2, Q represents a group represented by Q2-22, and $R^{6b}$ represents any one substituent described in Table 1A to Table 3A (hereinafter referred to as "Compound group SX576").

The Compound (L-9), wherein n represents 0, Q represents a group represented by Q2-23, and $R^{6b}$ represents any one substituent described in Table 1A to Table 3A (hereinafter referred to as "Compound group SX577").

The Compound (L-9), wherein n represents 1, Q represents a group represented by Q2-23, and $R^{6b}$ represents any one substituent described in Table 1A to Table 3A (hereinafter referred to as "Compound group SX578").

The Compound (L-9), wherein n represents 2, Q represents a group represented by Q2-23, and $R^{6b}$ represents any one substituent described in Table 1A to Table 3A (hereinafter referred to as "Compound group SX579").

The Compound (L-9), wherein n represents 0, Q represents a group represented by Q2-24, and $R^{6b}$ represents any one substituent described in Table 1A to Table 3A (hereinafter referred to as "Compound group SX580").

The Compound (L-9), wherein n represents 1, Q represents a group represented by Q2-24, and $R^{6b}$ represents any one substituent described in Table 1A to Table 3A (hereinafter referred to as "Compound group SX581").

The Compound (L-9), wherein n represents 2, Q represents a group represented by Q2-24, and $R^{6b}$ represents any one substituent described in Table 1A to Table 3A (hereinafter referred to as "Compound group SX582").

The Compound (L-9), wherein n represents 0, Q represents a group represented by Q2-25, and $R^{6b}$ represents any one substituent described in Table 1A to Table 3A (hereinafter referred to as "Compound group SX583").

The Compound (L-9), wherein n represents 1, Q represents a group represented by Q2-25, and $R^{6b}$ represents any one substituent described in Table 1A to Table 3A (hereinafter referred to as "Compound group SX584").

The Compound (L-9), wherein n represents 2, Q represents a group represented by Q2-25, and $R^{6b}$ represents any one substituent described in Table 1A to Table 3A (hereinafter referred to as "Compound group SX585").

The Compound (L-9), wherein n represents 0, Q represents a group represented by Q2-26, and $R^{6b}$ represents any one substituent described in Table 1A to Table 3A (hereinafter referred to as "Compound group SX586").

The Compound (L-9), wherein n represents 1, Q represents a group represented by Q2-26, and $R^{6b}$ represents any one substituent described in Table 1A to Table 3A (hereinafter referred to as "Compound group SX587").

The Compound (L-9), wherein n represents 2, Q represents a group represented by Q2-26, and $R^{6b}$ represents any one substituent described in Table 1A to Table 3A (hereinafter referred to as "Compound group SX588").

The Compound (L-9), wherein n represents 0, Q represents a group represented by Q2-27, and $R^{6b}$ represents any one substituent described in Table 1A to Table 3A (hereinafter referred to as "Compound group SX589").

The Compound (L-9), wherein n represents 1, Q represents a group represented by Q2-27, and $R^{6b}$ represents any one substituent described in Table 1A to Table 3A (hereinafter referred to as "Compound group SX590").

The Compound (L-9), wherein n represents 2, Q represents a group represented by Q2-27, and $R^{6b}$ represents any one substituent described in Table 1A to Table 3A (hereinafter referred to as "Compound group SX591").

The Compound (L-9), wherein n represents 0, Q represents a group represented by Q2-28, and $R^{6b}$ represents any one substituent described in Table 1A to Table 3A (hereinafter referred to as "Compound group SX592").

The Compound (L-9), wherein n represents 1, Q represents a group represented by Q2-28, and $R^{6b}$ represents any one substituent described in Table 1A to Table 3A (hereinafter referred to as "Compound group SX593").

The Compound (L-9), wherein n represents 2, Q represents a group represented by Q2-28, and $R^{6b}$ represents any one substituent described in Table 1A to Table 3A (hereinafter referred to as "Compound group SX594").

The Compound (L-9), wherein n represents 0, Q represents a group represented by Q2-29, and $R^{6b}$ represents any one substituent described in Table 1A to Table 3A (hereinafter referred to as "Compound group SX595").

The Compound (L-9), wherein n represents 1, Q represents a group represented by Q2-29, and $R^{6b}$ represents

any one substituent described in Table 1A to Table 3A (hereinafter referred to as "Compound group SX596").

The Compound (L-9), wherein n represents 2, Q represents a group represented by Q2-29, and $R^{6b}$ represents any one substituent described in Table 1A to Table 3A (hereinafter referred to as "Compound group SX597").

The Compound (L-9), wherein n represents 0, Q represents a group represented by Q2-30, and $R^{6b}$ represents any one substituent described in Table 1A to Table 3A (hereinafter referred to as "Compound group SX598").

The Compound (L-9), wherein n represents 1, Q represents a group represented by Q2-30, and $R^{6b}$ represents any one substituent described in Table 1A to Table 3A (hereinafter referred to as "Compound group SX599").

The Compound (L-9), wherein n represents 2, Q represents a group represented by Q2-30, and $R^{6b}$ represents any one substituent described in Table 1A to Table 3A (hereinafter referred to as "Compound group SX600").

The Compound (L-9), wherein n represents 0, Q represents a group represented by Q2-31, and $R^{6b}$ represents any one substituent described in Table 1A to Table 3A (hereinafter referred to as "Compound group SX601").

The Compound (L-9), wherein n represents 1, Q represents a group represented by Q2-31, and $R^{6b}$ represents any one substituent described in Table 1A to Table 3A (hereinafter referred to as "Compound group SX602").

The Compound (L-9), wherein n represents 2, Q represents a group represented by Q2-31, and $R^{6b}$ represents any one substituent described in Table 1A to Table 3A (hereinafter referred to as "Compound group SX603").

The Compound (L-9), wherein n represents 0, Q represents a group represented by Q2-32, and $R^{6b}$ represents any one substituent described in Table 1A to Table 3A (hereinafter referred to as "Compound group SX604").

The Compound (L-9), wherein n represents 1, Q represents a group represented by Q2-32, and $R^{6b}$ represents any one substituent described in Table 1A to Table 3A (hereinafter referred to as "Compound group SX605").

The Compound (L-9), wherein n represents 2, Q represents a group represented by Q2-32, and $R^{6b}$ represents any one substituent described in Table 1A to Table 3A (hereinafter referred to as "Compound group SX606").

The Compound (L-9), wherein n represents 0, Q represents a group represented by Q2-33, and $R^{6b}$ represents any one substituent described in Table 1A to Table 3A (hereinafter referred to as "Compound group SX607").

The Compound (L-9), wherein n represents 1, Q represents a group represented by Q2-33, and $R^{6b}$ represents any one substituent described in Table 1A to Table 3A (hereinafter referred to as "Compound group SX608").

The Compound (L-9), wherein n represents 2, Q represents a group represented by Q2-33, and $R^{6b}$ represents any one substituent described in Table 1A to Table 3A (hereinafter referred to as "Compound group SX609").

The Compound (L-9), wherein n represents 0, Q represents a group represented by Q2-34, and $R^{6b}$ represents any one substituent described in Table 1A to Table 3A (hereinafter referred to as "Compound group SX610").

The Compound (L-9), wherein n represents 1, Q represents a group represented by Q2-34, and $R^{6b}$ represents any one substituent described in Table 1A to Table 3A (hereinafter referred to as "Compound group SX611").

The Compound (L-9), wherein n represents 2, Q represents a group represented by Q2-34, and $R^{6b}$ represents any one substituent described in Table 1A to Table 3A (hereinafter referred to as "Compound group SX612").

The Compound (L-9), wherein n represents 0, Q represents a group represented by Q2-35, and $R^{6b}$ represents any one substituent described in Table 1A to Table 3A (hereinafter referred to as "Compound group SX613").

The Compound (L-9), wherein n represents 1, Q represents a group represented by Q2-35, and $R^{6b}$ represents any one substituent described in Table 1A to Table 3A (hereinafter referred to as "Compound group SX614").

The Compound (L-9), wherein n represents 2, Q represents a group represented by Q2-35, and $R^{6b}$ represents any one substituent described in Table 1A to Table 3A (hereinafter referred to as "Compound group SX615").

The Compound (L-9), wherein n represents 0, Q represents a group represented by Q2-36, and $R^{6b}$ represents any one substituent described in Table 1A to Table 3A (hereinafter referred to as "Compound group SX616").

The Compound (L-9), wherein n represents 1, Q represents a group represented by Q2-36, and $R^{6b}$ represents any one substituent described in Table 1A to Table 3A (hereinafter referred to as "Compound group SX617").

The Compound (L-9), wherein n represents 2, Q represents a group represented by Q2-36, and $R^{6b}$ represents any one substituent described in Table 1A to Table 3A (hereinafter referred to as "Compound group SX618").

The Compound (L-9), wherein n represents 0, Q represents a group represented by Q2-37, and $R^{6b}$ represents any one substituent described in Table 1A to Table 3A (hereinafter referred to as "Compound group SX619").

The Compound (L-9), wherein n represents 1, Q represents a group represented by Q2-37, and $R^{6b}$ represents any one substituent described in Table 1A to Table 3A (hereinafter referred to as "Compound group SX620").

The Compound (L-9), wherein n represents 2, Q represents a group represented by Q2-37, and $R^{6b}$ represents any one substituent described in Table 1A to Table 3A (hereinafter referred to as "Compound group SX621").

The Compound (L-9), wherein n represents 0, Q represents a group represented by Q2-38, and $R^{6b}$ represents any one substituent described in Table 1A to Table 3A (hereinafter referred to as "Compound group SX622").

The Compound (L-9), wherein n represents 1, Q represents a group represented by Q2-38, and $R^{6b}$ represents any one substituent described in Table 1A to Table 3A (hereinafter referred to as "Compound group SX623").

The Compound (L-9), wherein n represents 2, Q represents a group represented by Q2-38, and $R^{6b}$ represents any one substituent described in Table 1A to Table 3A (hereinafter referred to as "Compound group SX624").

The Compound (L-9), wherein n represents 0, Q represents a group represented by Q2-39, and $R^{6b}$ represents

any one substituent described in Table 1A to Table 3A (hereinafter referred to as "Compound group SX625").

The Compound (L-9), wherein n represents 1, Q represents a group represented by Q2-39, and $R^{6b}$ represents any one substituent described in Table 1A to Table 3A (hereinafter referred to as "Compound group SX626").

The Compound (L-9), wherein n represents 2, Q represents a group represented by Q2-39, and $R^{6b}$ represents any one substituent described in Table 1A to Table 3A (hereinafter referred to as "Compound group SX627").

The Compound (L-9), wherein n represents 0, Q represents a group represented by Q2-40, and $R^{6b}$ represents any one substituent described in Table 1A to Table 3A (hereinafter referred to as "Compound group SX628").

The Compound (L-9), wherein n represents 1, Q represents a group represented by Q2-40, and $R^{6b}$ represents any one substituent described in Table 1A to Table 3A (hereinafter referred to as "Compound group SX629").

The Compound (L-9), wherein n represents 2, Q represents a group represented by Q2-40, and $R^{6b}$ represents any one substituent described in Table 1A to Table 3A (hereinafter referred to as "Compound group SX630").

The Compound (L-9), wherein n represents 0, Q represents a group represented by Q2-41, and $R^{6b}$ represents any one substituent described in Table 1A to Table 3A (hereinafter referred to as "Compound group SX631").

The Compound (L-9), wherein n represents 1, Q represents a group represented by Q2-41, and $R^{6b}$ represents any one substituent described in Table 1A to Table 3A (hereinafter referred to as "Compound group SX632").

The Compound (L-9), wherein n represents 2, Q represents a group represented by Q2-41, and $R^{6b}$ represents any one substituent described in Table 1A to Table 3A (hereinafter referred to as "Compound group SX633").

The Compound (L-9), wherein n represents 0, Q represents a group represented by Q2-42, and $R^{6b}$ represents any one substituent described in Table 1A to Table 3A (hereinafter referred to as "Compound group SX634").

The Compound (L-9), wherein n represents 1, Q represents a group represented by Q2-42, and $R^{6b}$ represents any one substituent described in Table 1A to Table 3A (hereinafter referred to as "Compound group SX635").

The Compound (L-9), wherein n represents 2, Q represents a group represented by Q2-42, and $R^{6b}$ represents any one substituent described in Table 1A to Table 3A (hereinafter referred to as "Compound group SX636").

The Compound (L-9), wherein n represents 0, Q represents a group represented by Q2-43, and $R^{6b}$ represents any one substituent described in Table 1A to Table 3A (hereinafter referred to as "Compound group SX637").

The Compound (L-9), wherein n represents 1, Q represents a group represented by Q2-43, and $R^{6b}$ represents any one substituent described in Table 1A to Table 3A (hereinafter referred to as "Compound group SX638").

The Compound (L-9), wherein n represents 2, Q represents a group represented by Q2-43, and $R^{6b}$ represents any one substituent described in Table 1A to Table 3A (hereinafter referred to as "Compound group SX639").

The Compound (L-9), wherein n represents 0, Q represents a group represented by Q2-44, and $R^{6b}$ represents any one substituent described in Table 1A to Table 3A (hereinafter referred to as "Compound group SX640").

The Compound (L-9), wherein n represents 1, Q represents a group represented by Q2-44, and $R^{6b}$ represents any one substituent described in Table 1A to Table 3A (hereinafter referred to as "Compound group SX641").

The Compound (L-9), wherein n represents 2, Q represents a group represented by Q2-44, and $R^{6b}$ represents any one substituent described in Table 1A to Table 3A (hereinafter referred to as "Compound group SX642").

The Compound (L-9), wherein n represents 0, Q represents a group represented by Q2-45, and $R^{6b}$ represents any one substituent described in Table 1A to Table 3A (hereinafter referred to as "Compound group SX643").

The Compound (L-9), wherein n represents 1, Q represents a group represented by Q2-45, and $R^{6b}$ represents any one substituent described in Table 1A to Table 3A (hereinafter referred to as "Compound group SX644").

The Compound (L-9), wherein n represents 2, Q represents a group represented by Q2-45, and $R^{6b}$ represents any one substituent described in Table 1A to Table 3A (hereinafter referred to as "Compound group SX645").

The Compound (L-9), wherein n represents 0, Q represents a group represented by Q2-46, and $R^{6b}$ represents any one substituent described in Table 1A to Table 3A (hereinafter referred to as "Compound group SX646").

The Compound (L-9), wherein n represents 1, Q represents a group represented by Q2-46, and $R^{6b}$ represents any one substituent described in Table 1A to Table 3A (hereinafter referred to as "Compound group SX647").

The Compound (L-9), wherein n represents 2, Q represents a group represented by Q2-46, and $R^{6b}$ represents any one substituent described in Table 1A to Table 3A (hereinafter referred to as "Compound group SX648").

The Compound (L-9), wherein n represents 0, Q represents a group represented by Q2-47, and $R^{6b}$ represents any one substituent described in Table 1A to Table 3A (hereinafter referred to as "Compound group SX649").

The Compound (L-9), wherein n represents 1, Q represents a group represented by Q2-47, and $R^{6b}$ represents any one substituent described in Table 1A to Table 3A (hereinafter referred to as "Compound group SX650").

The Compound (L-9), wherein n represents 2, Q represents a group represented by Q2-47, and $R^{6b}$ represents any one substituent described in Table 1A to Table 3A (hereinafter referred to as "Compound group SX651").

The Compound (L-9), wherein n represents 0, Q represents a group represented by Q2-48, and $R^{6b}$ represents any one substituent described in Table 1A to Table 3A (hereinafter referred to as "Compound group SX652").

The Compound (L-9), wherein n represents 1, Q represents a group represented by Q2-48, and $R^{6b}$ represents any one substituent described in Table 1A to Table 3A (hereinafter referred to as "Compound group SX653").

The Compound (L-9), wherein n represents 2, Q represents a group represented by Q2-48, and $R^{6b}$ represents

any one substituent described in Table 1A to Table 3A (hereinafter referred to as "Compound group SX654").

The Compound (L-9), wherein n represents 0, Q represents a group represented by Q2-49, and $R^{6b}$ represents any one substituent described in Table 1A to Table 3A (hereinafter referred to as "Compound group SX655").

The Compound (L-9), wherein n represents 1, Q represents a group represented by Q2-49, and $R^{6b}$ represents any one substituent described in Table 1A to Table 3A (hereinafter referred to as "Compound group SX656").

The Compound (L-9), wherein n represents 2, Q represents a group represented by Q2-49, and $R^{6b}$ represents any one substituent described in Table 1A to Table 3A (hereinafter referred to as "Compound group SX657").

The Compound (L-9), wherein n represents 0, Q represents a group represented by Q2-50, and $R^{6b}$ represents any one substituent described in Table 1A to Table 3A (hereinafter referred to as "Compound group SX658").

The Compound (L-9), wherein n represents 1, Q represents a group represented by Q2-50, and $R^{6b}$ represents any one substituent described in Table 1A to Table 3A (hereinafter referred to as "Compound group SX659").

The Compound (L-9), wherein n represents 2, Q represents a group represented by Q2-50, and $R^{6b}$ represents any one substituent described in Table 1A to Table 3A (hereinafter referred to as "Compound group SX660").

The Compound (L-9), wherein n represents 0, Q represents a group represented by Q2-51, and $R^{6b}$ represents any one substituent described in Table 1A to Table 3A (hereinafter referred to as "Compound group SX661").

The Compound (L-9), wherein n represents 1, Q represents a group represented by Q2-51, and $R^{6b}$ represents any one substituent described in Table 1A to Table 3A (hereinafter referred to as "Compound group SX662").

The Compound (L-9), wherein n represents 2, Q represents a group represented by Q2-51, and $R^{6b}$ represents any one substituent described in Table 1A to Table 3A (hereinafter referred to as "Compound group SX663").

The Compound (L-9), wherein n represents 0, Q represents a group represented by Q2-52, and $R^{6b}$ represents any one substituent described in Table 1A to Table 3A (hereinafter referred to as "Compound group SX664").

The Compound (L-9), wherein n represents 1, Q represents a group represented by Q2-52, and $R^{6b}$ represents any one substituent described in Table 1A to Table 3A (hereinafter referred to as "Compound group SX665").

The Compound (L-9), wherein n represents 2, Q represents a group represented by Q2-52, and $R^{6b}$ represents any one substituent described in Table 1A to Table 3A (hereinafter referred to as "Compound group SX666").

The Compound (L-9), wherein n represents 0, Q represents a group represented by Q2-53, and $R^{6b}$ represents any one substituent described in Table 1A to Table 3A (hereinafter referred to as "Compound group SX667").

The Compound (L-9), wherein n represents 1, Q represents a group represented by Q2-53, and $R^{6b}$ represents any one substituent described in Table 1A to Table 3A (hereinafter referred to as "Compound group SX668").

The Compound (L-9), wherein n represents 2, Q represents a group represented by Q2-53, and $R^{6b}$ represents any one substituent described in Table 1A to Table 3A (hereinafter referred to as "Compound group SX669").

The Compound (L-9), wherein n represents 0, Q represents a group represented by Q2-54, and $R^{6b}$ represents any one substituent described in Table 1A to Table 3A (hereinafter referred to as "Compound group SX670").

The Compound (L-9), wherein n represents 1, Q represents a group represented by Q2-54, and $R^{6b}$ represents any one substituent described in Table 1A to Table 3A (hereinafter referred to as "Compound group SX671").

The Compound (L-9), wherein n represents 2, Q represents a group represented by Q2-54, and $R^{6b}$ represents any one substituent described in Table 1A to Table 3A (hereinafter referred to as "Compound group SX672").

The Compound (L-9), wherein n represents 0, Q represents a group represented by Q2-55, and $R^{6b}$ represents any one substituent described in Table 1A to Table 3A (hereinafter referred to as "Compound group SX673").

The Compound (L-9), wherein n represents 1, Q represents a group represented by Q2-55, and $R^{6b}$ represents any one substituent described in Table 1A to Table 3A (hereinafter referred to as "Compound group SX674").

The Compound (L-9), wherein n represents 2, Q represents a group represented by Q2-55, and $R^{6b}$ represents any one substituent described in Table 1A to Table 3A (hereinafter referred to as "Compound group SX675").

The Compound (L-9), wherein n represents 0, Q represents a group represented by Q2-56, and $R^{6b}$ represents any one substituent described in Table 1A to Table 3A (hereinafter referred to as "Compound group SX676").

The Compound (L-9), wherein n represents 1, Q represents a group represented by Q2-56, and $R^{6b}$ represents any one substituent described in Table 1A to Table 3A (hereinafter referred to as "Compound group SX677").

The Compound (L-9), wherein n represents 2, Q represents a group represented by Q2-56, and $R^{6b}$ represents any one substituent described in Table 1A to Table 3A (hereinafter referred to as "Compound group SX678").

The Compound (L-9), wherein n represents 0, Q represents a group represented by Q2-57, and $R^{6b}$ represents any one substituent described in Table 1A to Table 3A (hereinafter referred to as "Compound group SX679").

The Compound (L-9), wherein n represents 1, Q represents a group represented by Q2-57, and $R^{6b}$ represents any one substituent described in Table 1A to Table 3A (hereinafter referred to as "Compound group SX680").

The Compound (L-9), wherein n represents 2, Q represents a group represented by Q2-57, and $R^{6b}$ represents any one substituent described in Table 1A to Table 3A (hereinafter referred to as "Compound group SX681").

The Compound (L-9), wherein n represents 0, Q represents a group represented by Q2-58, and $R^{6b}$ represents any one substituent described in Table 1A to Table 3A (hereinafter referred to as "Compound group SX682").

The Compound (L-9), wherein n represents 1, Q represents a group represented by Q2-58, and $R^{6b}$ represents

any one substituent described in Table 1A to Table 3A (hereinafter referred to as "Compound group SX683").

The Compound (L-9), wherein n represents 2, Q represents a group represented by Q2-58, and $R^{6b}$ represents any one substituent described in Table 1A to Table 3A (hereinafter referred to as "Compound group SX684").

The Compound (L-9), wherein n represents 0, Q represents a group represented by Q2-59, and $R^{6b}$ represents any one substituent described in Table 1A to Table 3A (hereinafter referred to as "Compound group SX685").

The Compound (L-9), wherein n represents 1, Q represents a group represented by Q2-59, and $R^{6b}$ represents any one substituent described in Table 1A to Table 3A (hereinafter referred to as "Compound group SX686").

The Compound (L-9), wherein n represents 2, Q represents a group represented by Q2-59, and $R^{6b}$ represents any one substituent described in Table 1A to Table 3A (hereinafter referred to as "Compound group SX687").

The Compound (L-9), wherein n represents 0, Q represents a group represented by Q2-60, and $R^{6b}$ represents any one substituent described in Table 1A to Table 3A (hereinafter referred to as "Compound group SX688").

The Compound (L-9), wherein n represents 1, Q represents a group represented by Q2-60, and $R^{6b}$ represents any one substituent described in Table 1A to Table 3A (hereinafter referred to as "Compound group SX689").

The Compound (L-9), wherein n represents 2, Q represents a group represented by Q2-60, and $R^{6b}$ represents any one substituent described in Table 1A to Table 3A (hereinafter referred to as "Compound group SX690").

The Compound (L-9), wherein n represents 0, Q represents a group represented by Q2-61, and $R^{6b}$ represents any one substituent described in Table 1A to Table 3A (hereinafter referred to as "Compound group SX691").

The Compound (L-9), wherein n represents 1, Q represents a group represented by Q2-61, and $R^{6b}$ represents any one substituent described in Table 1A to Table 3A (hereinafter referred to as "Compound group SX692").

The Compound (L-9), wherein n represents 2, Q represents a group represented by Q2-61, and $R^{6b}$ represents any one substituent described in Table 1A to Table 3A (hereinafter referred to as "Compound group SX693").

The Compound (L-9), wherein n represents 0, Q represents a group represented by Q2-62, and $R^{6b}$ represents any one substituent described in Table 1A to Table 3A (hereinafter referred to as "Compound group SX694").

The Compound (L-9), wherein n represents 1, Q represents a group represented by Q2-62, and $R^{6b}$ represents any one substituent described in Table 1A to Table 3A (hereinafter referred to as "Compound group SX695").

The Compound (L-9), wherein n represents 2, Q represents a group represented by Q2-62, and $R^{6b}$ represents any one substituent described in Table 1A to Table 3A (hereinafter referred to as "Compound group SX696").

The Compound (L-9), wherein n represents 0, Q represents a group represented by Q2-63, and $R^{6b}$ represents any one substituent described in Table 1A to Table 3A (hereinafter referred to as "Compound group SX697").

The Compound (L-9), wherein n represents 1, Q represents a group represented by Q2-63, and $R^{6b}$ represents any one substituent described in Table 1A to Table 3A (hereinafter referred to as "Compound group SX698").

The Compound (L-9), wherein n represents 2, Q represents a group represented by Q2-63, and $R^{6b}$ represents any one substituent described in Table 1A to Table 3A (hereinafter referred to as "Compound group SX699").

The Compound (L-9), wherein n represents 0, Q represents a group represented by Q2-64, and $R^{6b}$ represents any one substituent described in Table 1A to Table 3A (hereinafter referred to as "Compound group SX700").

The Compound (L-9), wherein n represents 1, Q represents a group represented by Q2-64, and $R^{6b}$ represents any one substituent described in Table 1A to Table 3A (hereinafter referred to as "Compound group SX701").

The Compound (L-9), wherein n represents 2, Q represents a group represented by Q2-64, and $R^{6b}$ represents any one substituent described in Table 1A to Table 3A (hereinafter referred to as "Compound group SX702").

The Compound (L-9), wherein n represents 0, Q represents a group represented by Q2-65, and $R^{6b}$ represents any one substituent described in Table 1A to Table 3A (hereinafter referred to as "Compound group SX703").

The Compound (L-9), wherein n represents 1, Q represents a group represented by Q2-65, and $R^{6b}$ represents any one substituent described in Table 1A to Table 3A (hereinafter referred to as "Compound group SX704").

The Compound (L-9), wherein n represents 2, Q represents a group represented by Q2-65, and $R^{6b}$ represents any one substituent described in Table 1A to Table 3A (hereinafter referred to as "Compound group SX705").

The Compound (L-9), wherein n represents 0, Q represents a group represented by Q2-66, and $R^{6b}$ represents any one substituent described in Table 1A to Table 3A (hereinafter referred to as "Compound group SX706").

The Compound (L-9), wherein n represents 1, Q represents a group represented by Q2-66, and $R^{6b}$ represents any one substituent described in Table 1A to Table 3A (hereinafter referred to as "Compound group SX707").

The Compound (L-9), wherein n represents 2, Q represents a group represented by Q2-66, and $R^{6b}$ represents any one substituent described in Table 1A to Table 3A (hereinafter referred to as "Compound group SX708").

Q1-1 to Q1-18 and Q2-1 to Q2-66 are the following groups (wherein # represents the binding site to the sulfur atom, and • represents the binding site to Het).

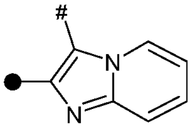
Q1-1

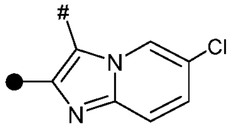
Q1-2

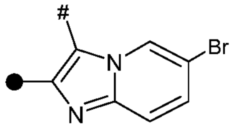
Q1-3

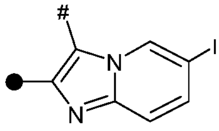
Q1-4

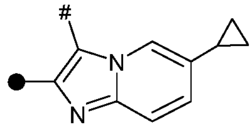
Q1-5

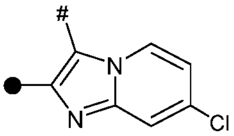
Q1-6

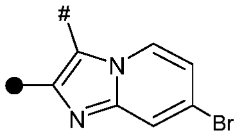
Q1-7

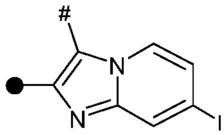
Q1-8

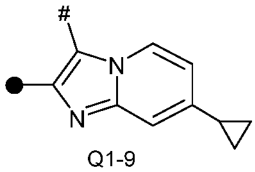
Q1-9

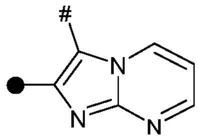
Q1-10

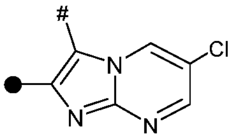
Q1-11

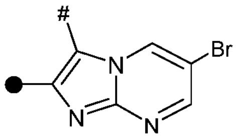
Q1-12

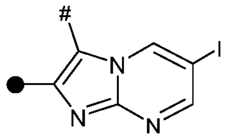
Q1-13

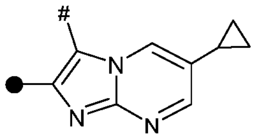
Q1-14

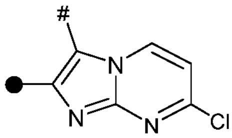
Q1-15

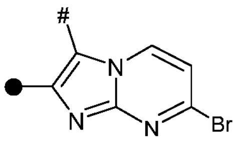
Q1-16

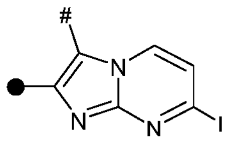
Q1-17

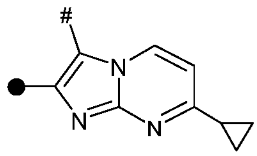
Q1-18

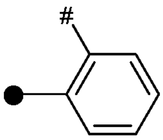
Q2-1

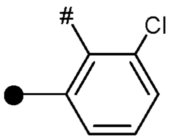
Q2-2

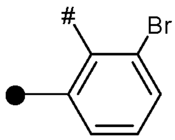
Q2-3

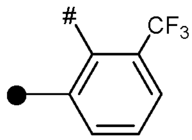
Q2-4

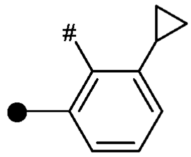
Q2-5

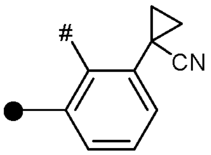
Q2-6

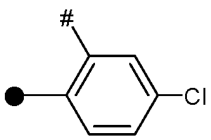
Q2-7

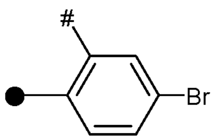
Q2-8

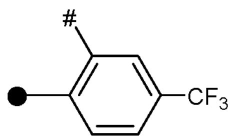
Q2-9

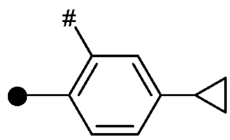
Q2-10

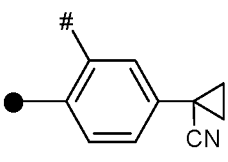
Q2-11

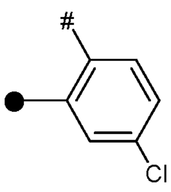
Q2-12

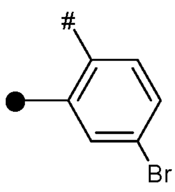
Q2-13

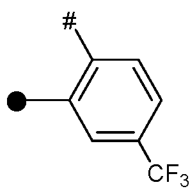
Q2-14

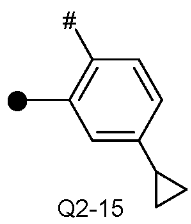
Q2-15

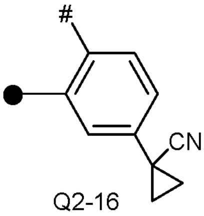
Q2-16
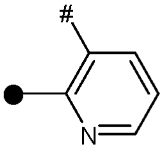
Q2-17
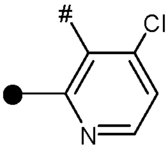
Q2-18
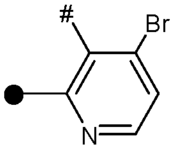
Q2-19
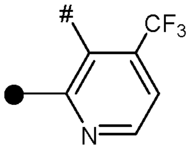
Q2-20

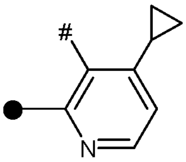
Q2-21
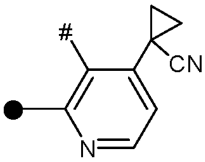
Q2-22
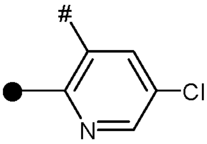
Q2-23
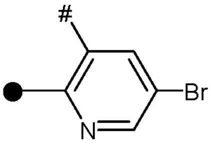
Q2-24
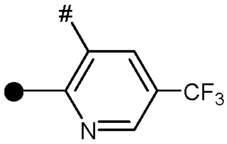
Q2-25

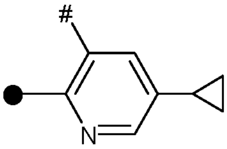
Q2-26
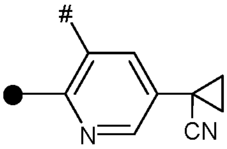
Q2-27
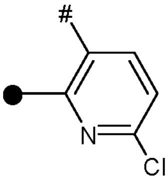
Q2-28
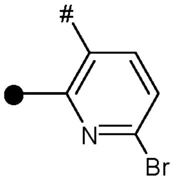
Q2-29
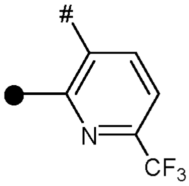
Q2-30

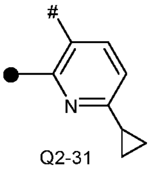
Q2-31
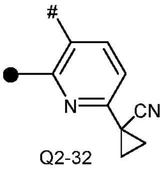
Q2-32
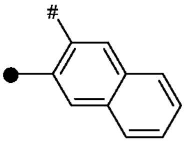
Q2-33
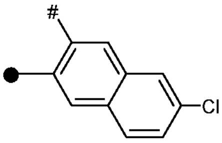
Q2-34
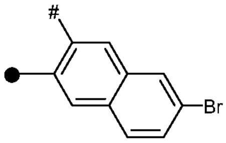
Q2-35

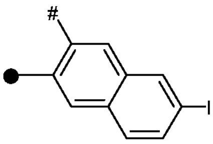
Q2-36
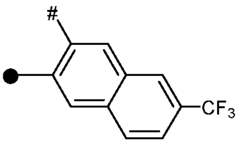
Q2-37
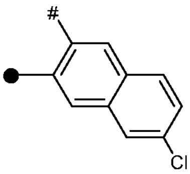
Q2-38
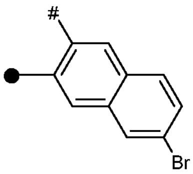
Q2-39
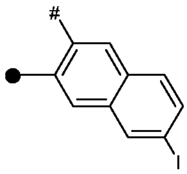
Q2-40

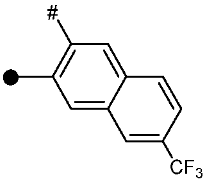
Q2-41
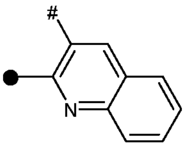
Q2-42
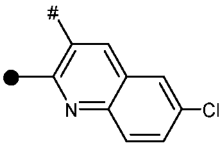
Q2-43
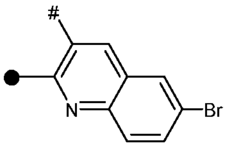
Q2-44
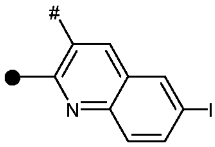
Q2-45

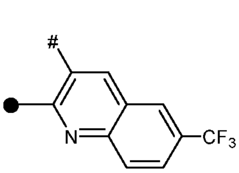
Q2-46
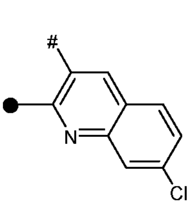
Q2-47
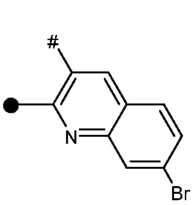
Q2-48
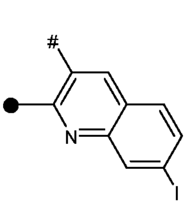
Q2-49
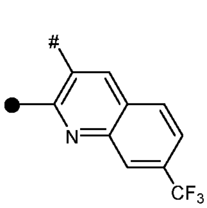
Q2-50

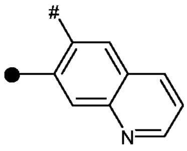
Q2-51

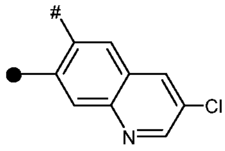
Q2-52

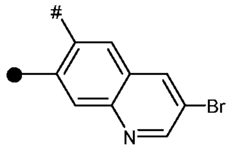
Q2-53

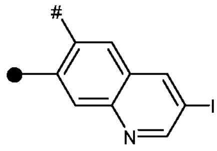
Q2-54

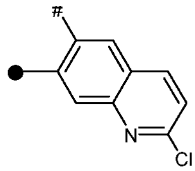
Q2-55

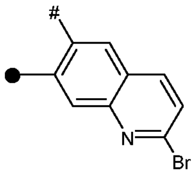
Q2-56

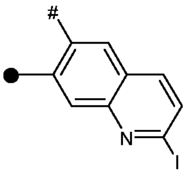
Q2-57

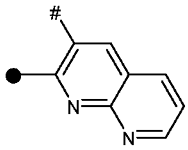
Q2-58

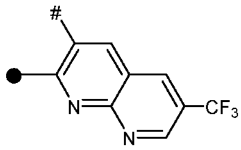
Q2-59

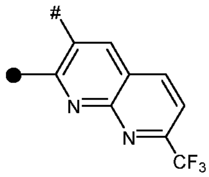
Q2-60

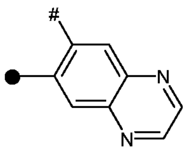
Q2-61

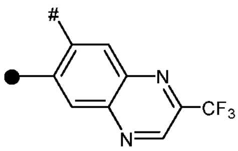
Q2-62

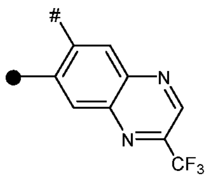
Q2-63

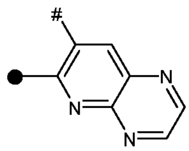
Q2-64

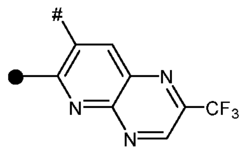
Q2-65

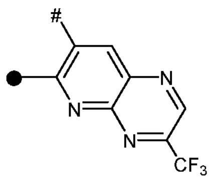
Q2-66

A compound represented by formula (L-10)

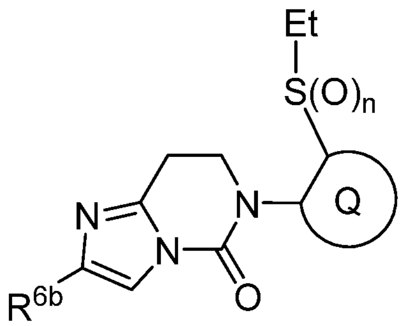
( L-10 )

(hereinafter referred to as "Compound (L-10)"), wherein n represents 0, Q represents a group represented by Q1-1, and $R^{6b}$ represents any one substituent described in Table 1A to Table 3A (hereinafter referred to as "Compound group SX709").

The Compound (L-10), wherein n represents 1, Q represents a group represented by Q1-1, and $R^{6b}$ represents any one substituent described in Table 1A to Table 3A (hereinafter referred to as "Compound group SX710").

The Compound (L-10), wherein n represents 2, Q represents a group represented by Q1-1, and $R^{6b}$ represents any one substituent described in Table 1A to Table 3A (hereinafter referred to as "Compound group SX711").

The Compound (L-10), wherein n represents 0, Q represents a group represented by Q1-2, and $R^{6b}$ represents any one substituent described in Table 1A to Table 3A (hereinafter referred to as "Compound group SX712").

The Compound (L-10), wherein n represents 1, Q represents a group represented by Q1-2, and $R^{6b}$ represents any one substituent described in Table 1A to Table 3A (hereinafter referred to as "Compound group SX713").

The Compound (L-10), wherein n represents 2, Q represents a group represented by Q1-2, and $R^{6b}$ represents any one substituent described in Table 1A to Table 3A (hereinafter referred to as "Compound group SX714").

The Compound (L-10), wherein n represents 0, Q represents a group represented by Q1-3, and $R^{6b}$ represents

any one substituent described in Table 1A to Table 3A (hereinafter referred to as "Compound group SX715").

The Compound (L-10), wherein n represents 1, Q represents a group represented by Q1-3, and $R^{6b}$ represents any one substituent described in Table 1A to Table 3A (hereinafter referred to as "Compound group SX716").

The Compound (L-10), wherein n represents 2, Q represents a group represented by Q1-3, and $R^{6b}$ represents any one substituent described in Table 1A to Table 3A (hereinafter referred to as "Compound group SX717").

The Compound (L-10), wherein n represents 0, Q represents a group represented by Q1-4, and $R^{6b}$ represents any one substituent described in Table 1A to Table 3A (hereinafter referred to as "Compound group SX718").

The Compound (L-10), wherein n represents 1, Q represents a group represented by Q1-4, and $R^{6b}$ represents any one substituent described in Table 1A to Table 3A (hereinafter referred to as "Compound group SX719").

The Compound (L-10), wherein n represents 2, Q represents a group represented by Q1-4, and $R^{6b}$ represents any one substituent described in Table 1A to Table 3A (hereinafter referred to as "Compound group SX720").

The Compound (L-10), wherein n represents 0, Q represents a group represented by Q1-5, and $R^{6b}$ represents any one substituent described in Table 1A to Table 3A (hereinafter referred to as "Compound group SX721").

The Compound (L-10), wherein n represents 1, Q represents a group represented by Q1-5, and $R^{6b}$ represents any one substituent described in Table 1A to Table 3A (hereinafter referred to as "Compound group SX722").

The Compound (L-10), wherein n represents 2, Q represents a group represented by Q1-5, and $R^{6b}$ represents any one substituent described in Table 1A to Table 3A (hereinafter referred to as "Compound group SX723").

The Compound (L-10), wherein n represents 0, Q represents a group represented by Q1-6, and $R^{6b}$ represents any one substituent described in Table 1A to Table 3A (hereinafter referred to as "Compound group SX724").

The Compound (L-10), wherein n represents 1, Q represents a group represented by Q1-6, and $R^{6b}$ represents any one substituent described in Table 1A to Table 3A (hereinafter referred to as "Compound group SX725").

The Compound (L-10), wherein n represents 2, Q represents a group represented by Q1-6, and $R^{6b}$ represents any one substituent described in Table 1A to Table 3A (hereinafter referred to as "Compound group SX726").

The Compound (L-10), wherein n represents 0, Q represents a group represented by Q1-7, and $R^{6b}$ represents any one substituent described in Table 1A to Table 3A (hereinafter referred to as "Compound group SX727").

The Compound (L-10), wherein n represents 1, Q represents a group represented by Q1-7, and $R^{6b}$ represents any one substituent described in Table 1A to Table 3A (hereinafter referred to as "Compound group SX728").

The Compound (L-10), wherein n represents 2, Q represents a group represented by Q1-7, and $R^{6b}$ represents any one substituent described in Table 1A to Table 3A (hereinafter referred to as "Compound group SX729").

The Compound (L-10), wherein n represents 0, Q represents a group represented by Q1-8, and $R^{6b}$ represents any one substituent described in Table 1A to Table 3A (hereinafter referred to as "Compound group SX730").

The Compound (L-10), wherein n represents 1, Q represents a group represented by Q1-8, and $R^{6b}$ represents any one substituent described in Table 1A to Table 3A (hereinafter referred to as "Compound group SX731").

The Compound (L-10), wherein n represents 2, Q represents a group represented by Q1-8, and $R^{6b}$ represents any one substituent described in Table 1A to Table 3A (hereinafter referred to as "Compound group SX732").

The Compound (L-10), wherein n represents 0, Q represents a group represented by Q1-9, and $R^{6b}$ represents any one substituent described in Table 1A to Table 3A (hereinafter referred to as "Compound group SX733").

The Compound (L-10), wherein n represents 1, Q represents a group represented by Q1-9, and $R^{6b}$ represents any one substituent described in Table 1A to Table 3A (hereinafter referred to as "Compound group SX734").

The Compound (L-10), wherein n represents 2, Q represents a group represented by Q1-9, and $R^{6b}$ represents any one substituent described in Table 1A to Table 3A (hereinafter referred to as "Compound group SX735").

The Compound (L-10), wherein n represents 0, Q represents a group represented by Q1-10, and $R^{6b}$ represents any one substituent described in Table 1A to Table 3A (hereinafter referred to as "Compound group SX736").

The Compound (L-10), wherein n represents 1, Q represents a group represented by Q1-10, and $R^{6b}$ represents any one substituent described in Table 1A to Table 3A (hereinafter referred to as "Compound group SX737").

The Compound (L-10), wherein n represents 2, Q represents a group represented by Q1-10, and $R^{6b}$ represents any one substituent described in Table 1A to Table 3A (hereinafter referred to as "Compound group SX738").

The Compound (L-10), wherein n represents 0, Q represents a group represented by Q1-11, and $R^{6b}$ represents any one substituent described in Table 1A to Table 3A (hereinafter referred to as "Compound group SX739").

The Compound (L-10), wherein n represents 1, Q represents a group represented by Q1-11, and $R^{6b}$ represents any one substituent described in Table 1A to Table 3A (hereinafter referred to as "Compound group SX740").

The Compound (L-10), wherein n represents 2, Q represents a group represented by Q1-11, and $R^{6b}$ represents any one substituent described in Table 1A to Table 3A (hereinafter referred to as "Compound group SX741").

The Compound (L-10), wherein n represents 0, Q represents a group represented by Q1-12, and $R^{6b}$ represents any one substituent described in Table 1A to Table 3A (hereinafter referred to as "Compound group SX742").

The Compound (L-10), wherein n represents 1, Q represents a group represented by Q1-12, and $R^{6b}$ represents any one substituent described in Table 1A to Table 3A (hereinafter referred to as "Compound group SX743").

The Compound (L-10), wherein n represents 2, Q represents a group represented by Q1-12, and $R^{6b}$ represents

any one substituent described in Table 1A to Table 3A (hereinafter referred to as "Compound group SX744").

The Compound (L-10), wherein n represents 0, Q represents a group represented by Q1-13, and $R^{6b}$ represents any one substituent described in Table 1A to Table 3A (hereinafter referred to as "Compound group SX745").

The Compound (L-10), wherein n represents 1, Q represents a group represented by Q1-13, and $R^{6b}$ represents any one substituent described in Table 1A to Table 3A (hereinafter referred to as "Compound group SX746").

The Compound (L-10), wherein n represents 2, Q represents a group represented by Q1-13, and $R^{6b}$ represents any one substituent described in Table 1A to Table 3A (hereinafter referred to as "Compound group SX747").

The Compound (L-10), wherein n represents 0, Q represents a group represented by Q1-14, and $R^{6b}$ represents any one substituent described in Table 1A to Table 3A (hereinafter referred to as "Compound group SX748").

The Compound (L-10), wherein n represents 1, Q represents a group represented by Q1-14, and $R^{6b}$ represents any one substituent described in Table 1A to Table 3A (hereinafter referred to as "Compound group SX749").

The Compound (L-10), wherein n represents 2, Q represents a group represented by Q1-14, and $R^{6b}$ represents any one substituent described in Table 1A to Table 3A (hereinafter referred to as "Compound group SX750").

The Compound (L-10), wherein n represents 0, Q represents a group represented by Q1-15, and $R^{6b}$ represents any one substituent described in Table 1A to Table 3A (hereinafter referred to as "Compound group SX751").

The Compound (L-10), wherein n represents 1, Q represents a group represented by Q1-15, and $R^{6b}$ represents any one substituent described in Table 1A to Table 3A (hereinafter referred to as "Compound group SX752").

The Compound (L-10), wherein n represents 2, Q represents a group represented by Q1-15, and $R^{6b}$ represents any one substituent described in Table 1A to Table 3A (hereinafter referred to as "Compound group SX753").

The Compound (L-10), wherein n represents 0, Q represents a group represented by Q1-16, and $R^{6b}$ represents any one substituent described in Table 1A to Table 3A (hereinafter referred to as "Compound group SX754").

The Compound (L-10), wherein n represents 1, Q represents a group represented by Q1-16, and $R^{6b}$ represents any one substituent described in Table 1A to Table 3A (hereinafter referred to as "Compound group SX755").

The Compound (L-10), wherein n represents 2, Q represents a group represented by Q1-16, and $R^{6b}$ represents any one substituent described in Table 1A to Table 3A (hereinafter referred to as "Compound group SX756").

The Compound (L-10), wherein n represents 0, Q represents a group represented by Q1-17, and $R^{6b}$ represents any one substituent described in Table 1A to Table 3A (hereinafter referred to as "Compound group SX757").

The Compound (L-10), wherein n represents 1, Q represents a group represented by Q1-17, and $R^{6b}$ represents any one substituent described in Table 1A to Table 3A (hereinafter referred to as "Compound group SX758").

The Compound (L-10), wherein n represents 2, Q represents a group represented by Q1-17, and $R^{6b}$ represents any one substituent described in Table 1A to Table 3A (hereinafter referred to as "Compound group SX759").

The Compound (L-10), wherein n represents 0, Q represents a group represented by Q1-18, and $R^{6b}$ represents any one substituent described in Table 1A to Table 3A (hereinafter referred to as "Compound group SX760").

The Compound (L-10), wherein n represents 1, Q represents a group represented by Q1-18, and $R^{6b}$ represents any one substituent described in Table 1A to Table 3A (hereinafter referred to as "Compound group SX761").

The Compound (L-10), wherein n represents 2, Q represents a group represented by Q1-18, and $R^{6b}$ represents any one substituent described in Table 1A to Table 3A (hereinafter referred to as "Compound group SX762").

The Compound (L-10), wherein n represents 0, Q represents a group represented by Q2-1, and $R^{6b}$ represents any one substituent described in Table 1A to Table 3A (hereinafter referred to as "Compound group SX763").

The Compound (L-10), wherein n represents 1, Q represents a group represented by Q2-1, and $R^{6b}$ represents any one substituent described in Table 1A to Table 3A (hereinafter referred to as "Compound group SX764").

The Compound (L-10), wherein n represents 2, Q represents a group represented by Q2-1, and $R^{6b}$ represents any one substituent described in Table 1A to Table 3A (hereinafter referred to as "Compound group SX765").

The Compound (L-10), wherein n represents 0, Q represents a group represented by Q2-2, and $R^{6b}$ represents any one substituent described in Table 1A to Table 3A (hereinafter referred to as "Compound group SX766").

The Compound (L-10), wherein n represents 1, Q represents a group represented by Q2-2, and $R^{6b}$ represents any one substituent described in Table 1A to Table 3A (hereinafter referred to as "Compound group SX767").

The Compound (L-10), wherein n represents 2, Q represents a group represented by Q2-2, and $R^{6b}$ represents any one substituent described in Table 1A to Table 3A (hereinafter referred to as "Compound group SX768").

The Compound (L-10), wherein n represents 0, Q represents a group represented by Q2-3, and $R^{6b}$ represents any one substituent described in Table 1A to Table 3A (hereinafter referred to as "Compound group SX769").

The Compound (L-10), wherein n represents 1, Q represents a group represented by Q2-3, and $R^{6b}$ represents any one substituent described in Table 1A to Table 3A (hereinafter referred to as "Compound group SX770").

The Compound (L-10), wherein n represents 2, Q represents a group represented by Q2-3, and $R^{6b}$ represents any one substituent described in Table 1A to Table 3A (hereinafter referred to as "Compound group SX771").

The Compound (L-10), wherein n represents 0, Q represents a group represented by Q2-4, and $R^{6b}$ represents any one substituent described in Table 1A to Table 3A (hereinafter referred to as "Compound group SX772").

The Compound (L-10), wherein n represents 1, Q represents a group represented by Q2-4, and $R^{6b}$ represents

any one substituent described in Table 1A to Table 3A (hereinafter referred to as "Compound group SX773").

The Compound (L-10), wherein n represents 2, Q represents a group represented by Q2-4, and $R^{6b}$ represents any one substituent described in Table 1A to Table 3A (hereinafter referred to as "Compound group SX774").

The Compound (L-10), wherein n represents 0, Q represents a group represented by Q2-5, and $R^{6b}$ represents any one substituent described in Table 1A to Table 3A (hereinafter referred to as "Compound group SX775").

The Compound (L-10), wherein n represents 1, Q represents a group represented by Q2-5, and $R^{6b}$ represents any one substituent described in Table 1A to Table 3A (hereinafter referred to as "Compound group SX776").

The Compound (L-10), wherein n represents 2, Q represents a group represented by Q2-5, and $R^{6b}$ represents any one substituent described in Table 1A to Table 3A (hereinafter referred to as "Compound group SX777").

The Compound (L-10), wherein n represents 0, Q represents a group represented by Q2-6, and $R^{6b}$ represents any one substituent described in Table 1A to Table 3A (hereinafter referred to as "Compound group SX778").

The Compound (L-10), wherein n represents 1, Q represents a group represented by Q2-6, and $R^{6b}$ represents any one substituent described in Table 1A to Table 3A (hereinafter referred to as "Compound group SX779").

The Compound (L-10), wherein n represents 2, Q represents a group represented by Q2-6, and $R^{6b}$ represents any one substituent described in Table 1A to Table 3A (hereinafter referred to as "Compound group SX780").

The Compound (L-10), wherein n represents 0, Q represents a group represented by Q2-7, and $R^{6b}$ represents any one substituent described in Table 1A to Table 3A (hereinafter referred to as "Compound group SX781").

The Compound (L-10), wherein n represents 1, Q represents a group represented by Q2-7, and $R^{6b}$ represents any one substituent described in Table 1A to Table 3A (hereinafter referred to as "Compound group SX782").

The Compound (L-10), wherein n represents 2, Q represents a group represented by Q2-7, and $R^{6b}$ represents any one substituent described in Table 1A to Table 3A (hereinafter referred to as "Compound group SX783").

The Compound (L-10), wherein n represents 0, Q represents a group represented by Q2-8, and $R^{6b}$ represents any one substituent described in Table 1A to Table 3A (hereinafter referred to as "Compound group SX784").

The Compound (L-10), wherein n represents 1, Q represents a group represented by Q2-8, and $R^{6b}$ represents any one substituent described in Table 1A to Table 3A (hereinafter referred to as "Compound group SX785").

The Compound (L-10), wherein n represents 2, Q represents a group represented by Q2-8, and $R^{6b}$ represents any one substituent described in Table 1A to Table 3A (hereinafter referred to as "Compound group SX786").

The Compound (L-10), wherein n represents 0, Q represents a group represented by Q2-9, and $R^{6b}$ represents any one substituent described in Table 1A to Table 3A (hereinafter referred to as "Compound group SX787").

The Compound (L-10), wherein n represents 1, Q represents a group represented by Q2-9, and $R^{6b}$ represents any one substituent described in Table 1A to Table 3A (hereinafter referred to as "Compound group SX788").

The Compound (L-10), wherein n represents 2, Q represents a group represented by Q2-9, and $R^{6b}$ represents any one substituent described in Table 1A to Table 3A (hereinafter referred to as "Compound group SX789").

The Compound (L-10), wherein n represents 0, Q represents a group represented by Q2-10, and $R^{6b}$ represents any one substituent described in Table 1A to Table 3A (hereinafter referred to as "Compound group SX790").

The Compound (L-10), wherein n represents 1, Q represents a group represented by Q2-10, and $R^{6b}$ represents any one substituent described in Table 1A to Table 3A (hereinafter referred to as "Compound group SX791").

The Compound (L-10), wherein n represents 2, Q represents a group represented by Q2-10, and $R^{6b}$ represents any one substituent described in Table 1A to Table 3A (hereinafter referred to as "Compound group SX792").

The Compound (L-10), wherein n represents 0, Q represents a group represented by Q2-11, and $R^{6b}$ represents any one substituent described in Table 1A to Table 3A (hereinafter referred to as "Compound group SX793").

The Compound (L-10), wherein n represents 1, Q represents a group represented by Q2-11, and $R^{6b}$ represents any one substituent described in Table 1A to Table 3A (hereinafter referred to as "Compound group SX794").

The Compound (L-10), wherein n represents 2, Q represents a group represented by Q2-11, and $R^{6b}$ represents any one substituent described in Table 1A to Table 3A (hereinafter referred to as "Compound group SX795").

The Compound (L-10), wherein n represents 0, Q represents a group represented by Q2-12, and $R^{6b}$ represents any one substituent described in Table 1A to Table 3A (hereinafter referred to as "Compound group SX796").

The Compound (L-10), wherein n represents 1, Q represents a group represented by Q2-12, and $R^{6b}$ represents any one substituent described in Table 1A to Table 3A (hereinafter referred to as "Compound group SX797").

The Compound (L-10), wherein n represents 2, Q represents a group represented by Q2-12, and $R^{6b}$ represents any one substituent described in Table 1A to Table 3A (hereinafter referred to as "Compound group SX798").

The Compound (L-10), wherein n represents 0, Q represents a group represented by Q2-13, and $R^{6b}$ represents any one substituent described in Table 1A to Table 3A (hereinafter referred to as "Compound group SX799").

The Compound (L-10), wherein n represents 1, Q represents a group represented by Q2-13, and $R^{6b}$ represents any one substituent described in Table 1A to Table 3A (hereinafter referred to as "Compound group SX800").

The Compound (L-10), wherein n represents 2, Q represents a group represented by Q2-13, and $R^{6b}$ represents any one substituent described in Table 1A to Table 3A (hereinafter referred to as "Compound group SX801").

The Compound (L-10), wherein n represents 0, Q represents a group represented by Q2-14, and $R^{6b}$ represents

any one substituent described in Table 1A to Table 3A (hereinafter referred to as "Compound group SX802").

The Compound (L-10), wherein n represents 1, Q represents a group represented by Q2-14, and $R^{6b}$ represents any one substituent described in Table 1A to Table 3A (hereinafter referred to as "Compound group SX803").

The Compound (L-10), wherein n represents 2, Q represents a group represented by Q2-14, and $R^{6b}$ represents any one substituent described in Table 1A to Table 3A (hereinafter referred to as "Compound group SX804").

The Compound (L-10), wherein n represents 0, Q represents a group represented by Q2-15, and $R^{6b}$ represents any one substituent described in Table 1A to Table 3A (hereinafter referred to as "Compound group SX805").

The Compound (L-10), wherein n represents 1, Q represents a group represented by Q2-15, and $R^{6b}$ represents any one substituent described in Table 1A to Table 3A (hereinafter referred to as "Compound group SX806").

The Compound (L-10), wherein n represents 2, Q represents a group represented by Q2-15, and $R^{6b}$ represents any one substituent described in Table 1A to Table 3A (hereinafter referred to as "Compound group SX807").

The Compound (L-10), wherein n represents 0, Q represents a group represented by Q2-16, and $R^{6b}$ represents any one substituent described in Table 1A to Table 3A (hereinafter referred to as "Compound group SX808").

The Compound (L-10), wherein n represents 1, Q represents a group represented by Q2-16, and $R^{6b}$ represents any one substituent described in Table 1A to Table 3A (hereinafter referred to as "Compound group SX809").

The Compound (L-10), wherein n represents 2, Q represents a group represented by Q2-16, and $R^{6b}$ represents any one substituent described in Table 1A to Table 3A (hereinafter referred to as "Compound group SX810").

The Compound (L-10), wherein n represents 0, Q represents a group represented by Q2-17, and $R^{6b}$ represents any one substituent described in Table 1A to Table 3A (hereinafter referred to as "Compound group SX811").

The Compound (L-10), wherein n represents 1, Q represents a group represented by Q2-17, and $R^{6b}$ represents any one substituent described in Table 1A to Table 3A (hereinafter referred to as "Compound group SX812").

The Compound (L-10), wherein n represents 2, Q represents a group represented by Q2-17, and $R^{6b}$ represents any one substituent described in Table 1A to Table 3A (hereinafter referred to as "Compound group SX813").

The Compound (L-10), wherein n represents 0, Q represents a group represented by Q2-18, and $R^{6b}$ represents any one substituent described in Table 1A to Table 3A (hereinafter referred to as "Compound group SX814").

The Compound (L-10), wherein n represents 1, Q represents a group represented by Q2-18, and $R^{6b}$ represents any one substituent described in Table 1A to Table 3A (hereinafter referred to as "Compound group SX815").

The Compound (L-10), wherein n represents 2, Q represents a group represented by Q2-18, and $R^{6b}$ represents any one substituent described in Table 1A to Table 3A (hereinafter referred to as "Compound group SX816").

The Compound (L-10), wherein n represents 0, Q represents a group represented by Q2-19, and $R^{6b}$ represents any one substituent described in Table 1A to Table 3A (hereinafter referred to as "Compound group SX817").

The Compound (L-10), wherein n represents 1, Q represents a group represented by Q2-19, and $R^{6b}$ represents any one substituent described in Table 1A to Table 3A (hereinafter referred to as "Compound group SX818").

The Compound (L-10), wherein n represents 2, Q represents a group represented by Q2-19, and $R^{6b}$ represents any one substituent described in Table 1A to Table 3A (hereinafter referred to as "Compound group SX819").

The Compound (L-10), wherein n represents 0, Q represents a group represented by Q2-20, and $R^{6b}$ represents any one substituent described in Table 1A to Table 3A (hereinafter referred to as "Compound group SX820").

The Compound (L-10), wherein n represents 1, Q represents a group represented by Q2-20, and $R^{6b}$ represents any one substituent described in Table 1A to Table 3A (hereinafter referred to as "Compound group SX821").

The Compound (L-10), wherein n represents 2, Q represents a group represented by Q2-20, and $R^{6b}$ represents any one substituent described in Table 1A to Table 3A (hereinafter referred to as "Compound group SX822").

The Compound (L-10), wherein n represents 0, Q represents a group represented by Q2-21, and $R^{6b}$ represents any one substituent described in Table 1A to Table 3A (hereinafter referred to as "Compound group SX823").

The Compound (L-10), wherein n represents 1, Q represents a group represented by Q2-21, and $R^{6b}$ represents any one substituent described in Table 1A to Table 3A (hereinafter referred to as "Compound group SX824").

The Compound (L-10), wherein n represents 2, Q represents a group represented by Q2-21, and $R^{6b}$ represents any one substituent described in Table 1A to Table 3A (hereinafter referred to as "Compound group SX825").

The Compound (L-10), wherein n represents 0, Q represents a group represented by Q2-22, and $R^{6b}$ represents any one substituent described in Table 1A to Table 3A (hereinafter referred to as "Compound group SX826").

The Compound (L-10), wherein n represents 1, Q represents a group represented by Q2-22, and $R^{6b}$ represents any one substituent described in Table 1A to Table 3A (hereinafter referred to as "Compound group SX827").

The Compound (L-10), wherein n represents 2, Q represents a group represented by Q2-22, and $R^{6b}$ represents any one substituent described in Table 1A to Table 3A (hereinafter referred to as "Compound group SX828").

The Compound (L-10), wherein n represents 0, Q represents a group represented by Q2-23, and $R^{6b}$ represents any one substituent described in Table 1A to Table 3A (hereinafter referred to as "Compound group SX829").

The Compound (L-10), wherein n represents 1, Q represents a group represented by Q2-23, and $R^{6b}$ represents any one substituent described in Table 1A to Table 3A (hereinafter referred to as "Compound group SX830").

The Compound (L-10), wherein n represents 2, Q represents a group represented by Q2-23, and $R^{6b}$ represents

any one substituent described in Table 1A to Table 3A (hereinafter referred to as "Compound group SX831").

The Compound (L-10), wherein n represents 0, Q represents a group represented by Q2-24, and $R^{6b}$ represents any one substituent described in Table 1A to Table 3A (hereinafter referred to as "Compound group SX832").

The Compound (L-10), wherein n represents 1, Q represents a group represented by Q2-24, and $R^{6b}$ represents any one substituent described in Table 1A to Table 3A (hereinafter referred to as "Compound group SX833").

The Compound (L-10), wherein n represents 2, Q represents a group represented by Q2-24, and $R^{6b}$ represents any one substituent described in Table 1A to Table 3A (hereinafter referred to as "Compound group SX834").

The Compound (L-10), wherein n represents 0, Q represents a group represented by Q2-25, and $R^{6b}$ represents any one substituent described in Table 1A to Table 3A (hereinafter referred to as "Compound group SX835").

The Compound (L-10), wherein n represents 1, Q represents a group represented by Q2-25, and $R^{6b}$ represents any one substituent described in Table 1A to Table 3A (hereinafter referred to as "Compound group SX836").

The Compound (L-10), wherein n represents 2, Q represents a group represented by Q2-25, and $R^{6b}$ represents any one substituent described in Table 1A to Table 3A (hereinafter referred to as "Compound group SX837").

The Compound (L-10), wherein n represents 0, Q represents a group represented by Q2-26, and $R^{6b}$ represents any one substituent described in Table 1A to Table 3A (hereinafter referred to as "Compound group SX838").

The Compound (L-10), wherein n represents 1, Q represents a group represented by Q2-26, and $R^{6b}$ represents any one substituent described in Table 1A to Table 3A (hereinafter referred to as "Compound group SX839").

The Compound (L-10), wherein n represents 2, Q represents a group represented by Q2-26, and $R^{6b}$ represents any one substituent described in Table 1A to Table 3A (hereinafter referred to as "Compound group SX840").

The Compound (L-10), wherein n represents 0, Q represents a group represented by Q2-27, and $R^{6b}$ represents any one substituent described in Table 1A to Table 3A (hereinafter referred to as "Compound group SX841").

The Compound (L-10), wherein n represents 1, Q represents a group represented by Q2-27, and $R^{6b}$ represents any one substituent described in Table 1A to Table 3A (hereinafter referred to as "Compound group SX842").

The Compound (L-10), wherein n represents 2, Q represents a group represented by Q2-27, and $R^{6b}$ represents any one substituent described in Table 1A to Table 3A (hereinafter referred to as "Compound group SX843").

The Compound (L-10), wherein n represents 0, Q represents a group represented by Q2-28, and $R^{6b}$ represents any one substituent described in Table 1A to Table 3A (hereinafter referred to as "Compound group SX844").

The Compound (L-10), wherein n represents 1, Q represents a group represented by Q2-28, and $R^{6b}$ represents any one substituent described in Table 1A to Table 3A (hereinafter referred to as "Compound group SX845").

The Compound (L-10), wherein n represents 2, Q represents a group represented by Q2-28, and $R^{6b}$ represents any one substituent described in Table 1A to Table 3A (hereinafter referred to as "Compound group SX846").

The Compound (L-10), wherein n represents 0, Q represents a group represented by Q2-29, and $R^{6b}$ represents any one substituent described in Table 1A to Table 3A (hereinafter referred to as "Compound group SX847").

The Compound (L-10), wherein n represents 1, Q represents a group represented by Q2-29, and $R^{6b}$ represents any one substituent described in Table 1A to Table 3A (hereinafter referred to as "Compound group SX848").

The Compound (L-10), wherein n represents 2, Q represents a group represented by Q2-29, and $R^{6b}$ represents any one substituent described in Table 1A to Table 3A (hereinafter referred to as "Compound group SX849").

The Compound (L-10), wherein n represents 0, Q represents a group represented by Q2-30, and $R^{6b}$ represents any one substituent described in Table 1A to Table 3A (hereinafter referred to as "Compound group SX850").

The Compound (L-10), wherein n represents 1, Q represents a group represented by Q2-30, and $R^{6b}$ represents any one substituent described in Table 1A to Table 3A (hereinafter referred to as "Compound group SX851").

The Compound (L-10), wherein n represents 2, Q represents a group represented by Q2-30, and $R^{6b}$ represents any one substituent described in Table 1A to Table 3A (hereinafter referred to as "Compound group SX852").

The Compound (L-10), wherein n represents 0, Q represents a group represented by Q2-31, and $R^{6b}$ represents any one substituent described in Table 1A to Table 3A (hereinafter referred to as "Compound group SX853").

The Compound (L-10), wherein n represents 1, Q represents a group represented by Q2-31, and $R^{6b}$ represents any one substituent described in Table 1A to Table 3A (hereinafter referred to as "Compound group SX854").

The Compound (L-10), wherein n represents 2, Q represents a group represented by Q2-31, and $R^{6b}$ represents any one substituent described in Table 1A to Table 3A (hereinafter referred to as "Compound group SX855").

The Compound (L-10), wherein n represents 0, Q represents a group represented by Q2-32, and $R^{6b}$ represents any one substituent described in Table 1A to Table 3A (hereinafter referred to as "Compound group SX856").

The Compound (L-10), wherein n represents 1, Q represents a group represented by Q2-32, and $R^{6b}$ represents any one substituent described in Table 1A to Table 3A (hereinafter referred to as "Compound group SX857").

The Compound (L-10), wherein n represents 2, Q represents a group represented by Q2-32, and $R^{6b}$ represents any one substituent described in Table 1A to Table 3A (hereinafter referred to as "Compound group SX858").

The Compound (L-10), wherein n represents 0, Q represents a group represented by Q2-33, and $R^{6b}$ represents any one substituent described in Table 1A to Table 3A (hereinafter referred to as "Compound group SX859").

The Compound (L-10), wherein n represents 1, Q represents a group represented by Q2-33, and $R^{6b}$ represents

any one substituent described in Table 1A to Table 3A (hereinafter referred to as "Compound group SX860").

The Compound (L-10), wherein n represents 2, Q represents a group represented by Q2-33, and $R^{6b}$ represents any one substituent described in Table 1A to Table 3A (hereinafter referred to as "Compound group SX861").

The Compound (L-10), wherein n represents 0, Q represents a group represented by Q2-34, and $R^{6b}$ represents any one substituent described in Table 1A to Table 3A (hereinafter referred to as "Compound group SX862").

The Compound (L-10), wherein n represents 1, Q represents a group represented by Q2-34, and $R^{6b}$ represents any one substituent described in Table 1A to Table 3A (hereinafter referred to as "Compound group SX863").

The Compound (L-10), wherein n represents 2, Q represents a group represented by Q2-34, and $R^{6b}$ represents any one substituent described in Table 1A to Table 3A (hereinafter referred to as "Compound group SX864").

The Compound (L-10), wherein n represents 0, Q represents a group represented by Q2-35, and $R^{6b}$ represents any one substituent described in Table 1A to Table 3A (hereinafter referred to as "Compound group SX865").

The Compound (L-10), wherein n represents 1, Q represents a group represented by Q2-35, and $R^{6b}$ represents any one substituent described in Table 1A to Table 3A (hereinafter referred to as "Compound group SX866").

The Compound (L-10), wherein n represents 2, Q represents a group represented by Q2-35, and $R^{6b}$ represents any one substituent described in Table 1A to Table 3A (hereinafter referred to as "Compound group SX867").

The Compound (L-10), wherein n represents 0, Q represents a group represented by Q2-36, and $R^{6b}$ represents any one substituent described in Table 1A to Table 3A (hereinafter referred to as "Compound group SX868").

The Compound (L-10), wherein n represents 1, Q represents a group represented by Q2-36, and $R^{6b}$ represents any one substituent described in Table 1A to Table 3A (hereinafter referred to as "Compound group SX869").

The Compound (L-10), wherein n represents 2, Q represents a group represented by Q2-36, and $R^{6b}$ represents any one substituent described in Table 1A to Table 3A (hereinafter referred to as "Compound group SX870").

The Compound (L-10), wherein n represents 0, Q represents a group represented by Q2-37, and $R^{6b}$ represents any one substituent described in Table 1A to Table 3A (hereinafter referred to as "Compound group SX871").

The Compound (L-10), wherein n represents 1, Q represents a group represented by Q2-37, and $R^{6b}$ represents any one substituent described in Table 1A to Table 3A (hereinafter referred to as "Compound group SX872").

The Compound (L-10), wherein n represents 2, Q represents a group represented by Q2-37, and $R^{6b}$ represents any one substituent described in Table 1A to Table 3A (hereinafter referred to as "Compound group SX873").

The Compound (L-10), wherein n represents 0, Q represents a group represented by Q2-38, and $R^{6b}$ represents any one substituent described in Table 1A to Table 3A (hereinafter referred to as "Compound group SX874").

The Compound (L-10), wherein n represents 1, Q represents a group represented by Q2-38, and $R^{6b}$ represents any one substituent described in Table 1A to Table 3A (hereinafter referred to as "Compound group SX875").

The Compound (L-10), wherein n represents 2, Q represents a group represented by Q2-38, and $R^{6b}$ represents any one substituent described in Table 1A to Table 3A (hereinafter referred to as "Compound group SX876").

The Compound (L-10), wherein n represents 0, Q represents a group represented by Q2-39, and $R^{6b}$ represents any one substituent described in Table 1A to Table 3A (hereinafter referred to as "Compound group SX877").

The Compound (L-10), wherein n represents 1, Q represents a group represented by Q2-39, and $R^{6b}$ represents any one substituent described in Table 1A to Table 3A (hereinafter referred to as "Compound group SX878").

The Compound (L-10), wherein n represents 2, Q represents a group represented by Q2-39, and $R^{6b}$ represents any one substituent described in Table 1A to Table 3A (hereinafter referred to as "Compound group SX879").

The Compound (L-10), wherein n represents 0, Q represents a group represented by Q2-40, and $R^{6b}$ represents any one substituent described in Table 1A to Table 3A (hereinafter referred to as "Compound group SX880").

The Compound (L-10), wherein n represents 1, Q represents a group represented by Q2-40, and $R^{6b}$ represents any one substituent described in Table 1A to Table 3A (hereinafter referred to as "Compound group SX881").

The Compound (L-10), wherein n represents 2, Q represents a group represented by Q2-40, and $R^{6b}$ represents any one substituent described in Table 1A to Table 3A (hereinafter referred to as "Compound group SX882").

The Compound (L-10), wherein n represents 0, Q represents a group represented by Q2-41, and $R^{6b}$ represents any one substituent described in Table 1A to Table 3A (hereinafter referred to as "Compound group SX883").

The Compound (L-10), wherein n represents 1, Q represents a group represented by Q2-41, and $R^{6b}$ represents any one substituent described in Table 1A to Table 3A (hereinafter referred to as "Compound group SX884").

The Compound (L-10), wherein n represents 2, Q represents a group represented by Q2-41, and $R^{6b}$ represents any one substituent described in Table 1A to Table 3A (hereinafter referred to as "Compound group SX885").

The Compound (L-10), wherein n represents 0, Q represents a group represented by Q2-42, and $R^{6b}$ represents any one substituent described in Table 1A to Table 3A (hereinafter referred to as "Compound group SX886").

The Compound (L-10), wherein n represents 1, Q represents a group represented by Q2-42, and $R^{6b}$ represents any one substituent described in Table 1A to Table 3A (hereinafter referred to as "Compound group SX887").

The Compound (L-10), wherein n represents 2, Q represents a group represented by Q2-42, and $R^{6b}$ represents any one substituent described in Table 1A to Table 3A (hereinafter referred to as "Compound group SX888").

The Compound (L-10), wherein n represents 0, Q represents a group represented by Q2-43, and $R^{6b}$ represents

any one substituent described in Table 1A to Table 3A (hereinafter referred to as "Compound group SX889").

The Compound (L-10), wherein n represents 1, Q represents a group represented by Q2-43, and $R^{6b}$ represents any one substituent described in Table 1A to Table 3A (hereinafter referred to as "Compound group SX890").

The Compound (L-10), wherein n represents 2, Q represents a group represented by Q2-43, and $R^{6b}$ represents any one substituent described in Table 1A to Table 3A (hereinafter referred to as "Compound group SX891").

The Compound (L-10), wherein n represents 0, Q represents a group represented by Q2-44, and $R^{6b}$ represents any one substituent described in Table 1A to Table 3A (hereinafter referred to as "Compound group SX892").

The Compound (L-10), wherein n represents 1, Q represents a group represented by Q2-44, and $R^{6b}$ represents any one substituent described in Table 1A to Table 3A (hereinafter referred to as "Compound group SX893").

The Compound (L-10), wherein n represents 2, Q represents a group represented by Q2-44, and $R^{6b}$ represents any one substituent described in Table 1A to Table 3A (hereinafter referred to as "Compound group SX894").

The Compound (L-10), wherein n represents 0, Q represents a group represented by Q2-45, and $R^{6b}$ represents any one substituent described in Table 1A to Table 3A (hereinafter referred to as "Compound group SX895").

The Compound (L-10), wherein n represents 1, Q represents a group represented by Q2-45, and $R^{6b}$ represents any one substituent described in Table 1A to Table 3A (hereinafter referred to as "Compound group SX896").

The Compound (L-10), wherein n represents 2, Q represents a group represented by Q2-45, and $R^{6b}$ represents any one substituent described in Table 1A to Table 3A (hereinafter referred to as "Compound group SX897").

The Compound (L-10), wherein n represents 0, Q represents a group represented by Q2-46, and $R^{6b}$ represents any one substituent described in Table 1A to Table 3A (hereinafter referred to as "Compound group SX898").

The Compound (L-10), wherein n represents 1, Q represents a group represented by Q2-46, and $R^{6b}$ represents any one substituent described in Table 1A to Table 3A (hereinafter referred to as "Compound group SX899").

The Compound (L-10), wherein n represents 2, Q represents a group represented by Q2-46, and $R^{6b}$ represents any one substituent described in Table 1A to Table 3A (hereinafter referred to as "Compound group SX900").

The Compound (L-10), wherein n represents 0, Q represents a group represented by Q2-47, and $R^{6b}$ represents any one substituent described in Table 1A to Table 3A (hereinafter referred to as "Compound group SX901").

The Compound (L-10), wherein n represents 1, Q represents a group represented by Q2-47, and $R^{6b}$ represents any one substituent described in Table 1A to Table 3A (hereinafter referred to as "Compound group SX902").

The Compound (L-10), wherein n represents 2, Q represents a group represented by Q2-47, and $R^{6b}$ represents any one substituent described in Table 1A to Table 3A (hereinafter referred to as "Compound group SX903").

The Compound (L-10), wherein n represents 0, Q represents a group represented by Q2-48, and $R^{6b}$ represents any one substituent described in Table 1A to Table 3A (hereinafter referred to as "Compound group SX904").

The Compound (L-10), wherein n represents 1, Q represents a group represented by Q2-48, and $R^{6b}$ represents any one substituent described in Table 1A to Table 3A (hereinafter referred to as "Compound group SX905").

The Compound (L-10), wherein n represents 2, Q represents a group represented by Q2-48, and $R^{6b}$ represents any one substituent described in Table 1A to Table 3A (hereinafter referred to as "Compound group SX906").

The Compound (L-10), wherein n represents 0, Q represents a group represented by Q2-49, and $R^{6b}$ represents any one substituent described in Table 1A to Table 3A (hereinafter referred to as "Compound group SX907").

The Compound (L-10), wherein n represents 1, Q represents a group represented by Q2-49, and $R^{6b}$ represents any one substituent described in Table 1A to Table 3A (hereinafter referred to as "Compound group SX908").

The Compound (L-10), wherein n represents 2, Q represents a group represented by Q2-49, and $R^{6b}$ represents any one substituent described in Table 1A to Table 3A (hereinafter referred to as "Compound group SX909").

The Compound (L-10), wherein n represents 0, Q represents a group represented by Q2-50, and $R^{6b}$ represents any one substituent described in Table 1A to Table 3A (hereinafter referred to as "Compound group SX910").

The Compound (L-10), wherein n represents 1, Q represents a group represented by Q2-50, and $R^{6b}$ represents any one substituent described in Table 1A to Table 3A (hereinafter referred to as "Compound group SX911").

The Compound (L-10), wherein n represents 2, Q represents a group represented by Q2-50, and $R^{6b}$ represents any one substituent described in Table 1A to Table 3A (hereinafter referred to as "Compound group SX912").

The Compound (L-10), wherein n represents 0, Q represents a group represented by Q2-51, and $R^{6b}$ represents any one substituent described in Table 1A to Table 3A (hereinafter referred to as "Compound group SX913").

The Compound (L-10), wherein n represents 1, Q represents a group represented by Q2-51, and $R^{6b}$ represents any one substituent described in Table 1A to Table 3A (hereinafter referred to as "Compound group SX914").

The Compound (L-10), wherein n represents 2, Q represents a group represented by Q2-51, and $R^{6b}$ represents any one substituent described in Table 1A to Table 3A (hereinafter referred to as "Compound group SX915").

The Compound (L-10), wherein n represents 0, Q represents a group represented by Q2-52, and $R^{6b}$ represents any one substituent described in Table 1A to Table 3A (hereinafter referred to as "Compound group SX916").

The Compound (L-10), wherein n represents 1, Q represents a group represented by Q2-52, and $R^{6b}$ represents any one substituent described in Table 1A to Table 3A (hereinafter referred to as "Compound group SX917").

The Compound (L-10), wherein n represents 2, Q represents a group represented by Q2-52, and $R^{6b}$ represents

any one substituent described in Table 1A to Table 3A (hereinafter referred to as "Compound group SX918").

The Compound (L-10), wherein n represents 0, Q represents a group represented by Q2-53, and $R^{6b}$ represents any one substituent described in Table 1A to Table 3A (hereinafter referred to as "Compound group SX919").

The Compound (L-10), wherein n represents 1, Q represents a group represented by Q2-53, and $R^{6b}$ represents any one substituent described in Table 1A to Table 3A (hereinafter referred to as "Compound group SX920").

The Compound (L-10), wherein n represents 2, Q represents a group represented by Q2-53, and $R^{6b}$ represents any one substituent described in Table 1A to Table 3A (hereinafter referred to as "Compound group SX921").

The Compound (L-10), wherein n represents 0, Q represents a group represented by Q2-54, and $R^{6b}$ represents any one substituent described in Table 1A to Table 3A (hereinafter referred to as "Compound group SX922").

The Compound (L-10), wherein n represents 1, Q represents a group represented by Q2-54, and $R^{6b}$ represents any one substituent described in Table 1A to Table 3A (hereinafter referred to as "Compound group SX923").

The Compound (L-10), wherein n represents 2, Q represents a group represented by Q2-54, and $R^{6b}$ represents any one substituent described in Table 1A to Table 3A (hereinafter referred to as "Compound group SX924").

The Compound (L-10), wherein n represents 0, Q represents a group represented by Q2-55, and $R^{6b}$ represents any one substituent described in Table 1A to Table 3A (hereinafter referred to as "Compound group SX925").

The Compound (L-10), wherein n represents 1, Q represents a group represented by Q2-55, and $R^{6b}$ represents any one substituent described in Table 1A to Table 3A (hereinafter referred to as "Compound group SX926").

The Compound (L-10), wherein n represents 2, Q represents a group represented by Q2-55, and $R^{6b}$ represents any one substituent described in Table 1A to Table 3A (hereinafter referred to as "Compound group SX927").

The Compound (L-10), wherein n represents 0, Q represents a group represented by Q2-56, and $R^{6b}$ represents any one substituent described in Table 1A to Table 3A (hereinafter referred to as "Compound group SX928").

The Compound (L-10), wherein n represents 1, Q represents a group represented by Q2-56, and $R^{6b}$ represents any one substituent described in Table 1A to Table 3A (hereinafter referred to as "Compound group SX929").

The Compound (L-10), wherein n represents 2, Q represents a group represented by Q2-56, and $R^{6b}$ represents any one substituent described in Table 1A to Table 3A (hereinafter referred to as "Compound group SX930").

The Compound (L-10), wherein n represents 0, Q represents a group represented by Q2-57, and $R^{6b}$ represents any one substituent described in Table 1A to Table 3A (hereinafter referred to as "Compound group SX931").

The Compound (L-10), wherein n represents 1, Q represents a group represented by Q2-57, and $R^{6b}$ represents any one substituent described in Table 1A to Table 3A (hereinafter referred to as "Compound group SX932").

The Compound (L-10), wherein n represents 2, Q represents a group represented by Q2-57, and $R^{6b}$ represents any one substituent described in Table 1A to Table 3A (hereinafter referred to as "Compound group SX933").

The Compound (L-10), wherein n represents 0, Q represents a group represented by Q2-58, and $R^{6b}$ represents any one substituent described in Table 1A to Table 3A (hereinafter referred to as "Compound group SX934").

The Compound (L-10), wherein n represents 1, Q represents a group represented by Q2-58, and $R^{6b}$ represents any one substituent described in Table 1A to Table 3A (hereinafter referred to as "Compound group SX935").

The Compound (L-10), wherein n represents 2, Q represents a group represented by Q2-58, and $R^{6b}$ represents any one substituent described in Table 1A to Table 3A (hereinafter referred to as "Compound group SX936").

The Compound (L-10), wherein n represents 0, Q represents a group represented by Q2-59, and $R^{6b}$ represents any one substituent described in Table 1A to Table 3A (hereinafter referred to as "Compound group SX937").

The Compound (L-10), wherein n represents 1, Q represents a group represented by Q2-59, and $R^{6b}$ represents any one substituent described in Table 1A to Table 3A (hereinafter referred to as "Compound group SX938").

The Compound (L-10), wherein n represents 2, Q represents a group represented by Q2-59, and $R^{6b}$ represents any one substituent described in Table 1A to Table 3A (hereinafter referred to as "Compound group SX939").

The Compound (L-10), wherein n represents 0, Q represents a group represented by Q2-60, and $R^{6b}$ represents any one substituent described in Table 1A to Table 3A (hereinafter referred to as "Compound group SX940").

The Compound (L-10), wherein n represents 1, Q represents a group represented by Q2-60, and $R^{6b}$ represents any one substituent described in Table 1A to Table 3A (hereinafter referred to as "Compound group SX941").

The Compound (L-10), wherein n represents 2, Q represents a group represented by Q2-60, and $R^{6b}$ represents any one substituent described in Table 1A to Table 3A (hereinafter referred to as "Compound group SX942").

The Compound (L-10), wherein n represents 0, Q represents a group represented by Q2-61, and $R^{6b}$ represents any one substituent described in Table 1A to Table 3A (hereinafter referred to as "Compound group SX943").

The Compound (L-10), wherein n represents 1, Q represents a group represented by Q2-61, and $R^{6b}$ represents any one substituent described in Table 1A to Table 3A (hereinafter referred to as "Compound group SX944").

The Compound (L-10), wherein n represents 2, Q represents a group represented by Q2-61, and $R^{6b}$ represents any one substituent described in Table 1A to Table 3A (hereinafter referred to as "Compound group SX945").

The Compound (L-10), wherein n represents 0, Q represents a group represented by Q2-62, and $R^{6b}$ represents any one substituent described in Table 1A to Table 3A (hereinafter referred to as "Compound group SX946").

The Compound (L-10), wherein n represents 1, Q represents a group represented by Q2-62, and $R^{6b}$ represents

any one substituent described in Table 1A to Table 3A (hereinafter referred to as "Compound group SX947").

The Compound (L-10), wherein n represents 2, Q represents a group represented by Q2-62, and $R^{6b}$ represents any one substituent described in Table 1A to Table 3A (hereinafter referred to as "Compound group SX948").

The Compound (L-10), wherein n represents 0, Q represents a group represented by Q2-63, and $R^{6b}$ represents any one substituent described in Table 1A to Table 3A (hereinafter referred to as "Compound group SX949").

The Compound (L-10), wherein n represents 1, Q represents a group represented by Q2-63, and $R^{6b}$ represents any one substituent described in Table 1A to Table 3A (hereinafter referred to as "Compound group SX950").

The Compound (L-10), wherein n represents 2, Q represents a group represented by Q2-63, and $R^{6b}$ represents any one substituent described in Table 1A to Table 3A (hereinafter referred to as "Compound group SX951").

The Compound (L-10), wherein n represents 0, Q represents a group represented by Q2-64, and $R^{6b}$ represents any one substituent described in Table 1A to Table 3A (hereinafter referred to as "Compound group SX952").

The Compound (L-10), wherein n represents 1, Q represents a group represented by Q2-64, and $R^{6b}$ represents any one substituent described in Table 1A to Table 3A (hereinafter referred to as "Compound group SX953").

The Compound (L-10), wherein n represents 2, Q represents a group represented by Q2-64, and $R^{6b}$ represents any one substituent described in Table 1A to Table 3A (hereinafter referred to as "Compound group SX954").

The Compound (L-10), wherein n represents 0, Q represents a group represented by Q2-65, and $R^{6b}$ represents any one substituent described in Table 1A to Table 3A (hereinafter referred to as "Compound group SX955").

The Compound (L-10), wherein n represents 1, Q represents a group represented by Q2-65, and $R^{6b}$ represents any one substituent described in Table 1A to Table 3A (hereinafter referred to as "Compound group SX956").

The Compound (L-10), wherein n represents 2, Q represents a group represented by Q2-65, and $R^{6b}$ represents any one substituent described in Table 1A to Table 3A (hereinafter referred to as "Compound group SX957").

The Compound (L-10), wherein n represents 0, Q represents a group represented by Q2-66, and $R^{6b}$ represents any one substituent described in Table 1A to Table 3A (hereinafter referred to as "Compound group SX958").

The Compound (L-10), wherein n represents 1, Q represents a group represented by Q2-66, and $R^{6b}$ represents any one substituent described in Table 1A to Table 3A (hereinafter referred to as "Compound group SX959").

The Compound (L-10), wherein n represents 2, Q represents a group represented by Q2-66, and $R^{6b}$ represents any one substituent described in Table 1A to Table 3A (hereinafter referred to as "Compound group SX960").

A compound represented by formula (L-11)

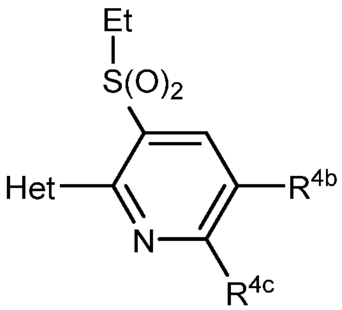

( L-11 )

(hereinafter referred to as "Compound (L-11)"), wherein Het represents a group represented by Het3, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX961").

Table 7A

| |
|---|
| F |
| Cl |
| Br |
| Me |
| Et |
| Pr |
| i-Pr |
| c-Pr |
| 1-CN-c-Pr |

(continued)

| |
|---|
| OMe |
| OEt |
| OPr |
| Oi-Pr |
| $CF_3$ |
| $CHF_2$ |
| $NH_2$ |
| $NHCH_2CF_3$ |
| CN |
| C(O)OEt |
| NHC(O)c-Pr |
| NMeC(O)c-Pr |
| CH=N-OH |
| CH=N-OMe |

Table 8A

| |
|---|
| Ph |
| 2-F-Ph |
| 3-F-Ph |
| 4-F-Ph |
| 2-Cl-Ph |
| 3-Cl-Ph |
| 4-Cl-Ph |
| 2-$CF_3$-Ph |
| 3-$CF_3$-Ph |
| 4-$CF_3$-Ph |
| 2-$CHF_2$-Ph |
| 3-$CHF_2$-Ph |
| 4-$CHF_2$-Ph |
| 2-$NMe_2$-Ph |
| 3-$NMe_2$-Ph |
| 4-$NMe_2$-Ph |
| 2-CN-Ph |
| 3-CN-Ph |
| 4-CN-Ph |
| 4-C(O)$NMe_2$-Ph |
| 4-NHC(O)Me-Ph |
| 3,4-$F_2$-Ph |
| 3,5-$F_2$-Ph |
| 2,4-$F_2$-Ph |

(continued)

| |
|---|
| 3,4,5-$F_3$-Ph |
| 3,4-$Cl_2$-Ph |
| 3,5-$Cl_2$-Ph |
| 3,5-$Cl_2$-4-F-Ph |
| OPh |
| O-2-F-Ph |

Table 9A

| |
|---|
| Py2 |
| 3-F-Py2 |
| 4-F-Py2 |
| 5-F-Py2 |
| 3-Cl-Py2 |
| 4-Cl-Py2 |
| 5-Cl-Py2 |
| 3-$CF_3$-Py2 |
| 4-$CF_3$-Py2 |
| 5-$CF_3$-Py2 |
| 6-$CF_3$-Py2 |
| 3-$CHF_2$-Py2 |
| 4-$CHF_2$-Py2 |
| 5-$CHF_2$-Py2 |
| 6-$CHF_2$-Py2 |
| 3-Me-Py2 |
| 4-Me-Py2 |
| 5-Me-Py2 |
| 6-Me-Py2 |
| 3-CN-Py2 |
| 4-CN-Py2 |
| 5-CN-Py2 |
| 6-CN-Py2 |
| S-$OCH_2CF_2CF_3$)- Py2 |
| 3,5-$F_2$-Py2 |
| Py3 |
| 6-$CF_3$-Py3 |
| 5-$CF_3$-Py3 |
| 6-F-Py3 |
| 6-Cl-Py3 |
| Py4 |
| OPy2 |

(continued)

| |
|---|
| OPy3 |

Table 10A

| |
|---|
| 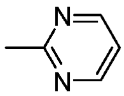 |
| 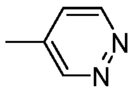 |
| 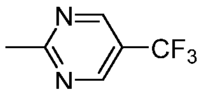 |
| 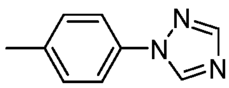 |
| 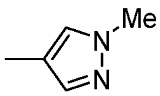 |
| 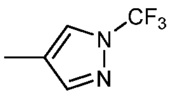 |
| 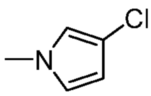 |
| 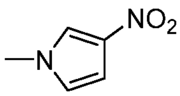 |

Table 11A

| |
|---|
| 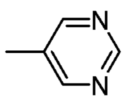 |
| 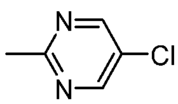 |
| 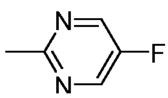 |

(continued)

| |
|---|
| 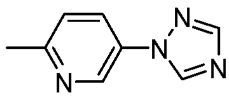 |
| 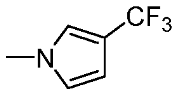 |
| 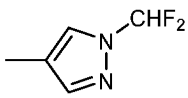 |
| 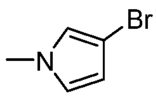 |
| 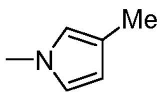 |

Table 12A

| |
|---|
| 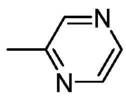 |
| 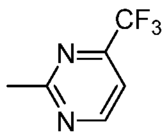 |
| 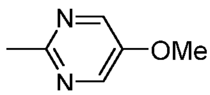 |
| 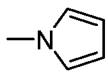 |
| 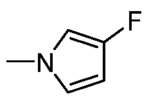 |
| 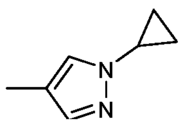 |
| 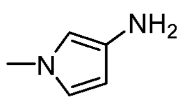 |

(continued)

| |
|---|
| 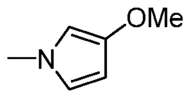 |

Table 13A

| |
|---|
| 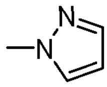 |
| 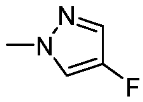 |
| 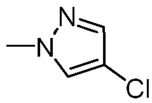 |
| 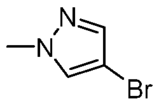 |
| 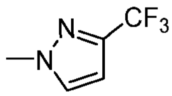 |
| 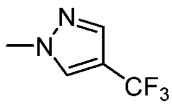 |
| 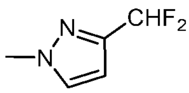 |
| 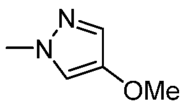 |
| 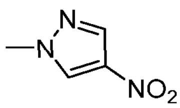 |
| 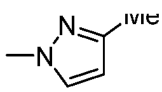 |

Table 14A

| |
|---|
| 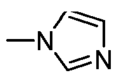 |

(continued)

| |
|---|
| 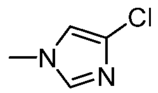 |
| 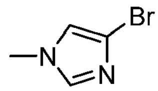 |
| 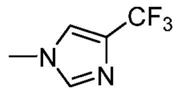 |
| 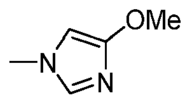 |
| 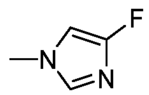 |
| 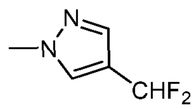 |
| 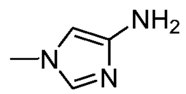 |
| 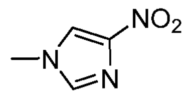 |
| 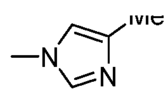 |

Table 15A

| |
|---|
| 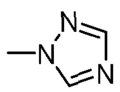 |
| 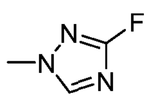 |
| 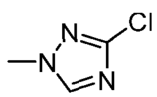 |
| 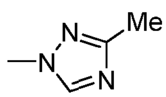 |

(continued)

| |
|---|
| 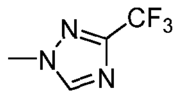 |
| 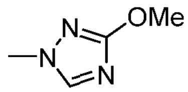 |
| 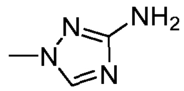 |
| 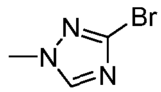 |
| 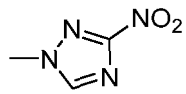 |

Het3 to Het57 are the following groups.

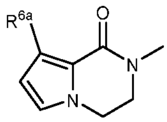
Het3

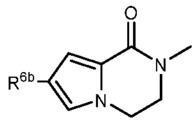
Het4

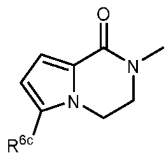
Het5

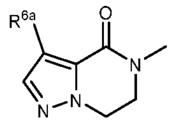
Het6

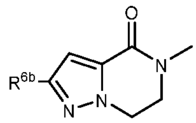
Het7

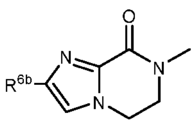
Het8

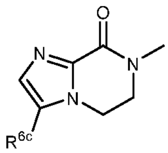
Het9

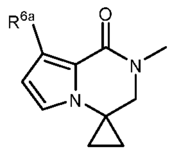
Het10

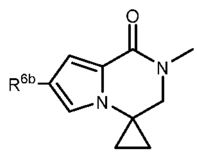
Het11

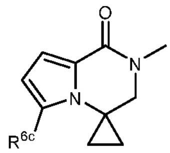
Het12

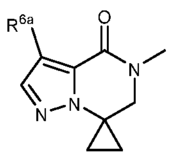
Het13

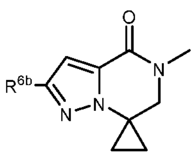
Het14

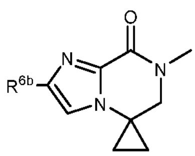
Het15

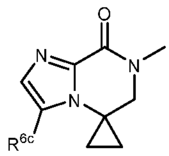
Het16

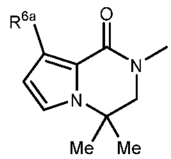
Het17

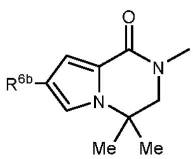
Het18

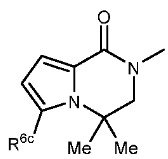
Het19

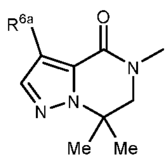
Het20

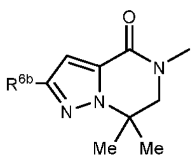
Het21

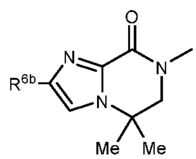
Het22

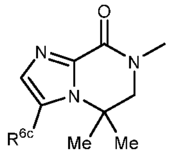
Het23

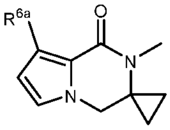
Het24

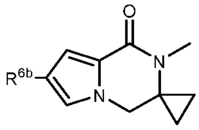
Het25

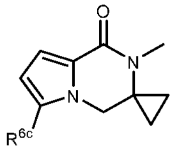
Het26

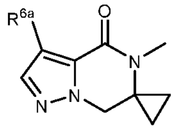
Het27

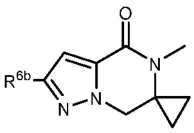
Het28

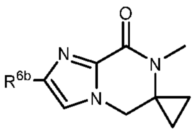
Het29

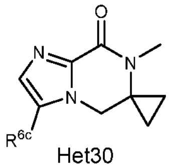
Het30

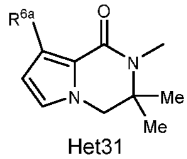
Het31

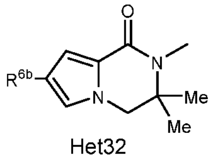
Het32

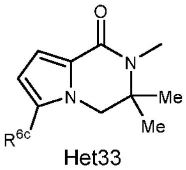
Het33

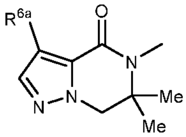
Het34

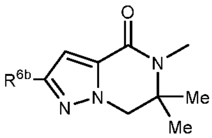
Het35

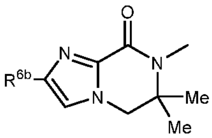
Het36

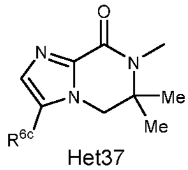
Het37

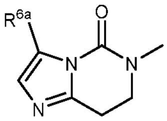
Het38

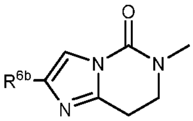
Het39

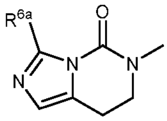
Het40

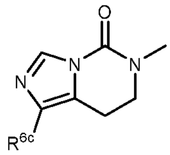
Het41

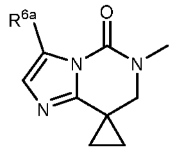
Het42

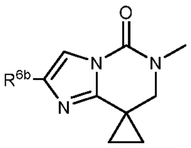
Het43

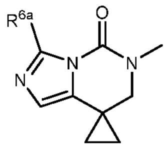
Het44

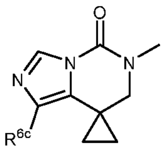
Het45

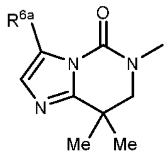
Het46

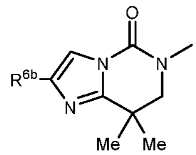
Het47

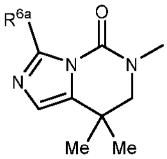
Het48

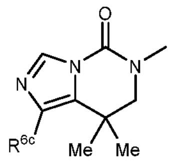
Het49

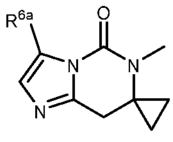
Het50

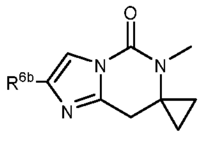
Het51

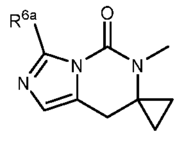
Het52

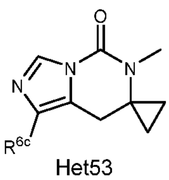
Het53

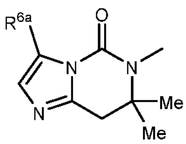
Het54

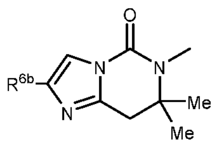
Het55

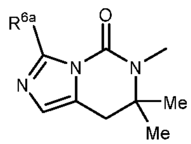
Het56

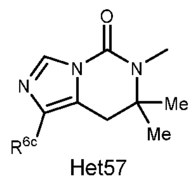
Het57

The Compound (L-11), wherein Het represents a group represented by Het3, $R^{6a}$ represents a difluoromethyl group, $R^{4b}$ represents any one substituent described in Table 7A to Table 15A, and $R^{4c}$ represents a hydrogen atom (hereinafter referred to as "Compound group SX962").

The Compound (L-11), wherein Het represents a group represented by Het3, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX963").

The Compound (L-11), wherein Het represents a group represented by Het3, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX964").

The Compound (L-11), wherein Het represents a group represented by Het4, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX965").

The Compound (L-11), wherein Het represents a group represented by Het4, $R^{6b}$ represents a difluoromethyl group, $R^{4b}$ represents any one substituent described in Table 7A to Table 15A, and $R^{4c}$ represents a hydrogen atom (hereinafter referred to as "Compound group SX966").

The Compound (L-11), wherein Het represents a group represented by Het4, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX967").

The Compound (L-11), wherein Het represents a group represented by Het4, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX968").

The Compound (L-11), wherein Het represents a group represented by Het5, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX969").

The Compound (L-11), wherein Het represents a group represented by Het5, $R^{6c}$ represents a difluoromethyl group, $R^{4b}$ represents any one substituent described in Table 7A to Table 15A, and $R^{4c}$ represents a hydrogen atom (hereinafter referred to as "Compound group SX970").

The Compound (L-11), wherein Het represents a group represented by Het5, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX971").

The Compound (L-11), wherein Het represents a group represented by Het5, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX972").

The Compound (L-11), wherein Het represents a group represented by Het6, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX973").

The Compound (L-11), wherein Het represents a group represented by Het6, $R^{6a}$ represents a difluoromethyl group, $R^{4b}$ represents any one substituent described in Table 7A to Table 15A, and $R^{4c}$ represents a hydrogen atom (hereinafter referred to as "Compound group SX974").

The Compound (L-11), wherein Het represents a group represented by Het6, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX975").

The Compound (L-11), wherein Het represents a group represented by Het6, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX976").

The Compound (L-11), wherein Het represents a group represented by Het7, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX977").

The Compound (L-11), wherein Het represents a group represented by Het7, $R^{6b}$ represents a difluoromethyl group, $R^{4b}$ represents any one substituent described in Table 7A to Table 15A, and $R^{4c}$ represents a hydrogen atom (hereinafter referred to as "Compound group SX978").

The Compound (L-11), wherein Het represents a group represented by Het7, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX979").

The Compound (L-11), wherein Het represents a group represented by Het7, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX980").

The Compound (L-11), wherein Het represents a group represented by Het8, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX981").

The Compound (L-11), wherein Het represents a group represented by Het8, $R^{6b}$ represents a difluoromethyl group, $R^{4b}$ represents any one substituent described in Table 7A to Table 15A, and $R^{4c}$ represents a hydrogen atom (hereinafter referred to as "Compound group SX982").

The Compound (L-11), wherein Het represents a group represented by Het8, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX983").

The Compound (L-11), wherein Het represents a group represented by Het8, $R^{6b}$ represents a difluoromethoxy

group, $R^{4b}$ represents any one substituent described in Table 7A to Table 15A, and $R^{4c}$ represents a hydrogen atom (hereinafter referred to as "Compound group SX984").

The Compound (L-11), wherein Het represents a group represented by Het9, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX985").

The Compound (L-11), wherein Het represents a group represented by Het9, $R^{6c}$ represents a difluoromethyl group, $R^{4b}$ represents any one substituent described in Table 7A to Table 15A, and $R^{4c}$ represents a hydrogen atom (hereinafter referred to as "Compound group SX986").

The Compound (L-11), wherein Het represents a group represented by Het9, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX987").

The Compound (L-11), wherein Het represents a group represented by Het9, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX988").

The Compound (L-11), wherein Het represents a group represented by Het10, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX989").

The Compound (L-11), wherein Het represents a group represented by Het10, $R^{6a}$ represents a difluoromethyl group, $R^{4b}$ represents any one substituent described in Table 7A to Table 15A, and $R^{4c}$ represents a hydrogen atom (hereinafter referred to as "Compound group SX990").

The Compound (L-11), wherein Het represents a group represented by Het10, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX991").

The Compound (L-11), wherein Het represents a group represented by Het10, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX992").

The Compound (L-11), wherein Het represents a group represented by Het11, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX993").

The Compound (L-11), wherein Het represents a group represented by Het11, $R^{6b}$ represents a difluoromethyl group, $R^{4b}$ represents any one substituent described in Table 7A to Table 15A, and $R^{4c}$ represents a hydrogen atom (hereinafter referred to as "Compound group SX994").

The Compound (L-11), wherein Het represents a group represented by Het11, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX995").

The Compound (L-11), wherein Het represents a group represented by Het11, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX996").

The Compound (L-11), wherein Het represents a group represented by Het12, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX997").

The Compound (L-11), wherein Het represents a group represented by Het12, $R^{6c}$ represents a difluoromethyl group, $R^{4b}$ represents any one substituent described in Table 7A to Table 15A, and $R^{4c}$ represents a hydrogen atom (hereinafter referred to as "Compound group SX998").

The Compound (L-11), wherein Het represents a group represented by Het12, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX999").

The Compound (L-11), wherein Het represents a group represented by Het12, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX1000").

The Compound (L-11), wherein Het represents a group represented by Het13, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1001").

The Compound (L-11), wherein Het represents a group represented by Het13, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1002").

The Compound (L-11), wherein Het represents a group represented by Het13, $R^{6a}$ represents a trifluoromethoxy group, $R^{4b}$ represents any one substituent described in Table 7A to Table 15A, and $R^{4c}$ represents a hydrogen atom

(hereinafter referred to as "Compound group SX1003").

The Compound (L-11), wherein Het represents a group represented by Het13, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1004").

The Compound (L-11), wherein Het represents a group represented by Het14, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1005").

The Compound (L-11), wherein Het represents a group represented by Het14, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1006").

The Compound (L-11), wherein Het represents a group represented by Het14, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1007").

The Compound (L-11), wherein Het represents a group represented by Het14, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1008").

The Compound (L-11), wherein Het represents a group represented by Het15, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1009").

The Compound (L-11), wherein Het represents a group represented by Het15, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1010").

The Compound (L-11), wherein Het represents a group represented by Het15, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1011").

The Compound (L-11), wherein Het represents a group represented by Het15, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1012").

The Compound (L-11), wherein Het represents a group represented by Het16, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX1013").

The Compound (L-11), wherein Het represents a group represented by Het16, $R^{6c}$ represents a difluoromethyl group, $R^{4b}$ represents any one substituent described in Table 7A to Table 15A, and $R^{4c}$ represents a hydrogen atom (hereinafter referred to as "Compound group SX1014").

The Compound (L-11), wherein Het represents a group represented by Het16, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX1015").

The Compound (L-11), wherein Het represents a group represented by Het16, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX1016").

The Compound (L-11), wherein Het represents a group represented by Het17, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1017").

The Compound (L-11), wherein Het represents a group represented by Het17, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1018").

The Compound (L-11), wherein Het represents a group represented by Het17, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1019").

The Compound (L-11), wherein Het represents a group represented by Het17, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1020").

The Compound (L-11), wherein Het represents a group represented by Het18, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1021").

The Compound (L-11), wherein Het represents a group represented by Het18, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1022").

The Compound (L-11), wherein Het represents a group represented by Het18, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1023").

The Compound (L-11), wherein Het represents a group represented by Het18, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1024").

The Compound (L-11), wherein Het represents a group represented by Het19, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX1025").

The Compound (L-11), wherein Het represents a group represented by Het19, $R^{6c}$ represents a difluoromethyl group, $R^{4b}$ represents any one substituent described in Table 7A to Table 15A, and $R^{4c}$ represents a hydrogen atom (hereinafter referred to as "Compound group SX1026").

The Compound (L-11), wherein Het represents a group represented by Het19, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX1027").

The Compound (L-11), wherein Het represents a group represented by Het19, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX1028").

The Compound (L-11), wherein Het represents a group represented by Het20, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1029").

The Compound (L-11), wherein Het represents a group represented by Het20, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1030").

The Compound (L-11), wherein Het represents a group represented by Het20, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1031").

The Compound (L-11), wherein Het represents a group represented by Het20, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1032").

The Compound (L-11), wherein Het represents a group represented by Het21, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1033").

The Compound (L-11), wherein Het represents a group represented by Het21, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1034").

The Compound (L-11), wherein Het represents a group represented by Het21, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1035").

The Compound (L-11), wherein Het represents a group represented by Het21, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1036").

The Compound (L-11), wherein Het represents a group represented by Het22, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1037").

The Compound (L-11), wherein Het represents a group represented by Het22, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1038").

The Compound (L-11), wherein Het represents a group represented by Het22, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1039").

The Compound (L-11), wherein Het represents a group represented by Het22, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1040").

The Compound (L-11), wherein Het represents a group represented by Het23, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX1041").

The Compound (L-11), wherein Het represents a group represented by Het23, $R^{6c}$ represents a difluoromethyl

group, $R^{4b}$ represents any one substituent described in Table 7A to Table 15A, and $R^{4c}$ represents a hydrogen atom (hereinafter referred to as "Compound group SX1042").

The Compound (L-11), wherein Het represents a group represented by Het23, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX1043").

The Compound (L-11), wherein Het represents a group represented by Het23, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX1044").

The Compound (L-11), wherein Het represents a group represented by Het24, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1045").

The Compound (L-11), wherein Het represents a group represented by Het24, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1046").

The Compound (L-11), wherein Het represents a group represented by Het24, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1047").

The Compound (L-11), wherein Het represents a group represented by Het24, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1048").

The Compound (L-11), wherein Het represents a group represented by Het25, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1049").

The Compound (L-11), wherein Het represents a group represented by Het25, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1050").

The Compound (L-11), wherein Het represents a group represented by Het25, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1051").

The Compound (L-11), wherein Het represents a group represented by Het25, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1052").

The Compound (L-11), wherein Het represents a group represented by Het26, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX1053").

The Compound (L-11), wherein Het represents a group represented by Het26, $R^{6c}$ represents a difluoromethyl group, $R^{4b}$ represents any one substituent described in Table 7A to Table 15A, and $R^{4c}$ represents a hydrogen atom (hereinafter referred to as "Compound group SX1054").

The Compound (L-11), wherein Het represents a group represented by Het26, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX1055").

The Compound (L-11), wherein Het represents a group represented by Het26, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX1056").

The Compound (L-11), wherein Het represents a group represented by Het27, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1057").

The Compound (L-11), wherein Het represents a group represented by Het27, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1058").

The Compound (L-11), wherein Het represents a group represented by Het27, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1059").

The Compound (L-11), wherein Het represents a group represented by Het27, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1060").

The Compound (L-11), wherein Het represents a group represented by Het28, $R^{6b}$ represents a trifluoromethyl group, $R^{4b}$ represents any one substituent described in Table 7A to Table 15A, and $R^{4c}$ represents a hydrogen atom

(hereinafter referred to as "Compound group SX1061").

The Compound (L-11), wherein Het represents a group represented by Het28, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1062").

The Compound (L-11), wherein Het represents a group represented by Het28, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1063").

The Compound (L-11), wherein Het represents a group represented by Het28, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1064").

The Compound (L-11), wherein Het represents a group represented by Het29, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1065").

The Compound (L-11), wherein Het represents a group represented by Het29, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1066").

The Compound (L-11), wherein Het represents a group represented by Het29, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1067").

The Compound (L-11), wherein Het represents a group represented by Het29, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1068").

The Compound (L-11), wherein Het represents a group represented by Het30, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX1069").

The Compound (L-11), wherein Het represents a group represented by Het30, $R^{6c}$ represents a difluoromethyl group, $R^{4b}$ represents any one substituent described in Table 7A to Table 15A, and $R^{4c}$ represents a hydrogen atom (hereinafter referred to as "Compound group SX1070").

The Compound (L-11), wherein Het represents a group represented by Het30, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX1071").

The Compound (L-11), wherein Het represents a group represented by Het30, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX1072").

The Compound (L-11), wherein Het represents a group represented by Het31, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1073").

The Compound (L-11), wherein Het represents a group represented by Het31, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1074").

The Compound (L-11), wherein Het represents a group represented by Het31, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1075").

The Compound (L-11), wherein Het represents a group represented by Het31, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1076").

The Compound (L-11), wherein Het represents a group represented by Het32, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1077").

The Compound (L-11), wherein Het represents a group represented by Het32, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1078").

The Compound (L-11), wherein Het represents a group represented by Het32, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1079").

The Compound (L-11), wherein Het represents a group represented by Het32, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1080").

The Compound (L-11), wherein Het represents a group represented by Het33, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX1081").

The Compound (L-11), wherein Het represents a group represented by Het33, $R^{6c}$ represents a difluoromethyl group, $R^{4b}$ represents any one substituent described in Table 7A to Table 15A, and $R^{4c}$ represents a hydrogen atom (hereinafter referred to as "Compound group SX1082").

The Compound (L-11), wherein Het represents a group represented by Het33, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX1083").

The Compound (L-11), wherein Het represents a group represented by Het33, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX1084").

The Compound (L-11), wherein Het represents a group represented by Het34, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1085").

The Compound (L-11), wherein Het represents a group represented by Het34, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1086").

The Compound (L-11), wherein Het represents a group represented by Het34, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1087").

The Compound (L-11), wherein Het represents a group represented by Het34, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1088").

The Compound (L-11), wherein Het represents a group represented by Het35, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1089").

The Compound (L-11), wherein Het represents a group represented by Het35, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1090").

The Compound (L-11), wherein Het represents a group represented by Het35, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1091").

The Compound (L-11), wherein Het represents a group represented by Het35, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1092").

The Compound (L-11), wherein Het represents a group represented by Het36, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1093").

The Compound (L-11), wherein Het represents a group represented by Het36, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1094").

The Compound (L-11), wherein Het represents a group represented by Het36, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1095").

The Compound (L-11), wherein Het represents a group represented by Het36, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1096").

The Compound (L-11), wherein Het represents a group represented by Het37, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX1097").

The Compound (L-11), wherein Het represents a group represented by Het37, $R^{6c}$ represents a difluoromethyl group, $R^{4b}$ represents any one substituent described in Table 7A to Table 15A, and $R^{4c}$ represents a hydrogen atom (hereinafter referred to as "Compound group SX1098").

The Compound (L-11), wherein Het represents a group represented by Het37, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX1099").

The Compound (L-11), wherein Het represents a group represented by Het37, $R^{6c}$ represents a difluoromethoxy

group, $R^{4b}$ represents any one substituent described in Table 7A to Table 15A, and $R^{4c}$ represents a hydrogen atom (hereinafter referred to as "Compound group SX1100").

The Compound (L-11), wherein Het represents a group represented by Het38, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1101").

The Compound (L-11), wherein Het represents a group represented by Het38, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1102").

The Compound (L-11), wherein Het represents a group represented by Het38, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1103").

The Compound (L-11), wherein Het represents a group represented by Het38, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1104").

The Compound (L-11), wherein Het represents a group represented by Het39, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1105").

The Compound (L-11), wherein Het represents a group represented by Het39, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1106").

The Compound (L-11), wherein Het represents a group represented by Het39, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1107").

The Compound (L-11), wherein Het represents a group represented by Het39, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1108").

The Compound (L-11), wherein Het represents a group represented by Het40, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1109").

The Compound (L-11), wherein Het represents a group represented by Het40, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1110").

The Compound (L-11), wherein Het represents a group represented by Het40, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1111").

The Compound (L-11), wherein Het represents a group represented by Het40, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1112").

The Compound (L-11), wherein Het represents a group represented by Het41, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX1113").

The Compound (L-11), wherein Het represents a group represented by Het41, $R^{6c}$ represents a difluoromethyl group, $R^{4b}$ represents any one substituent described in Table 7A to Table 15A, and $R^{4c}$ represents a hydrogen atom (hereinafter referred to as "Compound group SX1114").

The Compound (L-11), wherein Het represents a group represented by Het41, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX1115").

The Compound (L-11), wherein Het represents a group represented by Het41, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX1116").

The Compound (L-11), wherein Het represents a group represented by Het42, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1117").

The Compound (L-11), wherein Het represents a group represented by Het42, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1118").

The Compound (L-11), wherein Het represents a group represented by Het42, $R^{6a}$ represents a trifluoromethoxy group, $R^{4b}$ represents any one substituent described in Table 7A to Table 15A, and $R^{4c}$ represents a hydrogen atom

(hereinafter referred to as "Compound group SX1119").

The Compound (L-11), wherein Het represents a group represented by Het42, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1120").

The Compound (L-11), wherein Het represents a group represented by Het43, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1121").

The Compound (L-11), wherein Het represents a group represented by Het43, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1122").

The Compound (L-11), wherein Het represents a group represented by Het43, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1123").

The Compound (L-11), wherein Het represents a group represented by Het43, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1124").

The Compound (L-11), wherein Het represents a group represented by Het44, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1125").

The Compound (L-11), wherein Het represents a group represented by Het44, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1126").

The Compound (L-11), wherein Het represents a group represented by Het44, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1127").

The Compound (L-11), wherein Het represents a group represented by Het44, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1128").

The Compound (L-11), wherein Het represents a group represented by Het45, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX1129").

The Compound (L-11), wherein Het represents a group represented by Het45, $R^{6c}$ represents a difluoromethyl group, $R^{4b}$ represents any one substituent described in Table 7A to Table 15A, and $R^{4c}$ represents a hydrogen atom (hereinafter referred to as "Compound group SX1130").

The Compound (L-11), wherein Het represents a group represented by Het45, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX1131").

The Compound (L-11), wherein Het represents a group represented by Het45, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX1132").

The Compound (L-11), wherein Het represents a group represented by Het46, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1133").

The Compound (L-11), wherein Het represents a group represented by Het46, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1134").

The Compound (L-11), wherein Het represents a group represented by Het46, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1135").

The Compound (L-11), wherein Het represents a group represented by Het46, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1136").

The Compound (L-11), wherein Het represents a group represented by Het47, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1137").

The Compound (L-11), wherein Het represents a group represented by Het47, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1138").

The Compound (L-11), wherein Het represents a group represented by Het47, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1139").

The Compound (L-11), wherein Het represents a group represented by Het47, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1140").

The Compound (L-11), wherein Het represents a group represented by Het48, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1141").

The Compound (L-11), wherein Het represents a group represented by Het48, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1142").

The Compound (L-11), wherein Het represents a group represented by Het48, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1143").

The Compound (L-11), wherein Het represents a group represented by Het48, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1144").

The Compound (L-11), wherein Het represents a group represented by Het49, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX1145").

The Compound (L-11), wherein Het represents a group represented by Het49, $R^{6c}$ represents a difluoromethyl group, $R^{4b}$ represents any one substituent described in Table 7A to Table 15A, and $R^{4c}$ represents a hydrogen atom (hereinafter referred to as "Compound group SX1146").

The Compound (L-11), wherein Het represents a group represented by Het49, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX1147").

The Compound (L-11), wherein Het represents a group represented by Het49, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX1148").

The Compound (L-11), wherein Het represents a group represented by Het50, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1149").

The Compound (L-11), wherein Het represents a group represented by Het50, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1150").

The Compound (L-11), wherein Het represents a group represented by Het50, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1151").

The Compound (L-11), wherein Het represents a group represented by Het50, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1152").

The Compound (L-11), wherein Het represents a group represented by Het51, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1153").

The Compound (L-11), wherein Het represents a group represented by Het51, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1154").

The Compound (L-11), wherein Het represents a group represented by Het51, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1155").

The Compound (L-11), wherein Het represents a group represented by Het51, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1156").

The Compound (L-11), wherein Het represents a group represented by Het52, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1157").

The Compound (L-11), wherein Het represents a group represented by Het52, $R^{6a}$ represents a difluoromethyl

group, $R^{4b}$ represents any one substituent described in Table 7A to Table 15A, and $R^{4c}$ represents a hydrogen atom (hereinafter referred to as "Compound group SX1158").

The Compound (L-11), wherein Het represents a group represented by Het52, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1159").

The Compound (L-11), wherein Het represents a group represented by Het52, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1160").

The Compound (L-11), wherein Het represents a group represented by Het53, $R^{6c}$ represents a trifluoromethyl group, $R^{4b}$ represents any one substituent described in Table 7A to Table 15A, and $R^{4c}$ represents a hydrogen atom (hereinafter referred to as "Compound group SX1161").

The Compound (L-11), wherein Het represents a group represented by Het53, $R^{6c}$ represents a difluoromethyl group, $R^{4b}$ represents any one substituent described in Table 7A to Table 15A, and $R^{4c}$ represents a hydrogen atom (hereinafter referred to as "Compound group SX1162").

The Compound (L-11), wherein Het represents a group represented by Het53, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX1163").

The Compound (L-11), wherein Het represents a group represented by Het53, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX1164").

The Compound (L-11), wherein Het represents a group represented by Het54, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1165").

The Compound (L-11), wherein Het represents a group represented by Het54, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1166").

The Compound (L-11), wherein Het represents a group represented by Het54, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1167").

The Compound (L-11), wherein Het represents a group represented by Het54, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1168").

The Compound (L-11), wherein Het represents a group represented by Het55, $R^{6b}$ represents a trifluoromethyl group, $R^{4b}$ represents any one substituent described in Table 7A to Table 15A, and $R^{4c}$ represents a hydrogen atom (hereinafter referred to as "Compound group SX1169").

The Compound (L-11), wherein Het represents a group represented by Het55, $R^{6b}$ represents a difluoromethyl group, $R^{4b}$ represents any one substituent described in Table 7A to Table 15A, and $R^{4c}$ represents a hydrogen atom (hereinafter referred to as "Compound group SX1170").

The Compound (L-11), wherein Het represents a group represented by Het55, $R^{6b}$ represents a trifluoromethoxy group, $R^{4b}$ represents any one substituent described in Table 7A to Table 15A, and $R^{4c}$ represents a hydrogen atom (hereinafter referred to as "Compound group SX1171").

The Compound (L-11), wherein Het represents a group represented by Het55, $R^{6b}$ represents a difluoromethoxy group, $R^{4b}$ represents any one substituent described in Table 7A to Table 15A, and $R^{4c}$ represents a hydrogen atom (hereinafter referred to as "Compound group SX1172").

The Compound (L-11), wherein Het represents a group represented by Het56, $R^{6a}$ represents a trifluoromethyl group, $R^{4b}$ represents any one substituent described in Table 7A to Table 15A, and $R^{4c}$ represents a hydrogen atom (hereinafter referred to as "Compound group SX1173").

The Compound (L-11), wherein Het represents a group represented by Het56, $R^{6a}$ represents a difluoromethyl group, $R^{4b}$ represents any one substituent described in Table 7A to Table 15A, and $R^{4c}$ represents a hydrogen atom (hereinafter referred to as "Compound group SX1174").

The Compound (L-11), wherein Het represents a group represented by Het56, $R^{6a}$ represents a trifluoromethoxy group, $R^{4b}$ represents any one substituent described in Table 7A to Table 15A, and $R^{4c}$ represents a hydrogen atom (hereinafter referred to as "Compound group SX1175").

The Compound (L-11), wherein Het represents a group represented by Het56, $R^{6a}$ represents a difluoromethoxy group, $R^{4b}$ represents any one substituent described in Table 7A to Table 15A, and $R^{4c}$ represents a hydrogen atom (hereinafter referred to as "Compound group SX1176").

The Compound (L-11), wherein Het represents a group represented by Het57, $R^{6c}$ represents a trifluoromethyl group, $R^{4b}$ represents any one substituent described in Table 7A to Table 15A, and $R^{4c}$ represents a hydrogen atom

(hereinafter referred to as "Compound group SX1177").

The Compound (L-11), wherein Het represents a group represented by Het57, $R^{6c}$ represents a difluoromethyl group, $R^{4b}$ represents any one substituent described in Table 7A to Table 15A, and $R^{4c}$ represents a hydrogen atom (hereinafter referred to as "Compound group SX1178").

The Compound (L-11), wherein Het represents a group represented by Het57, $R^{6c}$ represents a trifluoromethoxy group, $R^{4b}$ represents any one substituent described in Table 7A to Table 15A, and $R^{4c}$ represents a hydrogen atom (hereinafter referred to as "Compound group SX1179").

The Compound (L-11), wherein Het represents a group represented by Het57, $R^{6c}$ represents a difluoromethoxy group, $R^{4b}$ represents any one substituent described in Table 7A to Table 15A, and $R^{4c}$ represents a hydrogen atom (hereinafter referred to as "Compound group SX1180").

The Compound (L-11), wherein Het represents a group represented by Het3, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1181").

The Compound (L-11), wherein Het represents a group represented by Het3, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1182").

The Compound (L-11), wherein Het represents a group represented by Het3, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1183").

The Compound (L-11), wherein Het represents a group represented by Het3, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1184").

The Compound (L-11), wherein Het represents a group represented by Het4, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1185").

The Compound (L-11), wherein Het represents a group represented by Het4, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1186").

The Compound (L-11), wherein Het represents a group represented by Het4, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1187").

The Compound (L-11), wherein Het represents a group represented by Het4, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1188").

The Compound (L-11), wherein Het represents a group represented by Het5, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1189").

The Compound (L-11), wherein Het represents a group represented by Het5, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1190").

The Compound (L-11), wherein Het represents a group represented by Het5, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1191").

The Compound (L-11), wherein Het represents a group represented by Het5, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1192").

The Compound (L-11), wherein Het represents a group represented by Het6, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1193").

The Compound (L-11), wherein Het represents a group represented by Het6, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1194").

The Compound (L-11), wherein Het represents a group represented by Het6, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1195").

The Compound (L-11), wherein Het represents a group represented by Het6, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1196").

The Compound (L-11), wherein Het represents a group represented by Het7, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1197").

The Compound (L-11), wherein Het represents a group represented by Het7, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1198").

The Compound (L-11), wherein Het represents a group represented by Het7, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1199").

The Compound (L-11), wherein Het represents a group represented by Het7, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1200").

The Compound (L-11), wherein Het represents a group represented by Het8, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1201").

The Compound (L-11), wherein Het represents a group represented by Het8, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1202").

The Compound (L-11), wherein Het represents a group represented by Het8, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1203").

The Compound (L-11), wherein Het represents a group represented by Het8, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1204").

The Compound (L-11), wherein Het represents a group represented by Het9, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1205").

The Compound (L-11), wherein Het represents a group represented by Het9, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1206").

The Compound (L-11), wherein Het represents a group represented by Het9, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1207").

The Compound (L-11), wherein Het represents a group represented by Het9, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1208").

The Compound (L-11), wherein Het represents a group represented by Het10, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1209").

The Compound (L-11), wherein Het represents a group represented by Het10, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1210").

The Compound (L-11), wherein Het represents a group represented by Het10, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1211").

The Compound (L-11), wherein Het represents a group represented by Het10, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1212").

The Compound (L-11), wherein Het represents a group represented by Het11, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1213").

The Compound (L-11), wherein Het represents a group represented by Het11, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1214").

The Compound (L-11), wherein Het represents a group represented by Het11, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1215").

The Compound (L-11), wherein Het represents a group represented by Het11, $R^{6b}$ represents a difluoromethoxy

group, $R^{4b}$ represents a hydrogen atom, and $R^{4c}$ represents any one substituent described in Table 7A to Table 15A (hereinafter referred to as "Compound group SX1216").

The Compound (L-11), wherein Het represents a group represented by Het12, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1217").

The Compound (L-11), wherein Het represents a group represented by Het12, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1218").

The Compound (L-11), wherein Het represents a group represented by Het12, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1219").

The Compound (L-11), wherein Het represents a group represented by Het12, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1220").

The Compound (L-11), wherein Het represents a group represented by Het13, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1221").

The Compound (L-11), wherein Het represents a group represented by Het13, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1222").

The Compound (L-11), wherein Het represents a group represented by Het13, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1223").

The Compound (L-11), wherein Het represents a group represented by Het13, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1224").

The Compound (L-11), wherein Het represents a group represented by Het14, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1225").

The Compound (L-11), wherein Het represents a group represented by Het14, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1226").

The Compound (L-11), wherein Het represents a group represented by Het14, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1227").

The Compound (L-11), wherein Het represents a group represented by Het14, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1228").

The Compound (L-11), wherein Het represents a group represented by Het15, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1229").

The Compound (L-11), wherein Het represents a group represented by Het15, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1230").

The Compound (L-11), wherein Het represents a group represented by Het15, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1231").

The Compound (L-11), wherein Het represents a group represented by Het15, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1232").

The Compound (L-11), wherein Het represents a group represented by Het16, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1233").

The Compound (L-11), wherein Het represents a group represented by Het16, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1234").

The Compound (L-11), wherein Het represents a group represented by Het16, $R^{6c}$ represents a trifluoromethoxy group, $R^{4b}$ represents a hydrogen atom, and $R^{4c}$ represents any one substituent described in Table 7A to Table 15A

(hereinafter referred to as "Compound group SX1235").

The Compound (L-11), wherein Het represents a group represented by Het16, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1236").

The Compound (L-11), wherein Het represents a group represented by Het17, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1237").

The Compound (L-11), wherein Het represents a group represented by Het17, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1238").

The Compound (L-11), wherein Het represents a group represented by Het17, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1239").

The Compound (L-11), wherein Het represents a group represented by Het17, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1240").

The Compound (L-11), wherein Het represents a group represented by Het18, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1241").

The Compound (L-11), wherein Het represents a group represented by Het18, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1242").

The Compound (L-11), wherein Het represents a group represented by Het18, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1243").

The Compound (L-11), wherein Het represents a group represented by Het18, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1244").

The Compound (L-11), wherein Het represents a group represented by Het19, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1245").

The Compound (L-11), wherein Het represents a group represented by Het19, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1246").

The Compound (L-11), wherein Het represents a group represented by Het19, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1247").

The Compound (L-11), wherein Het represents a group represented by Het19, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1248").

The Compound (L-11), wherein Het represents a group represented by Het20, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1249").

The Compound (L-11), wherein Het represents a group represented by Het20, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1250").

The Compound (L-11), wherein Het represents a group represented by Het20, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1251").

The Compound (L-11), wherein Het represents a group represented by Het20, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1252").

The Compound (L-11), wherein Het represents a group represented by Het21, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1253").

The Compound (L-11), wherein Het represents a group represented by Het21, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1254").

The Compound (L-11), wherein Het represents a group represented by Het21, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1255").

The Compound (L-11), wherein Het represents a group represented by Het21, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1256").

The Compound (L-11), wherein Het represents a group represented by Het22, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1257").

The Compound (L-11), wherein Het represents a group represented by Het22, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1258").

The Compound (L-11), wherein Het represents a group represented by Het22, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1259").

The Compound (L-11), wherein Het represents a group represented by Het22, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1260").

The Compound (L-11), wherein Het represents a group represented by Het23, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1261").

The Compound (L-11), wherein Het represents a group represented by Het23, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1262").

The Compound (L-11), wherein Het represents a group represented by Het23, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1263").

The Compound (L-11), wherein Het represents a group represented by Het23, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1264").

The Compound (L-11), wherein Het represents a group represented by Het24, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1265").

The Compound (L-11), wherein Het represents a group represented by Het24, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1266").

The Compound (L-11), wherein Het represents a group represented by Het24, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1267").

The Compound (L-11), wherein Het represents a group represented by Het24, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1268").

The Compound (L-11), wherein Het represents a group represented by Het25, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1269").

The Compound (L-11), wherein Het represents a group represented by Het25, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1270").

The Compound (L-11), wherein Het represents a group represented by Het25, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1271").

The Compound (L-11), wherein Het represents a group represented by Het25, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1272").

The Compound (L-11), wherein Het represents a group represented by Het26, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1273").

The Compound (L-11), wherein Het represents a group represented by Het26, $R^{6c}$ represents a difluoromethyl

group, $R^{4b}$ represents a hydrogen atom, and $R^{4c}$ represents any one substituent described in Table 7A to Table 15A (hereinafter referred to as "Compound group SX1274").

The Compound (L-11), wherein Het represents a group represented by Het26, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1275").

The Compound (L-11), wherein Het represents a group represented by Het26, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1276").

The Compound (L-11), wherein Het represents a group represented by Het27, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1277").

The Compound (L-11), wherein Het represents a group represented by Het27, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1278").

The Compound (L-11), wherein Het represents a group represented by Het27, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1279").

The Compound (L-11), wherein Het represents a group represented by Het27, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1280").

The Compound (L-11), wherein Het represents a group represented by Het28, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1281").

The Compound (L-11), wherein Het represents a group represented by Het28, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1282").

The Compound (L-11), wherein Het represents a group represented by Het28, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1283").

The Compound (L-11), wherein Het represents a group represented by Het28, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1284").

The Compound (L-11), wherein Het represents a group represented by Het29, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1285").

The Compound (L-11), wherein Het represents a group represented by Het29, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1286").

The Compound (L-11), wherein Het represents a group represented by Het29, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1287").

The Compound (L-11), wherein Het represents a group represented by Het29, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1288").

The Compound (L-11), wherein Het represents a group represented by Het30, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1289").

The Compound (L-11), wherein Het represents a group represented by Het30, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1290").

The Compound (L-11), wherein Het represents a group represented by Het30, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1291").

The Compound (L-11), wherein Het represents a group represented by Het30, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1292").

The Compound (L-11), wherein Het represents a group represented by Het31, $R^{6a}$ represents a trifluoromethyl group, $R^{4b}$ represents a hydrogen atom, and $R^{4c}$ represents any one substituent described in Table 7A to Table 15A

(hereinafter referred to as "Compound group SX1293").

The Compound (L-11), wherein Het represents a group represented by Het31, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1294").

The Compound (L-11), wherein Het represents a group represented by Het31, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1295").

The Compound (L-11), wherein Het represents a group represented by Het31, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1296").

The Compound (L-11), wherein Het represents a group represented by Het32, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1297").

The Compound (L-11), wherein Het represents a group represented by Het32, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1298").

The Compound (L-11), wherein Het represents a group represented by Het32, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1299").

The Compound (L-11), wherein Het represents a group represented by Het32, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1300").

The Compound (L-11), wherein Het represents a group represented by Het33, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1301").

The Compound (L-11), wherein Het represents a group represented by Het33, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1302").

The Compound (L-11), wherein Het represents a group represented by Het33, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1303").

The Compound (L-11), wherein Het represents a group represented by Het33, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1304").

The Compound (L-11), wherein Het represents a group represented by Het34, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1305").

The Compound (L-11), wherein Het represents a group represented by Het34, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1306").

The Compound (L-11), wherein Het represents a group represented by Het34, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1307").

The Compound (L-11), wherein Het represents a group represented by Het34, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1308").

The Compound (L-11), wherein Het represents a group represented by Het35, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1309").

The Compound (L-11), wherein Het represents a group represented by Het35, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1310").

The Compound (L-11), wherein Het represents a group represented by Het35, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1311").

The Compound (L-11), wherein Het represents a group represented by Het35, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1312").

The Compound (L-11), wherein Het represents a group represented by Het36, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1313").

The Compound (L-11), wherein Het represents a group represented by Het36, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1314").

The Compound (L-11), wherein Het represents a group represented by Het36, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1315").

The Compound (L-11), wherein Het represents a group represented by Het36, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1316").

The Compound (L-11), wherein Het represents a group represented by Het37, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1317").

The Compound (L-11), wherein Het represents a group represented by Het37, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1318").

The Compound (L-11), wherein Het represents a group represented by Het37, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1319").

The Compound (L-11), wherein Het represents a group represented by Het37, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1320").

The Compound (L-11), wherein Het represents a group represented by Het38, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1321").

The Compound (L-11), wherein Het represents a group represented by Het38, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1322").

The Compound (L-11), wherein Het represents a group represented by Het38, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1323").

The Compound (L-11), wherein Het represents a group represented by Het38, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1324").

The Compound (L-11), wherein Het represents a group represented by Het39, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1325").

The Compound (L-11), wherein Het represents a group represented by Het39, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1326").

The Compound (L-11), wherein Het represents a group represented by Het39, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1327").

The Compound (L-11), wherein Het represents a group represented by Het39, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1328").

The Compound (L-11), wherein Het represents a group represented by Het40, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1329").

The Compound (L-11), wherein Het represents a group represented by Het40, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1330").

The Compound (L-11), wherein Het represents a group represented by Het40, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1331").

The Compound (L-11), wherein Het represents a group represented by Het40, $R^{6a}$ represents a difluoromethoxy

group, $R^{4b}$ represents a hydrogen atom, and $R^{4c}$ represents any one substituent described in Table 7A to Table 15A (hereinafter referred to as "Compound group SX1332").

The Compound (L-11), wherein Het represents a group represented by Het41, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1333").

The Compound (L-11), wherein Het represents a group represented by Het41, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1334").

The Compound (L-11), wherein Het represents a group represented by Het41, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1335").

The Compound (L-11), wherein Het represents a group represented by Het41, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1336").

The Compound (L-11), wherein Het represents a group represented by Het42, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1337").

The Compound (L-11), wherein Het represents a group represented by Het42, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1338").

The Compound (L-11), wherein Het represents a group represented by Het42, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1339").

The Compound (L-11), wherein Het represents a group represented by Het42, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1340").

The Compound (L-11), wherein Het represents a group represented by Het43, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1341").

The Compound (L-11), wherein Het represents a group represented by Het43, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1342").

The Compound (L-11), wherein Het represents a group represented by Het43, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1343").

The Compound (L-11), wherein Het represents a group represented by Het43, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1344").

The Compound (L-11), wherein Het represents a group represented by Het44, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1345").

The Compound (L-11), wherein Het represents a group represented by Het44, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1346").

The Compound (L-11), wherein Het represents a group represented by Het44, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1347").

The Compound (L-11), wherein Het represents a group represented by Het44, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1348").

The Compound (L-11), wherein Het represents a group represented by Het45, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1349").

The Compound (L-11), wherein Het represents a group represented by Het45, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1350").

The Compound (L-11), wherein Het represents a group represented by Het45, $R^{6c}$ represents a trifluoromethoxy group, $R^{4b}$ represents a hydrogen atom, and $R^{4c}$ represents any one substituent described in Table 7A to Table 15A

(hereinafter referred to as "Compound group SX1351").

The Compound (L-11), wherein Het represents a group represented by Het45, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1352").

The Compound (L-11), wherein Het represents a group represented by Het46, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1353").

The Compound (L-11), wherein Het represents a group represented by Het46, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1354").

The Compound (L-11), wherein Het represents a group represented by Het46, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1355").

The Compound (L-11), wherein Het represents a group represented by Het46, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1356").

The Compound (L-11), wherein Het represents a group represented by Het47, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1357").

The Compound (L-11), wherein Het represents a group represented by Het47, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1358").

The Compound (L-11), wherein Het represents a group represented by Het47, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1359").

The Compound (L-11), wherein Het represents a group represented by Het47, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1360").

The Compound (L-11), wherein Het represents a group represented by Het48, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1361").

The Compound (L-11), wherein Het represents a group represented by Het48, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1362").

The Compound (L-11), wherein Het represents a group represented by Het48, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1363").

The Compound (L-11), wherein Het represents a group represented by Het48, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1364").

The Compound (L-11), wherein Het represents a group represented by Het49, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1365").

The Compound (L-11), wherein Het represents a group represented by Het49, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1366").

The Compound (L-11), wherein Het represents a group represented by Het49, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1367").

The Compound (L-11), wherein Het represents a group represented by Het49, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1368").

The Compound (L-11), wherein Het represents a group represented by Het50, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1369").

The Compound (L-11), wherein Het represents a group represented by Het50, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1370").

The Compound (L-11), wherein Het represents a group represented by Het50, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1371").

The Compound (L-11), wherein Het represents a group represented by Het50, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1372").

The Compound (L-11), wherein Het represents a group represented by Het51, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1373").

The Compound (L-11), wherein Het represents a group represented by Het51, $R^{6b}$ represents a difluoromethyl group, $R^{4b}$ represents a hydrogen atom, and $R^{4c}$ represents any one substituent described in Table 7A to Table 15A (hereinafter referred to as "Compound group SX1374").

The Compound (L-11), wherein Het represents a group represented by Het51, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1375").

The Compound (L-11), wherein Het represents a group represented by Het51, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1376").

The Compound (L-11), wherein Het represents a group represented by Het52, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1377").

The Compound (L-11), wherein Het represents a group represented by Het52, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1378").

The Compound (L-11), wherein Het represents a group represented by Het52, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1379").

The Compound (L-11), wherein Het represents a group represented by Het52, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1380").

The Compound (L-11), wherein Het represents a group represented by Het53, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1381").

The Compound (L-11), wherein Het represents a group represented by Het53, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1382").

The Compound (L-11), wherein Het represents a group represented by Het53, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1383").

The Compound (L-11), wherein Het represents a group represented by Het53, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1384").

The Compound (L-11), wherein Het represents a group represented by Het54, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1385").

The Compound (L-11), wherein Het represents a group represented by Het54, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1386").

The Compound (L-11), wherein Het represents a group represented by Het54, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1387").

The Compound (L-11), wherein Het represents a group represented by Het54, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1388").

The Compound (L-11), wherein Het represents a group represented by Het55, $R^{6b}$ represents a trifluoromethyl group, $R^{4b}$ represents a hydrogen atom, and $R^{4c}$ represents any one substituent described in Table 7A to Table 15A (hereinafter referred to as "Compound group SX1389").

The Compound (L-11), wherein Het represents a group represented by Het55, $R^{6b}$ represents a difluoromethyl

group, $R^{4b}$ represents a hydrogen atom, and $R^{4c}$ represents any one substituent described in Table 7A to Table 15A (hereinafter referred to as "Compound group SX1390").

The Compound (L-11), wherein Het represents a group represented by Het55, $R^{6b}$ represents a trifluoromethoxy group, $R^{4b}$ represents a hydrogen atom, and $R^{4c}$ represents any one substituent described in Table 7A to Table 15A (hereinafter referred to as "Compound group SX1391").

The Compound (L-11), wherein Het represents a group represented by Het55, $R^{6b}$ represents a difluoromethoxy group, $R^{4b}$ represents a hydrogen atom, and $R^{4c}$ represents any one substituent described in Table 7A to Table 15A (hereinafter referred to as "Compound group SX1392").

The Compound (L-11), wherein Het represents a group represented by Het56, $R^{6a}$ represents a trifluoromethyl group, $R^{4b}$ represents a hydrogen atom, and $R^{4c}$ represents any one substituent described in Table 7A to Table 15A (hereinafter referred to as "Compound group SX1393").

The Compound (L-11), wherein Het represents a group represented by Het56, $R^{6a}$ represents a difluoromethyl group, $R^{4b}$ represents a hydrogen atom, and $R^{4c}$ represents any one substituent described in Table 7A to Table 15A (hereinafter referred to as "Compound group SX1394").

The Compound (L-11), wherein Het represents a group represented by Het56, $R^{6a}$ represents a trifluoromethoxy group, $R^{4b}$ represents a hydrogen atom, and $R^{4c}$ represents any one substituent described in Table 7A to Table 15A (hereinafter referred to as "Compound group SX1395").

The Compound (L-11), wherein Het represents a group represented by Het56, $R^{6a}$ represents a difluoromethoxy group, $R^{4b}$ represents a hydrogen atom, and $R^{4c}$ represents any one substituent described in Table 7A to Table 15A (hereinafter referred to as "Compound group SX1396").

The Compound (L-11), wherein Het represents a group represented by Het57, $R^{6c}$ represents a trifluoromethyl group, $R^{4b}$ represents a hydrogen atom, and $R^{4c}$ represents any one substituent described in Table 7A to Table 15A (hereinafter referred to as "Compound group SX1397").

The Compound (L-11), wherein Het represents a group represented by Het57, $R^{6c}$ represents a difluoromethyl group, $R^{4b}$ represents a hydrogen atom, and $R^{4c}$ represents any one substituent described in Table 7A to Table 15A (hereinafter referred to as "Compound group SX1398").

The Compound (L-11), wherein Het represents a group represented by Het57, $R^{6c}$ represents a trifluoromethoxy group, $R^{4b}$ represents a hydrogen atom, and $R^{4c}$ represents any one substituent described in Table 7A to Table 15A (hereinafter referred to as "Compound group SX1399").

The Compound (L-11), wherein Het represents a group represented by Het57, $R^{6c}$ represents a difluoromethoxy group, $R^{4b}$ represents a hydrogen atom, and $R^{4c}$ represents any one substituent described in Table 7A to Table 15A (hereinafter referred to as "Compound group SX1400").

A compound represented by formula (L-12)

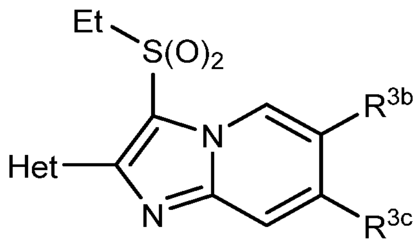

( L-12 )

(hereinafter referred to as "Compound (L-12)"), wherein Het represents a group represented by Het3, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1401").

The Compound (L-12), wherein Het represents a group represented by Het3, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1402").

The Compound (L-12), wherein Het represents a group represented by Het3, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1403").

The Compound (L-12), wherein Het represents a group represented by Het3, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1404").

The Compound (L-12), wherein Het represents a group represented by Het4, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1405").

The Compound (L-12), wherein Het represents a group represented by Het4, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1406").

The Compound (L-12), wherein Het represents a group represented by Het4, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1407").

The Compound (L-12), wherein Het represents a group represented by Het4, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1408").

The Compound (L-12), wherein Het represents a group represented by Het5, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1409").

The Compound (L-12), wherein Het represents a group represented by Het5, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1410").

The Compound (L-12), wherein Het represents a group represented by Het5, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1411").

The Compound (L-12), wherein Het represents a group represented by Het5, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1412").

The Compound (L-12), wherein Het represents a group represented by Het6, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1413").

The Compound (L-12), wherein Het represents a group represented by Het6, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1414").

The Compound (L-12), wherein Het represents a group represented by Het6, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1415").

The Compound (L-12), wherein Het represents a group represented by Het6, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1416").

The Compound (L-12), wherein Het represents a group represented by Het7, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1417").

The Compound (L-12), wherein Het represents a group represented by Het7, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1418").

The Compound (L-12), wherein Het represents a group represented by Het7, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1419").

The Compound (L-12), wherein Het represents a group represented by Het7, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1420").

The Compound (L-12), wherein Het represents a group represented by Het8, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1421").

The Compound (L-12), wherein Het represents a group represented by Het8, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1422").

The Compound (L-12), wherein Het represents a group represented by Het8, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1423").

The Compound (L-12), wherein Het represents a group represented by Het8, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1424").

The Compound (L-12), wherein Het represents a group represented by Het9, $R^{6c}$ represents a trifluoromethyl

group, $R^{3b}$ represents any one substituent described in Table 7A to Table 15A, and $R^{3c}$ represents a hydrogen atom (hereinafter referred to as "Compound group SX1425").

The Compound (L-12), wherein Het represents a group represented by Het9, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1426").

The Compound (L-12), wherein Het represents a group represented by Het9, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1427").

The Compound (L-12), wherein Het represents a group represented by Het9, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1428").

The Compound (L-12), wherein Het represents a group represented by Het10, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1429").

The Compound (L-12), wherein Het represents a group represented by Het10, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1430").

The Compound (L-12), wherein Het represents a group represented by Het10, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1431").

The Compound (L-12), wherein Het represents a group represented by Het10, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1432").

The Compound (L-12), wherein Het represents a group represented by Het11, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1433").

The Compound (L-12), wherein Het represents a group represented by Het11, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1434").

The Compound (L-12), wherein Het represents a group represented by Het11, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1435").

The Compound (L-12), wherein Het represents a group represented by Het11, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1436").

The Compound (L-12), wherein Het represents a group represented by Het12, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1437").

The Compound (L-12), wherein Het represents a group represented by Het12, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1438").

The Compound (L-12), wherein Het represents a group represented by Het12, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1439").

The Compound (L-12), wherein Het represents a group represented by Het12, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1440").

The Compound (L-12), wherein Het represents a group represented by Het13, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1441").

The Compound (L-12), wherein Het represents a group represented by Het13, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1442").

The Compound (L-12), wherein Het represents a group represented by Het13, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1443").

The Compound (L-12), wherein Het represents a group represented by Het13, $R^{6a}$ represents a difluoromethoxy group, $R^{3b}$ represents any one substituent described in Table 7A to Table 15A, and $R^{3c}$ represents a hydrogen atom

(hereinafter referred to as "Compound group SX1444").

The Compound (L-12), wherein Het represents a group represented by Het14, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1445").

The Compound (L-12), wherein Het represents a group represented by Het14, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1446").

The Compound (L-12), wherein Het represents a group represented by Het14, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1447").

The Compound (L-12), wherein Het represents a group represented by Het14, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1448").

The Compound (L-12), wherein Het represents a group represented by Het15, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1449").

The Compound (L-12), wherein Het represents a group represented by Het15, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1450").

The Compound (L-12), wherein Het represents a group represented by Het15, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1451").

The Compound (L-12), wherein Het represents a group represented by Het15, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1452").

The Compound (L-12), wherein Het represents a group represented by Het16, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1453").

The Compound (L-12), wherein Het represents a group represented by Het16, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1454").

The Compound (L-12), wherein Het represents a group represented by Het16, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1455").

The Compound (L-12), wherein Het represents a group represented by Het16, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1456").

The Compound (L-12), wherein Het represents a group represented by Het17, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1457").

The Compound (L-12), wherein Het represents a group represented by Het17, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1458").

The Compound (L-12), wherein Het represents a group represented by Het17, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1459").

The Compound (L-12), wherein Het represents a group represented by Het17, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1460").

The Compound (L-12), wherein Het represents a group represented by Het18, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1461").

The Compound (L-12), wherein Het represents a group represented by Het18, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1462").

The Compound (L-12), wherein Het represents a group represented by Het18, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1463").

The Compound (L-12), wherein Het represents a group represented by Het18, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1464").

The Compound (L-12), wherein Het represents a group represented by Het19, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1465").

The Compound (L-12), wherein Het represents a group represented by Het19, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1466").

The Compound (L-12), wherein Het represents a group represented by Het19, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1467").

The Compound (L-12), wherein Het represents a group represented by Het19, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1468").

The Compound (L-12), wherein Het represents a group represented by Het20, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1469").

The Compound (L-12), wherein Het represents a group represented by Het20, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1470").

The Compound (L-12), wherein Het represents a group represented by Het20, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1471").

The Compound (L-12), wherein Het represents a group represented by Het20, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1472").

The Compound (L-12), wherein Het represents a group represented by Het21, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1473").

The Compound (L-12), wherein Het represents a group represented by Het21, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1474").

The Compound (L-12), wherein Het represents a group represented by Het21, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1475").

The Compound (L-12), wherein Het represents a group represented by Het21, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1476").

The Compound (L-12), wherein Het represents a group represented by Het22, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1477").

The Compound (L-12), wherein Het represents a group represented by Het22, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1478").

The Compound (L-12), wherein Het represents a group represented by Het22, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1479").

The Compound (L-12), wherein Het represents a group represented by Het22, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1480").

The Compound (L-12), wherein Het represents a group represented by Het23, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1481").

The Compound (L-12), wherein Het represents a group represented by Het23, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1482").

The Compound (L-12), wherein Het represents a group represented by Het23, $R^{6c}$ represents a trifluoromethoxy

group, $R^{3b}$ represents any one substituent described in Table 7A to Table 15A, and $R^{3c}$ represents a hydrogen atom (hereinafter referred to as "Compound group SX1483").

The Compound (L-12), wherein Het represents a group represented by Het23, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1484").

The Compound (L-12), wherein Het represents a group represented by Het24, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1485").

The Compound (L-12), wherein Het represents a group represented by Het24, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1486").

The Compound (L-12), wherein Het represents a group represented by Het24, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1487").

The Compound (L-12), wherein Het represents a group represented by Het24, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1488").

The Compound (L-12), wherein Het represents a group represented by Het25, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1489").

The Compound (L-12), wherein Het represents a group represented by Het25, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1490").

The Compound (L-12), wherein Het represents a group represented by Het25, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1491").

The Compound (L-12), wherein Het represents a group represented by Het25, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1492").

The Compound (L-12), wherein Het represents a group represented by Het26, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1493").

The Compound (L-12), wherein Het represents a group represented by Het26, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1494").

The Compound (L-12), wherein Het represents a group represented by Het26, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1495").

The Compound (L-12), wherein Het represents a group represented by Het26, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1496").

The Compound (L-12), wherein Het represents a group represented by Het27, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1497").

The Compound (L-12), wherein Het represents a group represented by Het27, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1498").

The Compound (L-12), wherein Het represents a group represented by Het27, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1499").

The Compound (L-12), wherein Het represents a group represented by Het27, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1500").

The Compound (L-12), wherein Het represents a group represented by Het28, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1501").

The Compound (L-12), wherein Het represents a group represented by Het28, $R^{6b}$ represents a difluoromethyl group, $R^{3b}$ represents any one substituent described in Table 7A to Table 15A, and $R^{3c}$ represents a hydrogen atom

(hereinafter referred to as "Compound group SX1502").

The Compound (L-12), wherein Het represents a group represented by Het28, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1503").

The Compound (L-12), wherein Het represents a group represented by Het28, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1504").

The Compound (L-12), wherein Het represents a group represented by Het29, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1505").

The Compound (L-12), wherein Het represents a group represented by Het29, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1506").

The Compound (L-12), wherein Het represents a group represented by Het29, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1507").

The Compound (L-12), wherein Het represents a group represented by Het29, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1508").

The Compound (L-12), wherein Het represents a group represented by Het30, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1509").

The Compound (L-12), wherein Het represents a group represented by Het30, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1510").

The Compound (L-12), wherein Het represents a group represented by Het30, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1511").

The Compound (L-12), wherein Het represents a group represented by Het30, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1512").

The Compound (L-12), wherein Het represents a group represented by Het31, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1513").

The Compound (L-12), wherein Het represents a group represented by Het31, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1514").

The Compound (L-12), wherein Het represents a group represented by Het31, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1515").

The Compound (L-12), wherein Het represents a group represented by Het31, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1516").

The Compound (L-12), wherein Het represents a group represented by Het32, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1517").

The Compound (L-12), wherein Het represents a group represented by Het32, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1518").

The Compound (L-12), wherein Het represents a group represented by Het32, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1519").

The Compound (L-12), wherein Het represents a group represented by Het32, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1520").

The Compound (L-12), wherein Het represents a group represented by Het33, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1521").

The Compound (L-12), wherein Het represents a group represented by Het33, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1522").

The Compound (L-12), wherein Het represents a group represented by Het33, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1523").

The Compound (L-12), wherein Het represents a group represented by Het33, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1524").

The Compound (L-12), wherein Het represents a group represented by Het34, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1525").

The Compound (L-12), wherein Het represents a group represented by Het34, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1526").

The Compound (L-12), wherein Het represents a group represented by Het34, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1527").

The Compound (L-12), wherein Het represents a group represented by Het34, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1528").

The Compound (L-12), wherein Het represents a group represented by Het35, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1529").

The Compound (L-12), wherein Het represents a group represented by Het35, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1530").

The Compound (L-12), wherein Het represents a group represented by Het35, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1531").

The Compound (L-12), wherein Het represents a group represented by Het35, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1532").

The Compound (L-12), wherein Het represents a group represented by Het36, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1533").

The Compound (L-12), wherein Het represents a group represented by Het36, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1534").

The Compound (L-12), wherein Het represents a group represented by Het36, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1535").

The Compound (L-12), wherein Het represents a group represented by Het36, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1536").

The Compound (L-12), wherein Het represents a group represented by Het37, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1537").

The Compound (L-12), wherein Het represents a group represented by Het37, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1538").

The Compound (L-12), wherein Het represents a group represented by Het37, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1539").

The Compound (L-12), wherein Het represents a group represented by Het37, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1540").

The Compound (L-12), wherein Het represents a group represented by Het38, $R^{6a}$ represents a trifluoromethyl

group, $R^{3b}$ represents any one substituent described in Table 7A to Table 15A, and $R^{3c}$ represents a hydrogen atom (hereinafter referred to as "Compound group SX1541").

The Compound (L-12), wherein Het represents a group represented by Het38, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1542").

The Compound (L-12), wherein Het represents a group represented by Het38, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1543").

The Compound (L-12), wherein Het represents a group represented by Het38, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1544").

The Compound (L-12), wherein Het represents a group represented by Het39, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1545").

The Compound (L-12), wherein Het represents a group represented by Het39, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1546").

The Compound (L-12), wherein Het represents a group represented by Het39, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1547").

The Compound (L-12), wherein Het represents a group represented by Het39, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1548").

The Compound (L-12), wherein Het represents a group represented by Het40, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1549").

The Compound (L-12), wherein Het represents a group represented by Het40, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1550").

The Compound (L-12), wherein Het represents a group represented by Het40, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1551").

The Compound (L-12), wherein Het represents a group represented by Het40, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1552").

The Compound (L-12), wherein Het represents a group represented by Het41, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1553").

The Compound (L-12), wherein Het represents a group represented by Het41, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1554").

The Compound (L-12), wherein Het represents a group represented by Het41, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1555").

The Compound (L-12), wherein Het represents a group represented by Het41, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1556").

The Compound (L-12), wherein Het represents a group represented by Het42, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1557").

The Compound (L-12), wherein Het represents a group represented by Het42, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1558").

The Compound (L-12), wherein Het represents a group represented by Het42, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1559").

The Compound (L-12), wherein Het represents a group represented by Het42, $R^{6a}$ represents a difluoromethoxy group, $R^{3b}$ represents any one substituent described in Table 7A to Table 15A, and $R^{3c}$ represents a hydrogen atom

(hereinafter referred to as "Compound group SX1560").

The Compound (L-12), wherein Het represents a group represented by Het43, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1561").

The Compound (L-12), wherein Het represents a group represented by Het43, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1562").

The Compound (L-12), wherein Het represents a group represented by Het43, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1563").

The Compound (L-12), wherein Het represents a group represented by Het43, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1564").

The Compound (L-12), wherein Het represents a group represented by Het44, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1565").

The Compound (L-12), wherein Het represents a group represented by Het44, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1566").

The Compound (L-12), wherein Het represents a group represented by Het44, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1567").

The Compound (L-12), wherein Het represents a group represented by Het44, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1568").

The Compound (L-12), wherein Het represents a group represented by Het45, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1569").

The Compound (L-12), wherein Het represents a group represented by Het45, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1570").

The Compound (L-12), wherein Het represents a group represented by Het45, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1571").

The Compound (L-12), wherein Het represents a group represented by Het45, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1572").

The Compound (L-12), wherein Het represents a group represented by Het46, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1573").

The Compound (L-12), wherein Het represents a group represented by Het46, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1574").

The Compound (L-12), wherein Het represents a group represented by Het46, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1575").

The Compound (L-12), wherein Het represents a group represented by Het46, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1576").

The Compound (L-12), wherein Het represents a group represented by Het47, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1577").

The Compound (L-12), wherein Het represents a group represented by Het47, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1578").

The Compound (L-12), wherein Het represents a group represented by Het47, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1579").

The Compound (L-12), wherein Het represents a group represented by Het47, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1580").

The Compound (L-12), wherein Het represents a group represented by Het48, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1581").

The Compound (L-12), wherein Het represents a group represented by Het48, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1582").

The Compound (L-12), wherein Het represents a group represented by Het48, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1583").

The Compound (L-12), wherein Het represents a group represented by Het48, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1584").

The Compound (L-12), wherein Het represents a group represented by Het49, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1585").

The Compound (L-12), wherein Het represents a group represented by Het49, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1586").

The Compound (L-12), wherein Het represents a group represented by Het49, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1587").

The Compound (L-12), wherein Het represents a group represented by Het49, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1588").

The Compound (L-12), wherein Het represents a group represented by Het50, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1589").

The Compound (L-12), wherein Het represents a group represented by Het50, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1590").

The Compound (L-12), wherein Het represents a group represented by Het50, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1591").

The Compound (L-12), wherein Het represents a group represented by Het50, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1592").

The Compound (L-12), wherein Het represents a group represented by Het51, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1593").

The Compound (L-12), wherein Het represents a group represented by Het51, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1594").

The Compound (L-12), wherein Het represents a group represented by Het51, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1595").

The Compound (L-12), wherein Het represents a group represented by Het51, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1596").

The Compound (L-12), wherein Het represents a group represented by Het52, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1597").

The Compound (L-12), wherein Het represents a group represented by Het52, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1598").

The Compound (L-12), wherein Het represents a group represented by Het52, $R^{6a}$ represents a trifluoromethoxy

group, $R^{3b}$ represents any one substituent described in Table 7A to Table 15A, and $R^{3c}$ represents a hydrogen atom (hereinafter referred to as "Compound group SX1599").

The Compound (L-12), wherein Het represents a group represented by Het52, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1600").

The Compound (L-12), wherein Het represents a group represented by Het53, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1601").

The Compound (L-12), wherein Het represents a group represented by Het53, $R^{6c}$ represents a difluoromethyl group, $R^{3b}$ represents any one substituent described in Table 7A to Table 15A, and $R^{3c}$ represents a hydrogen atom (hereinafter referred to as "Compound group SX1602").

The Compound (L-12), wherein Het represents a group represented by Het53, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1603").

The Compound (L-12), wherein Het represents a group represented by Het53, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1604").

The Compound (L-12), wherein Het represents a group represented by Het54, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1605").

The Compound (L-12), wherein Het represents a group represented by Het54, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1606").

The Compound (L-12), wherein Het represents a group represented by Het54, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1607").

The Compound (L-12), wherein Het represents a group represented by Het54, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1608").

The Compound (L-12), wherein Het represents a group represented by Het55, $R^{6b}$ represents a trifluoromethyl group, $R^{3b}$ represents any one substituent described in Table 7A to Table 15A, and $R^{3c}$ represents a hydrogen atom (hereinafter referred to as "Compound group SX1609").

The Compound (L-12), wherein Het represents a group represented by Het55, $R^{6b}$ represents a difluoromethyl group, $R^{3b}$ represents any one substituent described in Table 7A to Table 15A, and $R^{3c}$ represents a hydrogen atom (hereinafter referred to as "Compound group SX1610").

The Compound (L-12), wherein Het represents a group represented by Het55, $R^{6b}$ represents a trifluoromethoxy group, $R^{3b}$ represents any one substituent described in Table 7A to Table 15A, and $R^{3c}$ represents a hydrogen atom (hereinafter referred to as "Compound group SX1611").

The Compound (L-12), wherein Het represents a group represented by Het55, $R^{6b}$ represents a difluoromethoxy group, $R^{3b}$ represents any one substituent described in Table 7A to Table 15A, and $R^{3c}$ represents a hydrogen atom (hereinafter referred to as "Compound group SX1612").

The Compound (L-12), wherein Het represents a group represented by Het56, $R^{6a}$ represents a trifluoromethyl group, $R^{3b}$ represents any one substituent described in Table 7A to Table 15A, and $R^{3c}$ represents a hydrogen atom (hereinafter referred to as "Compound group SX1613").

The Compound (L-12), wherein Het represents a group represented by Het56, $R^{6a}$ represents a difluoromethyl group, $R^{3b}$ represents any one substituent described in Table 7A to Table 15A, and $R^{3c}$ represents a hydrogen atom (hereinafter referred to as "Compound group SX1614").

The Compound (L-12), wherein Het represents a group represented by Het56, $R^{6a}$ represents a trifluoromethoxy group, $R^{3b}$ represents any one substituent described in Table 7A to Table 15A, and $R^{3c}$ represents a hydrogen atom (hereinafter referred to as "Compound group SX1615").

The Compound (L-12), wherein Het represents a group represented by Het56, $R^{6a}$ represents a difluoromethoxy group, $R^{3b}$ represents any one substituent described in Table 7A to Table 15A, and $R^{3c}$ represents a hydrogen atom (hereinafter referred to as "Compound group SX1616").

The Compound (L-12), wherein Het represents a group represented by Het57, $R^{6c}$ represents a trifluoromethyl group, $R^{3b}$ represents any one substituent described in Table 7A to Table 15A, and $R^{3c}$ represents a hydrogen atom (hereinafter referred to as "Compound group SX1617").

The Compound (L-12), wherein Het represents a group represented by Het57, $R^{6c}$ represents a difluoromethyl group, $R^{3b}$ represents any one substituent described in Table 7A to Table 15A, and $R^{3c}$ represents a hydrogen atom

(hereinafter referred to as "Compound group SX1618").

The Compound (L-12), wherein Het represents a group represented by Het57, $R^{6c}$ represents a trifluoromethoxy group, $R^{3b}$ represents any one substituent described in Table 7A to Table 15A, and $R^{3c}$ represents a hydrogen atom (hereinafter referred to as "Compound group SX1619").

The Compound (L-12), wherein Het represents a group represented by Het57, $R^{6c}$ represents a difluoromethoxy group, $R^{3b}$ represents any one substituent described in Table 7A to Table 15A, and $R^{3c}$ represents a hydrogen atom (hereinafter referred to as "Compound group SX1620").

The Compound (L-12), wherein Het represents a group represented by Het3, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1621").

The Compound (L-12), wherein Het represents a group represented by Het3, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1622").

The Compound (L-12), wherein Het represents a group represented by Het3, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1623").

The Compound (L-12), wherein Het represents a group represented by Het3, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1624").

The Compound (L-12), wherein Het represents a group represented by Het4, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1625").

The Compound (L-12), wherein Het represents a group represented by Het4, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1626").

The Compound (L-12), wherein Het represents a group represented by Het4, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1627").

The Compound (L-12), wherein Het represents a group represented by Het4, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1628").

The Compound (L-12), wherein Het represents a group represented by Het5, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1629").

The Compound (L-12), wherein Het represents a group represented by Het5, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1630").

The Compound (L-12), wherein Het represents a group represented by Het5, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1631").

The Compound (L-12), wherein Het represents a group represented by Het5, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1632").

The Compound (L-12), wherein Het represents a group represented by Het6, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1633").

The Compound (L-12), wherein Het represents a group represented by Het6, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1634").

The Compound (L-12), wherein Het represents a group represented by Het6, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1635").

The Compound (L-12), wherein Het represents a group represented by Het6, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1636").

The Compound (L-12), wherein Het represents a group represented by Het7, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1637").

The Compound (L-12), wherein Het represents a group represented by Het7, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1638").

The Compound (L-12), wherein Het represents a group represented by Het7, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1639").

The Compound (L-12), wherein Het represents a group represented by Het7, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1640").

The Compound (L-12), wherein Het represents a group represented by Het8, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1641").

The Compound (L-12), wherein Het represents a group represented by Het8, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1642").

The Compound (L-12), wherein Het represents a group represented by Het8, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1643").

The Compound (L-12), wherein Het represents a group represented by Het8, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1644").

The Compound (L-12), wherein Het represents a group represented by Het9, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1645").

The Compound (L-12), wherein Het represents a group represented by Het9, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1646").

The Compound (L-12), wherein Het represents a group represented by Het9, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1647").

The Compound (L-12), wherein Het represents a group represented by Het9, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1648").

The Compound (L-12), wherein Het represents a group represented by Het10, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1649").

The Compound (L-12), wherein Het represents a group represented by Het10, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1650").

The Compound (L-12), wherein Het represents a group represented by Het10, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1651").

The Compound (L-12), wherein Het represents a group represented by Het10, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1652").

The Compound (L-12), wherein Het represents a group represented by Het11, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1653").

The Compound (L-12), wherein Het represents a group represented by Het11, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1654").

The Compound (L-12), wherein Het represents a group represented by Het11, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1655").

The Compound (L-12), wherein Het represents a group represented by Het11, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1656").

The Compound (L-12), wherein Het represents a group represented by Het12, $R^{6c}$ represents a trifluoromethyl

group, $R^{3b}$ represents a hydrogen atom, and $R^{3c}$ represents any one substituent described in Table 7A to Table 15A (hereinafter referred to as "Compound group SX1657").

The Compound (L-12), wherein Het represents a group represented by Het12, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1658").

The Compound (L-12), wherein Het represents a group represented by Het12, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1659").

The Compound (L-12), wherein Het represents a group represented by Het12, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1660").

The Compound (L-12), wherein Het represents a group represented by Het13, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1661").

The Compound (L-12), wherein Het represents a group represented by Het13, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1662").

The Compound (L-12), wherein Het represents a group represented by Het13, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1663").

The Compound (L-12), wherein Het represents a group represented by Het13, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1664").

The Compound (L-12), wherein Het represents a group represented by Het14, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1665").

The Compound (L-12), wherein Het represents a group represented by Het14, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1666").

The Compound (L-12), wherein Het represents a group represented by Het14, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1667").

The Compound (L-12), wherein Het represents a group represented by Het14, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1668").

The Compound (L-12), wherein Het represents a group represented by Het15, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1669").

The Compound (L-12), wherein Het represents a group represented by Het15, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1670").

The Compound (L-12), wherein Het represents a group represented by Het15, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1671").

The Compound (L-12), wherein Het represents a group represented by Het15, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1672").

The Compound (L-12), wherein Het represents a group represented by Het16, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1673").

The Compound (L-12), wherein Het represents a group represented by Het16, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1674").

The Compound (L-12), wherein Het represents a group represented by Het16, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1675").

The Compound (L-12), wherein Het represents a group represented by Het16, $R^{6c}$ represents a difluoromethoxy group, $R^{3b}$ represents a hydrogen atom, and $R^{3c}$ represents any one substituent described in Table 7A to Table 15A

(hereinafter referred to as "Compound group SX1676").

The Compound (L-12), wherein Het represents a group represented by Het17, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1677").

The Compound (L-12), wherein Het represents a group represented by Het17, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1678").

The Compound (L-12), wherein Het represents a group represented by Het17, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1679").

The Compound (L-12), wherein Het represents a group represented by Het17, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1680").

The Compound (L-12), wherein Het represents a group represented by Het18, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1681").

The Compound (L-12), wherein Het represents a group represented by Het18, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1682").

The Compound (L-12), wherein Het represents a group represented by Het18, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1683").

The Compound (L-12), wherein Het represents a group represented by Het18, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1684").

The Compound (L-12), wherein Het represents a group represented by Het19, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1685").

The Compound (L-12), wherein Het represents a group represented by Het19, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1686").

The Compound (L-12), wherein Het represents a group represented by Het19, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1687").

The Compound (L-12), wherein Het represents a group represented by Het19, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1688").

The Compound (L-12), wherein Het represents a group represented by Het20, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1689").

The Compound (L-12), wherein Het represents a group represented by Het20, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1690").

The Compound (L-12), wherein Het represents a group represented by Het20, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1691").

The Compound (L-12), wherein Het represents a group represented by Het20, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1692").

The Compound (L-12), wherein Het represents a group represented by Het21, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1693").

The Compound (L-12), wherein Het represents a group represented by Het21, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1694").

The Compound (L-12), wherein Het represents a group represented by Het21, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1695").

The Compound (L-12), wherein Het represents a group represented by Het21, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1696").

The Compound (L-12), wherein Het represents a group represented by Het22, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1697").

The Compound (L-12), wherein Het represents a group represented by Het22, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1698").

The Compound (L-12), wherein Het represents a group represented by Het22, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1699").

The Compound (L-12), wherein Het represents a group represented by Het22, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1700").

The Compound (L-12), wherein Het represents a group represented by Het23, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1701").

The Compound (L-12), wherein Het represents a group represented by Het23, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1702").

The Compound (L-12), wherein Het represents a group represented by Het23, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1703").

The Compound (L-12), wherein Het represents a group represented by Het23, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1704").

The Compound (L-12), wherein Het represents a group represented by Het24, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1705").

The Compound (L-12), wherein Het represents a group represented by Het24, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1706").

The Compound (L-12), wherein Het represents a group represented by Het24, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1707").

The Compound (L-12), wherein Het represents a group represented by Het24, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1708").

The Compound (L-12), wherein Het represents a group represented by Het25, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1709").

The Compound (L-12), wherein Het represents a group represented by Het25, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1710").

The Compound (L-12), wherein Het represents a group represented by Het25, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1711").

The Compound (L-12), wherein Het represents a group represented by Het25, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1712").

The Compound (L-12), wherein Het represents a group represented by Het26, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1713").

The Compound (L-12), wherein Het represents a group represented by Het26, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1714").

The Compound (L-12), wherein Het represents a group represented by Het26, $R^{6c}$ represents a trifluoromethoxy

group, $R^{3b}$ represents a hydrogen atom, and $R^{3c}$ represents any one substituent described in Table 7A to Table 15A (hereinafter referred to as "Compound group SX1715").

The Compound (L-12), wherein Het represents a group represented by Het26, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1716").

The Compound (L-12), wherein Het represents a group represented by Het27, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1717").

The Compound (L-12), wherein Het represents a group represented by Het27, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1718").

The Compound (L-12), wherein Het represents a group represented by Het27, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1719").

The Compound (L-12), wherein Het represents a group represented by Het27, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1720").

The Compound (L-12), wherein Het represents a group represented by Het28, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1721").

The Compound (L-12), wherein Het represents a group represented by Het28, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1722").

The Compound (L-12), wherein Het represents a group represented by Het28, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1723").

The Compound (L-12), wherein Het represents a group represented by Het28, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1724").

The Compound (L-12), wherein Het represents a group represented by Het29, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1725").

The Compound (L-12), wherein Het represents a group represented by Het29, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1726").

The Compound (L-12), wherein Het represents a group represented by Het29, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1727").

The Compound (L-12), wherein Het represents a group represented by Het29, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1728").

The Compound (L-12), wherein Het represents a group represented by Het30, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1729").

The Compound (L-12), wherein Het represents a group represented by Het30, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1730").

The Compound (L-12), wherein Het represents a group represented by Het30, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1731").

The Compound (L-12), wherein Het represents a group represented by Het30, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1732").

The Compound (L-12), wherein Het represents a group represented by Het31, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1733").

The Compound (L-12), wherein Het represents a group represented by Het31, $R^{6a}$ represents a difluoromethyl group, $R^{3b}$ represents a hydrogen atom, and $R^{3c}$ represents any one substituent described in Table 7A to Table 15A

(hereinafter referred to as "Compound group SX1734").

The Compound (L-12), wherein Het represents a group represented by Het31, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1735").

The Compound (L-12), wherein Het represents a group represented by Het31, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1736").

The Compound (L-12), wherein Het represents a group represented by Het32, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1737").

The Compound (L-12), wherein Het represents a group represented by Het32, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1738").

The Compound (L-12), wherein Het represents a group represented by Het32, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1739").

The Compound (L-12), wherein Het represents a group represented by Het32, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1740").

The Compound (L-12), wherein Het represents a group represented by Het33, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1741").

The Compound (L-12), wherein Het represents a group represented by Het33, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1742").

The Compound (L-12), wherein Het represents a group represented by Het33, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1743").

The Compound (L-12), wherein Het represents a group represented by Het33, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1744").

The Compound (L-12), wherein Het represents a group represented by Het34, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1745").

The Compound (L-12), wherein Het represents a group represented by Het34, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1746").

The Compound (L-12), wherein Het represents a group represented by Het34, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1747").

The Compound (L-12), wherein Het represents a group represented by Het34, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1748").

The Compound (L-12), wherein Het represents a group represented by Het35, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1749").

The Compound (L-12), wherein Het represents a group represented by Het35, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1750").

The Compound (L-12), wherein Het represents a group represented by Het35, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1751").

The Compound (L-12), wherein Het represents a group represented by Het35, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1752").

The Compound (L-12), wherein Het represents a group represented by Het36, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1753").

The Compound (L-12), wherein Het represents a group represented by Het36, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1754").

The Compound (L-12), wherein Het represents a group represented by Het36, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1755").

The Compound (L-12), wherein Het represents a group represented by Het36, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1756").

The Compound (L-12), wherein Het represents a group represented by Het37, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1757").

The Compound (L-12), wherein Het represents a group represented by Het37, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1758").

The Compound (L-12), wherein Het represents a group represented by Het37, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1759").

The Compound (L-12), wherein Het represents a group represented by Het37, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1760").

The Compound (L-12), wherein Het represents a group represented by Het38, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1761").

The Compound (L-12), wherein Het represents a group represented by Het38, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1762").

The Compound (L-12), wherein Het represents a group represented by Het38, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1763").

The Compound (L-12), wherein Het represents a group represented by Het38, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1764").

The Compound (L-12), wherein Het represents a group represented by Het39, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1765").

The Compound (L-12), wherein Het represents a group represented by Het39, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1766").

The Compound (L-12), wherein Het represents a group represented by Het39, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1767").

The Compound (L-12), wherein Het represents a group represented by Het39, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1768").

The Compound (L-12), wherein Het represents a group represented by Het40, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1769").

The Compound (L-12), wherein Het represents a group represented by Het40, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1770").

The Compound (L-12), wherein Het represents a group represented by Het40, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1771").

The Compound (L-12), wherein Het represents a group represented by Het40, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1772").

The Compound (L-12), wherein Het represents a group represented by Het41, $R^{6c}$ represents a trifluoromethyl

group, $R^{3b}$ represents a hydrogen atom, and $R^{3c}$ represents any one substituent described in Table 7A to Table 15A (hereinafter referred to as "Compound group SX1773").

The Compound (L-12), wherein Het represents a group represented by Het41, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1774").

The Compound (L-12), wherein Het represents a group represented by Het41, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1775").

The Compound (L-12), wherein Het represents a group represented by Het41, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1776").

The Compound (L-12), wherein Het represents a group represented by Het42, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1777").

The Compound (L-12), wherein Het represents a group represented by Het42, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1778").

The Compound (L-12), wherein Het represents a group represented by Het42, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1779").

The Compound (L-12), wherein Het represents a group represented by Het42, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1780").

The Compound (L-12), wherein Het represents a group represented by Het43, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1781").

The Compound (L-12), wherein Het represents a group represented by Het43, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1782").

The Compound (L-12), wherein Het represents a group represented by Het43, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1783").

The Compound (L-12), wherein Het represents a group represented by Het43, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1784").

The Compound (L-12), wherein Het represents a group represented by Het44, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1785").

The Compound (L-12), wherein Het represents a group represented by Het44, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1786").

The Compound (L-12), wherein Het represents a group represented by Het44, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1787").

The Compound (L-12), wherein Het represents a group represented by Het44, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1788").

The Compound (L-12), wherein Het represents a group represented by Het45, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1789").

The Compound (L-12), wherein Het represents a group represented by Het45, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1790").

The Compound (L-12), wherein Het represents a group represented by Het45, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1791").

The Compound (L-12), wherein Het represents a group represented by Het45, $R^{6c}$ represents a difluoromethoxy group, $R^{3b}$ represents a hydrogen atom, and $R^{3c}$ represents any one substituent described in Table 7A to Table 15A

(hereinafter referred to as "Compound group SX1792").

The Compound (L-12), wherein Het represents a group represented by Het46, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1793").

The Compound (L-12), wherein Het represents a group represented by Het46, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1794").

The Compound (L-12), wherein Het represents a group represented by Het46, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1795").

The Compound (L-12), wherein Het represents a group represented by Het46, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1796").

The Compound (L-12), wherein Het represents a group represented by Het47, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1797").

The Compound (L-12), wherein Het represents a group represented by Het47, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1798").

The Compound (L-12), wherein Het represents a group represented by Het47, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1799").

The Compound (L-12), wherein Het represents a group represented by Het47, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1800").

The Compound (L-12), wherein Het represents a group represented by Het48, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1801").

The Compound (L-12), wherein Het represents a group represented by Het48, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1802").

The Compound (L-12), wherein Het represents a group represented by Het48, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1803").

The Compound (L-12), wherein Het represents a group represented by Het48, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1804").

The Compound (L-12), wherein Het represents a group represented by Het49, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1805").

The Compound (L-12), wherein Het represents a group represented by Het49, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1806").

The Compound (L-12), wherein Het represents a group represented by Het49, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1807").

The Compound (L-12), wherein Het represents a group represented by Het49, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1808").

The Compound (L-12), wherein Het represents a group represented by Het50, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1809").

The Compound (L-12), wherein Het represents a group represented by Het50, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1810").

The Compound (L-12), wherein Het represents a group represented by Het50, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1811").

The Compound (L-12), wherein Het represents a group represented by Het50, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1812").

The Compound (L-12), wherein Het represents a group represented by Het51, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1813").

The Compound (L-12), wherein Het represents a group represented by Het51, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1814").

The Compound (L-12), wherein Het represents a group represented by Het51, $R^{6b}$ represents a trifluoromethoxy group, $R^{3b}$ represents a hydrogen atom, and $R^{3c}$ represents any one substituent described in Table 7A to Table 15A (hereinafter referred to as "Compound group SX1815").

The Compound (L-12), wherein Het represents a group represented by Het51, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1816").

The Compound (L-12), wherein Het represents a group represented by Het52, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1817").

The Compound (L-12), wherein Het represents a group represented by Het52, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1818").

The Compound (L-12), wherein Het represents a group represented by Het52, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1819").

The Compound (L-12), wherein Het represents a group represented by Het52, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1820").

The Compound (L-12), wherein Het represents a group represented by Het53, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1821").

The Compound (L-12), wherein Het represents a group represented by Het53, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1822").

The Compound (L-12), wherein Het represents a group represented by Het53, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1823").

The Compound (L-12), wherein Het represents a group represented by Het53, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1824").

The Compound (L-12), wherein Het represents a group represented by Het54, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1825").

The Compound (L-12), wherein Het represents a group represented by Het54, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1826").

The Compound (L-12), wherein Het represents a group represented by Het54, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1827").

The Compound (L-12), wherein Het represents a group represented by Het54, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1828").

The Compound (L-12), wherein Het represents a group represented by Het55, $R^{6b}$ represents a trifluoromethyl group, $R^{3b}$ represents a hydrogen atom, and $R^{3c}$ represents any one substituent described in Table 7A to Table 15A (hereinafter referred to as "Compound group SX1829").

The Compound (L-12), wherein Het represents a group represented by Het55, $R^{6b}$ represents a difluoromethyl group, $R^{3b}$ represents a hydrogen atom, and $R^{3c}$ represents any one substituent described in Table 7A to Table 15A (hereinafter referred to as "Compound group SX1830").

The Compound (L-12), wherein Het represents a group represented by Het55, $R^{6b}$ represents a trifluoromethoxy

group, $R^{3b}$ represents a hydrogen atom, and $R^{3c}$ represents any one substituent described in Table 7A to Table 15A (hereinafter referred to as "Compound group SX1831").

The Compound (L-12), wherein Het represents a group represented by Het55, $R^{6b}$ represents a difluoromethoxy group, $R^{3b}$ represents a hydrogen atom, and $R^{3c}$ represents any one substituent described in Table 7A to Table 15A (hereinafter referred to as "Compound group SX1832").

The Compound (L-12), wherein Het represents a group represented by Het56, $R^{6a}$ represents a trifluoromethyl group, $R^{3b}$ represents a hydrogen atom, and $R^{3c}$ represents any one substituent described in Table 7A to Table 15A (hereinafter referred to as "Compound group SX1833").

The Compound (L-12), wherein Het represents a group represented by Het56, $R^{6a}$ represents a difluoromethyl group, $R^{3b}$ represents a hydrogen atom, and $R^{3c}$ represents any one substituent described in Table 7A to Table 15A (hereinafter referred to as "Compound group SX1834").

The Compound (L-12), wherein Het represents a group represented by Het56, $R^{6a}$ represents a trifluoromethoxy group, $R^{3b}$ represents a hydrogen atom, and $R^{3c}$ represents any one substituent described in Table 7A to Table 15A (hereinafter referred to as "Compound group SX1835").

The Compound (L-12), wherein Het represents a group represented by Het56, $R^{6a}$ represents a difluoromethoxy group, $R^{3b}$ represents a hydrogen atom, and $R^{3c}$ represents any one substituent described in Table 7A to Table 15A (hereinafter referred to as "Compound group SX1836").

The Compound (L-12), wherein Het represents a group represented by Het57, $R^{6c}$ represents a trifluoromethyl group, $R^{3b}$ represents a hydrogen atom, and $R^{3c}$ represents any one substituent described in Table 7A to Table 15A (hereinafter referred to as "Compound group SX1837").

The Compound (L-12), wherein Het represents a group represented by Het57, $R^{6c}$ represents a difluoromethyl group, $R^{3b}$ represents a hydrogen atom, and $R^{3c}$ represents any one substituent described in Table 7A to Table 15A (hereinafter referred to as "Compound group SX1838").

The Compound (L-12), wherein Het represents a group represented by Het57, $R^{6c}$ represents a trifluoromethoxy group, $R^{3b}$ represents a hydrogen atom, and $R^{3c}$ represents any one substituent described in Table 7A to Table 15A (hereinafter referred to as "Compound group SX1839").

The Compound (L-12), wherein Het represents a group represented by Het57, $R^{6c}$ represents a difluoromethoxy group, $R^{3b}$ represents a hydrogen atom, and $R^{3c}$ represents any one substituent described in Table 7A to Table 15A (hereinafter referred to as "Compound group SX1840").

Next, Formulation Examples of the Present compounds are shown below. The "part(s)" represents "part(s) by weight". Also, the expression of "Present compound S" represents the compounds described in the Compound groups SX1 to SX1840.

Formulation Example 1

A mixture of polyoxyethylene alkyl ether sulfate ammonium salt and silica (weight ratio of 1 : 1) (35 parts), any one of the Present compound S (10 parts), and water (55 parts) are mixed, and the resulting mixture is subjected to fine grinding according to a wet grinding method to obtain each formulation.

Formulation Example 2

Any one of the Present compound S (50 parts), calcium lignin sulfonate (3 parts), sodium lauryl sulfate (2 parts), and silica (45 parts) are ground and mixed to obtain each formulation.

Formulation Example 3

Any one of the Present compound S (5 parts), polyoxyethylene styryl phenyl ether (9 parts), polyoxyethylene decyl ether (number of added ethyleneoxide: 5) (5 parts), calcium dodecylbenzene sulfonate (6 parts), and xylene (75 parts) are mixed to obtain each formulation.

Formulation Example 4

Any one of the Present compound S (2 parts), silica (1 part), calcium lignin sulfonate (2 parts), bentonite (30 parts), and kaolin clay (65 parts) are ground and mixed, an appropriate amount of water is added thereto, the resulting mixture is kneaded, subjected to granulation with a granulator, and then dried to obtain each formulation.

Formulation Example 5

Any one of the Present compound S (10 parts), and a mixture of benzyl alcohol (18 parts) and DMSO (9 parts) are mixed, GERONOL (registered trademark) TE250 (6.3 parts), Ethylan (registered trademark) NS-500LQ (2.7 parts), and solvent naphtha (54 parts) are added thereto, and the resulting mixture is mixed to obtain each formulation.

Formulation Example 6

Any one of the Present compound S (0.1 part) is mixed with kerosene (39.9 parts) and dissolved therein, the resulting solution is placed into an aerosol container, and the container is filled with liquefied petroleum gas (a mixture of propane, butane, and isobutane; saturated vapor pressure: 0.47 MPa (25°C)) (60 parts) to obtain each formulation.

Formulation Example 7

Any one of the Present compound S (0.2 part), lees powder extracted from pyrethrum (50 parts), Tabu powder (30 parts), and wood powder (19.8 parts) are mixed, an appropriate amount of water is added thereto, the resulting mixture is kneaded, then subjected to an extruder to obtain a plate sheet, and the plate sheet is subjected to a punching machine to be converted into a spiral shape to obtain each formulation.

Next, Test Examples are used to show effects of the Present compounds on harmful arthropods. In the following Test Examples, the tests were carried out at 25°C.

Test Method 1

Each test compound is made to a formulation according to a similar method to that described in the Formulation Example 1, and thereto is added water containing 0.03 v/v % of Shindain (registered trademark) to prepare a diluted solution containing a prescribed concentration of the test compound.

Cucumber (Cucumis sativus) seedling (on the developmental stage of the second true leaf) is planted in a container and approximately 30 cotton aphids (Aphis gossypii) (all developmental stages of life) are released onto the leaves of the cucumber. After 1 day, the diluted solutions are sprayed to the seedling at a ratio of 10 mL/seedling. Further, after 5 days, the number of the surviving insects is examined and the controlling value is calculated by the following equation.

$$\text{Controlling value (\%)} = \{1 - (Cb \times Tai) / (Cai \times Tb)\} \times 100$$

wherein the symbols in the formula represent the following descriptions.

Cb: Number of the test insects in untreated group;
Cai: Number of the surviving insects at the time of the investigation in untreated group;
Tb: Number of the test insects in treated group;
Tai: Number of the surviving insects at the time of the investigation in treated group;
Here the "untreated group" represents a group where the similar treatment procedure to that of the treated group except not using the test compound is done.

Test Example 1-1

The test was conducted by making the prescribed concentration 500 ppm and using each of the below-mentioned Present compounds as a test compound according to the Test Method 1. As a result of the test, the below-mentioned Present compounds showed 90% or greater as the controlling value.
Present compounds: 1, 2, 4, 5, 6, 7, 8, 9, 10, 11, 12, 13, 14, 15, 19, 22, 23, 29, 31, 32, 33, 34, 35, 36, 38, 39, 52, 54, and 55

Test Method 2

Each test compound is made to a formulation according to a similar method to that described in the Formulation Example 5, and thereto is added water to prepare a diluted solution containing a prescribed concentration of the test compound.

Cucumber (Cucumis sativus) seedling (on the developmental stage of the second true leaf) is planted in a container, and the diluted solutions are drenched to the bottom of the seedling at a ratio of 5 mL/seedling. After 7 days, approximately 30 cotton aphids (Aphis gossypii) (all developmental stages of life) are released onto the leaf of the

seedling. After 6 days, the number of the surviving insects is examined and the controlling value is calculated by the following equation.

Controlling value (%) = {1 - (Cb × Tai) / (Cai × Tb)} × 100

wherein the symbols in the formula represent the following descriptions.

Cb: Number of the test insects in untreated group;
Cai: Number of the surviving insects at the time of the investigation in untreated group;
Tb: Number of the test insects in treated group;
Tai: Number of the surviving insects at the time of the investigation in treated group;
Here the "untreated group" represents a group where the similar treatment procedure to that of the treated group except not using the test compound is done.

Test Example 2-1

The test is conducted by making the prescribed concentration 1000 ppm and using each of the Present compounds as a test compound according to the Test Method 2. As a result of the test, insecticidal effects against cotton aphids (Aphis gossypii) can be confirmed.

Test Method 3

Each test compound is made to a formulation according to a similar method to that described in the Formulation Example 1, and thereto is added water containing 0.03 v/v% of Shindain (registered trademark) to prepare a diluted solution containing a prescribed concentration of the test compound.
Cabbage (Brassicae oleracea) seedling (on the developmental stage of the second to third true leaf) is planted in a container, and the diluted solutions are sprayed to the seedling at a ratio of 20 mL/seedling. Thereafter, the aerial part of the seedling is cut out and then is installed into a container in which a filter paper is placed at the bottom of the container. Five of second-instar larvae of cotton worm (Spodoptera litura) are released into the container. After 5 days, the number of the surviving insects is counted, and the mortality of insects is calculated by the following equation.

Mortality (%) = (1 - Number of surviving insects / 5) × 100

Test Example 3-1

The test was conducted by making the prescribed concentration 500 ppm and using each of the below-mentioned Present compounds as a test compound according to the Test Method 3. As a result of the test, the below-mentioned Present compounds showed 80% or greater as the mortality. Present compounds: 1, 2, 3, 4, 5, 6, 9, 10, 11, 12, 13, 14, 15, 19, 29, 31, 33, and 34

Test Method 4

Each test compound is made to a formulation according to a similar method to that described in the Formulation Example 1, and thereto is added water containing 0.03 v/v% of Shindain (registered trademark) to prepare a diluted solution containing a prescribed concentration of the test compound.
Cabbage (Brassicae oleracea) seedling (on the developmental stage of the second to third true leaf) is planted in a container, and the diluted solutions are sprayed to the seedling at a ratio of 20 mL/seedling. Thereafter, the aerial part of the seedling is cut out and then is installed into a container in which a filter paper is placed at the bottom of the container. Five of second-instar larvae of diamondback moth (Plutella xylostella) are released into the container. After 5 days, the number of the surviving insects is counted, and the mortality of insects is calculated by the following equation.

Mortality (%) = (1 - Number of surviving insects / 5) × 100

Test Example 4-1

The test was conducted by making the prescribed concentration 500 ppm and using each of the below-mentioned Present compounds as a test compound according to the Test Method 4. As a result of the test, the below-mentioned Present compounds showed 80% or greater as the mortality. Present compounds: 1, 2, 3, 4, 5, 6, 8,

9, 11, 12, 13, 15, 22, 27, 33, 34, 39, 43, 47, 52, 54, and 55

Test Method 5

Each 1 mg of the test compounds is dissolved in 50 μL of a mixed solution consisting of 5% of polyoxyethylene sorbitan mono-cocoate and 95% of acetone by volume ratio. Thereto is added water containing 0.03% by volume of Shindain (registered trademark) to prepare a diluted solution containing a prescribed concentration of the test compound.
A young seedling of corn (Zea mays) is immersed into the diluted solution for 30 seconds. Thereafter, two seedlings are installed in a plastic petri dish (90 mm radius), and 10 of second-instar larvae of Western corn rootworm (Diabrotica virgifera virgifera) are released into the dish. After 5 days, the number of the dead insects is counted, and the mortality of insects is calculated by the following equation.

Mortality (%) = (Number of dead insects / 10) × 100

Test Example 5-1

The test was conducted by making the prescribed concentration 200 ppm and using each of the below-mentioned Present compounds as a test compound according to the Test Method 5. As a result of the test, the below-mentioned Present compounds showed 80% or greater as the mortality. Present compounds: 2, 3, 5, 6, 7, 9, 11, and 28

Test Method 6

An acetone solution which is adjusted to 800 ppm of each test compound is poured into a 50 mL glass vial, and the test compound is coated uniformly on inner face of the vial so as to 40 mg/$m^2$ of the test compound treated, and the vial is then dried.
5 German cockroach (Blattella germanica) male adults are released into the treated vial, and the vial is then covered with lid. After the prescribed time, the state of the German cockroach is examined, and the mortality is calculated by the following equation.

Mortality (%) = (Number of dead insects / Number of tested insects) × 100

Test Example 6-1

The test is conducted according to the Test Method 6. As a result of the test, insecticidal effects against German cockroach (Blattella germanica) can be confirmed.

Test Method 7

An acetone solution which is adjusted to 2000 ppm of each test compound is poured into a 20 mL glass vial, and the test compound is coated uniformly on inner face of the vial so as to 100 mg/$m^2$ of the test compound treated, and the vial is then dried.
5 Haemaphysalis longicornis nymphs are released into the treated vial, and the vial is then covered with lid. After the prescribed time, the state of the Haemaphysalis longicornis is examined, and the mortality is calculated by the following equation.

Mortality (%) = (Number of dead insects / Number of tested insects) × 100

Test Example 7-1

The test is conducted according to the Test Method 7. As a result of the test, insecticidal effects against Haemaphysalis longicornis can be confirmed.

Test Method 8

An acetone solution which is adjusted to 800 ppm of each test compound is poured into a 20 mL glass vial, and the test compound is coated uniformly on inner face of the vial so as to 40 mg/$m^2$ of the test compound treated, and the vial is

then dried.

5 housefly (Musca domestica) female adults are released into the treated vial, and the vial is then covered with lid. After the prescribed time, the state of the housefly is examined, and the mortality is calculated by the following equation.

Mortality (%) = (Number of dead insects / Number of tested insects) $\times$ 100

Test Example 8-1

The test was conducted by making the prescribed time 1 day and using each of the below-mentioned Present compounds as a test compound according to the Test Method 8. As a result of the test, the below-mentioned Present compounds showed 80% or greater as the mortality.

Present compound: 1, 2, 3, 11

Test Method 9

Each test compound is made to a formulation according to a similar method to that described in the Formulation Example 5, and thereto is added water containing 0.03 v/v% of Shindain (registered trademark) to prepare a diluted solution containing a prescribed concentration of the test compound.

Cabbage (Brassicae oleracea) seedling (on the developmental stage of the third to fourth true leaf) is planted in a container, and the diluted solutions are sprayed to the seedling at a ratio of 20 mL/seedling. Thereafter, ten of third-instar larvae of cotton worm (Spodoptera litura) are released. After 6 days, the number of the surviving insects is counted, and the mortality of insects is calculated by the following equation.

Mortality (%) = (1 - Number of surviving insects / 10) $\times$ 100

Test Example 9-1

The test is conducted by making the prescribed concentration 200 ppm and using each of the Present compounds as a test compound according to the Test Method 9. As a result of the test, insecticidal effects against cotton worm (Spodoptera litura) can be confirmed.

Test Example 9-2

The test is conducted by making the prescribed concentration 50 ppm and using each of the Present compounds as a test compound according to the Test Method 9. As a result of the test, insecticidal effects against cotton worm (Spodoptera litura) can be confirmed.

Test Method 10

Each test compound is made to a formulation according to a similar method to that described in the Formulation Example 5, and thereto is added water containing 0.03 v/v% of Shindain (registered trademark) to prepare a diluted solution containing a prescribed concentration of the test compound.

Cabbage (Brassicae oleracea) seedling (on the developmental stage of the third to fourth true leaf) is planted in a container, and the diluted solutions are sprayed to the seedling at a ratio of 20 mL/seedling. Thereafter, ten of third-instar larvae of diamondback moth (Plutella xylostella) are released. After 6 days, the number of the surviving insects is counted, and the mortality of insects is calculated by the following equation.

Mortality (%) = (1 - Number of surviving insects / 10) $\times$ 100

Test Example 10-1

The test is conducted by making the prescribed concentration 200 ppm and using each of the Present compounds as a test compound according to the Test Method 10. As a result of the test, insecticidal effects against diamondback moth (Plutella xylostella) can be confirmed.

Test Example 10-2

The test is conducted by making the prescribed concentration 50 ppm and using each of the Present compounds as a test compound according to the Test Method 10. As a result of the test, insecticidal effects against diamondback moth (Plutella xylostella) can be confirmed.

Test Method 11

Each test compound is made to a formulation according to a similar method to that described in the Formulation Example 5, and thereto is added water containing 0.03 v/v% of Shindain (registered trademark) to prepare a diluted solution containing a prescribed concentration of the test compound.
Cucumber (Cucumis sativus) seedling (on the developmental stage of the second true leaf) is planted in a container and approximately 30 cotton aphids (Aphis gossypii) (all developmental stages of life) are released onto the leaves of the cucumber. After 1 day, the diluted solutions are sprayed to the seedling at a ratio of 10 mL/seedling. Further, after 5 days, the number of the surviving insects is examined and the controlling value is calculated by the following equation.

Controlling value (%) = $\{1 - (Cb \times Tai) / (Cai \times Tb)\} \times 100$

wherein the symbols in the formula represent the following descriptions.

Cb: Number of the test insects in untreated group;
Cai: Number of the surviving insects at the time of the investigation in untreated group;
Tb: Number of the test insects in treated group;
Tai: Number of the surviving insects at the time of the investigation in treated group;
Here the "untreated group" represents a group where the similar treatment procedure to that of the treated group except not using the test compound is done.

Test Example 11-1

The test was conducted by making the prescribed concentration 200 ppm and using each of the below-mentioned Present compounds as a test compound according to the Test Method 11. As a result of the test, the below-mentioned Present compounds showed 90% or greater as the controlling value.
Present compounds: 1, 2, 3, 4, 5, 6, 7, 8, 9, 10, 11, 16, 17, 19, 20, 21, 28, 36, 38, 39, 42, 43, 46, 54, 55

Test Example 11-2

The test was conducted by making the prescribed concentration 50 ppm and using the below-mentioned Present compound as a test compound according to the Test Method 11. As a result of the test, the below-mentioned Present compound showed 90% or greater as the controlling value. Present compound: 1

Test Method 12

Each test compound is formulated to a test formulation according to a similar method to that described in the Formulation Example 1, and water is added in preparing for a diluted solution containing a prescribed concentration of the test compound.
Into the diluted solution, 30 last instar larvae of common house mosquito (Culex pipiens pallens) are released, and after 1 day, the state of the house mosquito larvae is examined, and the mortality is calculated by the following equation.

Mortality (%) = (Number of dead insects / Number of tested insects) $\times$ 100

Test Example 12-1

The test was conducted by making the prescribed concentration 3.5 ppm and using each of the below-mentioned Present compounds as a test compound according to the Test Method 12. As a result of the test, the below-mentioned Present compounds showed greater than 90% as the mortality. Present compounds: 1, 2, 3, 5, 6, 7, 10, 12, 13, 14, 15, 19, 22, 23, 27, 29, 31, 32, 33, 34, 38, 39, 43, 52, and 55

Test Method 13

Each 1 mg of the Present compounds is dissolved in 10 μL of a mixed solution consisting of four ninths of xylene, four ninths of dimethylformamide, and one ninth of surfactant by volume ratio. Thereto is added water containing 0.02% by volume of a spreader to prepare diluted solution A containing a prescribed concentration of the Present compound.
Each 1 mg of the Present ingredients is dissolved in 10 μL of a mixed solution consisting of four ninths of xylene, four ninths of dimethylformamide, and one ninth of surfactant by volume ratio. Thereto is added water containing 0.02% by volume of a spreader to prepare diluted solution B containing a prescribed concentration of the Present ingredient.
The diluted solution A is mixed with the diluted solution B to prepare diluted solution C.
A leaf disc of cotyledon of cucumber (Cucumis sativus) (1.5 cm in length) is cut out and installed in each well of a 24-well microplate, and 2 apterous adults and 8 nymphs of cotton aphid (Aphis gossypii) are release onto the leaf disc in each well. The diluted solution C is sprayed to each leaf disc at the ratio of 20 μL / leaf disc. The procedure mentioned above represents the treated group.
Whereas, the untreated group represents a group where the similar treatment procedure to that of the treated group is done except 20 μL of water containing 0.02% by volume of a spreader is used instead of using the diluted solution C.
After the sprayed diluted solution C is dried, the microplate is covered with a film sheet. After 5 days, the number of the surviving insects in each well is examined and the controlling value is calculated by the following equation.

$$\text{Controlling value } (\%) = \{1 - (\text{Tai})/(\text{Cai})\} \times 100$$

wherein the symbols in the formula represent the following descriptions.

Cai: Number of the surviving insects at the time of the investigation in untreated group;
Tai: Number of the surviving insects at the time of the investigation in treated group.

Specific diluted solutions C, which can confirm their effects according to the Test Method 13, are described in the following 1) to 5).
1) The diluted solution C comprises the combination recited in List A wherein a concentration of the Present compound is 200 ppm and a concentration of the Present ingredient is 2000 ppm. In List A, Comp X represents any one compound selected from the Compound groups SX1 to SX1840. List A:

Comp X + Clothianidin; Comp X + thiamethoxam; Comp X + imidacloprid; Comp X + thiacloprid; Comp X + flupyradifurone;
Comp X + sulfoxaflor; Comp X + triflumezopyrim; Comp X + dicloromezotiaz; Comp X + beta-cyfluthrin; Comp X + tefluthrin; Comp X + fipronil; Comp X + chlorantraniliprole;
Comp X + cyantraniliprole; Comp X + tetraniliprole; Comp X + thiodicarb; Comp X + carbofuran; Comp X + fluxametamide;
Comp X + afoxolaner; Comp X + fluralaner; Comp X + broflanilide; Comp X + abamectin; Comp X + fluopyram; Comp X + fluensulfone; Comp X + fluazaindolizine; Comp X + tioxazafen; Comp X + flupyrimin; Comp X + Mycorrhizal Fungi;
Comp X + Bradyrhizobium japonicum TA-11; Comp X + Bacillus firmus; Comp X + Bacillus firmus I-1582; Comp X + Bacillus amyloliquefaciens; Comp X + Bacillus amyloliquefaciens FZB42; Comp X + Bacillus amyloliquefaciens PTA4838; Comp X + Pasteuria nishizawae; Comp X + Pasteuria nishizawae Pn1;
Comp X + Pasteuria penetrans; Comp X + tebuconazole; Comp X + prothioconazole; Comp X + metconazole; Comp X + ipconazole;
Comp X + triticonazole; Comp X + difenoconazole; Comp X + imazalil; Comp X + triadimenol; Comp X + tetraconazole; Comp X + flutriafol; Comp X + mandestrobin; Comp X + azoxystrobin;
Comp X + pyraclostrobin; Comp X + trifloxystrobin; Comp X + fluoxastrobin; Comp X + picoxystrobin; Comp X + fenamidone;
Comp X + metalaxyl; Comp X + metalaxyl-M; Comp X + fludioxonil; Comp X + sedaxane; Comp X + penflufen; Comp X + fluxapyroxad; Comp X + benzovindiflupyr; Comp X + boscalid;
Comp X + carboxin; Comp X + penthiopyrad; Comp X + flutolanil; Comp X + captan; Comp X + thiram; Comp X + tolclofos-methyl; Comp X + thiabendazole; Comp X + ethaboxam;
Comp X + mancozeb; Comp X + picarbutrazox; Comp X + oxathiapiprolin; Comp X + silthiofam; Comp X + inpyrfluxam.

2) The diluted solution C comprises the combination recited in List A wherein a concentration of the Present compound is 200 ppm and a concentration of the Present ingredient is 200 ppm.
3) The diluted solution C comprises the combination recited in List A wherein a concentration of the Present compound is 500 ppm and a concentration of the Present ingredient is 50 ppm.
4) The diluted solution C comprises the combination recited in List A wherein a concentration of the Present compound is 500 ppm and a concentration of the Present ingredient is 5 ppm.
5) The diluted solution C comprises the combination recited in List A wherein a concentration of the Present compound is 500 ppm and a concentration of the Present ingredient is 0.5 ppm.

INDUSTRIAL APPLICABILITY

The Present compounds have excellent control effects on harmful arthropods.

## Claims

**1.** A compound represented by formula (I)

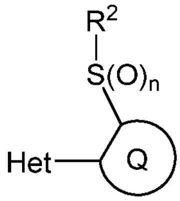

( I )

[wherein:

Q represented by the following formula

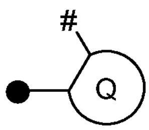

(wherein # represents the binding site to the sulfur atom, and • represents the binding site to Het) represents a group represented by formula Q1 or a group represented by formula Q2;

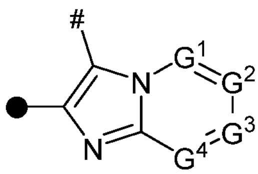

Q1

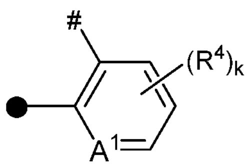

Q2

$A^1$ represents a nitrogen atom or $CR^8$;
$R^8$ represents a halogen atom or a hydrogen atom;
$R^2$ represents a C1-C6 alkyl group optionally substituted with one or more halogen atom(s), a cyclopropyl group, or a cyclopropylmethyl group;
n represents 0, 1, or 2;
$G^1$ represents a nitrogen atom or $CR^{32}$;
$G^2$ represents a nitrogen atom or $CR^{3\ b}$;
$G^3$ represents a nitrogen atom or $CR^{3\ c}$;
$G^4$ represents a nitrogen atom or $CR^{3\ d}$;
$R^{3\ a}$, $R^{3\ b}$, $R^{3\ c}$, and $R^{3\ d}$ are identical to or different from each other, and each represent a C1-C6 chain hydrocarbon group optionally substituted with one or more substituent(s) selected from Group L, a C3-C7 cycloalkyl group optionally substituted with one or more substituent(s) selected from Group E, a phenyl group

optionally substituted with one or more substituent(s) selected from Group H, a 5 or 6 membered aromatic heterocyclic group optionally substituted with one or more substituent(s) selected from Group H, $OR^{12}$, $NR^{11}R^{12}$, $NR^{11a}R^{12a}$, $NR^{24}NR^{11}R^{12}$, $NR^{24}OR^{11}$, $NR^{11}C(O)R^{13}$, $NR^{24}NR^{11}C(O)R^{13}$, $NR^{11}C(O)OR^{14}$, $NR^{24}NR^{11}C(O)OR^{14}$, $NR^{11}C(O)NR^{31}R^{32}$, $NR^{24}NR^{11}C(O)NR^{31}R^{32}$, $N=CHNR^{31}R^{32}$, $N=S(O)_pR^{15}R^{16}$, $C(O)R^{13}$, $C(O)OR^{17}$, $C(O)NR^{31}R^{32}$, $C(O)NR^{11}S(O)_2R^{23}$, $CR^{30}=NOR^{17}$, $NR^{11}CR^{24}=NOR^{17}$, $S(O)_mR^{23}$, a cyano group, a nitro group, a halogen atom, or a hydrogen atom;

p represents 0 or 1;

m represents 0, 1, or 2;

$R^{30}$ represents a C1-C6 chain hydrocarbon group optionally substituted with one or more halogen atom(s), a halogen atom, $OR^{35}$, $NR^{36}R^{37}$, or a hydrogen atom;

$R^{35}$ represents a C1-C6 chain hydrocarbon group optionally substituted with one or more halogen atom(s);

$R^{17}$ represents a C1-C6 chain hydrocarbon group optionally substituted with one or more halogen atom(s), a phenyl group optionally substituted with one or more substituent(s) selected from Group D, or a hydrogen atom;

$R^{11}$, $R^{24}$, $R^{36}$, and $R^{37}$ are identical to or different from each other, and each represent a C1-C6 chain hydrocarbon group optionally substituted with one or more halogen atom(s), or a hydrogen atom;

$R^{12}$ represents a C1-C6 chain hydrocarbon group optionally substituted with one or more substituent(s) selected from Group F, a C3-C7 cycloalkyl group optionally substituted with one or more substituent(s) selected from Group J, a C3-C7 cycloalkenyl group optionally substituted with one or more substituent(s) selected from Group J, a phenyl group optionally substituted with one or more substituent(s) selected from Group D, a 6 membered aromatic heterocyclic group optionally substituted with one or more substituent(s) selected from Group D, $S(O)_2R^{23}$, or a hydrogen atom;

$R^{23}$ represents a C1-C6 chain hydrocarbon group optionally substituted with one or more halogen atom(s), or a phenyl group optionally substituted with one or more substituent(s) selected from Group D;

$R^{11a}$ and $R^{12a}$ are combined with the nitrogen atom to which they are attached to form a 3-7 membered nonaromatic heterocyclic group optionally substituted with one or more substituent(s) selected from Group E;

$R^{13}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), a phenyl group optionally substituted with one or more substituent(s) selected from Group D, a 5 or 6 membered aromatic heterocyclic group optionally substituted with one or more substituent(s) selected from Group D, or a hydrogen atom;

$R^{14}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), or a phenyl C1-C3 alkyl group {wherein the phenyl moiety in said phenyl C1-C3 alkyl group is optionally substituted with one or more substituent(s) selected from Group D};

$R^{15}$ and $R^{16}$ are identical to or different from each other, and each represent a C1-C6 alkyl group optionally substituted with one or more halogen atom(s);

$R^{31}$ represents a C1-C6 alkyl group optionally substituted with one or more halogen atom(s), or a hydrogen atom;

$R^{32}$ represents a C1-C6 chain hydrocarbon group optionally substituted with one or more substituent(s) selected from Group F, a C3-C7 cycloalkyl group optionally substituted with one or more substituent(s) selected from Group J, $S(O)_2R^{23}$, or a hydrogen atom;

$R^4$ represents a C1-C6 chain hydrocarbon group optionally substituted with one or more substituent(s) selected from Group $L^2$, a C3-C7 cycloalkyl group optionally substituted with one or more substituent(s) selected from Group $E^2$, a phenyl group optionally substituted with one or more substituent(s) selected from Group $H^2$, a 5 or 6 membered aromatic heterocyclic group optionally substituted with one or more substituent(s) selected from Group $H^2$, $OR^{42}$, $NR^{41}R^{42}$, $NR^{41a}R^{42a}$, $NR^{54}NR^{41}R^{42}$, $NR^{54}OR^{41}$, $NR^{41}C(O)R^{43}$, $NR^{54}NR^{41}C(O)R^{43}$, $NR^{41}C(O)OR^{44}$, $NR^{54}NR^{41}C(O)OR^{4\ 4}$, $NR^{41}C(O)NR^{61}R^{62}$, $NR^{54}NR^{41}C(O)NR^{61}R^{62}$, $N=CHNR^{61}R^{62}$, $N=S(O)_rR^{45}R^{46}$, $C(O)R^{43}$, $C(O)OR^{47}$, $C(O)NR^{61}R^{62}$, $C(O)NR^{41}S(O)_2R^{53}$, $CR^{60}=NOR^{47}$, $NR^{41}CR^{54}=NOR^{47}$, $S(O)_tR^{53}$, a cyano group, a nitro group, or a halogen atom;

r represents 0 or 1;

t represents 0, 1, or 2;

$R^{60}$ represents a C1-C6 chain hydrocarbon group optionally substituted with one or more halogen atom(s), a halogen atom, $OR^{65}$, $NR^{66}R^{67}$, or a hydrogen atom;

$R^{65}$ represents a C1-C6 chain hydrocarbon group optionally substituted with one or more halogen atom(s);

$R^{47}$ represents a C1-C6 chain hydrocarbon group optionally substituted with one or more halogen atom(s), a phenyl group optionally substituted with one or more substituent(s) selected from Group $D^2$, or a hydrogen atom;

$R^{41}$, $R^{54}$, $R^{66}$, and $R^{67}$ are identical to or different from each other, and each represent a C1-C6 chain hydrocarbon group optionally substituted with one or more halogen atom(s), or a hydrogen atom;

$R^{42}$ represents a C1-C6 chain hydrocarbon group optionally substituted with one or more substituent(s) selected from Group $F^2$, a C3-C7 cycloalkyl group optionally substituted with one or more substituent(s) selected from Group $J^2$, a C3-C7 cycloalkenyl group optionally substituted with one or more substituent(s) selected from Group $J^2$, a phenyl group optionally substituted with one or more substituent(s) selected from Group $D^2$, a 6 membered aromatic heterocyclic group optionally substituted with one or more substituent(s) selected from Group $D^2$, a hydrogen atom, or $S(O)_2R^{53}$;
$R^{53}$ represents a C1-C6 chain hydrocarbon group optionally substituted with one or more halogen atom(s), or a phenyl group optionally substituted with one or more substituent(s) selected from Group $D^2$;
$R^{41a}$ and $R^{12a}$ are combined with the nitrogen atom to which they are attached to form a 3-7 membered nonaromatic heterocyclic group optionally substituted with one or more substituent(s) selected from Group $E^2$;
$R^{43}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), a phenyl group optionally substituted with one or more substituent(s) selected from Group $D^2$, a 5 or 6 membered aromatic heterocyclic group optionally substituted with one or more substituent(s) selected from Group $D^2$, or a hydrogen atom;
$R^{44}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), or a phenyl C1-C3 alkyl group {wherein the phenyl moiety in said phenyl C1-C3 alkyl group is optionally substituted with one or more substituent(s) selected from Group $D^2$};
$R^{45}$ and $R^{46}$ are identical to or different from each other, and each represent a C1-C6 alkyl group optionally substituted with one or more halogen atom(s);
$R^{61}$ represents a C1-C6 alkyl group optionally substituted with one or more halogen atom(s), or a hydrogen atom;
$R^{62}$ represents a C1-C6 chain hydrocarbon group optionally substituted with one or more substituent(s) selected from Group $F^2$, a C3-C7 cycloalkyl group optionally substituted with one or more substituent(s) selected from Group $J^2$, $S(O)_2R^{53}$, or a hydrogen atom;
k represents 0, 1, 2, or 3, wherein when k represents 2 or 3, then two or three $R^4$ are identical to or different from each other;
when two $R^4$ are bound to adjacent carbon atoms, then said two $R^4$ are optionally combined with two carbon atoms to which they are attached to form a benzene ring, a pyrrole ring, a furan ring, a thiophene ring, a pyrazole ring, an imidazole ring, an oxazole ring, an isoxazole ring, a thiazole ring, an oxadiazole ring, a thiadiazole ring, a pyridine ring, a pyridazine ring, a pyrimidine ring, or a pyrazine ring {wherein said benzene ring, said pyrrole ring, said furan ring, said thiophene ring, said pyrazole ring, said imidazole ring, said oxazole ring, said isoxazole ring, said thiazole ring, said pyridine ring, said pyridazine ring, said pyrimidine ring, and said pyrazine ring are optionally substituted with one or more substituent(s) selected from Group $H^2$}, or a triazole ring optionally substituted with one or more substituent(s) selected from Group P;
Het represents a group represented by formula Het1 or a group represented by formula Het2;

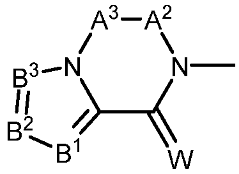
Het1

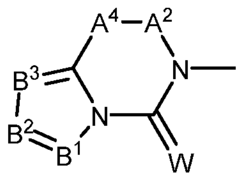
Het2

$A^2$ represents $-NR^{5a}-$ or $-CR^{7a}R^{7b}-$;
$A^3$ represents $-CR^{7c}R^{7d}-$, $-CR^{7e}R^{7f}-CR^{7g}R^{7h}-{*}^1$ , or - $CR^{7i}R^{7j}-CR^{7k}R^{7l}-CR^{7m}R^{7n}-{*}^1$ ;
${*}^1$ represents the binding position to $A^2$;
$A^4$ represents $-NR^{5b}-$, $-CR^{7o}R^{7p}-$, $-CR^{7q}R^{7r}-CR^{7s}R^{7t}-{*}^2$, $-NR^{5c}-CR^{7u}R^{7v}-{*}^2$, $-CR^{7w}R^{7x}-CR^{7y}R^{7z}-CR^{7aa}R^{7bb}-{*}^2$, or $-NR^{5d}-CR^{7cc}R^{7dd}-CR^{7ee}R^{7ff}-{*}^2$;
${*}^2$ represents the binding position to $A^2$;
$R^{5a}$, $R^{5b}$, $R^{5c}$, and $R^{5d}$ are identical to or different from each other, and each represent a C1-C6 chain hydrocarbon group optionally substituted with one or more substituent(s) selected from Group K, a C1-C6 alkoxy group optionally substituted with one or more halogen atom(s), a C3-C7 cycloalkyl group optionally substituted with one or more halogen atom(s), a (C3-C7 cycloalkyl) C1-C6 alkyl group optionally substituted with one or more halogen atom(s), or a hydrogen atom;
$R^{7a}$, $R^{7b}$, $R^{7c}$, $R^{7d}$, $R^{7e}$, $R^{7f}$, $R^{7g}$, $R^{7h}$, $R^{7i}$, $R^{7j}$, $R^{7k}$, $R^{7l}$, $R^{7m}$, $R^{7n}$, $R^{7o}$, $R^{7p}$, $R^{7q}$, $R^{7r}$, $R^{7s}$, $R^{7t}$, $R^{7u}$, $R^{7v}$, $R^{7w}$, $R^{7x}$,

$R^{7y}$, $R^{7z}$, $R^{7aa}$, $R^{7bb}$, $R^{7cc}$, $R^{7dd}$, $R^{7ee}$, and $R^{7ff}$ are identical to or different from each other, and each represent a C1-C6 chain hydrocarbon group optionally substituted with one or more substituent(s) selected from the group consisting of a cyano group and a halogen atom, a C3-C4 cycloalkyl group optionally substituted with one or more substituent(s) selected from the group consisting of a cyano group and a halogen atom, a nitro group, $OR^{69}$, $NR^{69}R^{70}$, a cyano group, a halogen atom, or a hydrogen atom;

$R^{7a}$ and $R^{7b}$, $R^{7c}$ and $R^{7d}$, $R^{7e}$ and $R^{7f}$, $R^{7g}$ and $R^{7h}$, $R^{7i}$ and $R^{7j}$, $R^{7k}$ and $R^{7l}$, $R^{7m}$ and $R^{7n}$, $R^{7o}$ and $R^{7p}$, $R^{7q}$ and $R^{7r}$, $R^{7s}$ and $R^{7t}$, $R^{7u}$ and $R^{7v}$, $R^{7w}$ and $R^{7x}$, $R^{7y}$ and $R^{7z}$, $R^{7aa}$ and $R^{7bb}$, $R^{7cc}$ and $R^{7dd}$, and $R^{7ee}$ and $R^{7ff}$ are each optionally combined with the carbon atom to which they are attached to form a C3-C7 cycloalkyl group optionally substituted with one or more substituent(s) selected from the group consisting of a cyano group and a halogen atom;

$R^{69}$ represents a C1-C6 chain hydrocarbon group optionally substituted with one or more substituent(s) selected from Group T, a C3-C7 cycloalkyl group optionally substituted with one or more substituent(s) selected from Group U, a C3-C7 cycloalkenyl group optionally substituted with one or more substituent(s) selected from Group U, a phenyl group optionally substituted with one or more substituent(s) selected from Group V, a 6 membered aromatic heterocyclic group optionally substituted with one or more substituent(s) selected from Group V, or a hydrogen atom;

$R^{70}$ represents a C1-C6 chain hydrocarbon group optionally substituted with one or more halogen atom(s), or a hydrogen atom;

W represents an oxygen atom or a sulfur atom;

$B^1$ represents $CR^{6a}$ or a nitrogen atom;

$B^2$ represents $CR^{6b}$ or a nitrogen atom;

$B^3$ represents $CR^{6c}$ or a nitrogen atom;

$R^{6a}$, $R^{6b}$, and $R^{6c}$ are identical to or different from each other, and each represent a C1-C6 chain hydrocarbon group optionally substituted with one or more substituent(s) selected from Group $L^3$, a C3-C7 cycloalkyl group optionally substituted with one or more substituent(s) selected from Group $E^3$, a phenyl group optionally substituted with one or more substituent(s) selected from Group $H^3$, a 5 or 6 membered aromatic heterocyclic group optionally substituted with one or more substituent(s) selected from Group $H^3$, $OR^{72}$, $NR^{71}R^{72}$, $NR^{71a}R^{72a}$, $NR^{84}NR^{71}R^{72}$, $NR^{84}OR^{71}$, $NR^{71}C(O)R^{73}$, $NR^{84}NR^{71}C(O)R^{73}$, $NR^{71}C(O)OR^{74}$, $NR^{84}NR^{71}C(O)OR^{74}$, $NR^{71}C(O)NR^{91}R^{92}$, $NR^{84}NR^{71}C(O)NR^{91}R^{92}$, $N=CHNR^{91}R^{92}$, $N=S(O)_qR^{75}R^{76}$, $C(O)R^{73}$, $C(O)OR^{77}$, $C(O)NR^{91}R^{92}$, $C(O)NR^{71}S(O)_2R^{83}$, $CR^{90}=NOR^{77}$, $NR^{71}CR^{84}=NOR^{77}$, $S(O)_vR^{83}$, a cyano group, a nitro group, a halogen atom, or a hydrogen atom;

q represents 0 or 1;

v represents 0, 1, or 2;

$R^{90}$ represents a C1-C6 chain hydrocarbon group optionally substituted with one or more halogen atom(s), a halogen atom, $OR^{95}$, $NR^{96}R^{97}$, or a hydrogen atom;

$R^{95}$ represents a C1-C6 chain hydrocarbon group optionally substituted with one or more halogen atom(s);

$R^{77}$ represents a C1-C6 chain hydrocarbon group optionally substituted with one or more halogen atom(s), a phenyl group optionally substituted with one or more substituent(s) selected from Group $D^3$, or a hydrogen atom;

$R^{7l}$, $R^{84}$, $R^{96}$, and $R^{97}$ are identical to or different from each other, and each represent a C1-C6 chain hydrocarbon group optionally substituted with one or more halogen atom(s), or a hydrogen atom;

$R^{72}$ represents a C1-C6 chain hydrocarbon group optionally substituted with one or more substituent(s) selected from Group $F^3$, a C3-C7 cycloalkyl group optionally substituted with one or more substituent(s) selected from Group $J^3$, a C3-C7 cycloalkenyl group optionally substituted with one or more substituent(s) selected from Group $J^3$, a phenyl group optionally substituted with one or more substituent(s) selected from Group $D^3$, a 6 membered aromatic heterocyclic group optionally substituted with one or more substituent(s) selected from Group $D^3$, $S(O)_2R^{83}$, or a hydrogen atom;

$R^{83}$ represents a C1-C6 chain hydrocarbon group optionally substituted with one or more halogen atom(s), or a phenyl group optionally substituted with one or more substituent(s) selected from Group $D^3$;

$R^{71a}$ and $R^{72a}$ are combined with the nitrogen atom to which they are attached to form a 3-7 membered nonaromatic heterocyclic group optionally substituted with one or more substituent(s) selected from Group $E^3$;

$R^{73}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), a phenyl group optionally substituted with one or more substituent(s) selected from Group $D^3$, a 5 or 6 membered aromatic heterocyclic group optionally substituted with one or more substituent(s) selected from Group $D^3$, or a hydrogen atom;

$R^{74}$ represents a C1-C6 chain hydrocarbon group optionally substituted with one or more halogen atom(s), a C3-C7 cycloalkyl group optionally substituted with one or more halogen atom(s), a (C3-C6 cycloalkyl) C1-C3 alkyl group optionally substituted with one or more halogen atom(s), or a phenyl C1-C3 alkyl group {wherein the phenyl

moiety in said phenyl C1-C3 alkyl group is optionally substituted with one or more substituent(s) selected from Group $D^3$};

$R^{75}$ and $R^{76}$ are identical to or different from each other, and each represent a C1-C6 alkyl group optionally substituted with one or more halogen atom(s);

$R^{91}$ represents a C1-C6 alkyl group optionally substituted with one or more halogen atom(s), or a hydrogen atom; and

$R^{92}$ represents a C1-C6 chain hydrocarbon group optionally substituted with one or more substituent(s) selected from Group $F^3$, a C3-C7 cycloalkyl group optionally substituted with one or more substituent(s) selected from Group $J^3$, $S(O)_2R^{83}$, or a hydrogen atom;

Group L: a group consisting of a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C3-C6 cycloalkyl group optionally substituted with one or more halogen atom(s), a cyano group, a hydroxy group, and a halogen atom;

Group M: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), and a halogen atom;

Group D: a group consisting of a C1-C6 chain hydrocarbon group optionally substituted with one or more halogen atom(s), a hydroxy group, a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a sulfanyl group, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), an amino group, $NHR^{21}$, $NR^{21}R^{22}$, $C(O)R^{21}$, $OC(O)R^{21}$, $C(O)OR^{21}$, a cyano group, a nitro group, and a halogen atom;

wherein $R^{21}$ and $R^{22}$ are identical to or different from each other, and each represent a C1-C6 alkyl group optionally substituted with one or more halogen atom(s);

Group E: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a halogen atom, an oxo group, a hydroxy group, a cyano group, and a nitro group;

Group F: a group consisting of a C1-C6 alkoxy group optionally substituted with one or more halogen atom(s), a phenyl group optionally substituted with one or more substituent(s) selected from Group D, a 5 or 6 membered aromatic heterocyclic group optionally substituted with one or more substituent(s) selected from Group D, a C3-C7 cycloalkyl group optionally substituted with one or more halogen atom(s), a 3-7 membered nonaromatic heterocyclic group optionally substituted with one or more substituent(s) selected from Group M, an amino group, $NHR^{21}$, $NR^{21}R^{22}$, a halogen atom, and a cyano group;

Group H: a group consisting of a C1-C6 alkyl group optionally substituted with one or more halogen atom(s), $OR^{10}$, $NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, an amino group, and a 5 or 6 membered aromatic heterocyclic group optionally substituted with one or more substituent(s) selected from Group W;

wherein $R^9$ represents a C1-C6 alkyl group optionally substituted with one or more halogen atom(s), or a C3-C6 cycloalkyl group optionally substituted with one or more halogen atom(s); and

$R^{10}$ represents a C1-C6 alkyl group optionally substituted with one or more halogen atom(s), a C3-C6 cycloalkyl group optionally substituted with one or more halogen atom(s), or a hydrogen atom;

Group P: a group consisting of a C2-C6 chain hydrocarbon group optionally substituted with one or more halogen atom(s), a C3-C6 cycloalkyl group optionally substituted with one or more halogen atom(s), a phenyl group optionally substituted with one or more substituent(s) selected from Group $D^2$, a 5 or 6 membered aromatic heterocyclic group optionally substituted with one or more substituent(s) selected from Group $D^2$, a C2-C6 alkylcarbonyl group optionally substituted with one or more halogen atom(s), a C2-C6 alkoxycarbonyl group optionally substituted with one or more halogen atom(s), an aminocarbonyl group, a (C1-C6 alkyl)aminocarbonyl group optionally substituted with one or more halogen atom(s), a methyl group, and a di(C1-C4 alkyl)aminocarbonyl group optionally substituted with one or more halogen atom(s);

Group J: a group consisting of a C1-C6 alkyl group optionally substituted with one or more halogen atom(s), a halogen atom, and a cyano group;

Group K: a group consisting of a C1-C6 alkoxy group optionally substituted with one or more halogen atom(s), a phenyl group optionally substituted with one or more substituent(s) selected from Group D, a 5 or 6 membered aromatic heterocyclic group optionally substituted with one or more substituent(s) selected from Group D, a C3-C7 cycloalkyl group optionally substituted with one or more halogen atom(s), and a 3-7 membered nonaromatic heterocyclic group optionally substituted with one or more substituent(s) selected from Group M;

Group T: a group consisting of a C1-C6 alkoxy group optionally substituted with one or more halogen atom(s), a phenyl group optionally substituted with one or more substituent(s) selected from Group D, a 5 or 6 membered aromatic heterocyclic group optionally substituted with one or more substituent(s) selected from Group D, a C3-C7 cycloalkyl group optionally substituted with one or more halogen atom(s), a 3-7 membered nonaromatic heterocyclic group optionally substituted with one or more substituent(s) selected from Group M, an amino group, $NHR^{21}$, $NR^{21}R^{22}$, a halogen atom, and a cyano group;

Group U: a group consisting of a C1-C6 alkyl group optionally substituted with one or more halogen atom(s), a halogen atom, and a cyano group;

Group V: a group consisting of a C1-C6 chain hydrocarbon group optionally substituted with one or more halogen atom(s), a hydroxy group, a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a sulfanyl group, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), an amino group, $NHR^{21}$, $NR^{21}R^{22}$, $C(O)R^{21}$, $OC(O)R^{21}$, $C(O)OR^{21}$, a cyano group, a nitro group, and a halogen atom;

Group W: a group consisting of a C1-C6 chain hydrocarbon group optionally substituted with one or more halogen atom(s), a hydroxy group, a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a sulfanyl group, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), an amino group, $NHR^{21}$, $NR^{21}R^{22}$, $C(O)R^{21}$, $OC(O)R^{21}$, $C(O)OR^{21}$, a cyano group, a nitro group, and a halogen atom;

Group $L^2$: a group consisting of a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C3-C6 cycloalkyl group optionally substituted with one or more halogen atom(s), a cyano group, a hydroxy group, and a halogen atom;

Group $D^2$: a group consisting of a C1-C6 chain hydrocarbon group optionally substituted with one or more halogen atom(s), a hydroxy group, a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a sulfanyl group, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), an amino group, $NHR^{21}$, $NR^{21}R^{22}$, $C(O)R^{21}$, $OC(O)R^{21}$, $C(O)OR^{21}$, a cyano group, a nitro group, and a halogen atom;

Group $E^2$: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a halogen atom, an oxo group, a hydroxy group, a cyano group, and a nitro group;

Group $F^2$: a group consisting of a C1-C6 alkoxy group optionally substituted with one or more halogen atom(s), a phenyl group optionally substituted with one or more substituent(s) selected from Group D, a 5 or 6 membered aromatic heterocyclic group optionally substituted with one or more substituent(s) selected from Group D, a C3-C7 cycloalkyl group optionally substituted with one or more halogen atom(s), a 3-7 membered nonaromatic heterocyclic group optionally substituted with one or more substituent(s) selected from Group M, an amino group, $NHR^{21}$, $NR^{21}R^{22}$, a halogen atom, and a cyano group;

Group $H^2$: a group consisting of a C1-C6 alkyl group optionally substituted with one or more halogen atom(s), $OR^{10}$, $NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, an amino group, and a 5 or 6 membered aromatic heterocyclic group optionally substituted with one or more substituent(s) selected from Group W;

Group $J^2$: a group consisting of a C1-C6 alkyl group optionally substituted with one or more halogen atom(s), a halogen atom, and a cyano group;

Group $L^3$: a group consisting of a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C3-C6 cycloalkyl group optionally substituted with one or more halogen atom(s), a cyano group, a hydroxy group, and a halogen atom;

Group $D^3$: a group consisting of a C1-C6 chain hydrocarbon group optionally substituted with one or more halogen atom(s), a hydroxy group, a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a sulfanyl group, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), an amino group, $NHR^{21}$, $NR^{21}R^{22}$, $C(O)R^{21}$, $OC(O)R^{21}$, $C(O)OR^{21}$, a cyano group, a nitro group, and a halogen atom;

Group $E^3$: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a halogen atom, an oxo group, a hydroxy group, a cyano group, and a nitro group;

Group $F^3$: a group consisting of a C1-C6 alkoxy group optionally substituted with one or more halogen atom(s), a phenyl group optionally substituted with one or more substituent(s) selected from Group D, a 5 or 6 membered aromatic heterocyclic group optionally substituted with one or more substituent(s) selected from Group D, a C3-C7 cycloalkyl group optionally substituted with one or more halogen atom(s), a 3-7 membered nonaromatic heterocyclic group optionally substituted with one or more substituent(s) selected from Group M, an amino group, $NHR^{21}$, $NR^{21}R^{22}$, a halogen atom, and a cyano group;

Group $H^3$: a group consisting of a C1-C6 alkyl group optionally substituted with one or more halogen atom(s), $OR^{10}$, $NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, an amino group, and a 5 or 6 membered aromatic heterocyclic group optionally substituted with one or more substituent(s) selected from Group W;

Group $J^3$: a group consisting of a C1-C6 alkyl group optionally substituted with one or more halogen atom(s), a halogen atom, and a cyano group]

or an N-oxide thereof.

**2.** The compound or an N-oxide thereof according to claim 1, wherein Q represents the group represented by formula Q1.

**3.** The compound or an N-oxide thereof according to claim 1, wherein Q represents the group represented by formula Q2.

**4.** The compound or an N-oxide thereof according to claim 1, wherein

Q represents the group represented by formula Q1;
$G^1$ represents $CR^{3a}$;
$G^2$ represents $CR^{3b}$;
$G^3$ represents $CR^{3c}$; and
$G^4$ represents a nitrogen atom or $CR^{3d}$.

**5.** The compound or an N-oxide thereof according to claim 1, wherein

Q represents the group represented by formula Q1;
$G^1$ represents $CR^{3a}$;
$G^2$ represents $CR^{3b}$;
$G^3$ represents $CR^{3c}$; and
$G^4$ represents $CR^{3d}$.

**6.** The compound or an N-oxide thereof according to claim 1, wherein

Q represents the group represented by formula Q2; and
$A^1$ represents a nitrogen atom.

**7.** The compound or an N-oxide thereof according to claim 1, wherein

Q represents the group represented by formula Q2; and
$A^1$ represents $CR^8$.

**8.** The compound or an N-oxide thereof according to any one of claims 1 to 7, wherein

Het represents the group represented by formula Het1;
$A^2$ represents $-CR^{7a}R^{7b}-$;
$A^3$ represents $-CR^{7c}R^{7d}-$ or $-CR^{7e}R^{7f}-CR^{7g}R^{7h}-*^1$; and
W represents an oxygen atom.

**9.** The compound or an N-oxide thereof according to any one of claims 1 to 7, wherein

Het represents the group represented by formula Het2;
$A^2$ represents $-CR^{7a}R^{7b}-$;
$A^4$ represents $-CR^{7o}R^{7p}-$ or $-CR^{7q}R^{7r}-CR^{7s}R^{7t}-*^2$; and
W represents an oxygen atom.

**10.** A composition for controlling a harmful arthropod comprising the compound or an N-oxide thereof according to any one of claims 1 to 9 and an inert carrier.

**11.** A composition comprising one or more ingredient(s) selected from the group consisting of Group (a), Group (b), Group (c), and Group (d), and the compound or an N-oxide thereof according to any one of claims 1 to 9:

Group (a): a group consisting of insecticidal active ingredients, miticidal active ingredients, and nematicidal active ingredients;
Group (b): fungicidal active ingredients;
Group (c): plant growth regulatory ingredients;
Group (d): repellent ingredients.

**12.** A method for controlling a harmful arthropod which comprises applying an effective amount of the compound or an N-oxide thereof according to any one of claims 1 to 9 or an effective amount of the composition according to claim 11 to a harmful arthropod or a habitat where a harmful arthropod lives.

**13.** A seed or a vegetative reproductive organ holding an effective amount of the compound or an N-oxide thereof according to any one of claims 1 to 9 or an effective amount of the composition according to claim 11.

**14.** A compound represented by formula (II)

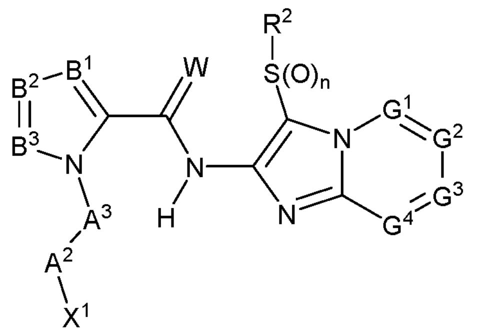

( II )

[wherein:

$X^1$ represents a chlorine atom, a bromine atom, an iodine atom, or a hydroxy group; and

the other symbols are the same as defined in claim 1] or a salt thereof.

**15.** A compound represented by formula (III)

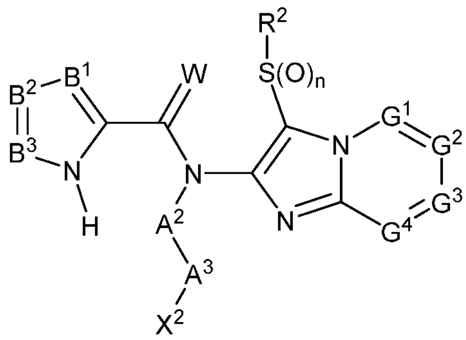

( III )

[wherein:

$X^2$ represents a chlorine atom, a bromine atom, an iodine atom, or a hydroxy group; and the other symbols are the same as defined in claim 1] or a salt thereof.

## INTERNATIONAL SEARCH REPORT

International application No.
**PCT/JP2023/039557**

**A. CLASSIFICATION OF SUBJECT MATTER**

***C07D 487/04***(2006.01)i; ***A01N 37/24***(2006.01)i; ***A01N 37/32***(2006.01)i; ***A01N 37/38***(2006.01)i; ***A01N 37/46***(2006.01)i; ***A01N 37/50***(2006.01)i; ***A01N 43/32***(2006.01)i; ***A01N 43/36***(2006.01)i; ***A01N 43/40***(2006.01)i; ***A01N 43/50***(2006.01)i; ***A01N 43/54***(2006.01)i; ***A01N 43/56***(2006.01)i; ***A01N 43/653***(2006.01)i; ***A01N 43/713***(2006.01)i; ***A01N 43/78***(2006.01)i; ***A01N 43/80***(2006.01)i; ***A01N 43/836***(2006.01)i; ***A01N 43/88***(2006.01)i; ***A01N 43/90***(2006.01)i; ***A01N 47/02***(2006.01)i; ***A01N 47/04***(2006.01)i; ***A01N 47/14***(2006.01)i; ***A01N 47/18***(2006.01)i; ***A01N 47/22***(2006.01)i; ***A01N 47/24***(2006.01)i; ***A01N 47/26***(2006.01)i; ***A01N 47/40***(2006.01)i; ***A01N 51/00***(2006.01)i; ***A01N 53/06***(2006.01)i; ***A01N 53/08***(2006.01)i; ***A01N 53/12***(2006.01)i; ***A01N 55/10***(2006.01)i; ***A01N 57/14***(2006.01)i; ***A01N 59/16***(2006.01)i; ***A01N 63/20***(2020.01)i; ***A01P 7/00***(2006.01)i; ***A01P 7/02***(2006.01)i; ***A01P 7/04***(2006.01)i; ***C07D 471/04***(2006.01)i; ***C07D 519/00***(2006.01)i

FI: C07D487/04 141; A01N37/24 101; A01N37/32 101; A01N37/38; A01N37/46; A01N37/50; A01N43/32; A01N43/36 A; A01N43/40 101A; A01N43/40 101C; A01N43/40 101D; A01N43/40 101E; A01N43/40 101Q; A01N43/50 C; A01N43/50 Q; A01N43/54 A; A01N43/56 C; A01N43/56 D; A01N43/653 B; A01N43/653 C; A01N43/653 G; A01N43/653 J; A01N43/653 Q; A01N43/713; A01N43/78 A; A01N43/78 C; A01N43/78 D; A01N43/80 101; A01N43/836; A01N43/88; A01N43/90 101; A01N43/90 103; A01N43/90 104; A01N47/02; A01N47/04 101; A01N47/14 C; A01N47/18 101A; A01N47/22 G; A01N47/24 G; A01N47/24 J; A01N47/26; A01N47/40 Z; A01N51/00; A01N53/06 110; A01N53/08 125; A01N53/12; A01N55/10 300; A01N57/14 C; A01N59/16 Z; A01N63/20; A01P7/00; A01P7/02; A01P7/04; C07D471/04 107Z; C07D487/04 144; C07D487/04 153; C07D519/00 CSP; C07D519/00 311

According to International Patent Classification (IPC) or to both national classification and IPC

**B. FIELDS SEARCHED**

Minimum documentation searched (classification system followed by classification symbols)

C07D487/04; A01N37/24; A01N37/32; A01N37/38; A01N37/46; A01N37/50; A01N43/32; A01N43/36; A01N43/40; A01N43/50; A01N43/54; A01N43/56; A01N43/653; A01N43/713; A01N43/78; A01N43/80; A01N43/836; A01N43/88; A01N43/90; A01N47/02; A01N47/04; A01N47/14; A01N47/18; A01N47/22; A01N47/24; A01N47/26; A01N47/40; A01N51/00; A01N53/06; A01N53/08; A01N53/12; A01N55/10; A01N57/14; A01N59/16; A01N63/20; A01P7/00; A01P7/02; A01P7/04; C07D471/04; C07D519/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 December 2023** | **26 December 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
**PCT/JP2023/039557**

C. DOCUMENTS CONSIDERED TO BE RELEVANT

| Category* | Citation of document, with indication, where appropriate, of the relevant passages | Relevant to claim No. |
|---|---|---|
| A | JP 2019-167374 A (SUMITOMO CHEMICAL CO) 03 October 2019 (2019-10-03) claims, examples | 1-15 |
| A | JP 2020-180131 A (SUMITOMO CHEMICAL CO) 05 November 2020 (2020-11-05) claims, examples | 1-15 |
| A | WO 2019/131587 A1 (SUMITOMO CHEMICAL CO) 04 July 2019 (2019-07-04) claims, examples | 1-15 |
| A | WO 2020/158889 A1 (SUMITOMO CHEMICAL CO) 06 August 2020 (2020-08-06) claims, examples | 1-15 |
| A | JP 2018-515440 A (BAYER CROPSCIENCE AKTIENGESELLSCHAFT) 14 June 2018 (2018-06-14) claims, examples | 1-15 |

Form PCT/ISA/210 (second sheet) (January 2015)

**INTERNATIONAL SEARCH REPORT**
**Information on patent family members**

International application No.
**PCT/JP2023/039557**

| Patent document cited in search report | Publication date (day/month/year) | Patent family member(s) | Publication date (day/month/year) |
|---|---|---|---|
| JP 2019-167374 A | 03 October 2019 | (Family: none) | |
| JP 2020-180131 A | 05 November 2020 | (Family: none) | |
| WO 2019/131587 A1 | 04 July 2019 | US 2020/0399278 A1<br>claims, examples<br>US 2022/0324871 A1<br>EP 3733672 A1<br>CN 111566108 A<br>CN 115772120 A | |
| WO 2020/158889 A1 | 06 August 2020 | US 2022/0095620 A1<br>claims, examples<br>EP 3918896 A1<br>CN 113412050 A | |
| JP 2018-515440 A | 14 June 2018 | US 2018/0116222 A1<br>claims, examples<br>WO 2016/162318 A1<br>EP 3280716 A1<br>CN 107810188 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022177697 A
- JP 2023088026 A
- JP 2023160980 A
- WO 2016129684 A
- WO 2013192346 A
- US 20180009778
- WO 2016121970 A
- WO 2015157093 A
- WO 2016109706 A
- WO 2020158889 A